# EUROPEAN PATENT SPECIFICATION

(11) **EP 3 499 212 B1**
(45) Date of publication and mention of the grant of the patent: **12.08.2020**
(21) Application number: 18199291.8
(22) Date of filing: 03.01.2014
(51) Int. Cl.: G01N 1/18, A61K 48/00, A61K 9/14

(54) **METHODS FOR ISOLATING MICROVESICLES**
VERFAHREN ZUR ISOLIERUNG VON MIKROVESIKELN
PROCÉDÉS D'ISOLEMENT DE MICROVÉSICULES

(30) Priority: 03.01.2013 US 201361748575 P
(43) Date of publication of application: 19.06.2019
(62) Divisional of application: 14735397.3
(73) Proprietor: Exosome Diagnostics, Inc., Waltham, MA 02451 (US)
(72) Inventor: ENDERLE, Daniel, Waltham, MA 02451 (DE); RAMACHANDRAN, Aparna, Iselin, NJ New Jersey 08830 (US); YAN, Haoheng, Bethesda, MD Maryland 20817 (US); BERGHOFF, Emily, Waltham, MA 02451 (US); WEI, Tai-Fen, Vernon Hills, IL Illinois 60061 (US); NOERHOLM, Mikkel, 82131 Gauting (DE); SKOG, Johan, Karl Olov, Waltham, MA 02451 (US)
(74) Representative: Cooley (UK) LLP

(56) References cited:
- WO-A1-2012/064993
- WO-A2-2010/065765
- WO-A2-2010/141862
- WO-A2-2012/006476
- US-A- 4 935 342
- ENDERLE DANIEL ET AL: "Characterization of RNA from Exosomes and Other Extracellular Vesicles Isolated by a Novel Spin Column-Based Method", PLOS ONE, vol. 10, no. 8, August 2015 (2015-08), XP002766816,

## Description

### FIELD OF THE INVENTION

The invention provides novel methods and kits for isolating microvesicles from a biological sample and for extracting nucleic acids from the microvesicles.

### BACKGROUND

Membrane vesicles that are shed by cells and are < 0.8µm in diameter are referred collectively as microvesicles. Microvesicles from various cell sources have been extensively studied with respect to protein and lipid content. Recently, microvesicles have been found to also contain both DNA and RNA, including genomic DNA, cDNA, mitochondrial DNA, microRNA (miRNA), and messenger RNA (mRNA).

Due to the genetic and proteomic information contained in microvesicles shed by cells, current research is directed at utilizing microvesicles to gain further insight into the status of these cells, for example, disease state or predisposition for a disease. Accordingly, there is a need for methods of isolating microvesicles from biological samples and methods of extracting high quality nucleic acids for accurate diagnosis of medical conditions and diseases.

### SUMMARY OF THE INVENTION

The present invention provides methods and kits for isolating microvesicles by capturing the microvesicles to a surface and subsequently lysing the microvesicles to release the nucleic acids contained therein. In some embodiments, the methods and kits isolate and extract the RNA from the microvesicle fraction. These methods and kits are referred to herein as EXO50 or EXO50-based methods and/or kits. In some embodiments, the methods and kits isolate and extract the DNA from the microvesicle fraction. The RNA can then be processed for further analysis. These methods and kits are referred to herein as EXO52 or EXO52-based methods and/or kits, and can include derivatives of the EXO52 methods and/or kits, referred to herein as EXO52.2. The DNA can then be processed for further analysis.

The present invention also provides methods and kits for isolating microvesicles by capturing the microvesicles to a surface and subsequently eluting the microvesicles from the capture surface. These methods and kits are referred to herein as EXO51. The microvesicles can then be processed for further analysis.

Previous procedures used to isolate microvesicle fractions and extract nucleic acids from the microvesicle fraction of a biological sample relied on the use of ultracentrifugation, e.g., spinning at less than 10,000 xg for 1-3 hrs, followed by removal of the supernatant, washing the pellet, lysing the pellet and purifying the nucleic acids, e.g., RNA on a column. These previous methods demonstrated several disadvantages such as being slow, tedious, subject to variability between batches, and not suited for scalability. The methods and kits for isolation and extraction overcome these disadvantages and provide a spin-based column for isolation and extraction that is fast, robust and easily scalable to large volumes.

The methods and kits isolate and extract RNA from a biological sample using the following the general procedure, which is referred to herein as "EXO50." First, the microvesicle fraction is bound to a membrane filter, and the filter is washed. Then, a reagent is used to perform on-membrane lysis and release of the RNA. Chloroform extraction is then performed using PLG tubes, followed by ethanol conditioning. The RNA is then bound to a silica column, washed and then eluted. The RNA can then be processed for further analysis.

The membranes used in the EXO50 methods and kits have large pores and, overall, are positively charged. In some embodiments, more than one membrane is used in the EXO50 methods and kits, for example, two or more membranes are used. In some embodiments, three membranes are used. In some embodiments, more than three membranes are used. In some embodiments, each layer of membrane is a different type of membrane. In some embodiments, each layer of membrane is the same type of membrane. In some embodiments, the layers of membranes are a combination of at least two different types of membranes. In some embodiments, each layer of membrane is composed of a different material. In some embodiments, each layer of membrane is composed of the same material. In some embodiments, each layer of membrane is charged. In some embodiments, at least one layer is not charged. In some embodiments, each layer of membrane has the same charge. In some embodiments, each layer of membrane has the same charge. In some embodiments, each layer of membrane has a different charge.

In some embodiments, the membrane or at least one layer of membrane is a positively charged membrane. In some embodiments, the capture surface or at least one layer is a regenerated cellulose, strong basic anion exchanger ("RC/SBAE") membrane, which is a positively charged membrane and is an anion exchanger with quaternary amines. For example, the RC/SBAE membrane is functionalized with quaternary ammonium, R-CH₂-N⁺(CH₃)₃. In some embodiments, the membrane has a pore size that is at least 3 um.

The number of membranes used in the EXO50 methods and kits correlates with the total volume of sample that can be analyzed at one time. The number of layers and the capacity, e.g., binding capacity, flowthrough rate or other measurement, of each layer used in the methods and kits affects the total volume of sample size that can be used. Where a layer or each layer has a higher binding capacity, more layers can be used in the methods and/or kits, and where a layer or each layer has a lower binding capacity, few layers can be used in the methods and/or kits. Furthermore, the viscosity and composition of the sample also affects the total volume of sample size that can be used. For example, in some embodiments where the sample is plasma, about 1 ml of samples is processed for each layer of membrane used in the EXO50 methods and kits.

In some embodiments, the agent used for on-membrane lysis is QIAzol. In some embodiments, the QIAzol is used at a volume of about 700 uL

The methods and kits isolate and extract DNA from a biological sample using the following the general procedure, which is referred to herein as "EXO52." First, the microvesicle fraction is bound to a membrane filter, and the filter is washed. Then, a reagent is used to perform on-membrane lysis and release of the nucleic acids, e.g., RNA. Ethanol precipitation is then performed, followed by lysis and protease digestion. The DNA is then bound to a silica column, washed and then eluted. The DNA can then be processed for further analysis.

The membranes used in the EXO52 methods and kits have large pores and, overall, are positively charged. In some embodiments, more than one membrane is used in the EXO52 methods and kits, for example, two or more membranes are used. In some embodiments, three membranes are used. In some embodiments, more than three membranes are used. In some embodiments, each layer of membrane is a different type of membrane. In some embodiments, each layer of membrane is the same type of membrane. In some embodiments, the layers of membranes are a combination of at least two different types of membranes. In some embodiments, each layer of membrane is composed of a different material. In some embodiments, each layer of membrane is composed of the same material. In some embodiments, each layer of membrane is charged. In some embodiments, at least one layer is not charged. In some embodiments, each layer of membrane has the same charge. In some embodiments, each layer of membrane has the same charge. In some embodiments, each layer of membrane has a different charge.

In some embodiments, the membrane or at least one layer of membrane is a positively charged membrane. In some embodiments, the capture surface or at least one layer is a regenerated cellulose, strong basic anion exchanger ("RC/SBAE") membrane, which is a positively charged membrane and is an anion exchanger with quaternary amines. For example, the RC/SBAE membrane is functionalized with quaternary ammonium, R-CH₂-N⁺(CH₃)₃. In some embodiments, the membrane has a pore size that is at least 3 um.

The number of membranes used in the EXO52 methods and kits correlates with the total volume of sample that can be analyzed at one time. The number of layers and the capacity, e.g., binding capacity, flowthrough rate or other measurement, of each layer used in the methods and kits affects the total volume of sample size that can be used. Where a layer or each layer has a higher binding capacity, more layers can be used in the methods and/or kits, and where a layer or each layer has a lower binding capacity, few layers can be used in the methods and/or kits. Furthermore, the viscosity and composition of the sample also affects the total volume of sample size that can be used. For example, in some embodiments where the sample is plasma, about 1 ml of samples is processed for each layer of membrane used in the EXO52 methods and kits.

In some embodiments, the agent used for on-membrane lysis is QIAzol. In some embodiments, the QIAzol is used at a volume of about 700 ul.

The membranes used in the EXO51 methods and kits have large pores and, overall, are positively charged. In some embodiments, more than one membrane is used in the EXO51 methods and kits, for example, two or more membranes are used. In some embodiments, three membranes are used. In some embodiments, more than three membranes are used. In some embodiments, each layer of membrane is a different type of membrane. In some embodiments, each layer of membrane is the same type of membrane. In some embodiments, the layers of membranes are a combination of at least two different types of membranes. In some embodiments, each layer of membrane is composed of a different material. In some embodiments, each layer of membrane is composed of the same material. In some embodiments, each layer of membrane is charged. In some embodiments, at least one layer is not charged. In some embodiments, each layer of membrane has the same charge. In some embodiments, each layer of membrane has the same charge. In some embodiments, each layer of membrane has a different charge.

In some embodiments, the membrane or at least one layer of membrane is a positively charged membrane. In some embodiments, the capture surface or at least one layer is a regenerated cellulose, strong basic anion exchanger ("RC/SBAE") membrane, which is a positively charged membrane and is an anion exchanger with quaternary amines. For example, the RC/SBAE membrane is functionalized with quaternary ammonium, R-CH₂-N⁺(CH₃)₃. In some embodiments, the membrane has a pore size that is at least 3 um.

The number of membranes used in the EXO51 methods and kits correlates with the total volume of sample that can be analyzed at one time. The number of layers and the capacity, e.g., binding capacity, flowthrough rate or other measurement, of each layer used in the methods and kits affects the total volume of sample size that can be used. Where a layer or each layer has a higher binding capacity, more layers can be used in the methods and/or kits, and where a layer or each layer has a lower binding capacity, few layers can be used in the methods and/or kits. Furthermore, the viscosity and composition of the sample also affects the total volume of sample size that can be used. For example, in some embodiments where the sample is plasma, about 1 ml of samples is processed for each layer of membrane used in the EXO51 methods and kits.

In some embodiments, the agent used for on-membrane lysis is QIAzol. In some embodiments, the QIAzol is used at a volume of about 700 ul.

Purification of the microvesicle fraction in any of these methods and/or kits is performed using ion exchange techniques. In some embodiments, the ion exchange technique is a technique selected from those shown in the working examples provided herein.

In one aspect, the method for extracting nucleic acids from a biological sample comprises (a) providing a biological sample; (b) contacting the biological sample with a capture surface under conditions sufficient to retain the microvesicle fraction on or in the capture surface; (c) lysing the microvesicle fraction while the microvesicles are on or in the capture surface; and (d) extracting the nucleic acids from the microvesicle fraction. Alternatively, the method for extracting nucleic acids from the biological sample further comprises eluting the microvesicle fraction from the capture surface after step (b), collecting the eluted microvesicle fraction, and extracting the nucleic acids from the eluted microvesicle fraction. Optionally, the eluted microvesicle fraction can be concentrated by a spin concentrator to obtain a concentrated microvesicle fraction, and the nucleic acids are subsequently extracted from the concentrated microvesicle fraction.

In one embodiment, the capture surface is positively charged. In another embodiment, the capture surface is negatively charged. In yet another embodiment, the capture surface is neutral. In some embodiments, the capture surface includes more than one layer. In some embodiments, more than one membrane is used in the EXO51 methods and kits, for example, two or more membranes are used. In some embodiments, three membranes are used. In some embodiments, more than three membranes are used. In some embodiments, each layer of membrane is a different type of membrane. In some embodiments, each layer of membrane is the same type of membrane. In some embodiments, the layers of membranes are a combination of at least two different types of membranes. In some embodiments, each layer of membrane is composed of a different material. In some embodiments, each layer of membrane is composed of the same material. In some embodiments, each layer of membrane is charged. In some embodiments, at least one layer is not charged. In some embodiments, each layer of membrane has the same charge. In some embodiments, each layer of membrane has the same charge. In some embodiments, each layer of membrane has a different charge.

In one embodiment, the capture surface is a bead. For example, the bead is magnetic. Alternatively, the bead is non-magnetic. In yet another embodiment, the bead is functionalized with an affinity ligand.

Preferably, the capture surface is a membrane. In one aspect, the membrane comprises regenerated cellulose. For example, the membrane has a pore size in the range of 3-5 um. In another aspect, the membrane comprises polyethersulfone (PES). For example, the membrane has a pore size in the range of 20 nm to 0.8 um. In another aspect, the membrane is positively charged.

In some aspects, the membrane is functionalized. For example, the membrane is functionalized with quaternary ammonium R-CH₂-N⁺(CH₃)₃.

In one embodiment, the capture surface comprises three membranes, wherein said three membranes are directly adjacent to one another.

Preferably, the biological sample is plasma, serum, urine, cerebrospinal fluid or cell culture supernatant.

In some aspects, the method and/or kit described herein further comprises contacting the biological sample with a loading buffer. The loading buffer is in the range of pH 4-8. In one aspect, the loading buffer has a neutral pH.

The methods and/or kits described herein provide for the extraction of nucleic acids from microvesicles. Preferably, the extracted nucleic acids are RNA. The extracted RNA may comprise messenger RNA, ribosomal RNA, transfer RNA, small RNAs such as microRNAs, noncoding RNA, and any other short RNAs and/or RNA fragments, or any combination thereof.

In some embodiments, the methods and/or kits are used to remove species of nucleic acids from a biological sample. For example, the EXO50 and/or kits are used to remove species of RNAs from a biological sample, including, by way of non-limiting example, vesicle-bound RNA from a biological sample to isolate reciprocal RNA(s) from the flow-through.

Various aspects and embodiments of the invention will now be described in detail. It will be appreciated that modification of the details may be made without departing from the scope of the invention. Further, unless otherwise required by context, singular terms shall include pluralities and plural terms shall include the singular.

All patents, patent applications, and publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representations as to the contents of these documents are based on the information available to the applicants and do not constitute any admission as to the correctness of the dates or contents of these documents.

### BRIEF DESCRIPTION OF THE FIGURES

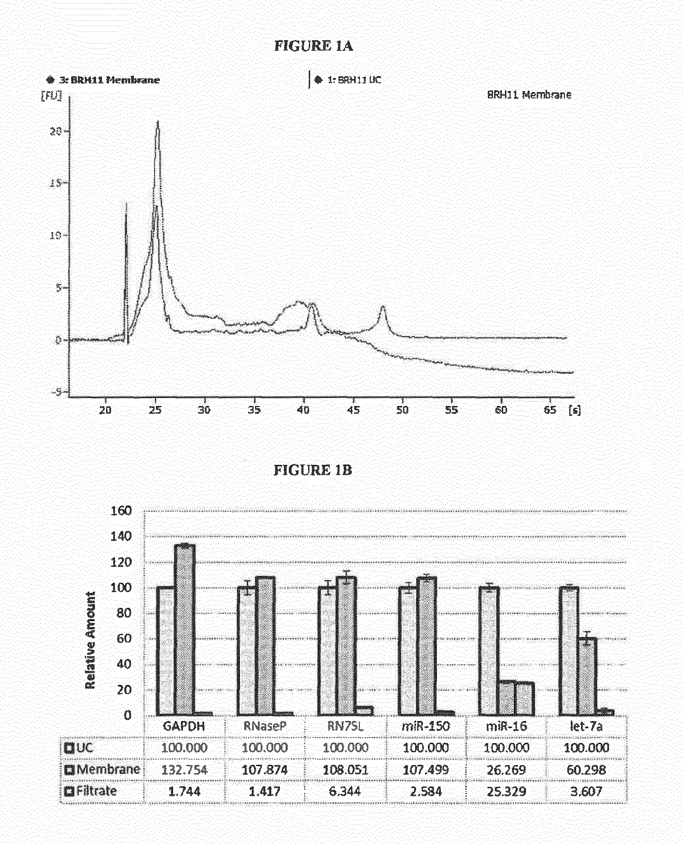

Figure 1 is a series of graphs depicting how plasma microvesicular RNA can be isolated using a 0.65um positively charged Q PES filter in a vacuum format (Millipore). Figure 1A depicts a bioanalyzer plot comparing the quality, concentration, and size distribution of microvesicle total RNA extracted from 4mL normal control plasma by ultracentrifugation and 0.65um positively charged Q polyethersulfone (PES)vacuum filtration (filter and filtrate). Relative fluorescence units (FU) arc plotted against time (s). The 25 s peak represents an internal standard. The most prominent peak represents small RNA. The peaks at ∼41 s and ∼47 s represent 18S and 28S, respectively. Figure 1B depicts levels of mRNA and mature miRNA that were analyzed using quantitative RT-PCR from the same samples. The relative amount value is presented as the mean ± SD.

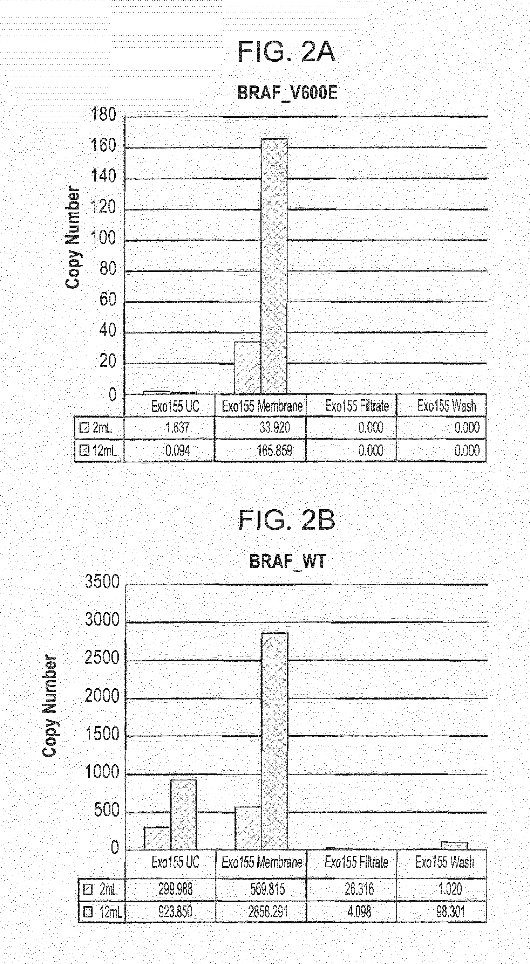

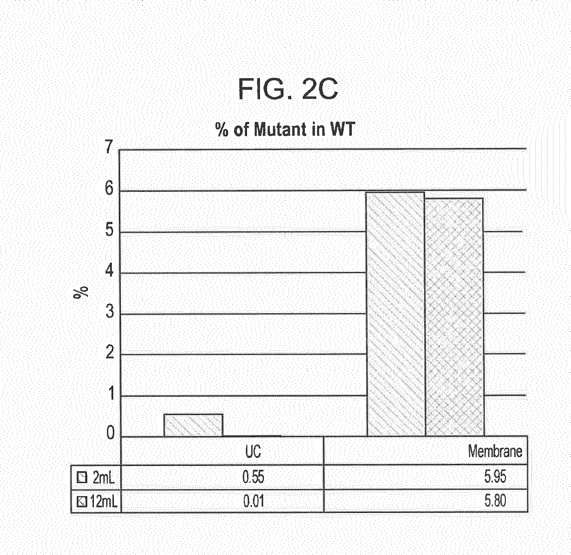

Figure 2 is a series of graphs depicting how BRAF V600E mutations can be detected in 2mL plasma and 12mL plasma using a 0.65um positively charged Q PES filter in a vacuum format (Millipore). BRAF V600E copy numbers were assessed using quantitative RT-PCR from 2mL and 12mL plasma extracted by ultracentrifugation and 0.65um positively charged Q PES vacuum filtration (filter, filtrate, and wash).

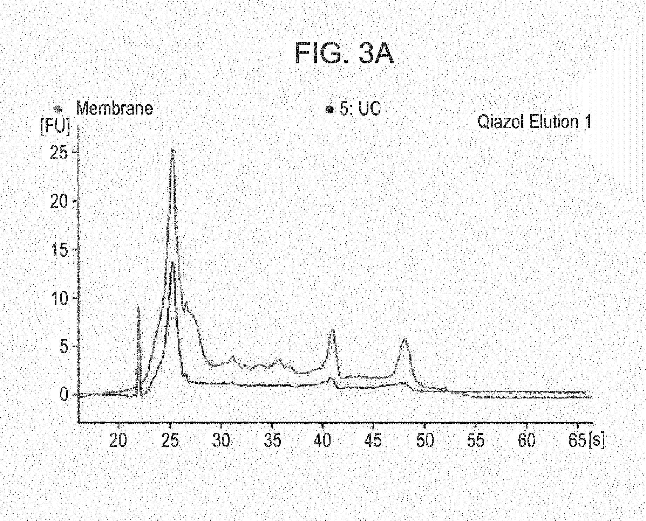

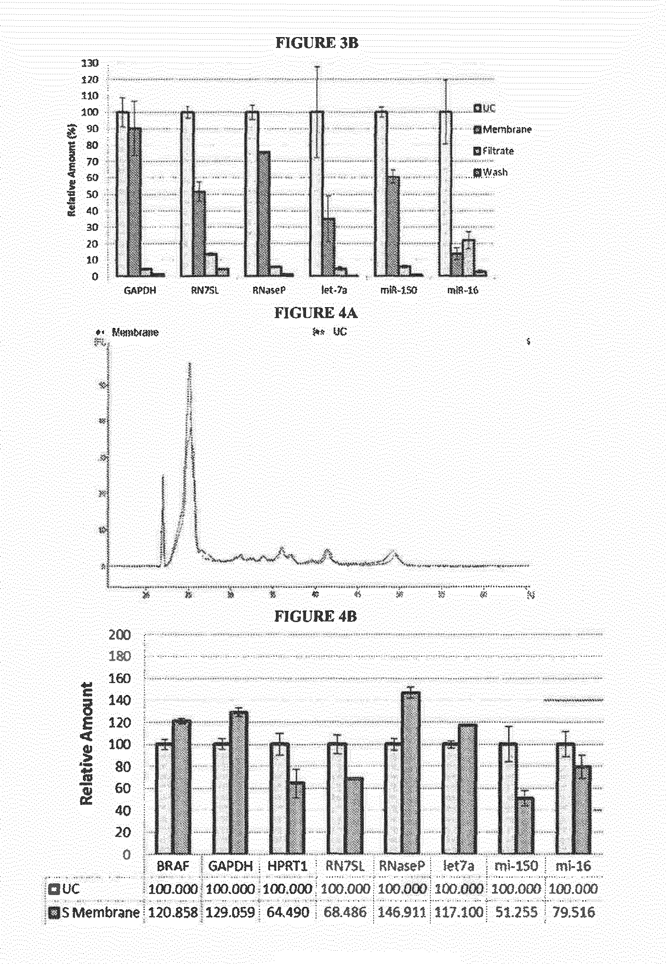

Figure 3 is a series of graphs depicting how plasma microvesicular RNA can be isolated using a 3-5um positively-charged Q regenerated cellulose filter in a spin column format (Sartorius). Figure 3A depicts a bioanalyzer plot comparing the quality, concentration, and size distribution of microvesicle total RNA extracted from 4mL normal control plasma by ultracentrifugation and 3-5um positively charged Q regenerated cellulose spin column filtration (filter, filtrate, and wash). Relative fluorescence units (FU) are plotted against time (s). The 25 s peak represents an internal standard. The most prominent peak represents small RNA. The peaks at ∼41 s and ∼47 s represent 18S and 28S, respectively. Figure 3B depicts levels of mRNA and mature miRNA that were analyzed using quantitative RT-PCR from the same samples. The relative amount value is presented as the mean ± SD.
Figure 4 is a series of graphs depicting how plasma microvesicular RNA can be isolated with a 0.8um negatively charged S PES filter (Pall) in a homemade spin column format. Figure 4A is a bioanalyzer plot comparing the quality, concentration, and size distribution of microvesicle total RNA extracted from 4mL normal control plasma by ultracentrifugation and a 0.8um negatively charged S PES spin column filtration. Relative fluorescence units (FU) are plotted against time (s). The 25 s peak represents an internal standard. The most prominent peak represents small RNA. The peaks at ∼41 s and ∼47 s represent 18S and 28S, respectively. Figure 4B depicts levels of mRNA and mature miRNA that were analyzed using quantitative RT-PCR from the same samples. The relative amount value is presented as the mean ± SD.

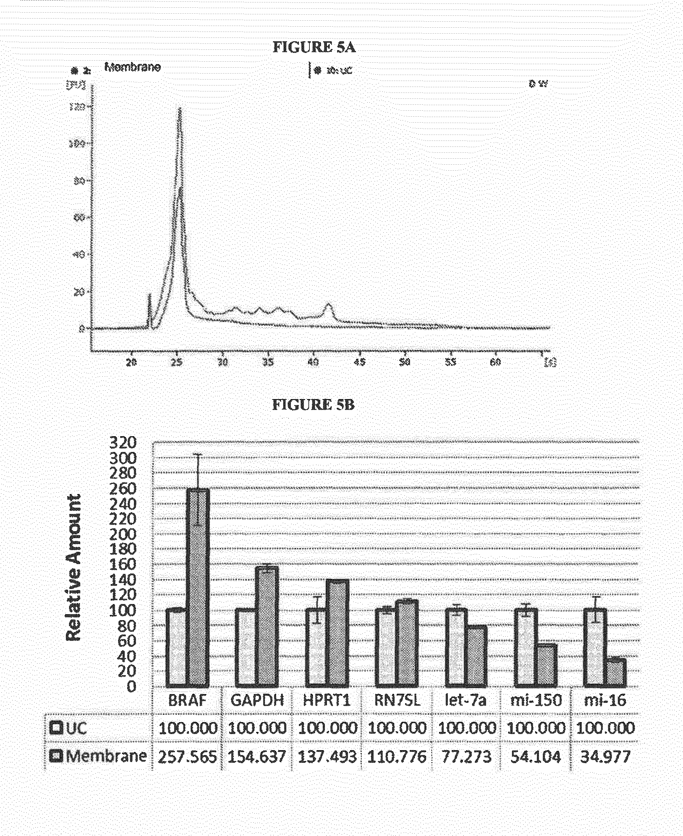

Figure 5 is a series of graphs depicting how plasma microvesicular RNA can be isolated with a 0.8um positively charged Q PES filter (Pall) in a homemade spin column format. Figure 5A is a bioanalyzer plot comparing the quality, concentration, and size distribution of microvesicle total RNA extracted from 4mL normal control plasma by ultracentrifugation and a 0.8um positively charged Q PES spin column filtration. Relative fluorescence units (FU) are plotted against time (s). The 25 s peak represents an internal standard. The most prominent peak represents small RNA. The peak at ∼41 s represents 18S. Figure 5B depicts levels of mRNA and mature miRNA that were analyzed using quantitative RT-PCR from the same samples. The relative amount value is presented as the mean ± SD.

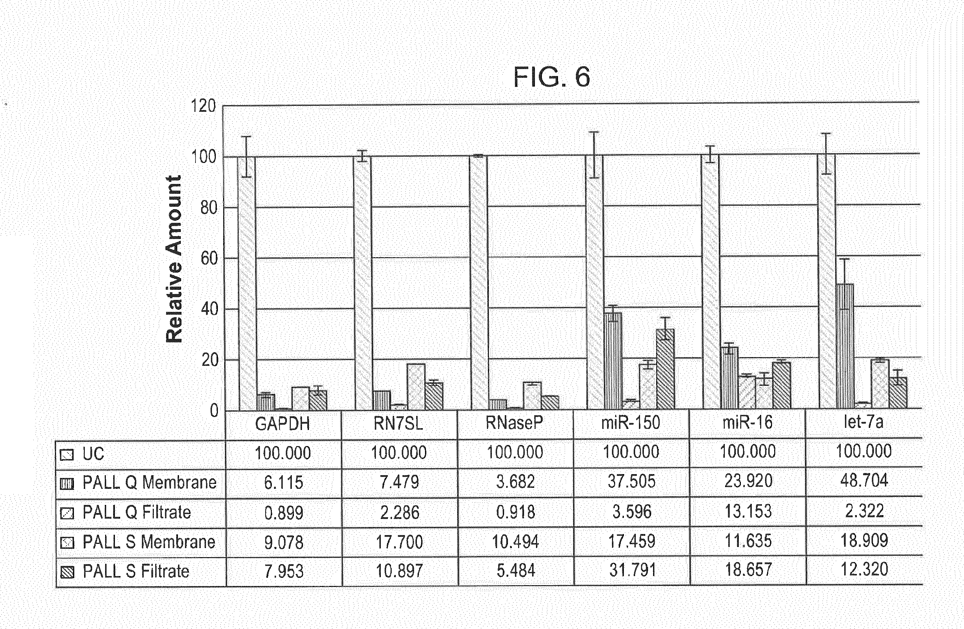

Figure 6 is a graph depicting how plasma microvesicular RNA can be isolated with a 0.8um positively charged Q PES syringe filter (Pall). Plasma microvesicular RNA can be isolated with a 0.8um negatively charged S PES syringe filter (Pall). Levels of mRNA and mature miRNA were analyzed using quantitative RT-PCR from 4mL plasma extracted by ultracentrifugation, 0.8um positively charged Q PES syringe filtration (filter and filtrate) and 0.8um negatively charged S PES syringe filtration (filter and filtrate). The relative amount value is presented as the mean ± SD.

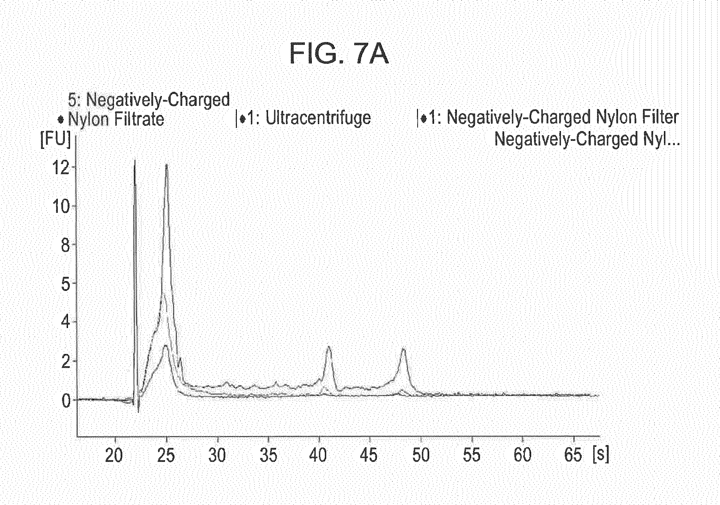

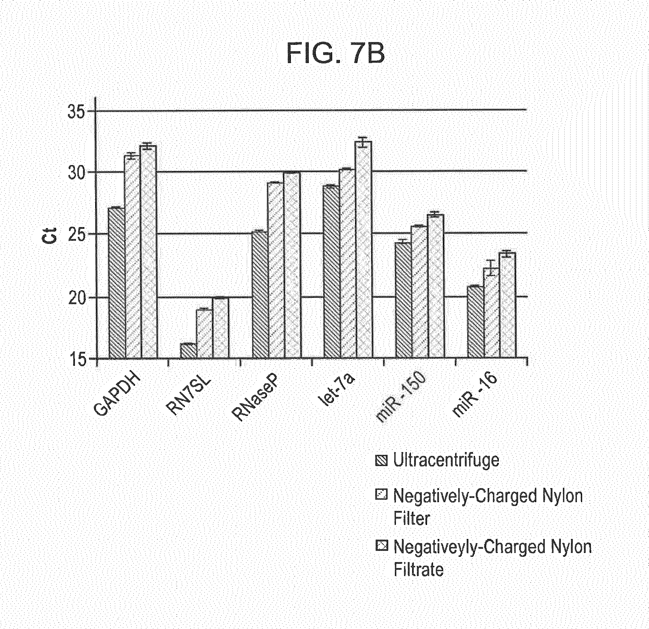

Figure 7 is a series of graphs depicting how plasma microvesicular RNA can be isolated using a charged nylon syringe filter. Figure 7A is a bioanalyzer plot comparing the quality, concentration, and size distribution of microvesicle total RNA extracted from 4mL normal control plasma by ultracentrifugation and negatively charged nylon syringe filtration (filter and filtrate). Relative fluorescence units (FU) are plotted against time (s). The 25 s peak represents an internal standard. The most prominent peak represents small RNA. The peaks at ∼41 s and ∼47 s represent 18S and 28S, respectively. Figure 7B depicts levels of mRNA and mature miRNA that were analyzed using quantitative RT-PCR on the same samples. The cycle threshold (Ct) value is presented as the mean ± SD.

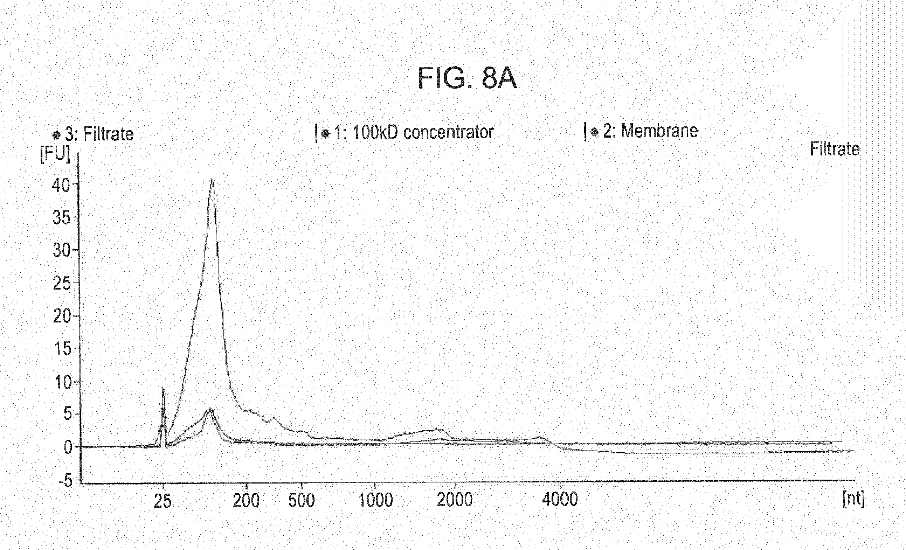

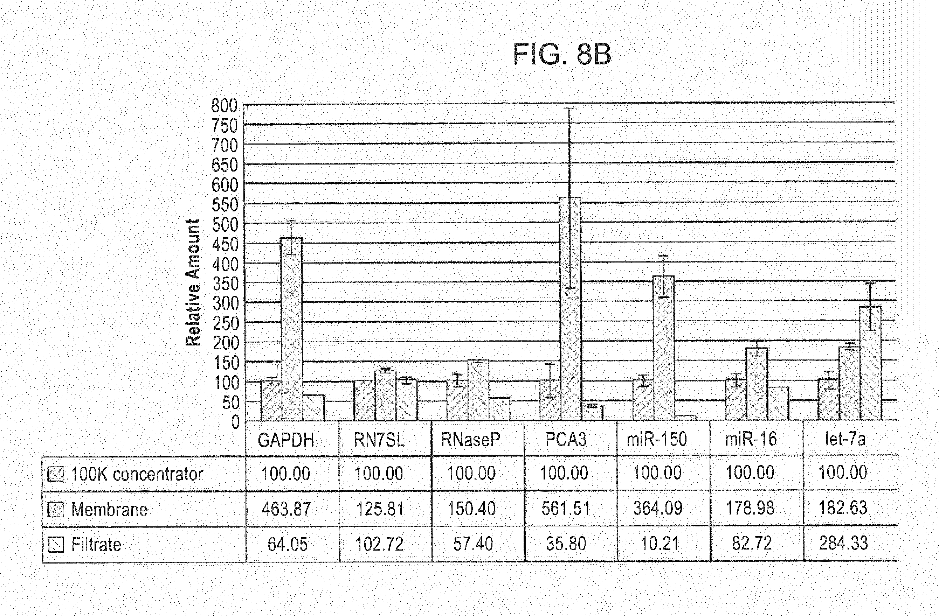

Figure 8 is a series of graphs depicting how urine microvesicular RNA can be isolated using a 0.65um positively charged Q PES filter in a vacuum format (Millipore). Figure 8A is a bioanalyzer plot comparing the quality, concentration, and size distribution of microvesicle total RNA extracted from 10mL normal control urine by ultracentrifugation and a 0.65um positively charged Q PES vacuum filtration (filter and filtrate). Relative fluorescence units (FU) are plotted against size (nt). The 25 s peak represents an internal standard. The most prominent peak represents small RNA. The peaks at ∼1900 nt and 3900 nt represent 18S and 28S, respectively. Figure 8B depicts levels of mRNA and mature miRNA that were analyzed using quantitative RT-PCR from the same samples. The relative amount value is presented as the mean ± SD.

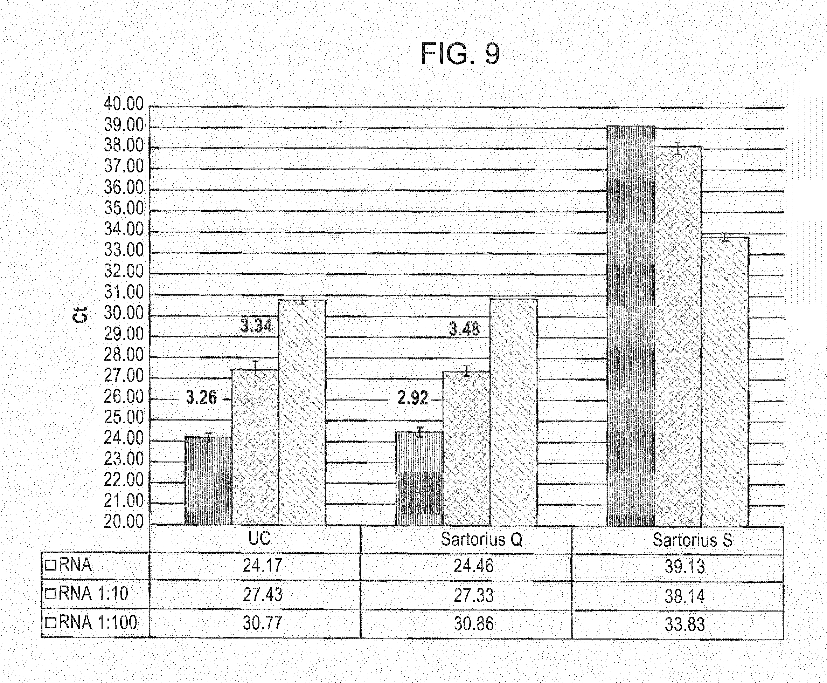

Figure 9 is a graph depicting how qRT-PCR is inhibited in samples plasma samples extracted using a negatively charged S regenerated cellulose filter in a spin column format (Thermo Scientific). Levels of GAPDH were analyzed using quantitative RT-PCR on 4mL plasma samples extracted using ultracentrifugation, 3-5um positively charged Q regenerated cellulose spin column filtration and 3-5um negatively charged S regenerated cellulose spin column filtration. All RNA samples were diluted 1:10 and 1:100 prior to cDNA synthesis. The cycle threshold (Ct) value is presented as the mean ± SD.

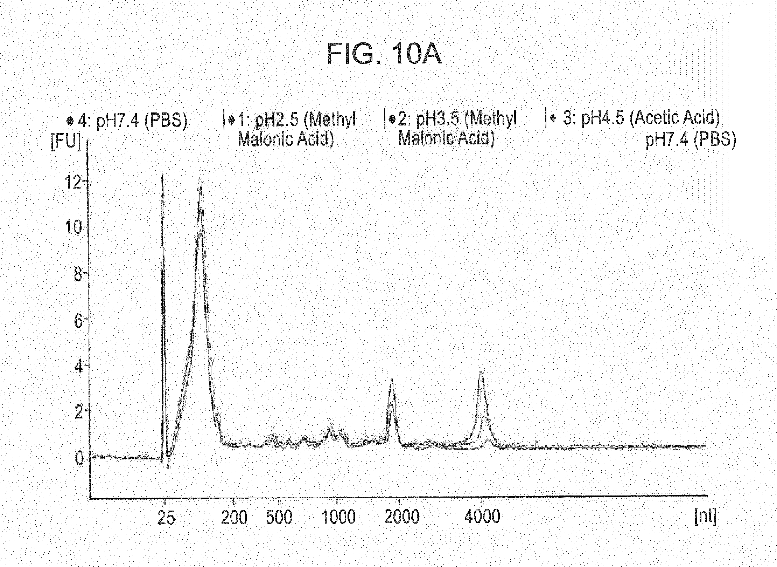

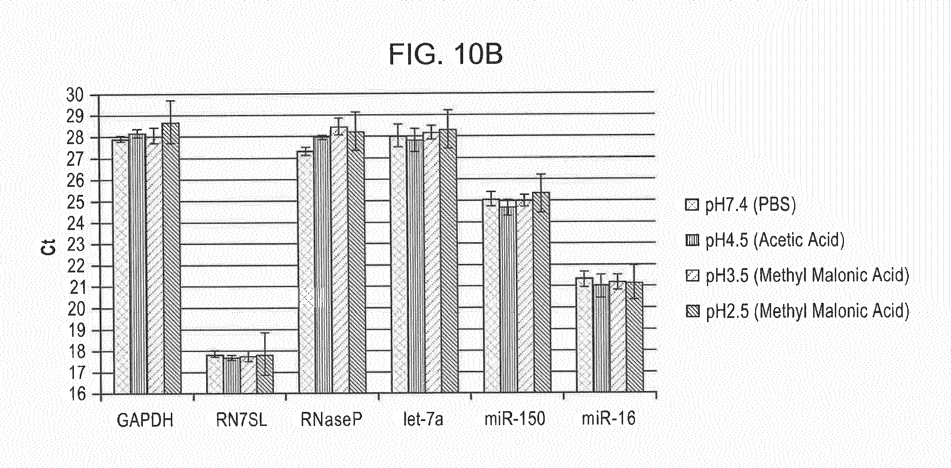

Figure 10 is a series of graphs depicting how microvesicles are stable in acidic pH. Figure 10A is a bioanalyzer plot comparing the quality, concentration, and size distribution of microvesicle total RNA extracted from 1.9mL normal control plasma by centrifugation Relative fluorescence units (FU) are plotted against size (nt). The 25 nt peak represents an internal standard. The most prominent peak represents small RNA. The peaks at ∼1900 nt and ∼4000 nt represent 18S and 28S, respectively. Figure 10B depicts levels of mRNA and mature miRNA that were analyzed using quantitative RT-PCR on the same samples. The cycle threshold (Ct) value is presented as the mean ± SD.

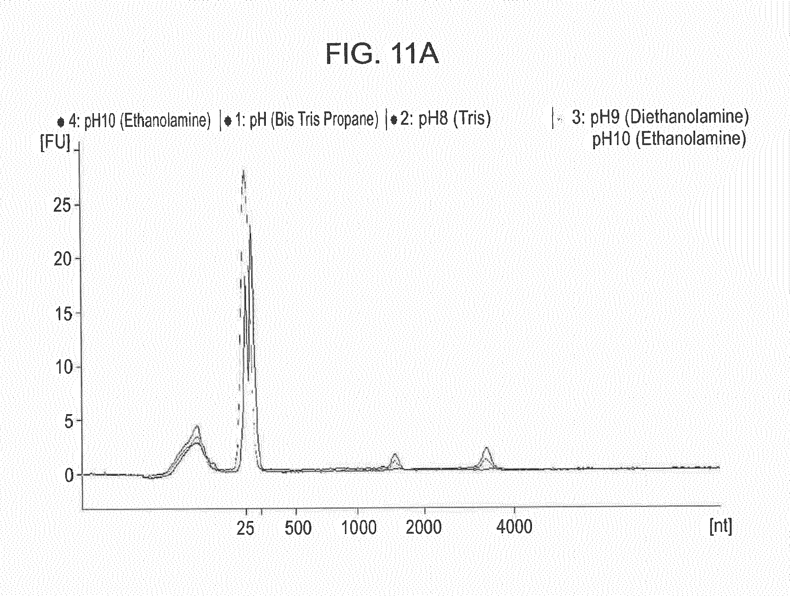

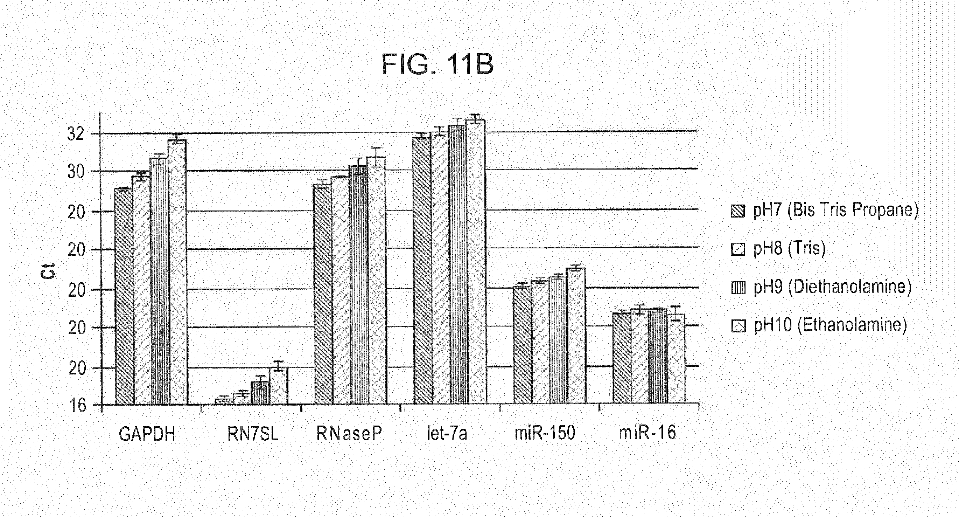

Figure 11 is a series of graphs depicting how microvesicles are not stable in basic pH. Figure 11A is a bioanalyzer plot comparing the quality, concentration, and size distribution of microvesicle total RNA extracted from 1.9mL normal control plasma by centrifugation. Relative fluorescence units (FU) are plotted against size (nt). The 25 nt peak represents an internal standard. The most prominent peak represents small RNA. It should be shown at ∼150 nt. However, due to a technical error the peak is shown ∼0 nt. In addition, due to a technical error the peaks at ∼1500 nt and ∼3700 nt represent 18S and 28S, respectively. Instead, the 18S and 28S peaks should be shown at ∼1900 nt and at ∼4700 nt, respectively. Figure 11B depicts levels of mRNA and mature miRNA that were analyzed using quantitative RT-PCR on the same samples. The cycle threshold (Ct) value is presented as the mean ± SD.

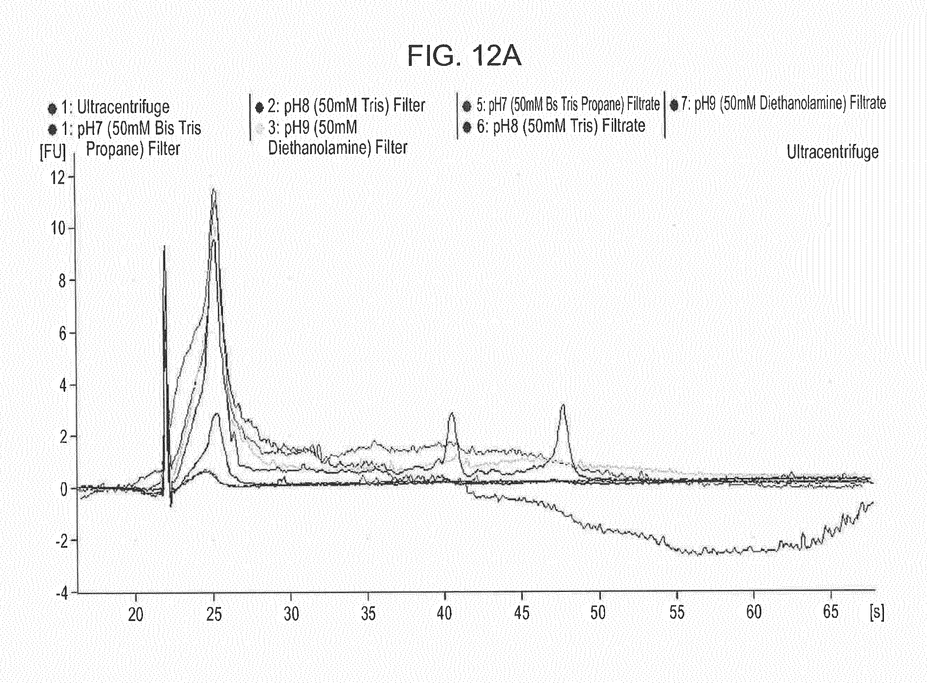

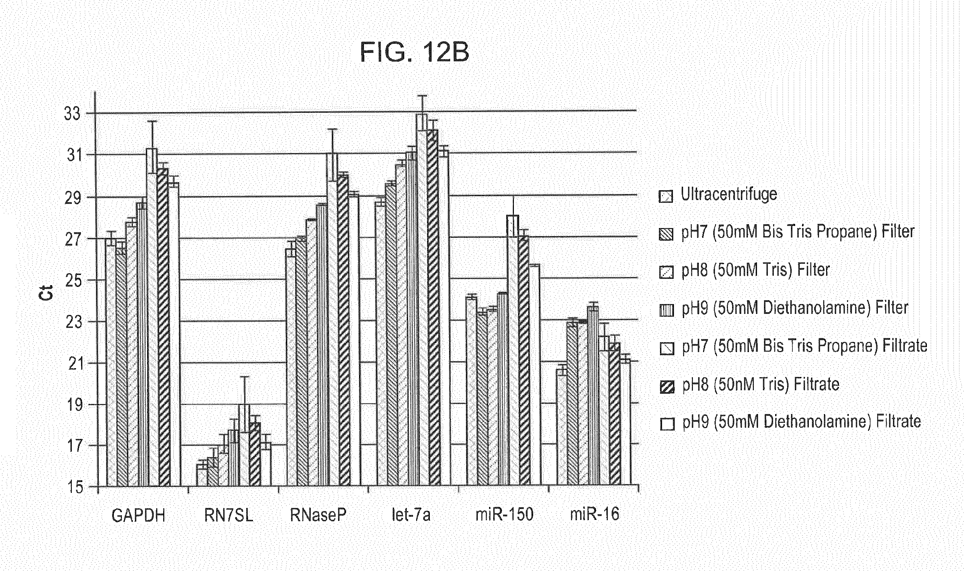

Figure 12 is a series of graphs depicting how microvesicle capture and/or microvesicle stability on a charged filter are affected by buffer pH and/or buffer concentration and/or buffer type (comparing buffers that are the same concentration, but NOT the same functional group concentration). Figure 12A is a bioanalyzer plot comparing the quality, concentration, and size distribution of microvesicle total RNA extracted from 4.8mL normal control plasma by ultracentrifugation and positively charged Q regenerated cellulose centrifugal filtration (filter and filtrate). Filtration samples were isolated with the following buffer sets:
   - 100mM Bis Tris Propane, 150mM NaCl, pH6.8 (2X Loading Buffer) and 50mM Bis Tris Propane, 150mM NaCl, pH7 (Equilibration and Wash Buffer)
   - 100mM Tris, 150mM NaCl, pH8 (2X Loading Buffer) and 50mM Tris, 150mM NaCl, pH8 (Equilibration and Wash Buffer)
   - 100mM Diethanolamine, 150mM NaCl, pH9 (2X Loading Buffer) and 50mM Diethanolamine, 150mM NaCl, pH9 (Equilibration and Wash Buffer)
In Figure 12A, the legend identifies the sample by the equilibration and wash buffer only. Relative fluorescence units (FU) are plotted against time (s). The 25 s peak represents an internal standard. The most prominent peak represents small RNA. The peaks at ∼41 s and ∼47 s represent 18S and 28S, respectively. Figure 12B depicts levels of mRNA and mature miRNA that were analyzed using quantitative RT-PCR from the same samples. The cycle threshold (Ct) value is presented as the mean ± SD.

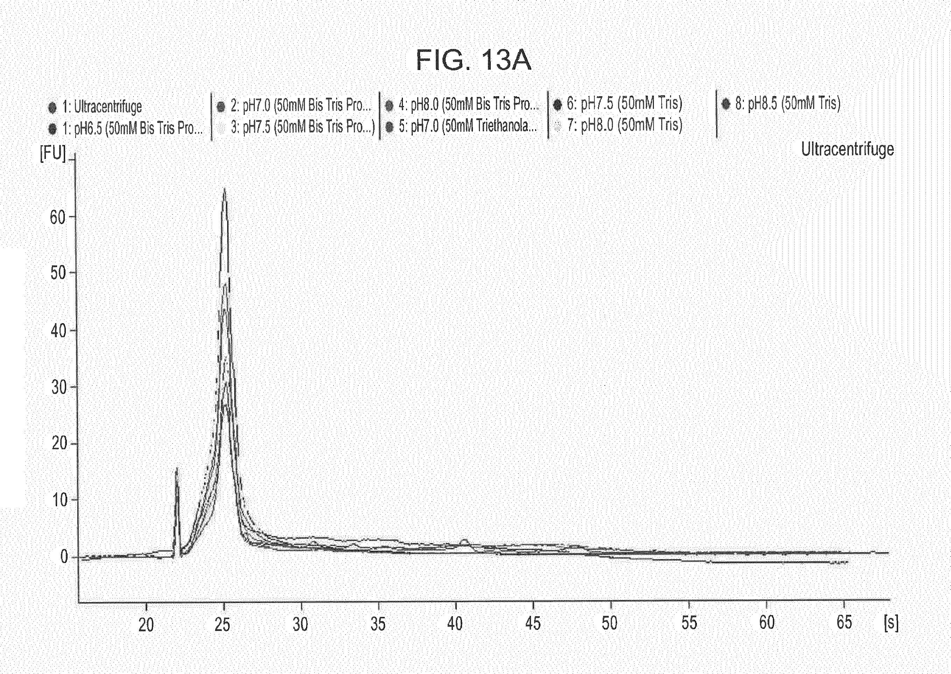

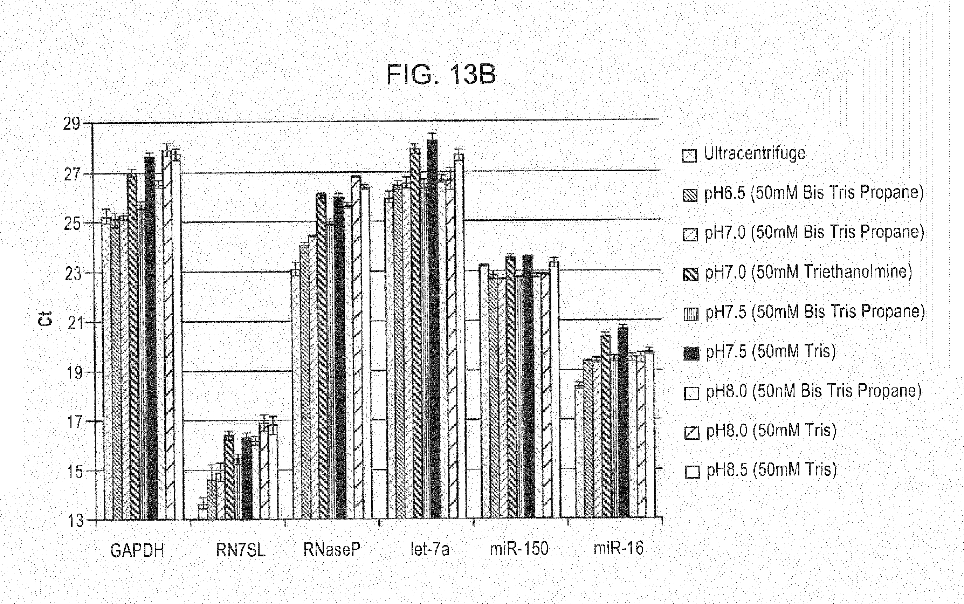

Figure 13 is a series of graphs depicting how microvesicle capture and/or microvesicle stability on a charged filter are affected by buffer pH and/or buffer concentration and/or buffer type (comparing buffers that are the same concentration, but NOT the same functional group concentration). Figure 13A is a bioanalyzer plot comparing the quality, concentration, and size distribution of microvesicle total RNA extracted from 3.8mL normal control plasma by ultracentrifugation and positively charged Q regenerated cellulose centrifugal filtration. Filtration samples were isolated with the following buffer sets:
   - 100mM Bis Tris Propane, 150mM NaCl, pH6 (2X Loading Buffer) and 50mM Bis Tris Propane, 150mM NaCl, pH6.5 (Equilibration and Wash Buffer)
   - 100mM Bis Tris Propane, 150mM NaCl, pH6.8 (2X Loading Buffer) and 50mM Bis Tris Propane, 150mM NaCl, pH7 (Equilibration and Wash Buffer)
   - 100mM Triethanolamine (TEA), 150mM NaCl, pH6.5 (2X Loading Buffer) and 50mM Triethanolamine, 150mM NaCl, pH7.0 (Equilibration and Wash Buffer)
   - 100mM Bis Tris Propane, 150mM NaCl, pH7.4 (2X Loading Buffer) and 50mM Bis Tris Propane, 150mM NaCl, pH7.5 (Equilibration and Wash Buffer)
   - 100mM Tris, 150mM NaCl, pH7.4 (2X Loading Buffer) and 50mM Tris, 150mM NaCl, pH7.5 (Equilibration and Wash Buffer)
   - 100mM Bis Tris Propane, 150mM NaCl, pH8 (2X Loading Buffer) and 50mM Bis Tris Propane, 150mM NaCl, pH8 (Equilibration and Wash Buffer)
   - 100mM Tris, 150mM NaCl, pH8 (2X Loading Buffer) and 50mM Tris, 150mM NaCl, pH8 (Equilibration and Wash Buffer)
   - 100mM Tris, 150mM NaCl, pH8.5 (2X Loading Buffer) and 50mM Tris, 150mM NaCl, pH8.5 (Equilibration and Wash Buffer)

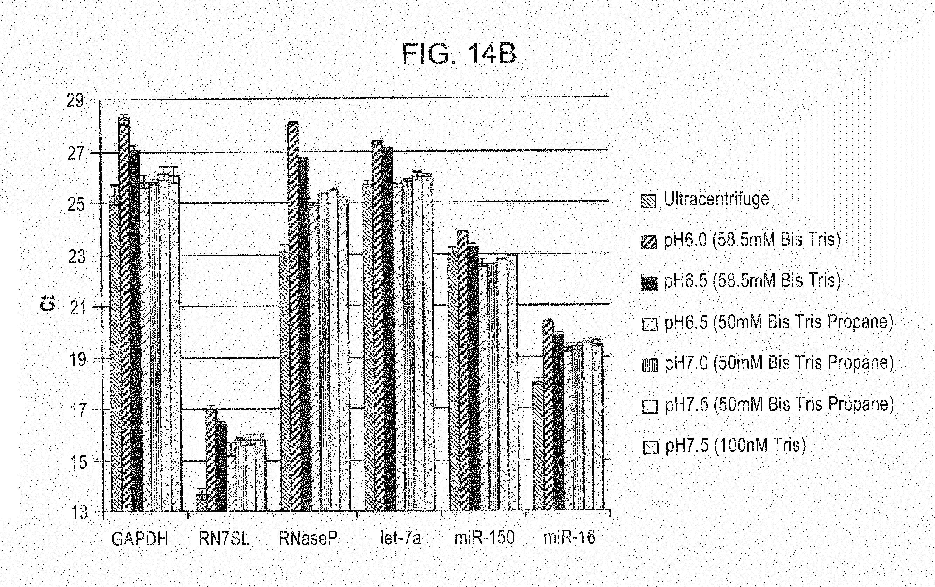

In Figure 13A, the legend identifies the sample by the equilibration and wash buffer only. Relative fluorescence units (FU) are plotted against time (s). The 25 s peak represents an internal standard. The most prominent peak represents small RNA. Figure 14B depicts levels of mRNA and mature miRNA that were analyzed using quantitative RT-PCR from the same samples. The cycle threshold (Ct) value is presented as the mean ± SD.

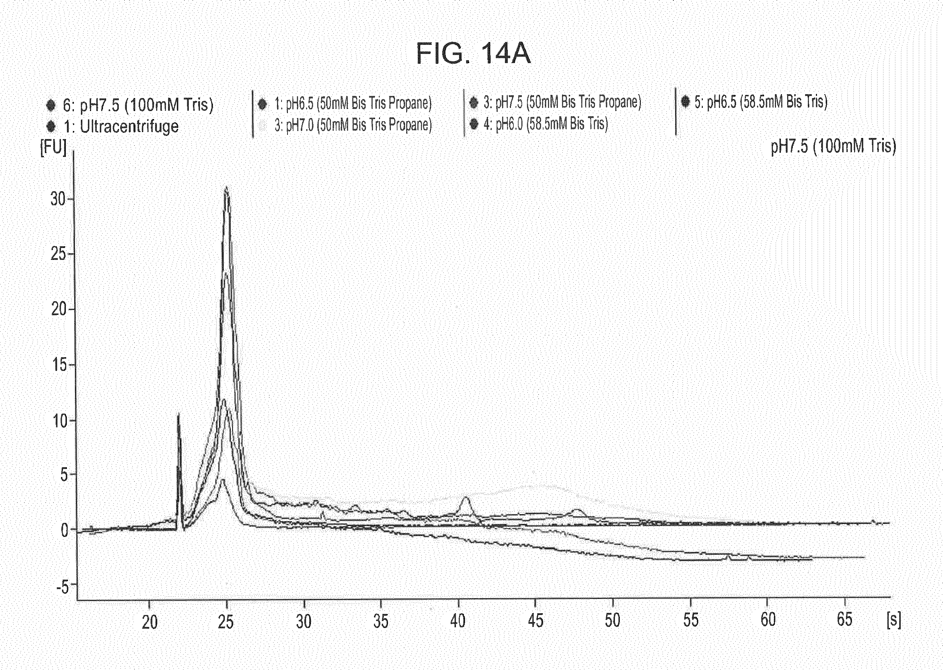

Figure 14 is a series of graphs depicting how microvesicle capture and/or microvesicle stability on a charged filter are affected by buffer pH and buffer concentration (comparing buffers that are the same functional group concentration, but not overall concentration). Figure 14A is a bioanalyzer plot comparing the quality, concentration, and size distribution of microvesicle total RNA extracted from 3.8mL normal control plasma by ultracentrifugation and positively charged Q regenerated cellulose centrifugal filtration. Filtration samples were isolated with the following buffer sets:
   - 117mM Bis Tris, 150mM NaCl, pH1.9 (2X Loading Buffer) and 58.5mM Bis Tris, 150mM NaCl, pH6 (Equilibration and Wash Buffer)
   - 1 17mM Bis Tris, 150mM NaCl, pH6.1 (2X Loading Buffer) and 58.5mM Bis Tris, 150mM NaCl, pH6.5 (Equilibration and Wash Buffer)
   - 100mM Bis Tris Propane, 150mM NaCl, pH6 (2X Loading Buffer) and 50mM Bis Tris Propane, 150mM NaCl, pH6.5 (Equilibration and Wash Buffer)
   - 100mM Bis Tris Propane, 150mM NaCl, pH6.8 (2X Loading Buffer) and 50mM Bis Tris Propane, 150mM NaCl, pH7 (Equilibration and Wash Buffer)
   - 100mM Bis Tris Propane, 150mM NaCl, pH7.4 (2X Loading Buffer) and 50mM Bis Tris Propane, 150mM NaCl, pH7.5 (Equilibration and Wash Buffer)
   - 200mM Tris, 150mM NaCl, pH7.5 (2X Loading Buffer) and 100mM Tris, 150mM NaCl, pH7.5 (Equilibration and Wash Buffer)
In Figure 14A, the legend identifies the sample by the equilibration and wash buffer only. Relative fluorescence units (FU) are plotted against time (s). The 25 s peak represents an internal standard. The most prominent peak represents small RNA. The peaks at ∼41 s and ∼47 s represent 18S and 28S, respectively. Figure 14B depicts levels of mRNA and mature miRNA that were analyzed using quantitative RT-PCR from the same samples. The cycle threshold (Ct) value is presented as the mean ± SD.

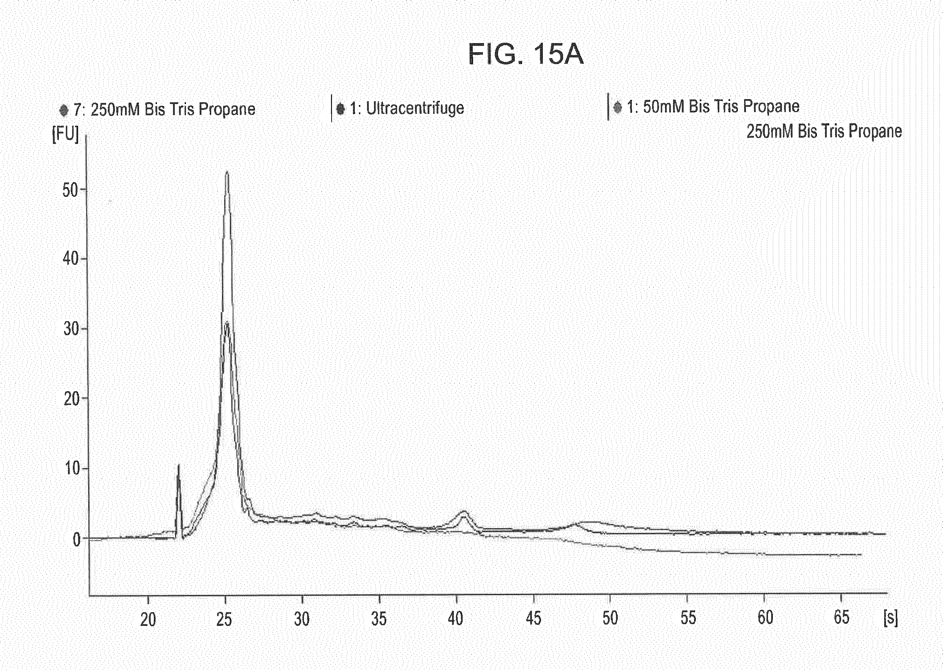

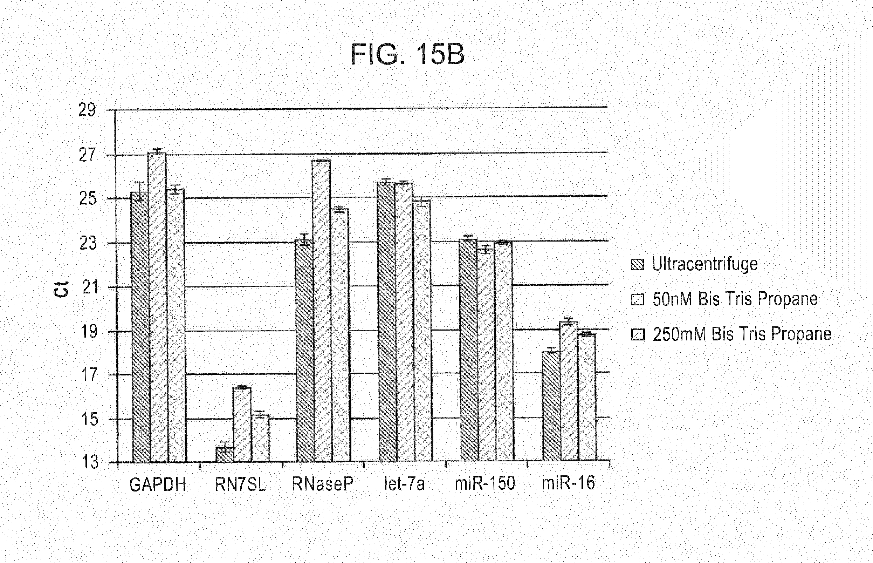

Figure 15 is a series of graphs depicting how microvesicle capture and microvesicle stability on a charged filter is affected by the concentration of buffer. Figure 15A is a bioanalyzer plot comparing the quality, concentration, and size distribution of microvesicle total RNA extracted from 3.8mL normal control plasma by ultracentrifugation and positively charged Q regenerated cellulose centrifugal filtration. Filtration samples were isolated with the following buffer sets:
   - 100mM Bis Tris Propane, 0.15mM NaCl, pH6 (2X Loading Buffer) and 50mM Bis Tris Propane, 0.15mM NaCl, pH6.5 (Equilibration and Wash Buffer)
   - 500mM Bis Tris Propane, 900mM NaCl, pH6.4 (2X Loading Buffer) and 250mM Bis Tris Propane, 450mM NaCl, pH6.5 (Equilibration and Wash Buffer)
In Figure 15A, the legend identifies the sample by the equilibration and wash buffer only. Relative fluorescence units (FU) are plotted against time (s). The 25 s peak represents an internal standard. The most prominent peak represents small RNA. The peaks at ∼41 s and ∼47 s represent 18S and 28S, respectively. Figure 15B depicts levels of mRNA and mature miRNA that were analyzed using quantitative RT-PCR from the same samples. The cycle threshold (Ct) value is presented as the mean ± SD.

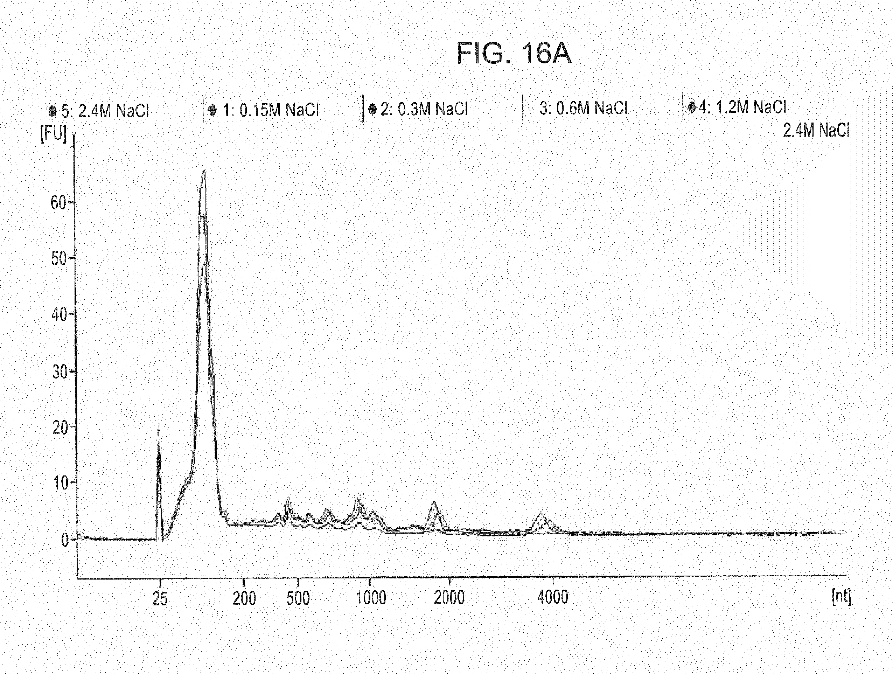

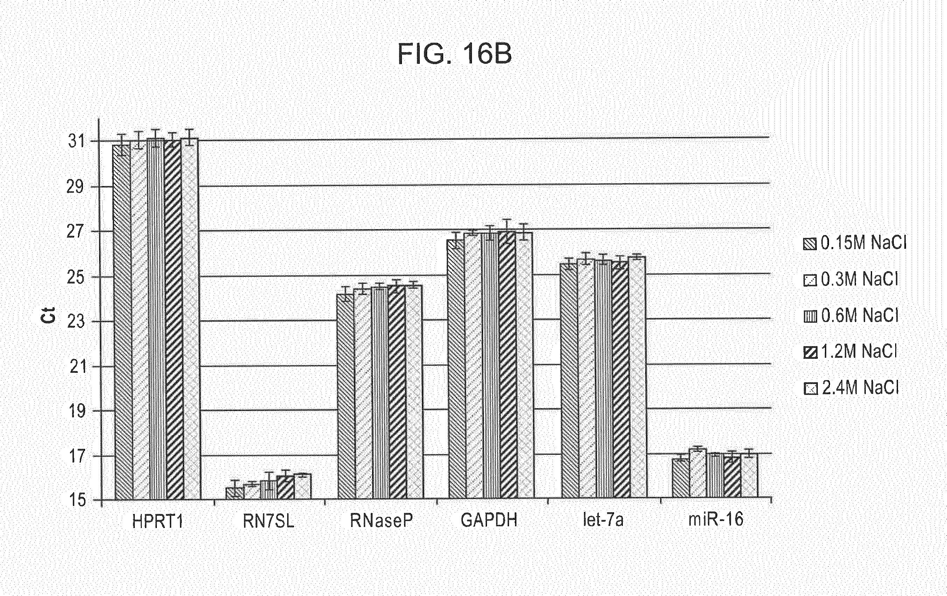

Figure 16 is a series of graphs depicting how microvesicles are stable and overall RNA yield is not affected by low to high concentrations of salt. Figure 16A is a bioanalyzer plot comparing the quality, concentration, and size distribution of microvesicle total RNA extracted from 1.9mL normal control plasma by centrifugation. Microvesicle pellets were resuspended in 50mM Bis Tris Propane, pH6.5 buffer with the following NaCl concentrations and incubated for 20 min prior to lysis: 0.15M NaCl, 0.3M NaCl, 0.6M NaCl, 1.2M NaCl and 2.4M NaCl.
   Relative fluorescence units (FU) are plotted against size (nt). The 25 nt peak represents an internal standard. The most prominent peak represents small RNA. The peaks at ∼1900 nt and ∼3900 nt represent 18S and 28S, respectively. Figure 16B depicts levels of mRNA and mature miRNA that were analyzed using quantitative RT-PCR on the same samples. The cycle threshold (Ct) value is presented as the mean ± SD.

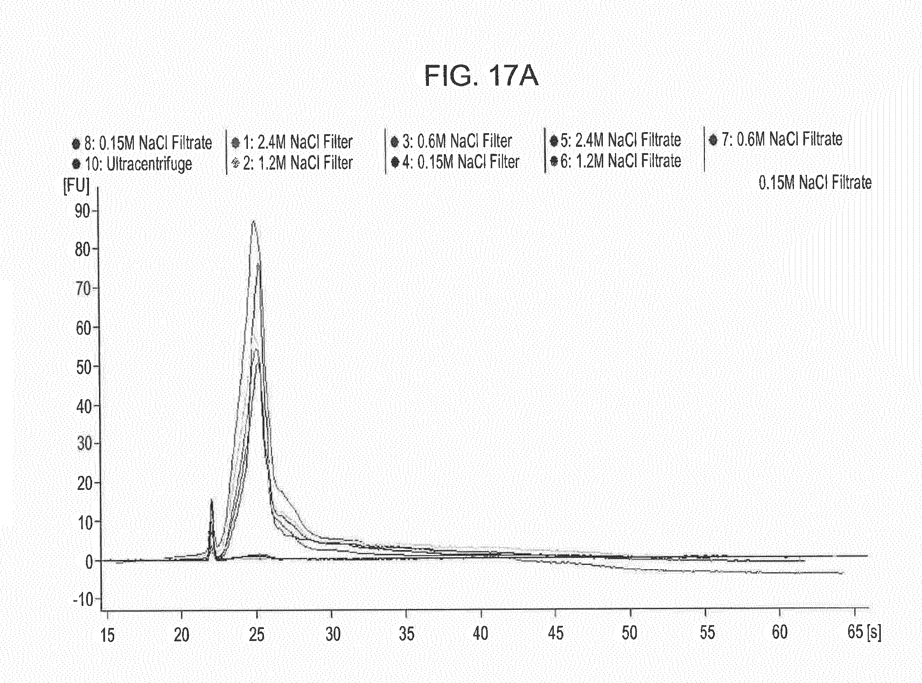

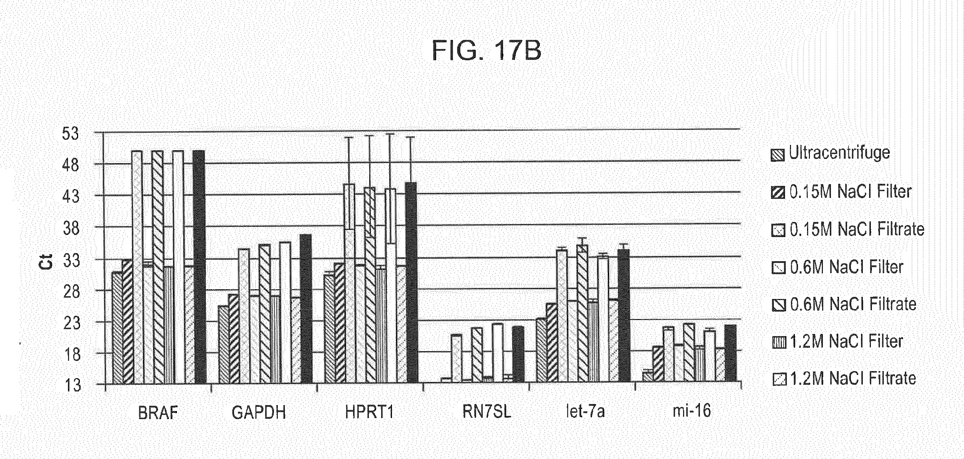

Figure 17 is a series of graphs depicting how microvesicle capture and microvesicle stability on a charged filter is not affected by salt concentration of the loading buffer. Figure 17A is a bioanalyzer plot comparing the quality, concentration, and size distribution of microvesicle total RNA extracted from 3.8mL normal control plasma by ultracentrifugation and positively charged Q regenerated cellulose centrifugal filtration (filter and filtrate). Filtration samples were isolated with the following buffer sets:
   - 100mM Bis Tris Propane, 0.15M NaCl, pH6.0 (2X Loading buffer) and 50mM Bis Tris Propane, 0.15M NaCl, pH6.5 (Equilibration buffer)
   - 100mM Bis Tris Propane, 1.05M NaCl, pH6.0 (2X Loading buffer) and 50mM Bis Tris Propane, 0.6M NaCl, pH6.5 (Equilibration buffer)
   - 100mM Bis Tris Propane, 2.25M NaCl, pH6.0 (2X Loading buffer) and 50mM Bis Tris Propane, 1.2M NaCl, pH6.5 (Equilibration buffer)
   - 100mM Bis Tris Propane, 4.65M NaCl, pH6.0 (2X Loading buffer) and 50mM Bis Tris Propane, 2.4M NaCl, pH6.5 (Equilibration buffer)

   The filtration samples were not washed before elution. In Figure 17A, the legend identifies the samples by the equilibration buffer only. Relative fluorescence units (FU) are plotted against time (s). The 25 s peak represents an internal standard. The most prominent peak represents small RNA. Figure 17B depicts levels of mRNA and mature miRNA that were analyzed using quantitative RT-PCR from the same samples. The cycle threshold (Ct) value is presented as the mean ± SD.

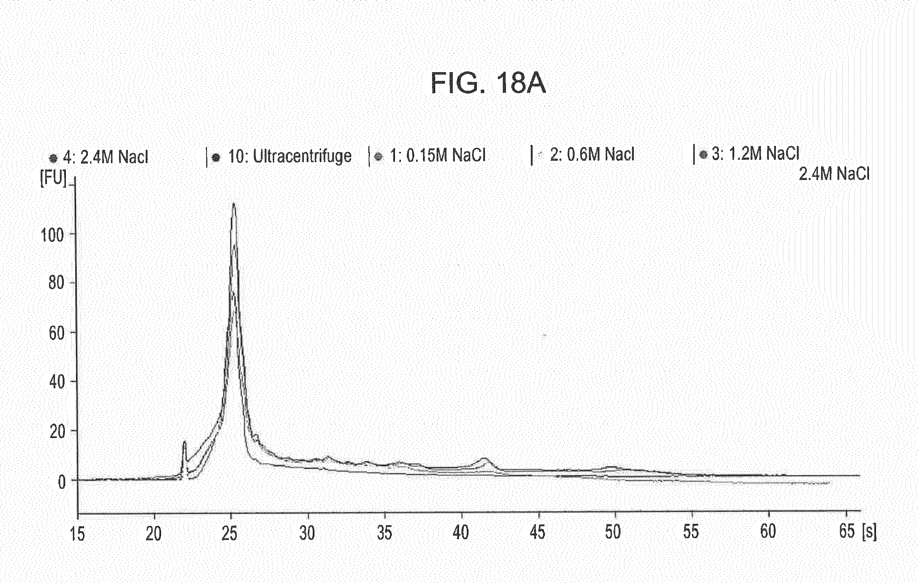

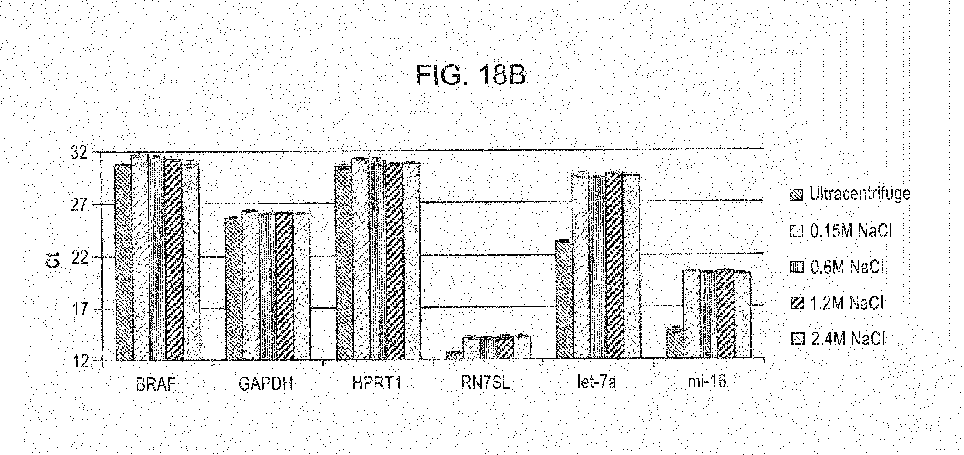

Figure 18 is a series of graphs depicting how microvesicle capture and microvesicle stability on a charged filter is not affected by salt concentration of the loading or wash buffer. Figure 18A is a bioanalyzer plot comparing the quality, concentration, and size distribution of microvesicle total RNA extracted from 3.8mL normal control plasma by ultracentrifugation and positively charged Q regenerated cellulose centrifugal filtration. Filtration samples were isolated with the following buffer sets:
   - 100mM Bis Tris Propane, 0.15mM NaCl, pH6 (2X Loading Buffer) and 50mM Bis Tris Propane, 0.15mM NaCl, pH6.5 (Equilibration and Wash Buffer)
   - 100mM Bis Tris Propane, 1.05M NaCl, pH6 (2X Loading Buffer) and 50mM Bis Tris Propane, 0.6M NaCl, pH6.5 (Equilibration and Wash Buffer)
   - 100mM Bis Tris Propane, 2.25M NaCl, pH6 (2X Loading Buffer) and 50mM Bis Tris Propane, 1.2M NaCl, pH6.5 (Equilibration and Wash Buffer)
   - 100mM Bis Tris Propane, 4.65M NaCl, pH6 (2X Loading Buffer) and 50mM Bis Tris Propane, 2.4M NaCl, pH6.5 (Equilibration and Wash Buffer).
In Figure 18A, the legend identifies the samples by the NaCl concentration in the equilibration and wash buffer only. Relative fluorescence units (FU) are plotted against time (s). The 25 s peak represents an internal standard. The most prominent peak represents small RNA. The peaks at ∼42 s and ∼50 s represent 18S and 28S, respectively. Figure 18B depicts levels of mRNA and mature miRNA that were analyzed using quantitative RT-PCR from the same samples. The cycle threshold (Ct) value is presented as the mean ± SD.
Figure 19 is a graph depicting how the RNA lysis buffer affects the RNA yield from microvesicles isolated with a charged filter. Levels of mRNA and mature miRNA that were analyzed using quantitative RT-PCR from 4mL plasma extracted by ultracentrifugation and positively charged Q PES vacuum filtration. Filtration samples were isolated with Qiazol or Promega lysis buffer. The relative amount value is presented as the mean ± SD.

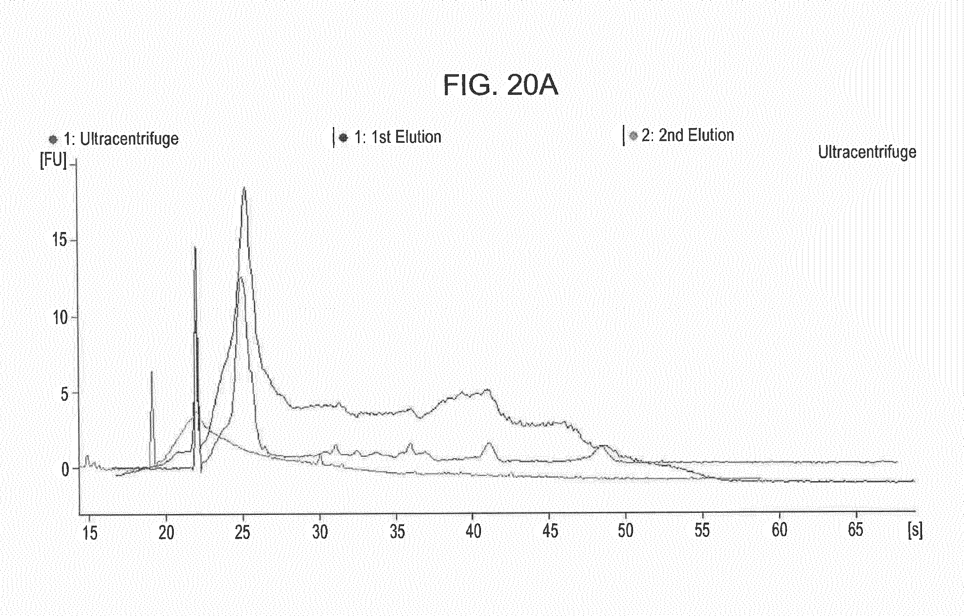

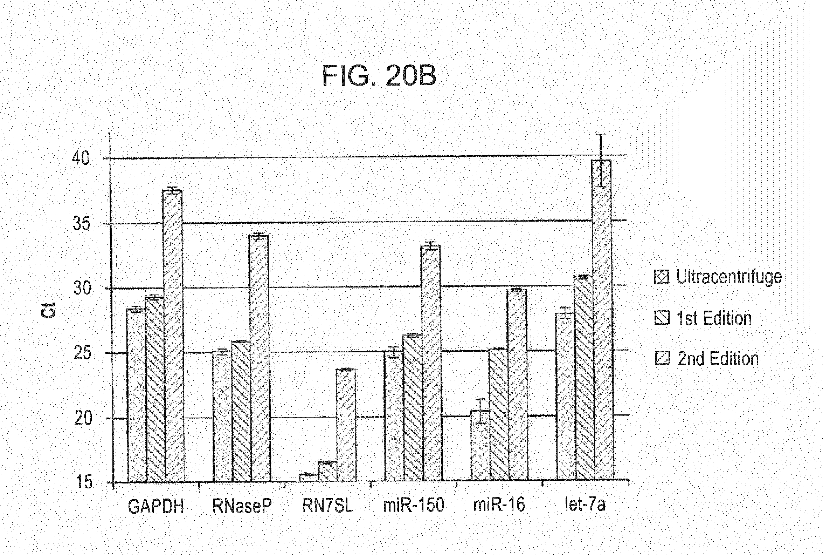

Figure 20 is a series of graphs depicting how a second volume of Qiazol does not significantly improve the RNA yields when isolating microvesicles on a charged filter. Figure 20A is a bioanalyzer plot comparing the quality, concentration, and size distribution of microvesicle total RNA extracted from 2mL normal control plasma by ultracentrifugation and positively charged Q PES vacuum filtration. Filter samples were isolated with two volumes of Qiazol lysis buffer. Relative fluorescence units (FU) are plotted against time (s). The 25 s peak represents an internal standard. The most prominent peak represents small RNA. The peaks at ∼41 s and ∼47 s represent 18S and 28S, respectively. There were technical difficulties with the second Qiazol elution sample. The internal standard is instead at ∼18 s and the small RNA peak is at 25 s. Figure 20B depicts levels of mRNA and mature miRNA that were analyzed using quantitative RT-PCR from the same samples. The cycle threshold (Ct) value is presented as the mean ± SD.

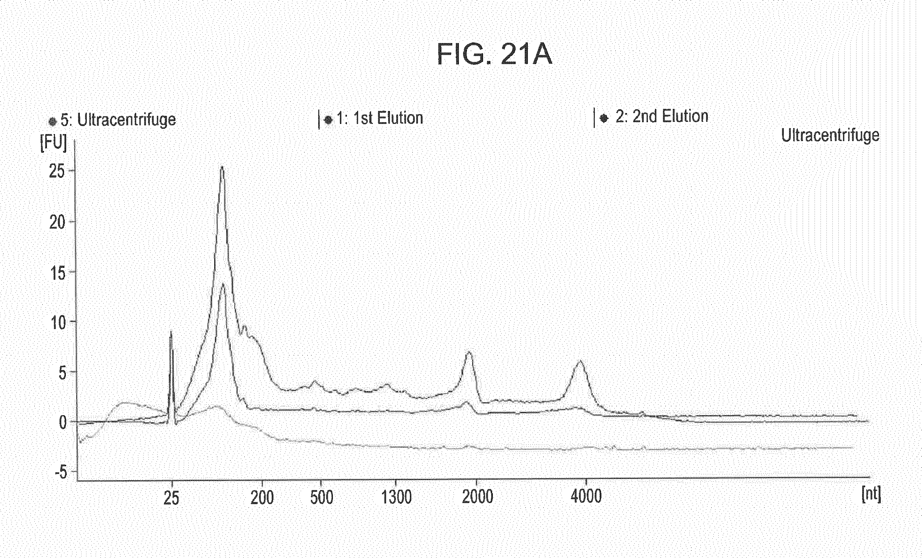

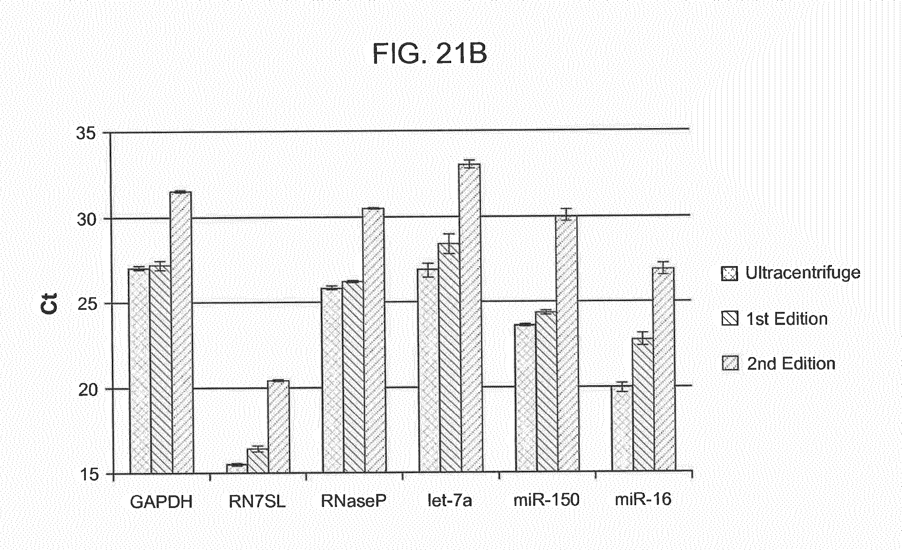

Figure 21 is a series of graphs depicting how a second volume of Qiazol does not significantly improve the RNA yields when isolating microvesicles on a charged filter. Figure 21A is a bioanalyzer plot comparing the quality, concentration, and size distribution of microvesicle total RNA extracted from 4mL normal control plasma by ultracentrifugation and positively charged Q regenerated cellulose centrifugal filtration. Filtration samples were isolated with two volumes of Qiazol lysis buffer. Relative fluorescence units (FU) are plotted against size (nt). The 25 nt peak represents an internal standard. The most prominent peak represents small RNA. The peaks at -1900 nt and -3900 nt represent 18S and 28S, respectively. Figure 21B depicts levels of mRNA and mature miRNA that were analyzed using quantitative RT-PCR from the same samples. The cycle threshold (Ct) value is presented as the mean ± SD.

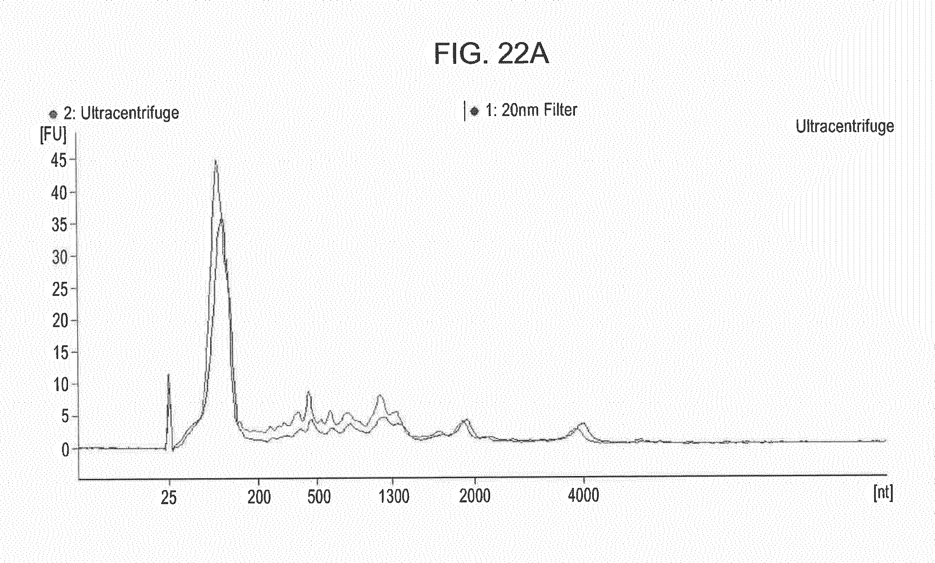

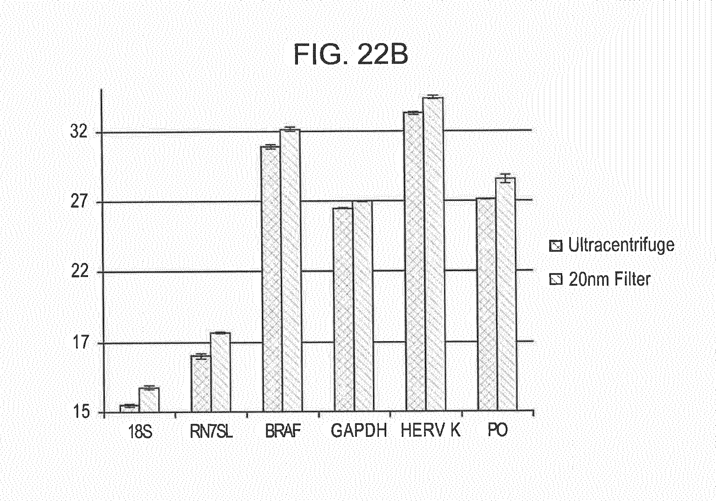

Figure 22 is a series of graphs depicting how microvesicular RNA can be isolated using a 20nm PES neutral syringe filter. Figure 22A is a bioanalyzer plot comparing the quality, concentration, and size distribution of microvesicle total RNA extracted from 4mL normal control plasma by ultracentrifugation and 20nm neutral PES syringe filtration. Relative fluorescence units (FU) are plotted against size (nt). The 25 nt peak represents an internal standard. The most prominent peak represents small RNA. The peaks at ∼1900 nt and ∼3900 nt represent 18S and 28S, respectively. Figure 22B depicts levels of mRNA were analyzed using quantitative RT-PCR from the same samples. The cycle threshold (Ct) value is presented as the mean ± SD.

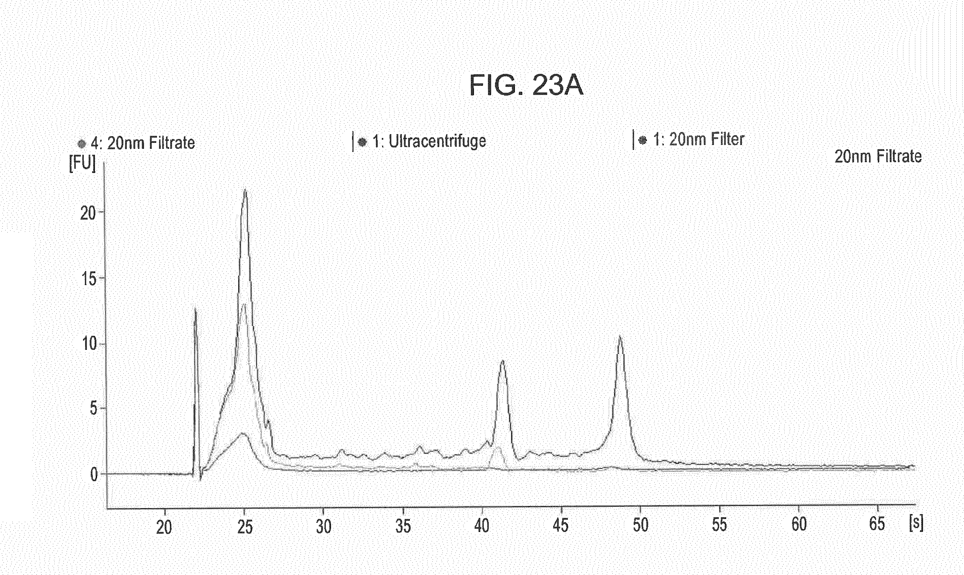

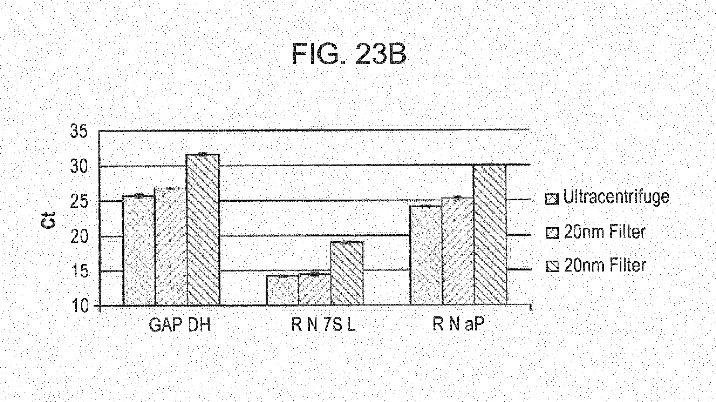

Figure 23 is a series of graphs depicting how microvesicular RNA can be isolated using a 20nm PES neutral syringe filter. Figure 23A is a bioanalyzer plot comparing the quality, concentration, and size distribution of microvesicle total RNA extracted from 4mL normal control plasma by ultracentrifugation and 20nm neutral PES syringe filtration. Relative fluorescence units (FU) are plotted against time (s). The 25 s peak represents an internal standard. The most prominent peak represents small RNA. The peaks at ∼41 s and ∼47 s represent 18S and 28S, respectively. Figure 23B depicts levels of mRNA were analyzed using quantitative RT-PCR from the same samples. The cycle threshold (Ct) value is presented as the mean ± SD.

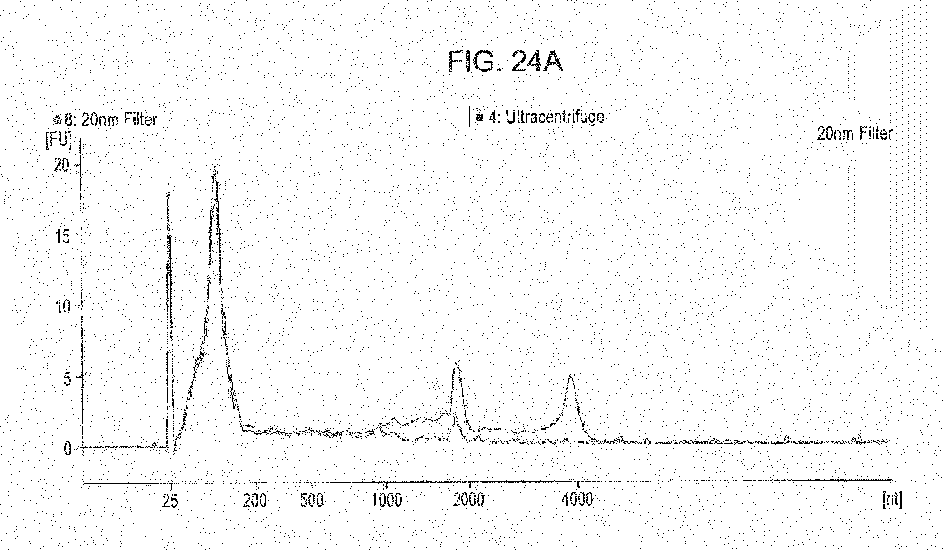

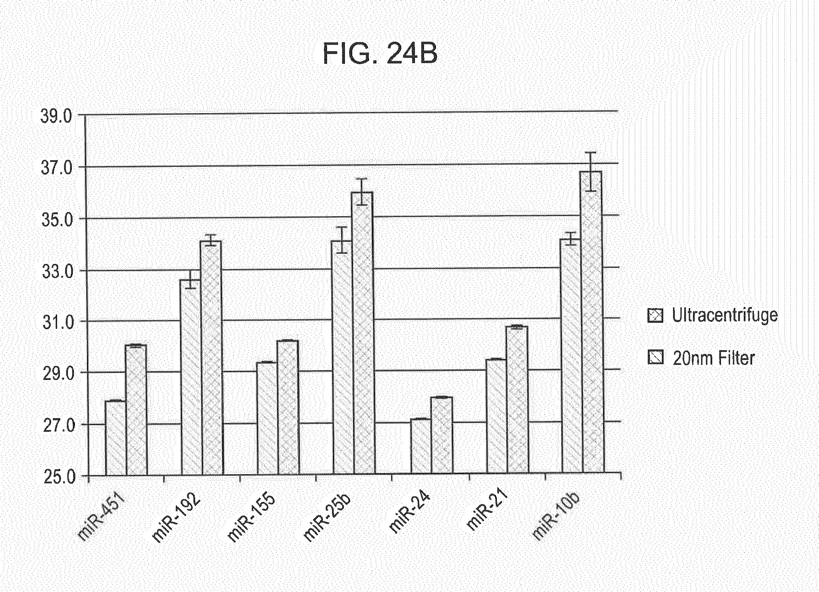

Figure 24 is a series of graphs depicting how microvesicular miRNA can be isolated using a 20nm PES neutral syringe filter (Tisch). Figure 24A is a bioanalyzer plot comparing the quality, concentration, and size distribution of microvesicle total RNA extracted from 2mL normal control plasma by ultracentrifugation and 20nm neutral PES syringe filtration. Relative fluorescence units (FU) are plotted against size (nt). The 25 nt peak represents an internal standard. The most prominent peak represents small RNA. The peaks at ∼1900 nt and ∼3900 nt represent 18S and 28S, respectively. Figure 24B depicts levels of mature miRNA that were analyzed using quantitative RT-PCR from the same samples. The cycle threshold (Ct) value is presented as the mean ± SD.

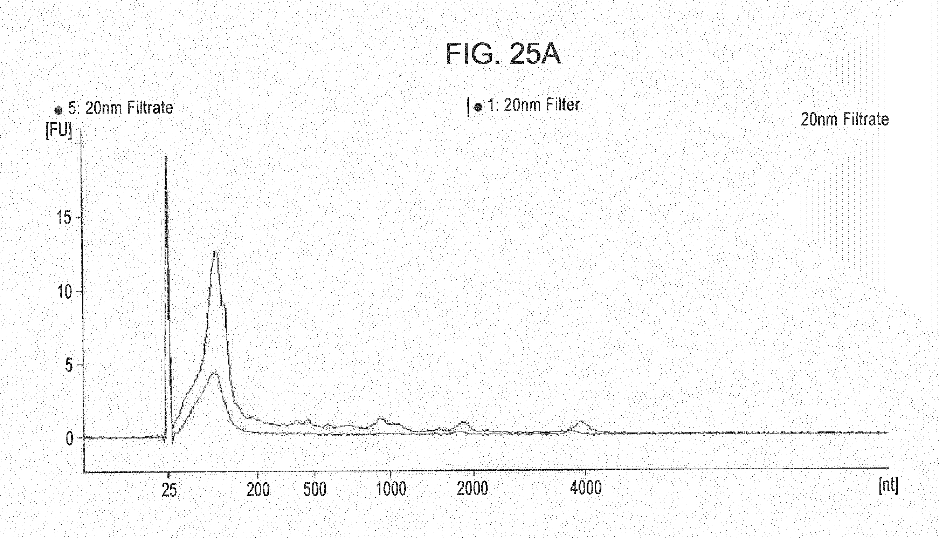

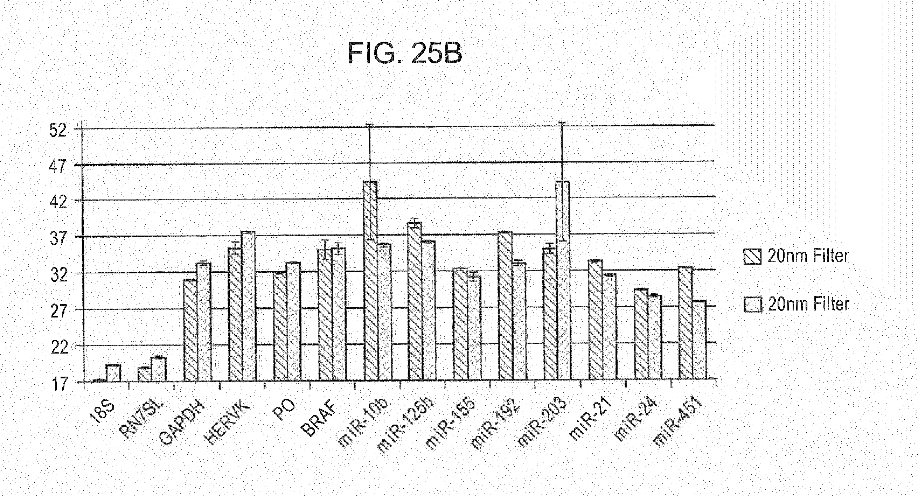

Figure 25 is a series of graphs depicting how microvesicle capture is RNA dependent. Some RNAs are more efficiently captured on the filter compared to others (GAPDH vs. miR-451). This may depend on whether the RNA is protected by proteins and/or microvesicles and on microvesicle size. Figure 25A is a bioanalyzer plot comparing the quality, concentration, and size distribution of microvesicle total RNA extracted from 2mL normal control plasma by neutral PES syringe filtration (filter and filtrate). Relative fluorescence units (FU) are plotted against size (nt). The 25 nt peak represents an internal standard. The most prominent peak represents small RNA. The peaks at ∼1900 nt and ∼3900 nt represent 18S and 28S, respectively. Figure 25B depicts levels of mRNA and mature miRNA that were analyzed using quantitative RT-PCR from the same samples. The cycle threshold (Ct) value is presented as the mean ± SD.

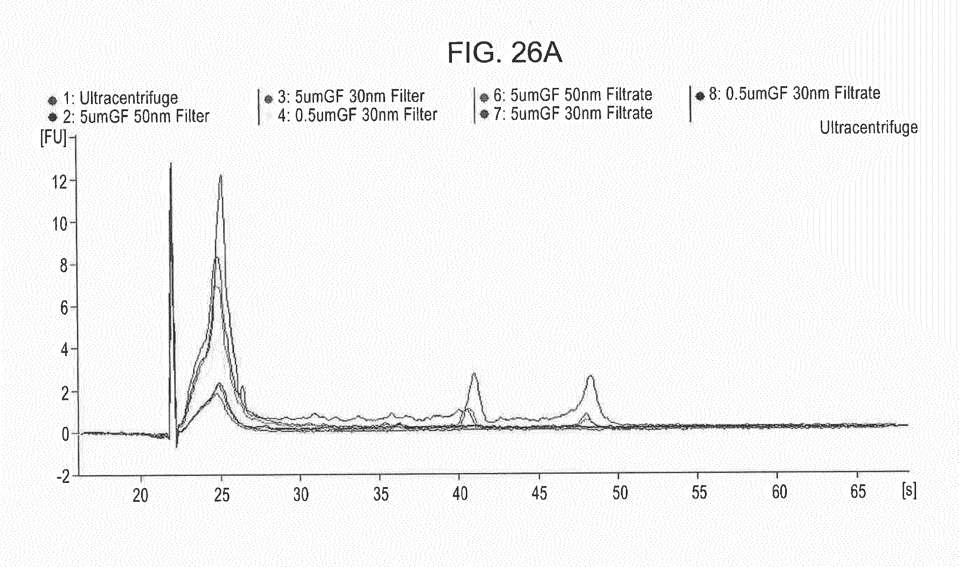

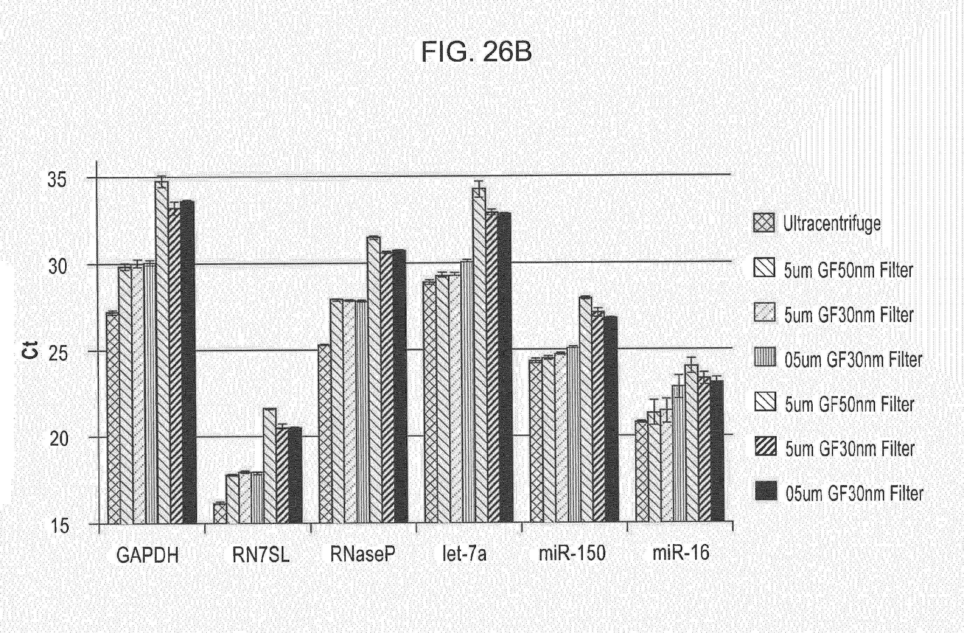

Figure 26 is a series of graphs depicting how microvesicular RNA can be isolated using a 30nm and a 50nm PES neutral syringe filter. Figure 26A is a bioanalyzer plot comparing the quality, concentration, and size distribution of microvesicle total RNA extracted from 2mL normal control plasma by ultracentrifugation and 30nm (5um or 0.05um glass fiber (GF) prefilter) and 50nm (5um GF prefilter) neutral PES syringe filtration (filter and filtrate). Relative fluorescence units (FU) are plotted against time (s). The 25 s peak represents an internal standard. The most prominent peak represents small RNA. The peaks at ∼41 s and ∼47 s represent 18S and 28S, respectively. Figure 26B depicts levels of mRNA and mature miRNA that were analyzed using quantitative RT-PCR from the same samples. The cycle threshold (Ct) value is presented as the mean ± SD.

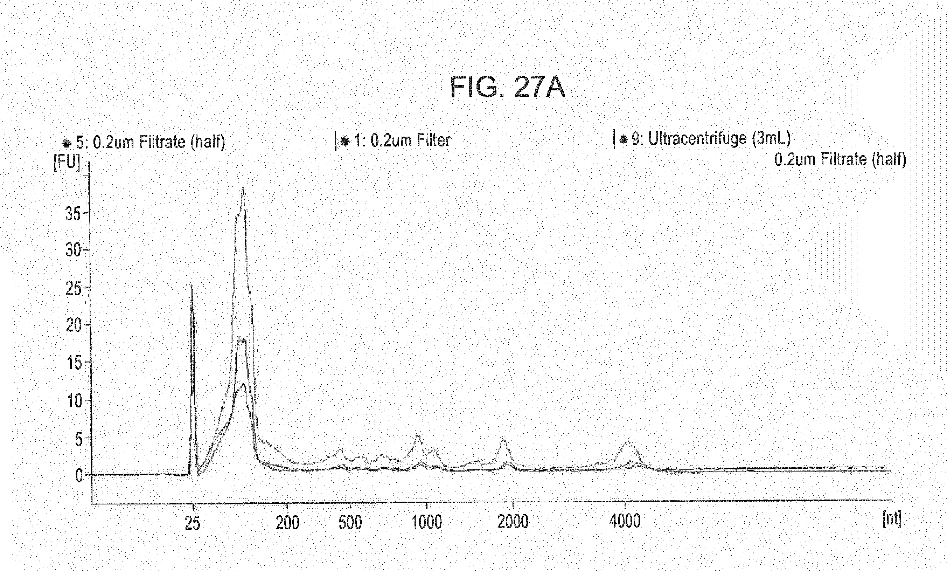

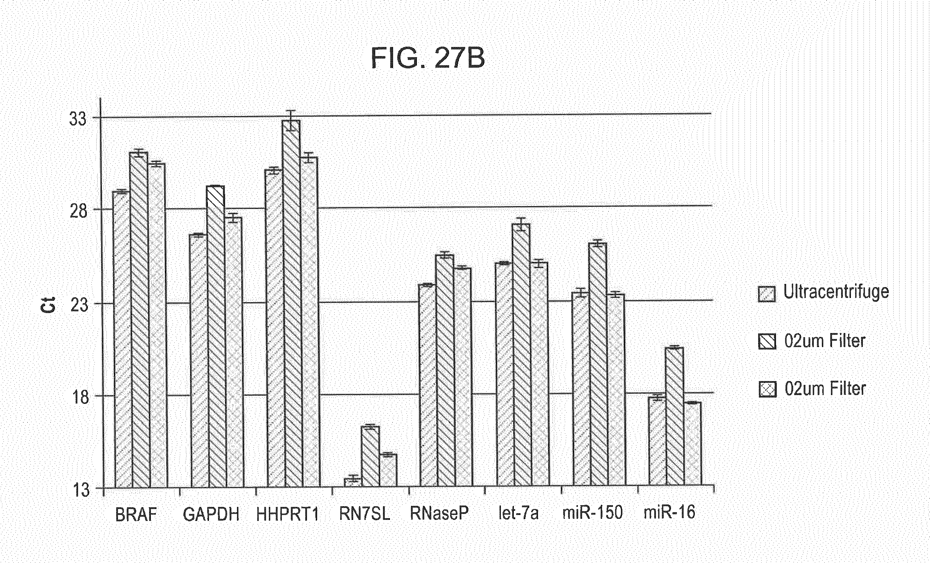

Figure 27 is a series of graphs depicting how microvesicular RNA can be isolated using a 0.2um PES neutral filter in a spin column format (Pall). Figure 27A is a bioanalyzer plot comparing the quality, concentration, and size distribution of microvesicle total RNA extracted from 4mL normal control plasma by ultracentrifugation and neutral 0.2um PES centrifugal filtration (filter and filtrate). Relative fluorescence units (FU) are plotted against size (nt). The 25 nt peak represents an internal standard. The most prominent peak represents small RNA. The peaks at ∼1900 nt and ∼4200 nt represent 18S and 28S, respectively. Figure 27B depicts levels of mRNA and mature miRNA that were analyzed using quantitative RT-PCR from the same samples. The cycle threshold (Ct) value is presented as the mean ± SD.

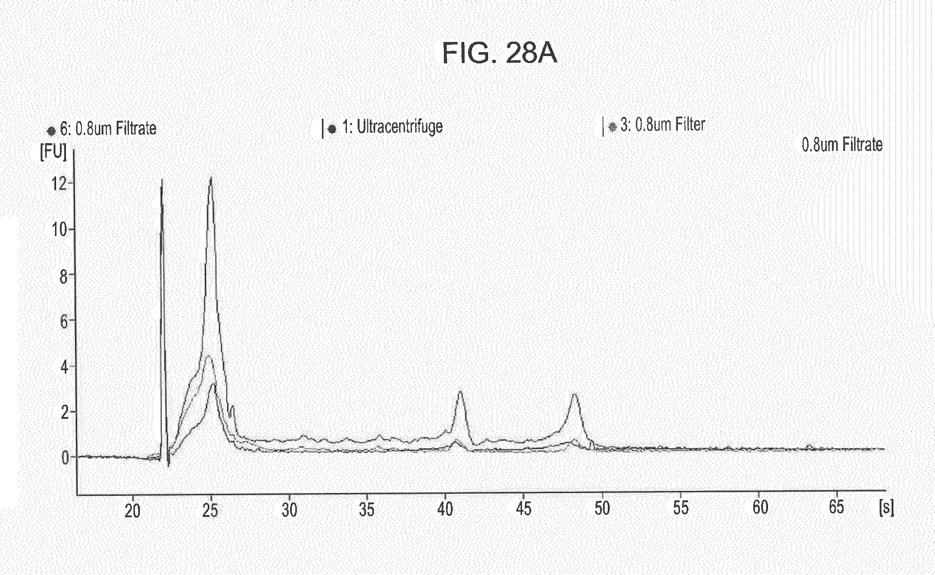

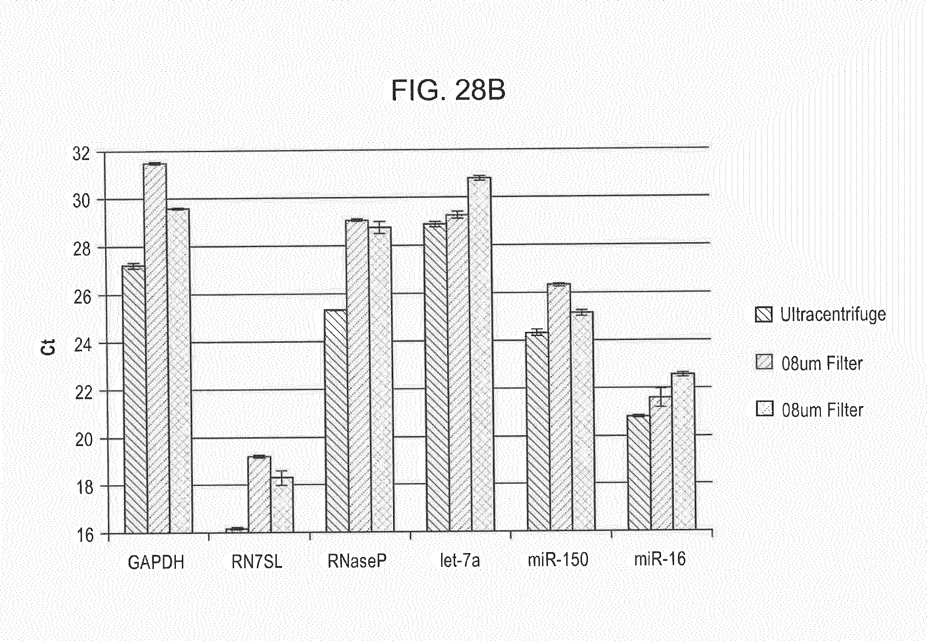

Figure 28 is a series of graphs depicting how microvesicular RNA can be isolated using a 0.8um PES neutral syringe filter. Figure 28A is a bioanalyzer plot comparing the quality, concentration, and size distribution of microvesicle total RNA extracted from 4mL normal control plasma by ultracentrifugation and neutral 0.8um PES syringe filtration (filter and filtrate). Relative fluorescence units (FU) are plotted against time (s). The 25 s peak represents an internal standard. The most prominent peak represents small RNA. The peaks at ∼41 s and ∼47 s represent 18S and 28S, respectively. Figure 28B depicts levels of mRNA and mature miRNA that were analyzed using quantitative RT-PCR from the same samples. The cycle threshold (Ct) value is presented as the mean ± SD.

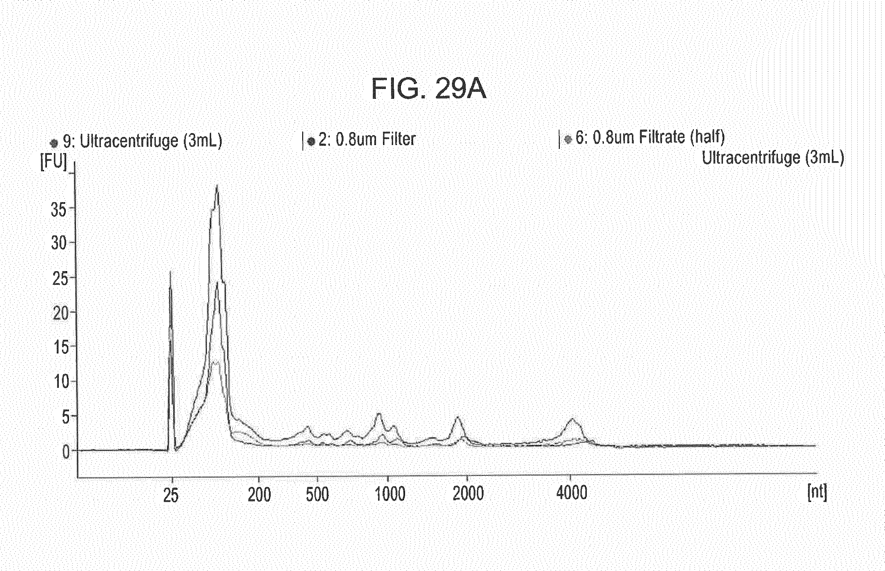

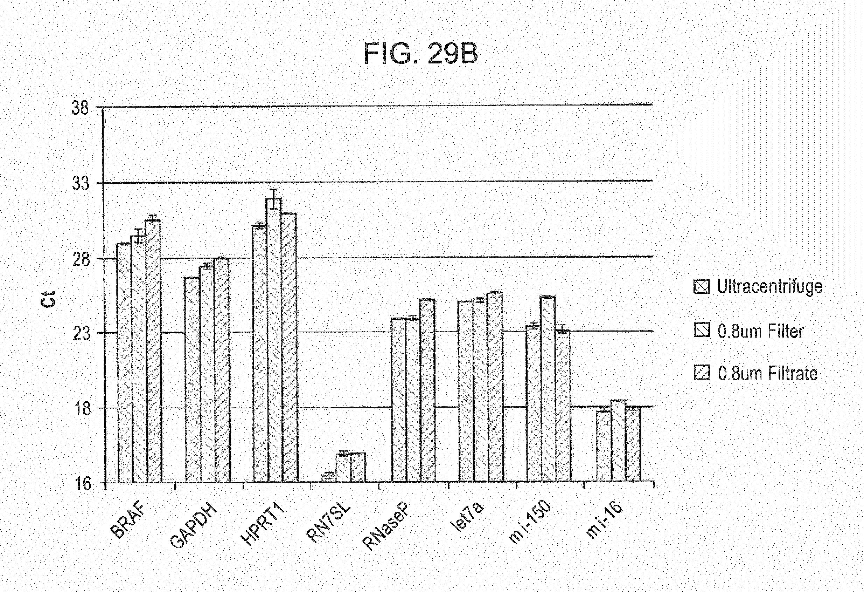

Figure 29 is a series of graphs depicting how microvesicular RNA can be isolated using a 0.8um PES neutral filter in a spin column format (Pall). Figure 29A is a bioanalyzer plot comparing the quality, concentration, and size distribution of microvesicle total RNA extracted from 4mL normal control plasma by ultracentrifugation and neutral 0.8um PES centrifugal filtration (filter and filtrate). The 0.8um filtrate sample was only isolated from half of the total sample volume. Relative fluorescence units (FU) are plotted against size (nt). The 25 nt peak represents an internal standard. The most prominent peak represents small RNA. The peaks at ∼1900 nt and ∼4200 nt represent 18S and 28S, respectively. Figure 29B depicts levels of mRNA and mature miRNA that were analyzed using quantitative RT-PCR from the same samples. The cycle threshold (Ct) value is presented as the mean ± SD. The "0.8um Filter" and "Ultracentrifugation" samples Ct values have been adjusted to account for only a partial isolation.

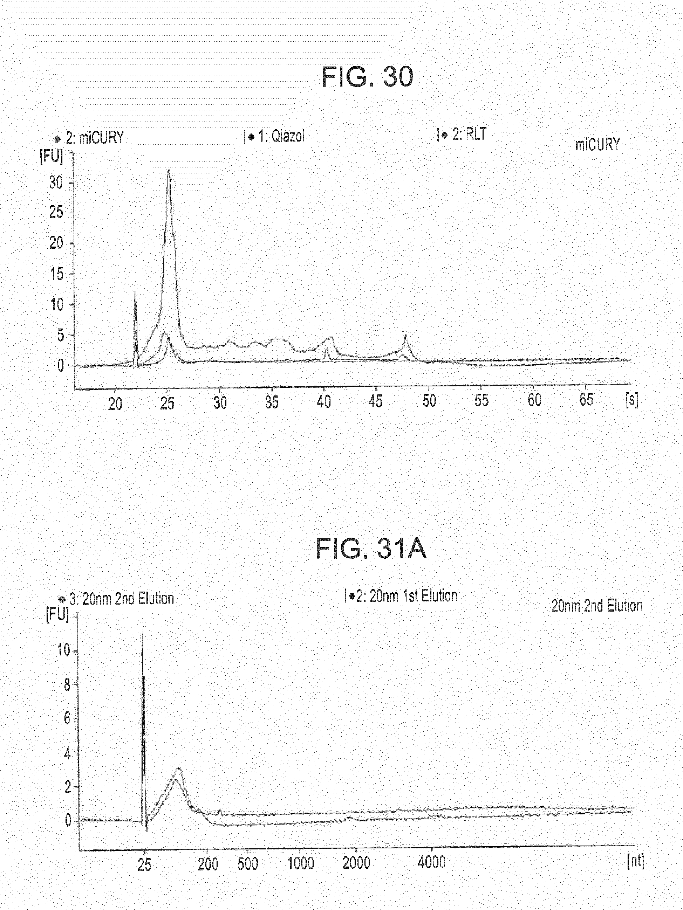

Figure 30 is a graph depicting how microvesicular RNA yield is affected by a lysis buffer type when isolating microvesicles on a neutral PES filter. Figure 30 is a bioanalyzer plot comparing the quality, concentration, and size distribution of microvesicle total RNA extracted from 6mL normal control plasma by neutral PES syringe filtration. Filtration samples were lysed with Qiazol (Qiagen), RLT (Qiagen), or miCURY (Exiqon). Relative fluorescence units (FU) are plotted against time (s). The 25 s peak represents an internal standard. The most prominent peak represents small RNA. The peaks at ∼41 s and ∼47 s represent 18S and 28S, respectively.

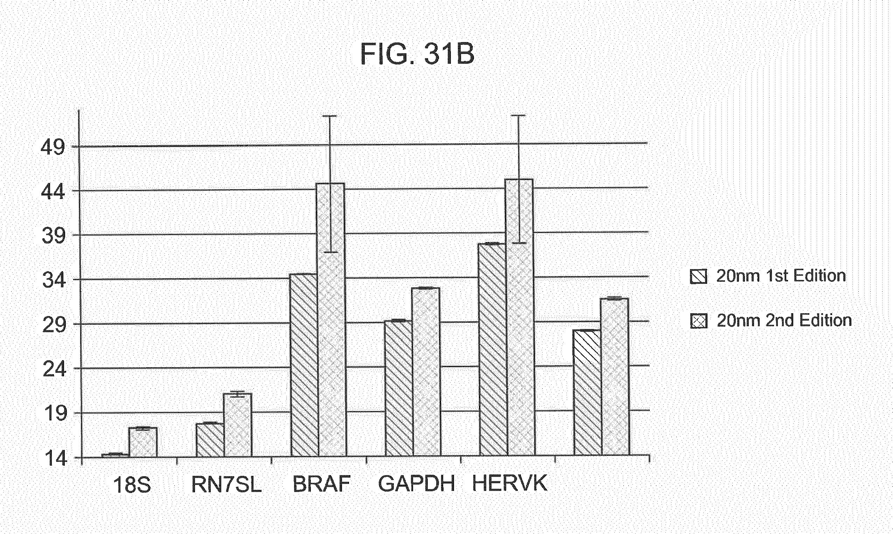

Figure 31 is a series of graphs depicting how an additional elution with Qiazol does not significantly improve the RNA yields in the isolation of microvesicular RNA on a 20nm PES neutral syringe filter. Figure 31A is a bioanalyzer plot comparing the quality, concentration, and size distribution of microvesicle total RNA extracted from 6mL normal control plasma by neutral PES syringe filtration. Filtration samples were lysed with two volumes of Qiazol. Relative fluorescence units (FU) are plotted against size (nt). The 25 nt peak represents an internal standard. The most prominent peak represents small RNA. The peaks at ∼1900 nt and ∼4200 nt represent 18S and 28S, respectively. Figure 31B depicts levels of mRNA were analyzed using quantitative RT-PCR from the same samples. The cycle threshold (Ct) value is presented as the mean ± SD.

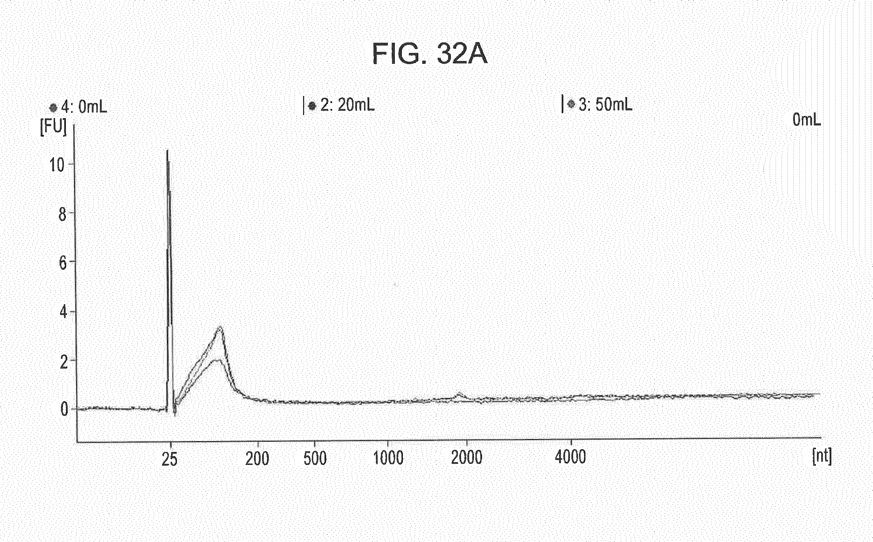

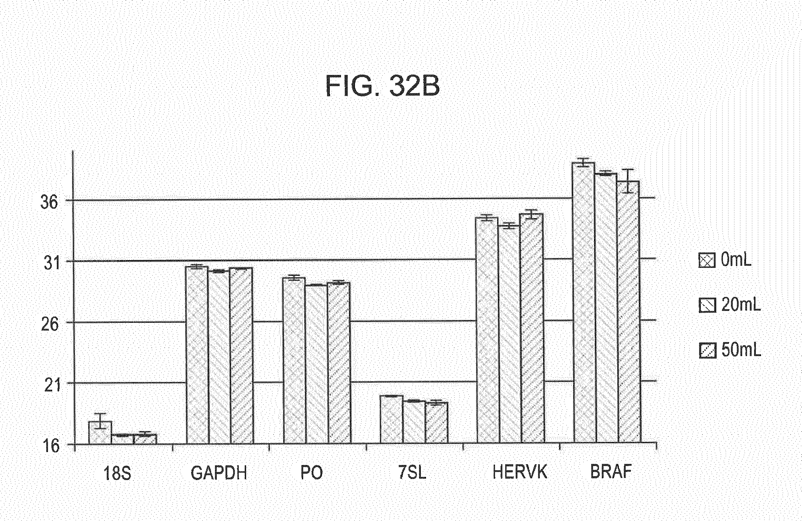

Figure 32 is a series of graphs depicting how microvesicle stability and/or microvesicular RNA yield is affected by a wash step when isolating microvesicles on a neutral filter. Figure 32A is a bioanalyzer plot comparing the quality, concentration, and size distribution of microvesicle total RNA extracted from 6mL normal control plasma by neutral PES syringe filtration. Filtration samples were washed with 0mL, 20mL, or 50mL of 10mM Sodium phosphate, 2mM Potassium phosphate, 2.7mM KCl, 137mM NaCl, pH 7.4 buffer. Relative fluorescence units (FU) are plotted against size (nt). The 25 nt peak represents an internal standard. The most prominent peak represents small RNA. The peaks at ∼1900 nt and ∼4200 nt represent 18S and 28S, respectively. Figure 32B depicts levels of mRNA and mature miRNA that were analyzed using quantitative RT-PCR from the same samples. The cycle threshold (Ct) value is presented as the mean ± SD.

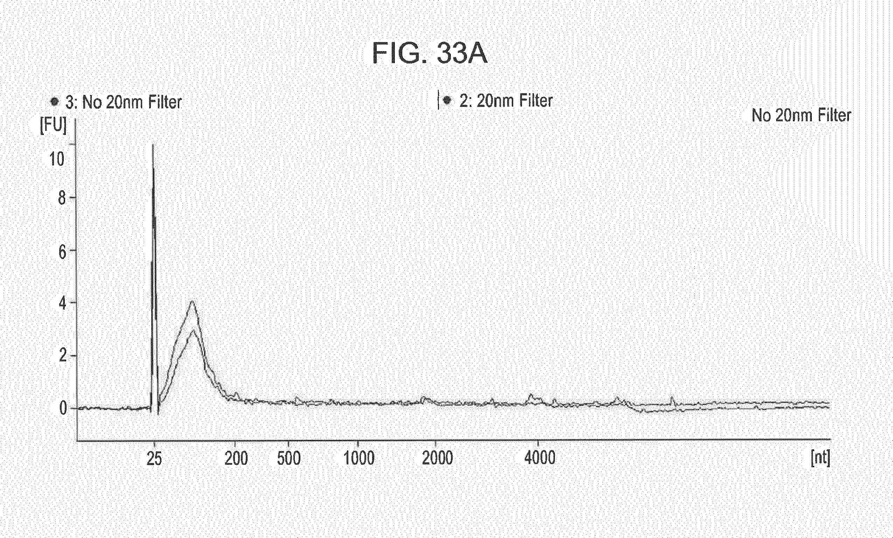

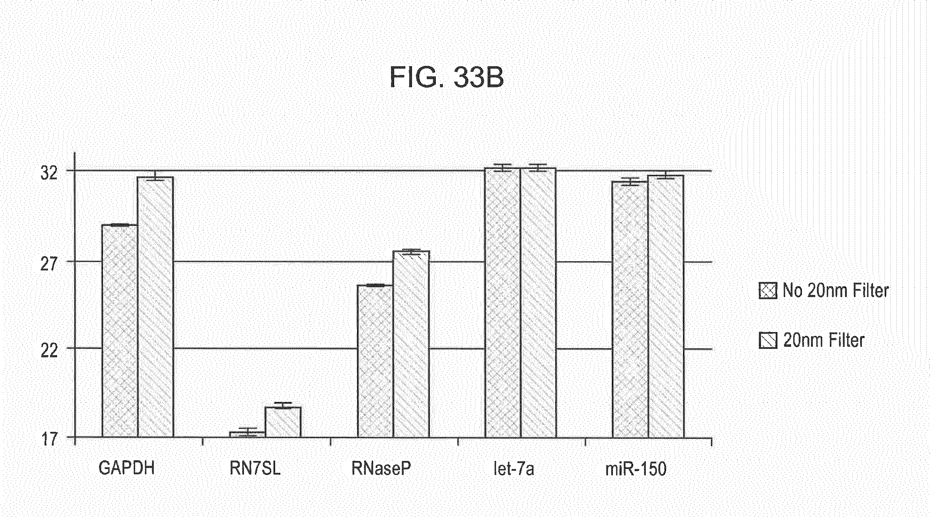

Figure 33 is a series of graphs depicting how RNA gets stuck on the 20nm PES filter in a syringe format and cannot be easily eluted off. Larger RNA (ex. GAPDH) is harder to elute off than smaller RNA (let-7a). Figure 33A is a bioanalyzer plot comparing the quality, concentration, and size distribution of lOng control total RNA isolated by resuspension in Qiazol and subsequent RNA isolation or by 20nm neutral PES syringe filtration followed elution in Qiazol and subsequent RNA isolation. Relative fluorescence units (FU) are plotted against size (nt). The 25 nt peak represents an internal standard. The most prominent peak represents small RNA. The peaks at ∼1900 nt and ∼3900 nt represent 18S and 28S, respectively. Figure 33B depicts levels of mRNA and mature miRNA that were analyzed using quantitative RT-PCR from the same samples. The cycle threshold (Ct) value is presented as the mean ± SD.
Figure 34 is a schematic representation of the general flow chart for microvesicle isolation with beads and RNA extraction.
Figure 35 is a graph depicting the isolation of microvesicles using different types of magnetic beads.
Figure 36 is a graph depicting the recovery of microvesicles using different types of magnetic beads.
Figure 37 is a schematic representation of the flow chart for microvesicle isolation with magnetic beads and RNA extraction.
Figure 38 is a graph depicting microvesicle isolation with TEA vs. imidazole treated epoxy beads tested on selected mRNA targets with RT-qPCR (threshold = 0.1).
Figure 39 is a graph depicting recovery of selected mRNA targets from microvesicles isolated with TEA vs. imidazole treated epoxy beads.
Figure 40 is a graph depicting microvesicle isolation with TEA vs. imidazole treated epoxy beads tested on selected micro RNA targets with RT-qPCR (threshold = 0.1).
Figure 41 is a graph depicting recovery of selected micro RNA targets from microvesicles isolated with TEA vs. imidazole treated epoxy beads.
Figure 42 is a graph depicting microvesicle isolation with non-magnetic beads tested on selected mRNA with RT-qPCR, threshold = 0.1, X: cationic, R: anionic.
Figure 43 is a graph depicting recovery of selected mRNA targets from microvesicles isolated with non-magnetic cationic/anionic exchange resins.
Figure 44 is a graph depicting microvesicle isolation with non-magnetic beads tested on micro RNA with RT-qPCR, threshold = 0.1, X: cationic, R: anionic.
Figure 45 is a graph depicting recovery analysis on micro RNA cationic/anionic exchange resin.
Figure 46 is a graph depicting microvesicle isolation with control beads tested on selected mRNA with RT-qPCR, threshold = 0.1).
Figure 47 is a graph depicting recovery of selected mRNA targets from microvesicles isolated with control beads.
Figure 48 is a graph depicting microvesicle isolation with control beads tested on micro RNA with RT-qPCR, threshold = 0.1.
Figure 49 is a graph depicting recovery of micro RNA targets from microvesicles isolated with control beads.
Figure 50 is a schematic representation depicting beads titration on microvesicle isolation and RNA extraction.
Figures 51A-51G are a series of graphs depicting evaluation of microvesicle capture using RT-qPCR for RN7SL (Fig. 51A), GAPDH (Fig. 51B), RNaseP (Fig. 51C), B2M (Fig. 51D), GUSB (Fig. 51E), HPRT1 (Fig. 51F), and Let-7a (Fig. 51G).
Figure 52 is a graph depicting Ct comparisons between individual and mixed beads (bead/microvesicle (B/E) ratio at 2:1 for all targets).
Figure 53 is a graph depicting the recovery comparisons between individual vs. mixed beads at B/E ratio 2:1.
Figure 54 is a schematic representation depicting the microvesicle isolation with magnetic beads (MBs) and RNA extraction.
Figures 55A-55G are a series of graphs depicting the evaluation of microvesicle recovery at a fixed bead to microvesicle ratio (B/E) with plasma titration for RN7SL (Fig. 55A), GAPDH (Fig. 55B), RNaseP (Fig. 55C), B2M (Fig. 55D), GUSB (Fig. 55E), HPRT1 (Fig. 55F), and Let-7a (Fig. 55G).
Figure 56 is a graph depicting recovery comparisons for various targets at B/E ratio 5:1 plasma titrations from 0.4 mL, 1 mL, to 4 mL.
Figure 57 is a graph depicting time course study of MB binding on microvesicle isolation.
Figure 58 is a graph depicting a time course study on epoxy beads-microvesicle binding (1 min, 5 min, 15 min, 30 min, and 60 min).
Figure 59 is a graph depicting a time course study of beads-microvesicle binding (B/E = 5:1, 0.4 mL Plasma).
Figure 60 is a graph depicting a time course study of beads-microvesicle binding (B/E = 5:1, 0.4 mL Plasma).
Figure 61 is a graph depicting a sense check study on microvesicle-epoxy MB binding.
Figure 62 is a graph depicting an average Ct of selected RNA targets in collected fractions (epoxy beads only).
Figure 63 is a graph depicting a % recovery of selected RNA targets (epoxy beads only).
Figure 64 is a series of schematics showing the EXO50 column. The panel on the left shows the outside of the filter holder. The middle panel shows a cross section, which shows the placement of the inner tube within the filter holder. The upper right panel shows the membrane filter that is held in place at the bottom of the filter holder, between the inner tube (above the membrane) and the outer frit (bottom of the column). The lower right panel shows the configuration of the outer frit that allows liquids to flow-through.
Figure 65 is a series of photographs showing the complete assembly of the EXO50 column with collection tube. The collection tube shown in the left and middle pictures is a 50 mL conical tube. The right picture shows the top view of the EXO50 filter holder.
Figure 66 is a graph depicting different functionalized membranes while using the same buffer conditions. The y-axis shows the Ct values of select mRNA targets extracted from microvesicles isolated using the different functionalized membranes listed in the x-axis. The functionalized membranes are: Q, S, D, IDA, aldehyde, and DE81.
Figure 67 is a graph depicting the binding capacity of the EXO50 column. Increasing volumes (0.5ml, 1.0 ml, 2.0ml, 4.0ml, 8.0ml, and 16ml) of plasma were added to the EXO50 column. Extracted RNA was assessed for Ct values of select mRNA targets. The graph demonstrates that volumes 0.5ml to 4.0 ml resulted in linear increase in expression signal
Figure 68 is a graph depicting the binding capacity of the EXO50 column, as demonstrated by detection of copy number relative to increasing volume of biological sample (plasma).
Figure 69 is a graph depicting the loading capacity of a column with 3 layers of membrane. The first set of bar values for each volume represents the expression detected from the plasma sample, while the second set of bar values for each volume represents the expression detected from the flowthrough after the first microvesicle fraction capture. The percentage of the copy number from the flow-through in relation to the normal sample loading step was calculated using 2^{ΔCT}.
Figure 70 is a graph demonstrating the number of layers of membrane required to capture all of the microvesicles from 4ml of plasma, measured by RNA detection of specific mRNA targets from the microvesicles captured on the membrane.
Figure 71 is a scanning electron microscopy picture showing exosomes captured on and in the membrane of a loaded EXO50 column.
Figure 72 shows is a graph showing that a large range of types of loading buffers for the biological samples is compatible with the EXO50 procedure. The y-axis represents the Ct values for the tested mRNA targets. Replicate experiments are shown.
Figure 73 is a graph showing that the EXO50 procedure tolerates low pH buffer conditions. The y-axis represents the Ct values for the tested mRNA targets. Replicate experiments are shown.
Figure 74 is a graph showing that the EXO50 procedure also tolerates varying concentrations of detergent, such as SDS, Tween20, and TritonX-100, in the buffer system. The y-axis represents the Ct values for the tested mRNA targets.

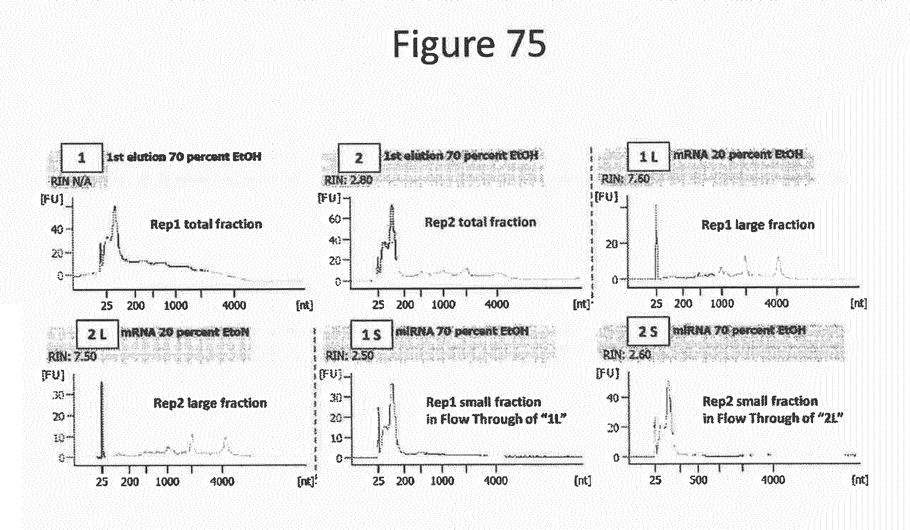

Figure 75 is a graph showing a series of bioanalyzer plots demonstrating that the total RNA isolated from EXO50 can be separated into a large and small fraction by using different ethanol concentration in the silica column binding buffer during extraction.
Figure 76 is a graph showing that the RNA purified by EXO50 is PCR-amplifiable RNA, ie., suitable for amplification and PCR processing. Expression of the tested mRNA targets was detected by amplification-based qPCR.

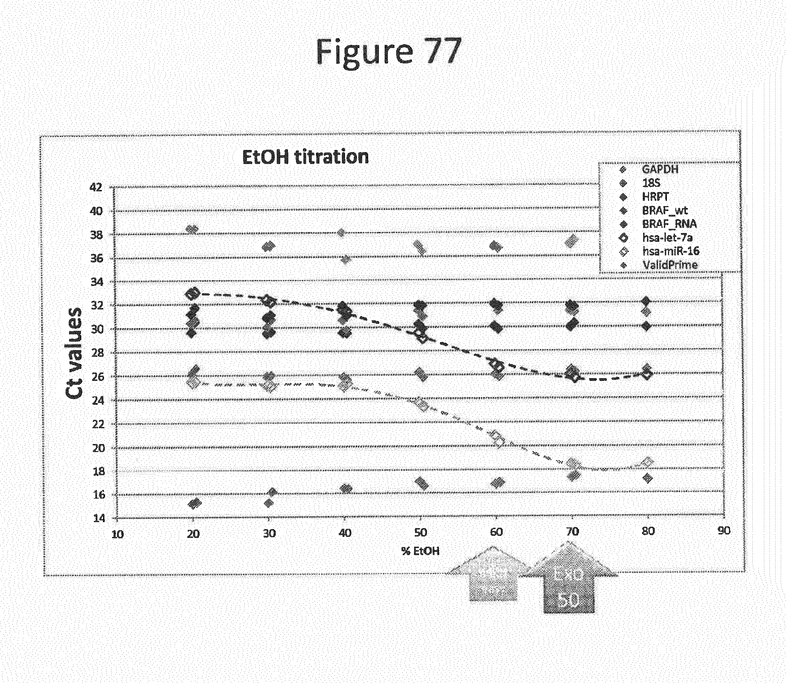

Figure 77 is a graph showing that ethanol titration can be optimized to isolate mRNA and miRNA.
Figure 78 is a graph showing that EXO50 purifies 100% of mRNA from commercially available cancer exosomes (at 3000, 1500, and 150 ng dry weight). Total RNA extracted (Direct lysis) is compared to EXO50 isolation (EXO50), and extraction from flow through (EXO Flow Through).
Figure 79 is a graph showing that the EXO50 procedure without any additional process steps isolates very little DNA. Incubation with turbo DNase or RNase A is compared to EXO50. Negative controls are represented by RT- (without reverse transcriptase). Replicate isolations are shown.
Figure 80 is a graph demonstrating that EXO50 is robust to parallel processing of many samples. 8 EXO50 replicates was performed with adding 3 minutes of delay for pipetting for each single step in the isolation. The standard deviation for individual assays between the isolation replicates is <0.5 Cts.
Figure 81 is a series of graphs that show the EXO50 analysis for two input volumes (0.2ml and 2ml) that was performed in different labs on different continents with different operators and PCR chemistry reagents. (A) MUC; (B) MSP; (C) CMH; (D) MEM; (E) IND; (F) LAX; (G) SAN; (H) AUS. Experiments were performed in triplicate.

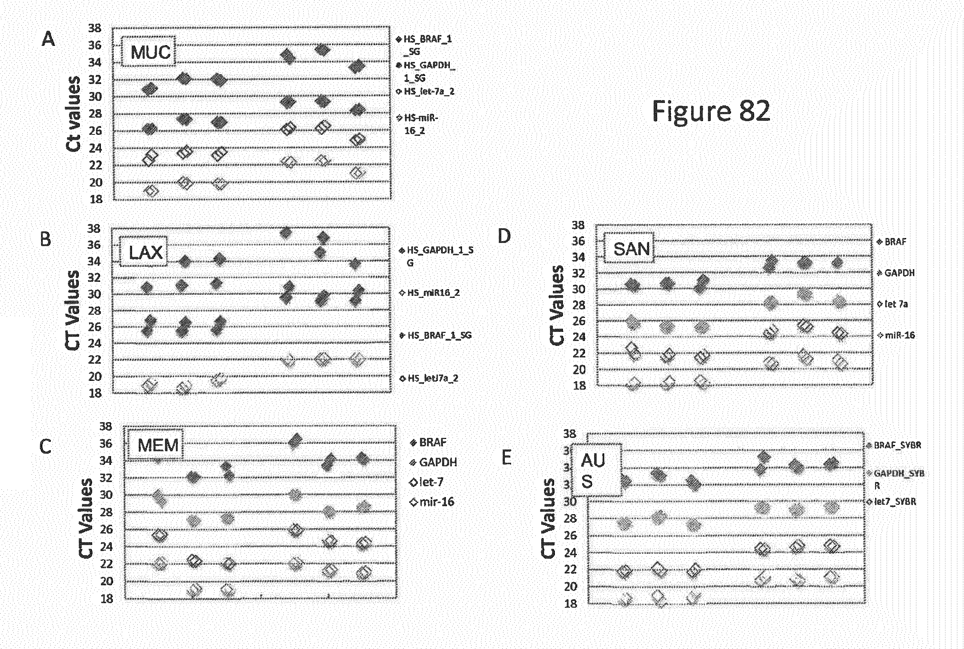

Figure 82 is a series of graphs showing that EXO50 analysis performed in different labs by first-time users with identical plasma and PCR chemistry reagents.
Figure 83 is a graph demonstrating that EXO50 can be adapted to a mini-spin column format. Ct values were compared between EXO50 mini-spin columns and ultracentrifugation for target mRNAs.
Figure 84 is a graph demonstrating different functionalized membranes in miniature regenerated cellulose columns compared to ultracentrifugation for two different input sample volumes.
Figure 85 is two scanning electron microscopy pictures showing the microvesicles ultracentrifugation (left) and EXO50 (right) isolation methods.
Figure 86 is two bioanalyzer plots showing that the profiles from RNA extracted from plasma by EXO50 and ultracentrifugation have similar RNA size-distributions. (A) Cancer patient #1; (B) Cancer patient #2.
Figure 87 is one graph showing the comparison between RNA extractions from plasma by self-assembled column, EXO50, and ultracentrifugation. Replicate experiments were performed.
Figure 88 is one graph showing the comparison between RNA extractions from 200ul of plasma by ultracentrifugation, direct lysis, and EXO50. RNA extraction from EXO50 flow-through was also analyzed.
Figure 89 is one graph showing the comparison between RNA extractions from 200ul of plasma by the EXO50 kit and miRNeasy, and 4 ml of plasma by the EXO50 kit and ultracentrifugation.
Figure 90 is one graph showing that EXO50 isolates RNA containing mutations from melanoma, BRAF V600E, compared to ultracentrifugation.
Figure 91 is one graph showing RNA isolation after elution from the EXO50 column yields as much RNA as the EXO50 process.
Figure 92 is a schematic demonstrating the EXO52 protocol for isolating a microvesicle fraction, releasing the microvesicle nucleic acids, and extracting RNA and DNA using two separate protocols.
Figure 93 is a schematic demonstrating the EXO52.2 protocol for isolating a microvesicle fraction, releasing the microvesicle nucleic acids, and extracting RNA and DNA using a single protocol.
Figure 94 is a graph showing the effect of chloroform concentration in phase separation for isolating microvesicle RNA and DNA in a single extraction, as demonstrated by detection of wild-type BRAF RNA and DNA.
Figure 95 is a graph showing the effect of chloroform concentration in phase separation for isolating microvesicle RNA and DNA in a single extraction, as demonstrated by detection of GAPDH RNA and DNA.
Figure 96 is a graph showing that the adjustment of pH in phase separation influences the DNA extraction and detection.
Figure 97 is a graph showing the effect of titration of sample volume of cerebrospinal fluid (CSF) on microvesicle RNA extraction and detection.

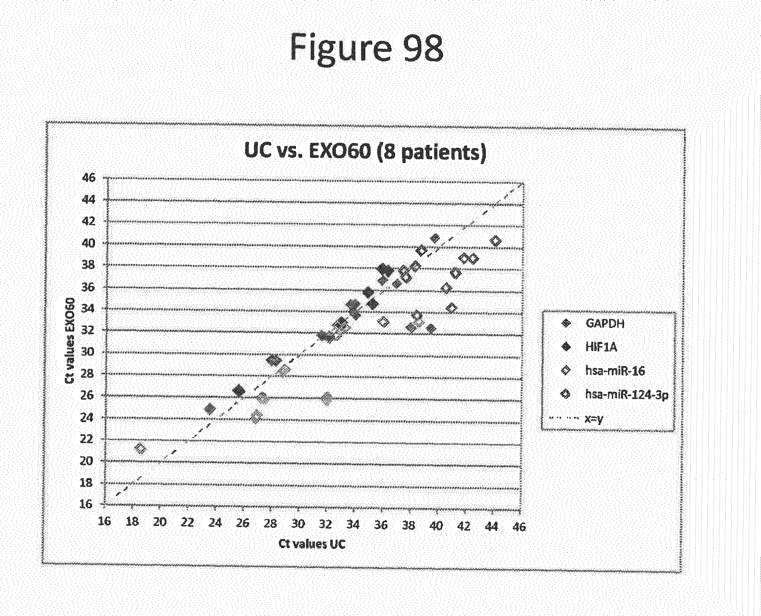

Figure 98 is a graph showing the comparison of detection of microvesicle RNA targets from ultracentrifugation and EXO60 isolation methods.
Figure 99 is a graph showing the comparison of detection of microvesicle RNA targets from ultracentrifugation and EXO60 isolation methods for different patient CSF samples. Patient samples are designated by patient ID. Varying sample volumes were utilized. (*) indicates post-mortem sample.
Figure 100 is a graph showing the effect of CSF sample volume (0.25ml, 0.5ml, 1.0ml and 2.0ml) on different microvesicle RNA isolation and extraction methods. UC (ultracentrifugation), uCSC (urine filtration method), and EXO60.
Figure 101 is a series ofbioanalyzer plots depicting the RNA profiles from extraction from 2 different urine samples using the EXO70 protocol compared to the urine circulating stem cell (uCSC) method.
Figure 102 is a graph showing the correlation between RNA detection after isolation and extraction by EXO70 compared to the urineCSC method.
Figure 103 is two graphs showing the detection of different RNA targets after isolation and extraction by EXO70 or uCSC method. RNA was extracted and analyzed from the isolated microvesicle fraction (EXO70 or uCSC) and the flow-through or supernatant fraction after isolation (EXO70 flow or uCSC flow). (A) mRNA targets; (B) miRNA targets.
Figure 104 is a graph depicting CTs (y-axis) for 4 mRNAs across 4 sample types. All points are the average of experimental duplicates on each of 2 microvesicle isolations. "SJCRH" is plasma from a pediatric patient, "DAOY" is a medulloblastoma cell line and "DAOY MED" is microvesicles from the medium of those cells. Commercially available plasma gave no results.
Figure 105 is a graph depicting the ability of EXO50 to isolate all mRNA from 100 µL cell culture supernatant.

### DETAILED DESCRIPTION OF THE INVENTION

The present invention provides methods of isolating microvesicles by capturing the microvesicles to a surface and subsequently lysing the microvesicles to release the nucleic acids, particularly RNA, contained therein. Microvesicles are shed by eukaryotic cells, or budded off of the plasma membrane, to the exterior of the cell. These membrane vesicles are heterogeneous in size with diameters ranging from about 10 nm to about 5000 nm. All membrane vesicles shed by cells < 0.8µm in diameter are referred to herein collectively as "microvesicles." These microvesicles include microvesicles, microvesicle-like particles, prostasomes, dexosomes, texosomes, ectosomes, oncosomes, apoptotic bodies, retrovirus-like particles, and human endogenous retrovirus (HERV) particles. Small microvesicles (approximately 10 to 1000nm, and more often 30 to 200 nm in diameter) that are released by exocytosis of intracellular multivesicular bodies are referred to in the art as "microvesicles."

Current methods of isolating microvesicles include ultracentrifugation, ultrafiltration, *e.g.*, using 100 kD filters, polymer precipitation techniques, and/or filtration based on size. However, there exists a need for alternative methods that are efficient and effective for isolating microvesicles and, optionally, extracting the nucleic acids contained therein, preferably microvesicle RNA, for use in a variety of applications, including diagnostic purposes.

In some embodiments, the present invention provides methods of isolating microvesicles by capturing the microvesicles to a surface and subsequently lysing the microvesicles to release the nucleic acids, particularly RNA, contained therein. In some embodiments, the present invention provides methods of isolating microvesicles by capturing the microvesicles to a surface and subsequently eluting the intact microvesicles from the capture surface.

Microvesicles are a rich source of high quality nucleic acids, excreted by all cells and present in all human biofluids. The RNA in microvesicles provides a snapshot of the transcriptome of primary tumors, metastases and the surrounding microenvironment in real-time. Thus, accurate assessment of the RNA profile of microvesicles by assays provides companion diagnostics and real-time monitoring of disease. This development has been stalled by the current standard of isolating exosomes which is slow, tedious, variable and not suited for a diagnostic environment.

The isolation and extraction methods and/or kits provided herein referred to as the EXO50 plasma exosome RNA isolation methods and/or kits use a spin-column based purification process using an affinity membrane that binds microvesicles. The methods and kits of the disclosure allow for the capability to run large numbers of clinical samples in parallel, using volumes from 0.2 up to 4 mL on a single column. The isolated RNA is highly pure, protected by a vesicle membrane until lysis, and intact vesicles can be eluted from the EXO50 membrane. The EXO50 procedure is able to deplete all mRNA from plasma input, and is equal or better in mRNA/miRNA yield when compared to ultracentrifugation or direct lysis. In contrast, the EXO50 methods and/or kits enrich for the microvesicle bound fraction of miRNAs, and they are easily scalable to large amounts of input material. This ability to scale up enables research on interesting, low abundant transcripts. In comparison with other commercially available products on the market, the methods and kits of the disclosure provide unique capabilities that are demonstrated by the examples provided herein.

The EXO50 methods and kits isolate and extract nucleic acids, e.g., RNA from a biological sample using the following the general procedure. First, the microvesicle fraction is bound to a membrane filter, and the filter is washed. Then, a reagent is used to perform on-membrane lysis and release of the nucleic acids, e.g., RNA. Chloroform extraction is then performed using PLG tubes, followed by ethanol conditioning. The nucleic acids, e.g., RNA, is then bound to a silica column, washed and then eluted.

A schematic representation of the general flow through of using a capture surface method to isolate microvesicle RNA from a biological sample is shown below:

Biological Sample
+
Loading Buffer (optional)
Capture
Surface
Wash
(optional)
Direct Lyse
on Capture Surface
Standard
RNA isolation
Pure RNA
Downstream Assay

A schematic representation of the general flow through of using membrane method to isolate microvesicle RNA from plasma sample is shown below:

Plasma
+
Loading Buffer
Membrane
Wash(optional))
Direct Lyse
on Membrane
Standard
RNA isolation
Pure RNA
Downstream Assay

A schematic representation of the general flow through of using a bead method to isolate microvesicle RNA from plasma sample is shown below:

Plasma
+
Loading Buffer
Bead
Wash
(optional)
Direct Lyse
on Bead
Standard
RNA isolation
Pure RNA
Downstream Assay

A schematic representation of the general flow through of using a capture surface method to isolate microvesicle RNA from a urine sample is shown below:

Urine Sample
Capture
Surface
Wash
(optional)
Direct Lyse
on Capture Surface
Standard
RNA isolation
Pure RNA
Downstream Assay

As demonstrated by the working examples provided herein, the format of the capturing surface, e.g., beads or a filter (also referred to herein as a membrane), does not affect the ability of the methods provided herein to efficiently capture microvesicles from a biological sample. Moreover, the surface charge of the capturing surface does not affect the ability of the methods provided herein to efficiently capture microvesicles. In addition, the working examples demonstrate that the ionic strength of the binding and wash buffer is of limited importance in the methods provided herein.

A wide range of surfaces are capable of capturing microvesicles according to the methods provided herein, but not all surfaces will capture microvesicles (some surfaces do not capture anything).

The present disclosure also describes a device for isolating and concentrating microvesicles from biological or clinical samples using disposable plastic parts and centrifuge equipment. For example, the device comprises a column comprising a capture surface (*i.e*., a membrane filter), a holder that secures the capture surface between the outer frit and an inner tube, and a collection tube. The outer frit comprises a large net structure to allow passing of liquid, and is preferably at one end of the column. The inner tube holds the capture surface in place, and preferably is slightly conus-shaped. The collection tube may be commercially available, *i.e*., 50ml Falcon tube. The column is preferably suitable for spinning, *i.e.*, the size is compatible with standard centrifuge and micro-centrifuge machines.

In embodiments where the capture surface is a membrane, the device for isolating the microvesicle fraction from a biological sample contains at least one membrane. In some embodiments, the device comprises one, two, three, four, five or six membranes. Preferably, the device comprises three membranes. In embodiments where the device comprises more than one membrane, the membranes are all directly adjacent to one another at one end of the column. In embodiments where the device comprises more than one membrane, the membranes are all identical to each other, *i.e.*, are of the same charge and/or have the same functional group.

As demonstrated by working Examples 1-32 provided herein, the choice of buffer component(s), the pH of the buffer and the choice of lysis buffer affect the ability of the methods provided herein to efficiently capture microvesicles and release the nucleic acids, particularly RNA, contained therein. In some embodiments, the lysis buffer is a phenol-based lysis buffer. For example, the lysis buffer is QIAzol® lysis reagent (Qiagen).

Working Examples 1-32 provided herein also demonstrate that capture by filtering through a pore size smaller than the microvesicles is not the primary mechanism of capture by the methods provided herein. However, filter pore size is nevertheless very important, e.g. because mRNA gets stuck on a 20nm filter and cannot be recovered, whereas microRNAs can easily be eluted off, and e.g. because the filter pore size is an important parameter in available surface capture area.

Working Examples 1-32 provided herein have also demonstrated the feasibility of microvesicle isolation with beads (magnetic or non-magnetic) that would be a more suitable format in clinical utility. Results showed that positively charged, negatively charged, and neutral beads all showed good microvesicle capturing efficiencies. This observation suggests that they may act on different ligands on the surface of microvesicle, thus one microvesicle can be captured with different (functionalized) particles via different (multiple) forces.

The methods provided herein use any of a variety of capture surfaces. In some embodiments, the capture surface is a membrane, also referred to herein as a filter or a membrane filter. In some embodiments, the capture surface is a commercially available membrane. In some embodiments, the capture surface is a charged commercially available membrane. In some embodiments, the capture surface is neutral. In some embodiments, the capture surface is selected from Mustang® Ion Exchange Membrane from PALL Corporation; Vivapure ® Q membrane from Sartorius AG; Sartobind Q, or Vivapure® Q Maxi H; Sartobind ® D from Sartorius AG, Sartobind (S) from Sartorius AG, Sartobind ® Q from Sartorius AG, Sartobind ® IDA from Sartorius AG, Sartobind® Aldehyde from Sartorius AG, Whatman® DE81 from Sigma, Fast Trap Virus Purification column from EMD Millipore; Thermo Scientific* Pierce Strong Cation and Anion Exchange Spin Columns.

In embodiments where the capture surface is charged, the capture surface can be a charged filter selected from the group consisting of 0.65um positively charged Q PES vacuum filtration, 3-5um positively charged Q RC spin column filtration, 0.8um positively charged Q PES homemade spin column filtration, 0.8um positively charged Q PES syringe filtration, 0.8um negatively charged S PES homemade spin column filtration, 0.8um negatively charged S PES syringe filtration, and 50nm negatively charged nylon syringe filtration. Preferably, the charged filter is not housed in a syringe filtration apparatus, as Qiazol/RNA is harder to get out of the filter in these embodiments. Preferably, the charged filter is housed at one end of a column.

In embodiments where the capture surface is a membrane, the membrane can be made from a variety of suitable materials. In some embodiments, the membrane is polyethersulfone (PES) (e.g., from Millipore or PALL Corp.). In some embodiments, the membrane is regenerated cellulose (RC) (e.g., from Sartorius or Pierce).

In some embodiments, the capture surface is a positively charged membrane. In some embodiments, the capture surface is a Q membrane, which is a positively charged membrane and is an anion exchanger with quaternary amines. For example, the Q membrane is functionalized with quaternary ammonium, R-CH₂-N⁺(CH₃)₃. In some embodiments, the capture surface is a negatively charged membrane. In some embodiments, the capture surface is an S membrane, which is a negatively charged membrane and is a cation exchanger with sulfonic acid groups. For example, the S membrane is functionalized with sulfonic acid, R-CH₂-SO₃⁻, In some embodiments, the capture surface is a D membrane, which is a weak basic anion exchanger with diethylamine groups, R-CH₂-NH⁺(C₂H₅)₂. In some embodiments, the capture surface is a metal chelate membrane. For example, the membrane is an IDA membrane, functionalized with minodiacetic acid-N(CH₂COOH⁻)₂. In some embodiments, the capture surface is a microporous membrane, functionalized with aldehyde groups, -CHO. In other embodiments, the membrane is a weak basic anion exchanger, with diethylaminoethyl (DEAE) cellulose. Not all charged membranes are suitable for use in the methods provided herein, e.g., RNA isolated using a regenerated cellulose, strong acidic cation exchanger ("RC/SACE") membrane spin column showed RT-qPCR inhibition and, thus, unsuitable for PCR related downstream assay.

In embodiments where the capture surface is charged, microvesicles can be isolated with a positively charged filter. In embodiments where the capture surface is charged, the capture surface is preferably not a negatively charged regenerated cellulose S filter, as this embodiment causes PCR inhibition. In contrast, negatively charged PES filters do not exhibit this phenomenon.

In embodiments where the capture surface is charged, the pH during microvesicle capture is a pH ≤7. In some embodiments, the pH is greater than 4 and less than or equal to 8.

In embodiments where the capture surface is a positively charged Q filter, the buffer system includes a wash buffer comprising 250mM Bis Tris Propane, pH6.5-7.0. In embodiments where the capture surface is a positively charged Q filter, the lysis buffer is Qiazol. In embodiments where the capture surface is a positively charged Q filter, the lysis buffer is present at one volume. In embodiments where the capture surface is a positively charged Q filter, the lysis buffer is present at more than one volume.

In embodiments where the capture surface is neutral, the capture surface is a filter selected from the group consisting of 20nm neutral PES syringe filtration (Tisch and Exomir), 30nm neutral PES syringe filtration (Sterlitech), 50nm neutral PES syringe filtration (Sterlitech), 0.2um neutral PES homemade spin column filtration (Pall), 0.8um neutral PES homemade spin column filtration (Pall) and 0.8um neutral PES syringe filtration (Pall). In embodiments where the capture surface is neutral, preferably the neutral capture surface is not housed in a syringe filter.

In embodiments where the capture surface is neutral, the lysis buffer is Qiazol. In embodiments where the capture surface is neutral, the lysis buffer is present at one volume. In embodiments where the capture surface is neutral, the lysis buffer is present at more than one volume. In embodiments where the capture surface is neutral, the methods include a wash step.

Depending on the membrane material, the pore sizes of the membrane range from 3 µm to 20 nm. For example, in embodiments where the capture surface is a commercially available PES membrane, the membrane has a pore size of 20nm (Exomir), 0.65µm (Millipore) or 0.8µm (Pall). In embodiments where the capture surface is a commercially available RC membrane, the membrane has a pore size in the range of 3-5µm (Sartorius, Pierce).

The surface charge of the capture surface can be positive, negative or neutral. In some embodiments, the capture surface is a positively charged bead or beads. In some embodiments, the capture surface is a negatively charged bead or beads. In some embodiments, the capture surface is a neutral bead or beads. In some embodiments, the capture surface is a neutral filter, e.g., a PES membrane that is not surface modified with a charged group.

In some embodiments, the methods provided herein include a variety of buffers including loading and wash buffers. In some embodiments, loading and wash buffers can be of high or low ionic strength. In some embodiments, the salt concentration, e.g., NaCl concentration, can be from 0 to 2.4M. The buffers can include a variety of components. In some embodiments, the buffers include one or more of the following components: Tris, Bis-Tris, Bis-Tris-Propane, Imidazole, Citrate, Methyl Malonic Acid, Acetic Acid, Ethanolamine, Diethanolamine, Triethanolamine (TEA) and Sodium phosphate. In the methods provided herein, the pH of loading and wash buffers is important. Filters tend to clog when plasma samples at set to pH ≤ 5.5 before loading (the plasma will not spin through the column at all), and at higher pH microvesicle RNA recovery is lower due to instability of the microvesicles. At neutral pH, the RNA recovery from microvesicles is optimal. In some embodiments, the buffer used is at 1X concentration, 2X concentration, 3X concentration, or 4X concentration. For example, the loading or binding buffer is at 2X concentration while the wash buffer is at 1X concentration.

In some embodiments, the methods include one or more wash steps, for example, after contacting the biological sample with the capture surface. In some embodiments, detergents are added to the wash buffer to facilitate removing the nonspecific binding (*i.e*., contaminants, cell debris, and circulating protein complexes or nucleic acids), to obtain a more pure microvesicle fraction. Detergents suitable for use include, but are not limited to, sodium dodecyl sulfate (SDS), Tween-20, Tween-80, Triton X-100, Nonidet P-40 (NP-40),, Brij-35, Brij-58, octyl glucoside, octyl thioglucoside, CHAPS or CHAPSO.

In some embodiments, the capture surface, e.g., membrane, is housed within a device used for centrifugation; e.g. spin columns, or for vacuum system e.g. vacuum filter holders, or for filtration with pressure e.g. syringe filters. In a preferred embodiment, the capture surface is housed in a spin column or vacuum system.

The isolation of microvesicles from a biological sample prior to extraction of nucleic acids is advantageous for the following reasons: 1) extracting nucleic acids from microvesicles provides the opportunity to selectively analyze disease or tumor-specific nucleic acids obtained by isolating disease or tumor-specific microvesicles apart from other microvesicles within the fluid sample; 2) nucleic acid-containing microvesicles produce significantly higher yields of nucleic acid species with higher integrity as compared to the yield/integrity obtained by extracting nucleic acids directly from the fluid sample without first isolating microvesicles; 3) scalability, e.g., to detect nucleic acids expressed at low levels, the sensitivity can be increased by concentrating microvesicles from a larger volume of sample using the methods and/or kits described herein; 4) more pure or higher quality/integrity of extracted nucleic acids in that proteins, lipids, cell debris, cells and other potential contaminants and PCR inhibitors that are naturally found within biological samples are excluded before the nucleic acid extraction step; and 5) more choices in nucleic acid extraction methods can be utilized as isolated microvesicle fractions can be of a smaller volume than that of the starting sample volume, making it possible to extract nucleic acids from these fractions or pellets using small volume column filters.

Several methods of isolating microvesicles from a biological sample have been described in the art. For example, a method of differential centrifugation is described in a paper by Raposo et al. (Raposo et al., 1996), a paper by Skog et. al.(Skog et al., 2008) and a paper by Nilsson et. al.(Nilsson et al., 2009). Methods of ion exchange and/or gel permeation chromatography are described in US Patent Nos. 6,899,863 and 6,812,023. Methods of sucrose density gradients or organelle electrophoresis are described in U.S. Patent No. 7,198,923. A method of magnetic activated cell sorting (MACS) is described in a paper by Taylor and Gercel Taylor (Taylor and Gercel-Taylor, 2008). A method of nanomembrane ultrafiltration concentration is described in a paper by Cheruvanky et al. (Cheruvanky et al., 2007). A method of Percoll gradient isolation is described in a publication by Miranda et al. (Miranda et al., 2010). Further, microvesicles may be identified and isolated from bodily fluid of a subject by a microfluidic device (Chen et al., 2010). In research and development, as well as commercial applications of nucleic acid biomarkers, it is desirable to extract high quality nucleic acids from biological samples in a consistent, reliable, and practical manner.

An object of the present invention is therefore to provide a method for quick and easy isolation of nucleic acid-containing particles from biological samples such as body fluids and extraction of high quality nucleic acids from the isolated particles. The method of the invention may be suitable for adaptation and incorporation into a compact device or instrument for use in a laboratory or clinical setting, or in the field.

In the working examples provided herein, human plasma from normal healthy controls was obtained from Bioreclamation LLC with a blood collection SOP developed by Exosome Diagnostic Inc. Briefly, blood was collected into K2 EDTA tubes and mixed well with complete inversions. Then the tubes were centrifuged at 1300xg for 10min to separate the blood cells and plasma. Then plasma was removed and filtered through a 0.8µm filter to remove cell debris and platelets. All plasma samples were then aliquoted into 1ml cryovials and store at -80°C until use.

Before RNA isolation, the membrane was conditioned by passing through equilibrium buffer. The thawed plasma sample was diluted with loading buffer. The diluted plasma sample was slowly passed through the membrane that adsorbs the microvesicles. The membrane was then washed with a wash buffer to remove any weakly bound plasma components. Then a lysis reagent was passed through the membrane to lyse the microvesicles. RNA was isolated using the miRNeasy kit (Qiagen).

RNA was assessed for quality and concentration with the 2100 Bioanalyzer (Agilent) using a RNA 6000 Pico Chip. The relative quantity of the extracted RNA was measured by RT-qPCR using selected human gene expression assays from Applied Biosystems (Taqman Assay).

While the examples provided herein used plasma samples, the skilled artisan will appreciate that these methods are applicable to a variety of biological samples. Other suitable biological samples include urine, cerebrospinal fluid, blood including blood components, e.g., plasma and serum, sputum, pleural fluid, nipple aspirates, lymph fluid, fluid of the respiratory, intestinal, and genitourinary tracts, tear fluid, saliva, breast milk, fluid from the lymphatic system, semen, intraorgan system fluid, ascitic fluid, tumor cyst fluid, amniotic fluid, cell culture supernatant and combinations thereof.

The methods and kits of the disclosure are suitable for use with samples derived from a human subject. The methods and kits of the disclosure are suitable for use with samples derived from a non-human subject such as, for example, a rodent, a non-human primate, a companion animal (e.g., cat, dog, horse), and/or a farm animal (e.g., chicken).

In some embodiments, a pre-processing step prior to isolation, purification or enrichment of the microvesicles is performed to remove large unwanted particles, cells and/or cell debris and other contaminants present in the biological sample. The pre-processing steps may be achieved through one or more centrifugation steps (e.g., differential centrifugation) or one or more filtration steps (e.g., ultrafiltration), or a combination thereof. Where more than one centrifugation pre-processing steps are performed, the biological sample may be centrifuged first at the lower speed and then at the higher speed. If desired, further suitable centrifugation pre-processing steps may be carried out. Alternatively or in addition to the one or more centrifugation pre-processing steps, the biological sample may be filtered. For example, a biological sample may be first centrifuged at 20,000g for 1 hour to remove large unwanted particles; the sample can then be filtered, for example, through a 0.8 µm filter.

In some embodiments, one or more centrifugation steps are performed before or after contacting the biological sample with the capture surface to separate microvesicles and concentrate the microvesicles isolated from the biological fraction. For example, the sample is centrifuged at 20,000 g for 1 hour at 4°C. To remove large unwanted particles, cells, and/or cell debris, the samples may be centrifuged at a low speed of about 100-500g, preferably about 250-300g. Alternatively or in addition, the samples may be centrifuged at a higher speed. Suitable centrifugation speeds are up to about 200,000g; for example from about 2,000g to less than about 200,000g. Speeds of above about 15,000g and less than about 200,000g or above about 15,000g and less than about 100,000g or above about 15,000g and less than about 50,000g are preferred. Speeds of from about 18,000g to about 40,000g or about 30,000g; and from about 18,000g to about 25,000g are more preferred. Particularly preferred is a centrifugation speed of about 20,000g. Generally, suitable times for centrifugation are from about 5 minutes to about 2 hours, for example, from about 10 minutes to about 1.5 hours, or more preferably from about 15 minutes to about 1 hour. A time of about 0.5 hours may be preferred. It is sometimes preferred to subject the biological sample to centrifugation at about 20,000g for about 0.5 hours. However the above speeds and times can suitably be used in any combination (e.g., from about 18,000g to about 25,000g, or from about 30,000g to about 40,000g for about 10 minutes to about 1.5 hours, or for about 15 minutes to about 1 hour, or for about 0.5 hours, and so on). The centrifugation step or steps may be carried out at below-ambient temperatures, for example at about 0-10°C, preferably about 1-5 °C, e.g., about 3 °C or about 4°C.

In some embodiments, one or more filtration steps are performed before or after contacting the biological sample with the capture surface. A filter having a size in the range about 0.1 to about 1.0 µm may be employed, preferably about 0.8 µm or 0.22 µm. The filtration may also be performed with successive filtrations using filters with decreasing porosity.

In some embodiments, one or more concentration steps are performed, in order to reduce the volumes of sample to be treated during the chromatography stages, before or after contacting the biological sample with the capture surface. Concentration may be through centrifugation of the sample at high speeds, e.g. between 10,000 and 100,000 g, to cause the sedimentation of the microvesicles. This may consist of a series of differential centrifugations. The microvesicles in the pellet obtained may be reconstituted with a smaller volume and in a suitable buffer for the subsequent steps of the process. The concentration step may also be performed by ultrafiltration. In fact, this ultrafiltration both concentrates the biological sample and performs an additional purification of the microvesicle fraction. In another embodiment, the filtration is an ultrafiltration, preferably a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibers (Amicon, Millipore, Sartorius, Pall, GF, Sepracor). Within the scope of the invention, the use of membranes with a cut-off threshold below 1000 kDa, preferably between 100 kDa and 1000 kDa, or even more preferably between 100 kDa and 600 kDa, is advantageous.

In some embodiments, one or more size-exclusion chromatography step or gel permeation chromatography steps are performed before or after contacting the biological sample with the capture surface. To perform the gel permeation chromatography step, a support selected from silica, acrylamide, agarose, dextran, ethylene glycol-methacrylate copolymer or mixtures thereof, e.g., agarose-dextran mixtures, are preferably used. For example, such supports include, but are not limited to: SUPERDEX® 200HR (Pharmacia), TSK G6000 (TosoHaas) or SEPHACRYL® S (Pharmacia).

In some embodiments, one or more affinity chromatography steps are performed before or after contacting the biological sample with the capture surface. Some microvesicles can also be characterized by certain surface molecules. Because microvesicles form from budding of the cell plasma membrane, these microvesicles often share many of the same surface molecules found on the cells they originated from. As used herein, "surface molecules" refers collectively to antigens, proteins, lipids, carbohydrates, and markers found on the surface or in or on the membrane of the microvesicle. These surface molecules can include, for example, receptors, tumor-associated antigens, membrane protein modifications (e.g., glycosylated structures). For example, microvesicles that bud from tumor cells often display tumor-associated antigens on their cell surface. As such, affinity chromatography or affinity exclusion chromatography can also be utilized in combination with the methods provided herein to isolate, identify, and or enrich for specific populations of microvesicles from a specific donor cell type (Al-Nedawi et al., 2008; Taylor and Gercel-Taylor, 2008). For example, tumor (malignant or non-malignant) microvesicles carry tumor-associated surface antigens and may be detected, isolated and/or enriched via these specific tumor-associated surface antigens. In one example, the surface antigen is epithelial cell adhesion molecule (EpCAM), which is specific to microvesicles from carcinomas of long, colorectal, breast, prostate, head and neck, and hepatic origin, but not of hematological cell origin (Balzar et al., 1999; Went et aL, 2004). Additionally, tumor-specific microvesicles can also be characterized by the lack of certain surface markers, such as CD80 and CD86. In these cases, microvesicles with these markers may be excluded for further analysis of tumor specific markers, e.g., by affinity exclusion chromatography. Affinity chromatography can be accomplished, for example, by using different supports, resins, beads, antibodies, aptamers, aptamer analogs, molecularly imprinted polymers, or other molecules known in the art that specifically target desired surface molecules on microvesicles.

Optionally, control particles may be added to the sample prior to microvesicle isolation or nucleic acid extraction to serve as an internal control to evaluate the efficiency or quality of microvesicle purification and/or nucleic acid extraction. The methods and/or kits described herein provide for the efficient isolation and the control particles along with the microvesicle fraction. These control particles include Q-beta bacteriophage, virus particles, or any other particle that contains control nucleic acids (e.g., at least one control target gene) that may be naturally occurring or engineered by recombinant DNA techniques. In some embodiments, the quantity of control particles is known before the addition to the sample. The control target gene can be quantified using real-time PCR analysis. Quantification of a control target gene can be used to determine the efficiency or quality of the microvesicle purification or nucleic acid extraction processes.

Preferably, the control particle is a Q-beta bacteriophage, referred to herein as "Q-beta particle." The Q-beta particle used in the methods and/or kits described herein may be a naturally-occurring virus particle or may be a recombinant or engineered virus, in which at least one component of the virus particle (e.g., a portion of the genome or coat protein) is synthesized by recombinant DNA or molecular biology techniques known in the art. Q-beta is a member of the leviviridae family, characterized by a linear, single-stranded RNA genome that consists of 3 genes encoding four viral proteins: a coat protein, a maturation protein, a lysis protein, and RNA replicase. Due to its similar size to average microvesicles, Q-beta can be easily purified from a biological sample using the same purification methods used to isolate microvesicles, as described herein. In addition, the low complexity of the Q-beta viral single-stranded gene structure is advantageous for its use as a control in amplification-based nucleic acid assays. The Q-beta particle contains a control target gene or control target sequence to be detected or measured for the quantification of the amount of Q-beta particle in a sample. For example, the control target gene is the Q-beta coat protein gene. After addition of the Q-beta particles to the biological sample, the nucleic acids from the Q-beta particle are extracted along with the nucleic acids from the biological sample using the extraction methods and/or kits described herein. Detection of the Q-beta control target gene can be determined by RT-PCR analysis, for example, simultaneously with the biomarkers of interest (*i.e.*, BRAF). A standard curve of at least 2, 3, or 4 known concentrations in 10-fold dilution of a control target gene can be used to determine copy number. The copy number detected and the quantity of Q-beta particle added can be compared to determine the quality of the isolation and/or extraction process.

In a preferred embodiment, the Q-beta particles are added to the urine sample prior to nucleic extraction. For example, the Q-beta particles are added to the urine sample prior to ultrafiltration and/or after the pre-filtration step.

In some embodiments, 50, 100, 150, 200, 250, 300, 350, 400, 450, 500, 1,000 or 5,000 copies of Q-beta particles added to a bodily fluid sample. In a preferred embodiment, 100 copies of Q-beta particles are added to a bodily fluid sample. The copy number of Q-beta particles can be calculated based on the ability of the Q-beta bacteriophage to infect target cells. Thus, the copy number of Q-beta particles is correlated to the colony forming units of the Q-beta bacteriophage.

### Nucleic Acid Extraction

The present invention is directed towards the use of a capture surface for the improved isolation, purification, or enrichment of microvesicles. The methods disclosed herein provide a highly enriched microvesicle fraction for extraction of high quality nucleic acids from said microvesicles. The nucleic acid extractions obtained by the methods and/or kits described herein may be useful for various applications in which high quality nucleic acid extractions are required or preferred, such as for use in the diagnosis, prognosis, or monitoring of diseases or medical conditions.

Recent studies reveal that nucleic acids within microvesicles have a role as biomarkers. For example, WO 2009/100029 describes, among other things, the use of nucleic acids extracted from microvesicles in GBM patient serum for medical diagnosis, prognosis and therapy evaluation. WO 2009/100029 also describes the use of nucleic acids extracted from microvesicles in human urine for the same purposes. The use of nucleic acids extracted from microvesicles is considered to potentially circumvent the need for biopsies, highlighting the enormous diagnostic potential of microvesicle biology (Skog et al., 2008).

The quality or purity of the isolated microvesicles can directly affect the quality of the extracted microvesicle nucleic acids, which then directly affects the efficiency and sensitivity of biomarker assays for disease diagnosis, prognosis, and/or monitoring. Given the importance of accurate and sensitive diagnostic tests in the clinical field, methods for isolating highly enriched microvesicle fractions from biological samples are needed. To address this need, the present invention provides methods for isolating microvesicles from biological sample for the extraction of high quality nucleic acids from a biological sample. As shown herein, highly enriched microvesicle fractions are isolated from biological samples by methods and/or kits described herein, and wherein high quality nucleic acids subsequently extracted from the highly enriched microvesicle fractions. These high quality extracted nucleic acids are useful for measuring or assessing the presence or absence of biomarkers for aiding in the diagnosis, prognosis, and/or monitoring of diseases or other medical conditions.

As used herein, the term "biological sample" refers to a sample that contains biological materials such as DNA, RNA and protein. In some embodiments, the biological sample may suitably comprise a bodily fluid from a subject. The bodily fluids can be fluids isolated from anywhere in the body of the subject, preferably a peripheral location, including but not limited to, for example, blood, plasma, serum, urine, sputum, spinal fluid, cerebrospinal fluid, pleural fluid, nipple aspirates, lymph fluid, fluid of the respiratory, intestinal, and genitourinary tracts, tear fluid, saliva, breast milk, fluid from the lymphatic system, semen, intra-organ system fluid, ascitic fluid, tumor cyst fluid, amniotic fluid and cell culture supernatant, and combinations thereof. In some embodiments, the preferred body fluid for use as the biological sample is urine. In other embodiments, the preferred body fluid is serum. In yet other embodiments, the preferred body fluid is plasma. In other embodiments, the preferred body fluid is cerebrospinal fluid. Suitably a sample volume of about 0.1ml to about 30ml fluid may be used. The volume of fluid may depend on a few factors, e.g., the type of fluid used. For example, the volume of serum samples may be about 0.1ml to about 4ml, preferably about 0.2ml to 4ml. The volume of plasma samples may be about 0.1ml to about 4ml, preferably 0.5ml to 4ml. The volume of urine samples may be about 10 ml to about 30ml, preferably about 20 ml. Biological samples can also include fecal or cecal samples, or supernatants isolated therefrom.

The term "subject" is intended to include all animals shown to or expected to have nucleic acid-containing particles. In particular embodiments, the subject is a mammal, a human or nonhuman primate, a dog, a cat, a horse, a cow, other farm animals, or a rodent (e.g. mice, rats, guinea pig. etc.). A human subject may be a normal human being without observable abnormalities, e.g., a disease. A human subject may be a human being with observable abnormalities, e.g., a disease. The observable abnormalities may be observed by the human being himself, or by a medical professional. The term "subject," "patient," and "individual" are used interchangeably herein.

As used herein, the term "nucleic acids" refer to DNA and RNA. The nucleic acids can be single stranded or double stranded. In some instances, the nucleic acid is DNA. In some instances, the nucleic acid is RNA. RNA includes, but is not limited to, messenger RNA, transfer RNA, ribosomal RNA, non-coding RNAs, microRNAs, and HERV elements.

As used herein, the term "high quality" in reference to nucleic acid extraction means an extraction in which one is able to detect 18S and 28S rRNA, preferably in a ratio of approximately 1:1 to approximately 1:2; and more preferably, approximately 1:2. Ideally, high quality nucleic acid extractions obtained by the methods and/or kits described herein will also have an RNA integrity number of greater than or equal to 5 for a low protein biological sample (e.g., urine), or greater than or equal to 3 for a high protein biological sample (e.g., serum), and a nucleic acid yield of greater than or equal to 50 pg/ml from a 20 ml low protein biological sample or a 1 ml high protein biological sample.

High quality RNA extractions are desirable because RNA degradation can adversely affect downstream assessment of the extracted RNA, such as in gene expression and mRNA analysis, as well as in analysis of non-coding RNA such as small RNA and microRNA. The new methods and/or kits described herein enable one to extract high quality nucleic acids from microvesicles isolated from a biological sample so that an accurate analysis of nucleic acids within the microvesicles can be performed.

Following the isolation of microvesicles from a biological sample, nucleic acid may be extracted from the isolated or enriched microvesicle fraction. To achieve this, in some embodiments, the microvesicles may first be lysed. The lysis of microvesicles and extraction of nucleic acids may be achieved with various methods known in the art. In one embodiment, the lysis and extraction steps may be achieved using a commercially available Qiagen RNeasy Plus kit. In another embodiment, the lysis and extraction steps may be achieved using a commercially available Qiagen miRNeasy kit. In yet another embodiment, the nucleic acid extraction may be achieved using phenol:chloroform according to standard procedures and techniques known in the art. Such methods may also utilize a nucleic acid-binding column to capture the nucleic acids contained within the microvesicles. Once bound, the nucleic acids can then be eluted using a buffer or solution suitable to disrupt the interaction between the nucleic acids and the binding column, thereby successfully eluting the nucleic acids.

In some embodiments, the nucleic acid extraction methods also include the step of removing or mitigating adverse factors that prevent high quality nucleic acid extraction from a biological sample. Such adverse factors are heterogeneous in that different biological samples may contain various species of adverse factors. In some biological samples, factors such as excessive DNA may affect the quality of nucleic acid extractions from such samples. In other samples, factors such as excessive endogenous RNase may affect the quality of nucleic acid extractions from such samples. Many agents and methods may be used to remove these adverse factors. These methods and agents are referred to collectively herein as an "extraction enhancement operations." In some instances, the extraction enhancement operation may involve the addition of nucleic acid extraction enhancement agents to the biological sample. To remove adverse factors such as endogenous RNases, such extraction enhancement agents as defined herein may include, but are not limited to, an RNase inhibitor such as Superase-In (commercially available from Ambion Inc.) or RNaseINplus (commercially available from Promega Corp.), or other agents that function in a similar fashion; a protease (which may function as an RNase inhibitor); DNase; a reducing agent; a decoy substrate such as a synthetic RNA and/or carrier RNA; a soluble receptor that can bind RNase; a small interfering RNA (siRNA); an RNA binding molecule, such as an anti-RNA antibody, a basic protein or a chaperone protein; an RNase denaturing substance, such as a high osmolarity solution, a detergent, or a combination thereof.

For example, the extraction enhancement operation may include the addition of an RNase inhibitor to the biological sample, and/or to the isolated microvesicle fraction, prior to extracting nucleic acid; preferably the RNase inhibitor has a concentration of greater than 0.027 AU (I X) for a sample equal to or more than 1 µl in volume; alternatively, greater than or equal to 0. 1 35 AU (5X) for a sample equal to or more than 1 µl; alternatively, greater than or equal to 0.27 AU (10X) for a sample equal to or more than I µl; alternatively, greater than or equal to 0.675 AU (25X) for a sample equal to or more than 1 µl; and alternatively, greater than or equal to 1 .35 AU (50X) for a sample equal to or more than 1 µl; wherein the I X concentration refers to an enzymatic condition wherein 0.027 AU or more RNase inhibitor is used to treat microvesicles isolated from 1 µl or more bodily fluid, the 5X concentration refers to an enzymatic condition wherein 0.135 AU or more RNase inhibitor is used to treat microvesicles isolated from 1 µl or more bodily fluid, the 10X protease concentration refers lo an enzymatic condition wherein 0.27 AU or more RNase inhibitor is used to treat particles isolated from 1 µl or more bodily fluid, the 25X concentration refers to an enzymatic condition wherein 0.675 AU or more RNase inhibitor is used to treat microvesicles isolated from 1 µl or more bodily fluid, and the 50X protease concentration refers to an enzymatic condition wherein 1 .35 AU or more RNase inhibitor is used to treat particles isolated from 1 µl or more bodily fluid. Preferably, the RNase inhibitor is a protease, in which case, 1 AU is the protease activity that releases folin-positive amino acids and peptides corresponding to 1 µmol tyrosine per minute.

These enhancement agents may exert their functions in various ways, e.g., through inhibiting RNase activity (e.g., RNase inhibitors), through a ubiquitous degradation of proteins (e.g., proteases), or through a chaperone protein (e.g., a RNA-binding protein) that binds and protects RNAs. In all instances, such extraction enhancement agents remove or at least mitigate some or all of the adverse factors in the biological sample or associated with the isolated particles that would otherwise prevent or interfere with the high quality extraction of nucleic acids from the isolated particles.

In some embodiments, the quantification of 18S and 28S rRNAs extracted can be used determine the quality of the nucleic acid extraction.

### Detection of nucleic acid biomarkers

In some embodiments, the extracted nucleic acid comprises RNA. In this instance, the RNA is preferably reverse-transcribed into complementary DNA (cDNA) before further amplification. Such reverse transcription may be performed alone or in combination with an amplification step. One example of a method combining reverse transcription and amplification steps is reverse transcription polymerase chain reaction (RT-PCR), which may be further modified to be quantitative, e.g., quantitative RT-PCR as described in US Patent No. 5,639,606, which is incorporated herein by reference for this teaching. Another example of the method comprises two separate steps: a first of reverse transcription to convert RNA into cDNA and a second step of quantifying the amount of cDNA using quantitative PCR. As demonstrated in the examples that follow, the RNAs extracted from nucleic acid-containing particles using the methods disclosed herein include many species of transcripts including, but not limited to, ribosomal 18S and 28S rRNA, microRNAs, transfer RNAs, transcripts that are associated with diseases or medical conditions, and biomarkers that are important for diagnosis, prognosis and monitoring of medical conditions.

For example, RT-PCR analysis determines a Ct (cycle threshold) value for each reaction. In RT-PCR, a positive reaction is detected by accumulation of a fluorescence signal. The Ct value is defined as the number of cycles required for the fluorescent signal to cross the threshold (i.e., exceeds background level). Ct levels are inversely proportional to the amount of target nucleic acid, or control nucleic acid, in the sample (i.e., the lower the Ct level, the greater the amount of control nucleic acid in the sample).

In another embodiment, the copy number of the control nucleic acid can be measured using any of a variety of art-recognized techniques, including, but not limited to, RT-PCR. Copy number of the control nucleic acid can be determined using methods known in the art, such as by generating and utilizing a calibration, or standard curve.

In some embodiments, one or more biomarkers can be one or a collection of genetic aberrations, which is used herein to refer to the nucleic acid amounts as well as nucleic acid variants within the nucleic acid-containing particles. Specifically, genetic aberrations include, without limitation, over-expression of a gene (e.g., an oncogene) or a panel of genes, under-expression of a gene (e.g., a tumor suppressor gene such as p53 or RB) or a panel of genes, alternative production of splice variants of a gene or a panel of genes, gene copy number variants (CNV) (e.g., DNA double minutes) (Hahn, 1993), nucleic acid modifications (e.g., methylation, acetylation and phosphorylations), single nucleotide polymorphisms (SNPs), chromosomal rearrangements (e.g., inversions, deletions and duplications), and mutations (insertions, deletions, duplications, missense, nonsense, synonymous or any other nucleotide changes) of a gene or a panel of genes, which mutations, in many cases, ultimately affect the activity and function of the gene products, lead to alternative transcriptional splice variants and/or changes of gene expression level, or combinations of any of the foregoing.

The analysis of nucleic acids present in the isolated particles is quantitative and/or qualitative. For quantitative analysis, the amounts (expression levels), either relative or absolute, of specific nucleic acids of interest within the isolated particles are measured with methods known in the art (described below). For qualitative analysis, the species of specific nucleic acids of interest within the isolated microvesicles, whether wild type or variants, are identified with methods known in the art.

The present invention also includes various uses of the new methods of isolating microvesicles from a biological sample for high quality nucleic acid extraction from a for (i) aiding in the diagnosis of a subject, (ii) monitoring the progress or reoccurrence of a disease or other medical condition in a subject, or (iii) aiding in the evaluation of treatment efficacy for a subject undergoing or contemplating treatment for a disease or other medical condition; wherein the presence or absence of one or more biomarkers in the nucleic acid extraction obtained from the method is determined, and the one or more biomarkers are associated with the diagnosis, progress or reoccurrence, or treatment efficacy, respectively, of a disease or other medical condition.

### Kits for isolating microvesicles from a biological sample

One aspect of the present invention is further directed to kits for use in the methods disclosed herein. The kit comprises a capture surface apparatus sufficient to separate microvesicles from a biological sample from unwanted particles, debris, and small molecules that are also present in the biological sample. The present invention also optionally includes instructions for using the foregoing reagents in the isolation and optional subsequent nucleic acid extraction process.

### EXAMPLES

While the examples provided herein use a variety of membranes and devices used for centrifugation and/or filtration purposes, it is to be understood that these methods can be used with any capture surface and/or housing device that allows for the efficient capture of microvesicles and release of the nucleic acids, particularly, RNA, contained therein.

### Example 1: Plasma Microvesicular RNA can be isolated using a 0.65um positively charged Q PES filter in a vacuum format

Normal control plasma was obtained from Bioreclamation LLC. Blood was collected into K2 EDTA tubes and mixed well with complete inversions. The tubes were centrifuged at 1300xg for 10min to separate the blood cells and plasma. Then, the plasma was filtered through a 0.8µm mixed cellulose ester filter (Millipore, Billerica, Ma., USA) to remove cell debris and platelets, and divided into 1 mL aliquots. Aliquots were frozen at - 80°C until needed.

Isolation of 4mL plasma microvesicular RNA was conducted using ultracentrifugation or Fast Trap Adenovirus purification and concentration kit (Millipore, Billerica, Ma., USA) 0.65um positively charged Q polyethersulfone vacuum filtration.

In the ultracentrifugation method, one 1 mL aliquots of plasma from four subjects were transferred to a 5mL polyallomer tube (Beckman-Coulter, Miami, Fl., USA) containing 8 µL RNasin Plus (40 U/µl, Promega, Madison, Wi., USA) RNase inhibitor, and incubated for 5 minutes at room temp. Following incubation, the plasma was diluted in 1 mL PBS (10mM Sodium phosphate, 2mM Potassium phosphate, 2.7mM KCl, 137mM NaCl, pH 7.4). Microvesicles were pelleted by ultracentrifugation at 120,000g for 80 minutes at 8°C. The microvesicle pellet was washed in 42 µL PBS and 8 µL RNasin Plus, and incubated for 20 minutes at room temp. The microvesicle pellet was lysed in 700ul Qiazol Reagent (Qiagen, Valencia, Ca., USA).

In the 0.65um positively charged Q polyethersulfone vacuum filtration method, one 1mL aliquots of plasma from the same four subjects used in the ultracentrifugation method were pooled, diluted with 5mL PBS, and mixed with 1 mL 10X Binding Buffer (Millipore). Before the plasma sample was applied, the filter was conditioned by passing through 25mL Equilibration Buffer (Millipore) by vacuum. Then, the plasma sample was passed through the filter by vacuum. The filtrate was saved for further analysis. The filter was washed with 20mL Wash Buffer (Millipore) by vacuum. Then the microvesicles were lysed with Qiazol. 2.25mL Qiazol was applied to the membrane and distributed by drawing approximately 5 drops of Qiazol through the filter by vacuum. The filter was then incubated for 5 min at room temp and eluted by vacuum.

The filtrate samples were transferred to two 5mL polyallomer tubes (Beckman-Coulter, Miami, Fl., USA) containing 8 µL RNasin Plus (40 U/µl, Promega, Madison, Wi, USA) RNase inhibitor, and incubated for 5 minutes at room temp. Microvesicles were pelleted by ultracentrifugation at 120,000g for 80 minutes at 8°C. The microvesicle pellets were washed in 42 µL PBS and 8 µL RNasin Plus, and incubated for 20 minutes at room temp. The microvesicle pellets were lysed in 700ul Qiazol Reagent (Qiagen, Valencia, Ca., USA).

For the ultracentrifugation and filtration (filter and filtrate) samples, microvesicular RNA was isolated using the miRNeasy kit (Qiagen) according to the manufacturer's recommendation. The RNA quality and concentration was assessed with the 2100 Bioanalyzer (Agilent, Palo Alto, Ca., USA) using a RNA 6000 Pico Chip (FIG 1A). Two-thirds and two-ninths of the total volume of microvesicular RNA was converted to cDNA using the SuperScript VILO cDNA Synthesis Kit (Invitrogen, Carlsbad, Ca., USA) and the Taqman MicroRNA Reverse Transcription Kit (Applied Biosystems, Foster City, Ca., USA), respectively, according to the manufacturer's protocol.

To compare microvesicular RNA isolated by filtration (filter and filtrate) and ultracentrifugation, RT-PCRs were performed on mRNA and non-coding RNA (including microRNA) using primers and probes specific to GAPDH, RN7SL, RNaseP, miR-16, miR-150, and let-7a. For GAPDH and RNaseP, the amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:15 fraction of the cDNA reverse transcribed with the SuperScript VILO cDNA Synthesis Kit. For RN7SL, the amplification reactions were performed in a 20ul volume containing 2X Taqman Gene Expression Master Mix, 900nM RN7SL Forward CAAAACTCCCGTGCTGATCA (SEQ ID NO 1), 900nM RN7SL Reverse GGCTGGAGTGCAGTGGCTAT (SEQ ID NO 2), 250nM RN7SL Probe TGGGATCGCGCCTGT (SEQ ID NO 3), and a 1:15 fraction of the cDNA reverse transcribed with the SuperScript VILO cDNA Synthesis Kit. For miR-16, miR-150, and let-7a, the amplification reactions were performed in a 20ul volume containing 2X Taqman Universal PCR Master Mix, 20X Taqman MicroRNA Assay (Applied Biosystems, Foster City, Ca., USA), and a 1:20 fraction of the cDNA reverse transcribed with the Taqman MicroRNA Reverse Transcription Kit. Amplification conditions consisted of: 1 cycle of 50°C, 2 min; 1 cycle of 95°C, 10 min; 40 cycles of 95°C, 10 min and 60°C, 1 min. See FIG 1B.

### Example 2: Detecting BRAF_V600E mutation in plasma sample from melanoma patient using Millipore Fast Trap Virus Purification Column

Melanoma plasma was obtained from Ludwig Maximilians University in accordance with protocols approved by the IRB and as described in EXAMPLE 1.

Isolation of 2mL and 12mL plasma microvesicular RNA was conducted using ultracentrifugation or Fast Trap Adenovirus purification and concentration kit (Millipore, Billerica, Ma., USA) 0.65um positively charged Q polyethersulfone vacuum filtration.

In the ultracentrifugation method, 2mL and 3X4mL plasma were transferred to a 5mL polyallomer tube (Beckman-Coulter, Miami, FL, USA) containing 8 µL RNasin Plus (40 U/µl, Promega, Madison, Wi., USA) RNase inhibitor, and incubated for 5 minutes at room temp. Following incubation, the 2mL and each 4mL plasma was diluted in 3mL and 1mL PBS, respectively. Microvesicles were pelleted and lysed as described in

### EXAMPLE 1.

In the 0.65um positively charged Q polyethersulfone vacuum filtration method, 2mL and 12mL plasma was diluted with 2.5 mL and 15mL PBS, respectively, and mixed with 500ul and 3mL 10X Binding Buffer (Millipore), respectively. Before the plasma samples were applied, the filters were conditioned by passing through 25mL Equilibration Buffer (Millipore) by vacuum. Then, the plasma samples were passed through the filter by vacuum. The filtrates were saved for further analysis. The filters were washed with 20mL Wash Buffer (Millipore) by vacuum. The washes were saved for further analysis. The filters were lysed as described in EXAMPLE 1.

The 2mL and 12mL plasma filtrate samples were transferred to one and six 5mL polyallomer tubes (Beckman-Coulter, Miami, Fl., USA), respectively. The wash samples were each transferred to four 5mL polyallomer tubes. The polyallomer tubes were incubated for 5 min at room temp with 8 µL RNasin Plus (40 U/µl, Promega, Madison, Wi., USA) RNase inhibitor. Microvesicles were pelleted and lysed as described in EXAMPLE 1.

For the ultracentrifugation and filtration (filter, filtrate, and wash) samples, microvesicular RNA was isolated, and the quality and concentration were assessed, as described in EXAMPLE 1.

For the ultracentrifugation and filtration (filter, filtrate, and wash) samples, microvesicular RNA was isolated, and the quality and concentration were assessed, as described in EXAMPLE 1. See FIG. 12A. Six-sevenths of the total volume of microvesicular RNA was converted to cDNA using the SuperScript VILO cDNA Synthesis Kit (Invitrogen, Carlsbad, Ca., USA) according to the manufacturer's protocol.

To compare the ability of microvesicular RNA isolated by filtration (filter, filtrate, and wash) and ultracentrifugation to detect BRAF mutation, RT-PCRs were performed on wild-type and mutant BRAF mRNA. The BRAF mutation assay uses ARMS (Amplificatory refractive and Mutation System) allele specific PCR. The assay was adapted from AstraZeneca (Cheshire, UK). The mutation assay contains primers specifically for amplifying BRAF T1799A (detecting V600E, V600K or V600D, depending on additional mutations present at 1798 or 1800 positions). The amplification reactions were performed in a 20ul volume containing 2X Taqman Universal PCR Master Mix, 900nM BRAF WT Forward AAAAATAGGTGATTTTGGTCTAGCTACAGT (SEQ ID NO 4), 900nM BRAF MT ARMS Forward AAAAATAGGTGATTTTGGTCTAGCTACATA (SEQ ID NO 5), 900nM BRAF JS E15 Reverse TGGATCCAGACAACTGTTCAA (SEQ ID NO 6), 250nM BRAF AZ E15 Probe GATGGAGTGGGTCCCATCAG (SEQ ID NO 7), and a 1:20 fraction of the cDNA. Amplification conditions consisted of: 1 cycle of 50°C, 2 min; 1 cycle of 95°C, 10 min; 50 cycles of 95°C, 10 min and 60°C, 1 min. See FIG 2.

### Example 3: Regenerated Cellulose Spin Column

Normal control plasma was obtained from Bioreclamation LLC, as described in EXAMPLE 1.

Isolation of 4mL plasma microvesicular RNA was conducted using ultracentrifugation or 3um positively charged Q regenerated cellulose centrifugal filtration.

The ultracentrifugation sample was prepared, pelleted, and lysed as described in EXAMPLE 1.

In the 3um positively charged Q regenerated cellulose centrifugal filtration method, 1mL aliquots of plasma from the same four subjects used in the ultracentrifugation method were pooled and diluted with 444ul 10X Loading Buffer (Sartorius). Before the plasma sample was applied, the filter was conditioned by passing through 5mL 1X Washing Buffer (Sartorius) at 500xg for 5 min. Then, the plasma sample was passed through the filter at 500xg for 5 min. The filtrate was saved for further analysis. The filter was washed twice with 18mL Washing Buffer (Sartorius) at 500xg for 5 min. The first wash was saved for further analysis. Then the microvesicles were lysed with Qiazol. 1mL Qiazol was applied to the membrane and distributed at 100xg for 1 sec. The filter was then incubated for 10 min at room temp and eluted at 500xg for 5 min.

The filtrate and wash samples were transferred to one and four 5mL polyallomer tubes (Beckman-Coulter, Miami, Fl., USA), respectively. The polyallomer tubes were incubated for 5 min at room temp with 8 µL RNasin Plus (40 U/µl, Promega, Madison, Wi., USA) RNase inhibitor. The wash polyallomer tubes were diluted with 500ul PBS. Microvesicles were pelleted and lysed as described in EXAMPLE 1.

For the ultracentrifugation and filtration (filter, filtrate, and wash) samples, microvesicular RNA was isolated, and the quality and concentration were assessed, as described in EXAMPLE 1. See FIG. 3A. cDNA was synthesized as described in EXAMPLE 1.

To compare microvesicular RNA isolated by filtration (filter, filtrate, and wash) and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See Fig. 3B.

### Example 4: Pall Mustang S membrane in Spin Column (Homemade Column)

Normal control plasma was obtained from Bioreclamation LLC, as described in EXAMPLE 1.

Isolation of 4mL plasma microvesicular RNA was conducted using ultracentrifugation or Mustang S (Pall, Port Washington, Ny., USA) 0.8um negatively charged S polyethersulfone centrifugal filtration using a homemade spin column device.

The ultracentrifugation sample was prepared, pelleted, and lysed as described in EXAMPLE 1.

In the 0.8um negatively charged S polyethersulfone centrifugal filtration method, the spin column was made by placing three cut filter layers in a spin column case and sealing with a Viton O-ring. One 1mL aliquots of plasma from the same four subjects used in the ultracentrifugation method were pooled and diluted 2X Loading Buffer (100mM sodium phosphate, pH6.8). Before the plasma sample was applied, the filter was conditioned by passing through 18mL Equilibration Buffer (50mM Sodium Phosphate, 150mM NaCl, pH6.8) at 500xg for 5 min. Then, the plasma sample was passed through the filter at 200xg for 2 min. The filter was washed with 18mL Wash Buffer (50mM Sodium Phosphate, 150mM NaCl, pH6.8) at 200xg for 2 min. Then the microvesicles were lysed with Qiazol. 1mL Qiazol was applied to the membrane and distributed at 100xg for 1 min. The filter was then incubated for 10 min at room temp and eluted at 100xg for 2 min followed by 2,000xg for 2 min.

For the ultracentrifugation and filtration samples, microvesicular RNA was isolated, and the quality and concentration were assessed, as described in EXAMPLE 1. See FIG. 4A. cDNA was synthesized as described in EXAMPLE 1.

To compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 4B.

### Example 5: Pall Mustang Membrane Q in Spin Column

Normal control plasma was obtained from Bioreclamation LLC, as described in EXAMPLE 1.

Isolation of 4mL plasma microvesicular RNA was conducted using ultracentrifugation or 0.8um Mustang Q (Pall) positively charged Q polyethersulfone centrifugal filtration using a homemade spin column device.

The ultracentrifugation sample was prepared, pelleted, and lysed as described in EXAMPLE 1.

In the 0.8um positively charged Q polyethersulfone centrifugal filtration method, the spin column was made as described in EXAMPLE 4. One 1mL aliquots of plasma from the same four subjects used in the ultracentrifugation method were pooled and diluted with 5mL PBS and mixed with 1mL 10X Loading Buffer (1M Tris, 1M NaCl, 0.1M MaCl₂, pH8). Before the plasma sample was applied, the filter was conditioned by passing through 18mL Equilibration Buffer (0.1M Tris, 0.1M NaCl, 0.01M MaCl₂, pH8) at 500xg for 5 min. Then, the plasma sample was passed through the filter at 200xg for 2 min. The filter was washed with 18mL Wash Buffer (0.1M Tris, 0.2M NaCl, 0.01M MgCl₂, pH8) at 200xg for 2 min. Then the microvesicles were lysed with Qiazol. 1mL Qiazol was applied to the membrane and distributed at 100xg for 1 sec. The filter was then incubated for 10 min at room temp and eluted at 100xg for 2 min followed by 2,000xg for 2 min.

For the ultracentrifugation and filtration samples, microvesicular RNA was isolated, and the quality and concentration were assessed, as described in EXAMPLE 1. See FIG. 5A. cDNA was synthesized as described in EXAMPLE 1.

To compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 5B.

### Example 6: Pall Mustang membrane Q or S in syringe filter

Normal control plasma was obtained from Bioreclamation LLC, as described in EXAMPLE 1.

Isolation of 4mL plasma microvesicular RNA was conducted using ultracentrifugation or Mustang Q (Pall) 0.8um positively charged Q polyethersulfone syringe filtration or Mustang S (Pall) 0.8um negatively charged S polyethersulfone syringe filtration.

The ultracentrifugation sample was prepared, pelleted, and lysed as described EXAMPLE 1.

In the 0.8um positively charged Q polyethersulfone syringe filtration method and the 0.8um negatively charged S polyethersulfone syringe filtration method, two 1mL aliquots of plasma from the same four subjects used in the ultracentrifugation method were pooled and diluted with 5mL PBS and mixed with 1mL 10X Loading Buffer (Q: 200mM Tris, 100mM NaCl, pH7.4; S: 200mM Phosphate, 100mM NaCl, pH7.4). Before the plasma samples were applied, the filters were conditioned by passing through 20mL of the appropriate Equilibration Buffer (Q: 20mM Tris, 100mM NaCl, pH7.4; S: 20mM Phosphate, 100mM NaCl, pH7.4) by syringe. Then, the plasma samples were passed through the filters by syringe. The filtrates were saved for further analysis. The filters were washed with 20mL of the appropriate Wash Buffer (Q: 20mM Tris, 100mM NaCl, pH7.4; S: 20mM Phosphate, 100mM NaCl, pH7.4) by syringe. Then, residual fluid was removed from the filters and the microvesicles were lysed with Qiazol. 700ul Qiazol was applied to the membranes and distributed by syringe. The filters were then incubated for 5 min at room temp and eluted by syringe.

The filtrate samples were prepared, pelleted, and lysed as described in EXAMPLE 1.

For the ultracentrifugation and filtration (filter and filtrate) samples, microvesicular RNA was isolated, and the quality and concentration were assessed, as described in EXAMPLE 1. cDNA was synthesized as described in EXAMPLE 1.

To compare microvesicular RNA isolated by filtration (filter and filtrate) and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 6.

### Example 7: Negatively-Charged Nylon (Syringe Filter)

Normal control plasma was obtained from Bioreclamation LLC, as described in EXAMPLE 1.

Isolation of 4mL plasma microvesicular RNA was conducted using ultracentrifugation or 50nm negatively charged nylon syringe filtration (Sterlitech, Kent, Wa., USA).

The ultracentrifugation sample was prepared, pelleted, and lysed as described in EXAMPLE 1.

In the 50nm negatively charged nylon syringe filtration method, one 1mL aliquots of plasma from the same four subjects used in the ultracentrifugation method were pooled. The plasma sample was passed through the filter by syringe. The filtrate was saved for further analysis. The filter was washed with 20mL PBS by syringe. Then, residual fluid was removed from the filter by syringe. For lysis, 700ul Qiazol was applied to the membranes and distributed by syringe. The filter was then incubated for 20 sec at room temp and eluted by syringe.

The filtrate was transferred to a 5mL polyallomer tube (Beckman-Coulter, Miami, Fl., USA) containing 8 µL RNasin Plus (40 U/µl, Promega, Madison, Wi., USA) RNase inhibitor and incubated for 5 min at room temp. Following incubation, the filtrate was diluted with 1mL PBS. Microvesicles were pelleted and lysed as described in EXAMPLE 1.

For the ultracentrifugation and filtration (filter and filtrate) samples, microvesicular RNA was isolated, and the quality and concentration were assessed, as described in EXAMPLE 1. See FIG. 7A. cDNA was synthesized as described in EXAMPLE 1.

To compare microvesicular RNA isolated by filtration (filter and filtrate) and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 7B.

### Example 8: Isolation of microvesicle RNA from Urine Sample

Normal control urine was obtained in house and was filtered through a 0.8 µm mixed cellulose ester filter (Millipore) to remove cell debris. The filtered urine was stored at 4°C until needed.

Isolation of 10mL urine microvesicular RNA was conducted using 100K centrifugal filter unit concentration (Millipore) or Fast Trap Adenovirus purification and concentration kit (Millipore) 0.65um positively charged Q polyethersulfone vacuum filtration.

In the 100K centrifugal filter unit concentration, 10mL urine transferred to a 100K centrifugal filter unit and concentrated at 4,500xg for 5 min. The concentrated microvesicle sample was then lysed in 700ul Qiazol.

In the 0.65um positively charged Q polyethersulfone vacuum filtration method, 10mL urine from the same subject used in the 100K centrifugal filter unit concentration was diluted with 8mL PBS and mixed with 2 mL 10X Binding Buffer (Millipore). The filter was equilibrated, loaded with the plasma sample, washed, and lysed as described in EXAMPLE 1.

The filtrate sample was transferred to four 5mL polyallomer tubes (Beckman-Coulter, Miami, Fl., USA) containing 8 µL RNasin Plus (40 U/µl, Promega, Madison, Wi., USA) RNase inhibitor and incubated for 5 min at room temp. Microvesicles were pelleted and lysed as described in EXAMPLE 1.

For the ultracentrifugation and filtration (filter and filtrate) samples, microvesicular RNA was isolated, and the quality and concentration were assessed, as described in EXAMPLE 1. See FIG. 8A. cDNA was synthesized as described in EXAMPLE 1.

To compare urine microvesicular RNA isolated by filtration (filter and filtrate) and 100K centrifugal filter unit concentration, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 8B.

### Example 9: Surface Charge (S Inhibition)

Normal control plasma was obtained from Bioreclamation LLC, as described in EXAMPLE 1.

Isolation of 4mL plasma microvesicular RNA was conducted using ultracentrifugation or modified 3um positively charged Q regenerated cellulose centrifugal filtration or modified 3um negatively charged S regenerated cellulose centrifugal filtration.

The ultracentrifugation sample was prepared, pelleted and lysed as described in EXAMPLE 1.

In the 3um positively charged Q regenerated centrifugal filtration method and the 3um negatively charged S regenerated cellulose centrifugal filtration method, the spin columns were each modified by removing 21 out of 24 filter layers. One 1mL aliquots of plasma from the same four subjects used in the ultracentrifugation method were pooled.

For the ultracentrifugation and filtration samples, microvesicular RNA was isolated, and the quality and concentration were assessed, as described in EXAMPLE 1. Prior to cDNA synthesis, the RNA was diluted 1:10 and 1:100. Three-fifths of the total volume of microvesicular RNA, the 1:10 RNA dilution, and the 1:100 RNA dilution were converted to cDNA using the SuperScript VILO cDNA Synthesis Kit (Invitrogen, Carlsbad, Ca., USA), according to the manufacturer's protocol.

To determine if there was any inhibition of RT-PCR at the RNA level, dilutions of microvesicular RNA isolated by filtration and ultracentrifugation were compared. RT-PCRs were performed on GAPDH. The amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:15 fraction of the cDNA reverse transcribed with the SuperScript VILO cDNA Synthesis Kit. Amplification conditions were as described in EXAMPLE 1. See FIG. 9.

If there was not any inhibition of RT-PCR at the RNA level, each 1:10 dilution would result in a 3.3Ct increase, as shown in ultracentrifugation and 3um positively charged Q regenerated cellulose filtration. However, the average Ct value decreased with dilution for the 3um negatively charged S regenerated cellulose filtration, indication RT-PCR inhibition at the RNA level.

It was observed that the filters tended to clog when plasma samples were at a pH set to less than or equal to 5.5 before loading, and the plasma would not spin through the column.

### Example 10: Microvesicles are stable in acidic pH

Normal control plasma was obtained from Bioreclamation LLC, as described in EXAMPLE 1.

Isolation of 1.9mL plasma microvesicular RNA was conducted using centrifugation. The pellets were resuspended, in duplicate, in the following buffers:
- 50mM Methyl Malonic Acid, 150mM NaCl, pH2.5
- 50mM Methyl Malonic Acid, 150mM NaCl, pH3.5
- 50mM Acetic Acid, 150mM NaCl, pH4.5
- 10mM Sodium phosphate, 2mM Potassium phosphate, 2.7mM KCl, 137mM NaCl, pH 7.4

Specifically, 16 1mL aliquots of plasma from one subject was pooled. 1.9mL of the pooled plasma was transferred to eight 2.0mL Eppendorf tubes and pelleted at 21,130xg for 30 min at 8°C. The microvesicle pellet was resuspended in 150ul of the appropriate buffer and incubated for 20 min at room temp. The microvesicle pellet was then lysed in 700ul Qiazol Reagent (Qiagen, Valencia, Ca., USA).

The microvesicular RNA was isolated, and the quality and concentration were assessed, as described in EXAMPLE 1. See FIG. 10A. cDNA was synthesized as described in EXAMPLE 1.

To determine the stability of plasma microvesicles in acidic pH, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 10B.

### Example 11: Microvesicles are not stable in basic pH.

Normal control plasma was obtained from Bioreclamation LLC, as described in EXAMPLE 1.

To determine the stability of plasma microvesicles in basic pH, microvesicular pellets were resuspended in the following buffers:
- 50mM Bis Tris Propane, 150mM NaCl, pH7
- 50mM Tris, 150mM NaCl, pH8
- 50mM Diethanolamine, 150mM NaCl, pH9
- 50mM Ethanolamine, 150mM NaCl, pH10

Plasma microvesicular RNA isolation and analysis was performed as described in EXAMPLE 10. See FIG. 11A and 11B.

### Example 12: Microvesicle capture and/or microvesicle stability on a charged membrane are affected by buffer pH and/or buffer concentration and/or buffer type.

Normal control plasma was obtained from Bioreclamation LLC, as described in EXAMPLE 1.

Isolation of 4.8mL plasma microvesicle RNA was conducted using ultracentrifugation or 3um positively charged Q regenerated cellulose centrifugal filtration.

In the ultracentrifugation method, 1 mL aliquots of plasma from five subjects were pooled. 4.8mL of the pooled plasma was transferred to a 5mL polyallomer tube (Beckman-Coulter, Miami, Fl., USA) containing 8 µL RNasin Plus (40 U/µl, Promega, Madison, Wi., USA) RNase inhibitor, and incubated for 5 minutes at room temp. Microvesicles were pelleted and lysed as described in EXAMPLE 1.

In the 3um positively charged Q regenerated cellulose centrifugal filtration method, equilibration, loading, and wash buffers were tested with different buffer types and pHs. The following buffer sets were used:
- 100mM Bis Tris Propane, 150mM NaCl, pH6.8 (2X Loading Buffer) and 50mM Bis Tris Propane, 150mM NaCl, pH7 (Equilibration and Wash Buffer),
- 100mM Tris, 150mM NaCl, pH8 (2X Loading Buffer) and 50mM Tris, 150mM NaCl, pH8 (Equilibration and Wash Buffer), or
- 100mM Diethanolamine, 150mM NaCl, pH9 (2X Loading Buffer) and 50mM Diethanolamine, 150mM NaCl, pH9 (Equilibration and Wash Buffer).

First, three 1mL aliquots of plasma from the same five subjects used in the ultracentrifugation method were pooled. 4.8mL of the pooled plasma was aliquoted into separate 50mL conical tubes for each buffer set and mixed with 4.8mL of the appropriate 2X loading buffer. The pH of each diluted plasma sample was assessed for accuracy. Before the plasma sample was applied, the filter was conditioned by passing through 5mL of the appropriate equilibration buffer at 500xg for 5 min. Then, the plasma sample was passed through the filter at 500xg for 5min. The filtrate was saved for further analysis. The filter was washed twice with 18mL of the appropriate wash buffer at 500xg for 5min. Then the microvesicles were lysed with Qiazol. 1mL Qiazol was applied to the membrane and distributed by centrifugation at 100xg for 1 sec. The filter was then incubated for 10 min at room temp and eluted at 500xg for 5min.

The filtrate samples were each prepared, pelleted and lysed as described in EXAMPLE 1.

For the ultracentrifugation and filtration (filter and filtrate) samples, microvesicular RNA was isolated, and the quality and concentration were assessed, as described in EXAMPLE 1. See FIG. 12A. cDNA was synthesized as described in EXAMPLE 1.

To determine the preferred pH for microvesicular RNA filtration and to compare microvesicular RNA isolated by filtration (filter and filtrate) and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1.

As shown in FIG. 12B, pH and/or buffer type has a significant effect on the filtration samples for GAPDH, RN7SL, RNaseP, let-7a, and miR-150. The 100mM Bis Tris Propane, 150mM NaCl, pH6.8 (2X Loading Buffer) and 50mM Bis Tris Propane, 150mM NaCl, pH7 (Equilibration and Wash Buffer) buffer set is preferred for the filter samples. pH and/or buffer type does not have a significant effect on the filtration samples for miR-16. Additionally, the levels of RNA for GAPDH, RN7SL, and RNaseP are similar for filter and ultracentrifuge samples. The average Ct-value was 0.9Ct lower for let-7a and 0.7Ct higher for miR-150 in the ultracentrifuge sample compared to the preferred filter sample. The average Ct-value was 2.3Ct lower for miR-16 in the ultracentrifuge sample compared to the preferred filter sample. This is most likely due to differences in packaging of microRNA in microvesicles. Some microRNAs are found in ribonucleoprotein complexes in plasma and would not necessarily be captured by positively charged filtration.

### Example 13: Microvesicle capture and/or microvesicle stability on a charged filter are affected by buffer pH and/or buffer concentration and/or buffer type.

Normal control plasma was obtained from Bioreclamation LLC, as described in EXAMPLE 1.

Isolation of 3.8mL plasma microvesicle RNA was conducted using ultracentrifugation or 3um positively charged Q regenerated cellulose centrifugal filtration.

In the ultracentrifugation method, 1 mL aliquots of plasma from four subjects were pooled. 3.8mL of the pooled plasma was transferred to a 5mL polyallomer tube (Beckman-Coulter, Miami, Fl., USA) containing 8 µL RNasin Plus (40 U/µl, Promega, Madison, Wi., USA) RNase inhibitor, and incubated for 5 minutes at room temp. Following incubation, the plasma was diluted in 1.2mL PBS. Microvesicles were pelleted and lysed as described in EXAMPLE 1.

In the 3um positively charged Q regenerated cellulose centrifugal filtration method, equilibration, loading, and wash buffers were tested with different buffer types and pHs. The following buffer sets were used:
- 100mM Bis Tris Propane, 150mM NaCl, pH6 (2X Loading Buffer) and 50mM Bis Tris Propane, 150mM NaCl, pH6.5 (Equilibration and Wash Buffer)
- 100mM Bis Tris Propane, 150mM NaCl, pH6.8 (2X Loading Buffer) and 50mM Bis Tris Propane, 150mM NaCl, pH7 (Equilibration and Wash Buffer)
- 100mM Triethanolamine, 150mM NaCl, pH6.5 (2X Loading Buffer) and 50mM Triethanolamine, 150mM NaCl, pH7.0 (Equilibration and Wash Buffer)
- 100mM Bis Tris Propane, 150mM NaCl, pH7.4 (2X Loading Buffer) and 50mM Bis Tris Propane, 150mM NaCl, pH7.5 (Equilibration and Wash Buffer)
- 100mM Tris, 150mM NaCl, pH7.4 (2X Loading Buffer) and 50mM Tris, 150mM NaCl, pH7.5 (Equilibration and Wash Buffer)
- 100mM Bis Tris Propane, 150mM NaCl, pH8 (2X Loading Buffer) and 50mM Bis Tris Propane, 150mM NaCl, pH8 (Equilibration and Wash Buffer)
- 100mM Tris, 150mM NaCl, pH8 (2X Loading Buffer) and 50mM Tris, 150mM NaCl, pH8 (Equilibration and Wash Buffer)
- 100mM Tris, 150mM NaCl, pH8.5 (2X Loading Buffer) and 50mM Tris, 150mM NaCl, pH8.5 (Equilibration and Wash Buffer)

First, eight 1mL aliquots of plasma from the same four subjects used in the ultracentrifugation method were pooled. 3.8mL of the pooled plasma was aliquoted into separate 50mL conical tubes for each buffer set and mixed with 3.8mL of the appropriate 2X loading buffer. The pH of each diluted plasma sample was assessed for accuracy. Before the plasma sample was applied, the filter was conditioned bypassing through 5mL of the appropriate equilibration buffer at 500xg for 5 min. Then, the plasma sample was passed through the filter at 500xg for 5min. The filter was washed twice with 18mL of the appropriate wash buffer at 500xg for 5min. Then the microvesicles were lysed with Qiazol. 1mL Qiazol was applied to the membrane and distributed by centrifugation at 100xg for 1 sec. The filter was then incubated for 10 min at room temp and eluted at 500xg for 5min.

For the ultracentrifugation and filtration samples, microvesicular RNA was isolated, and the quality and concentration were assessed, as described in EXAMPLE 1. See FIG. 13A. cDNA was synthesized as described in EXAMPLE 1.

To determine the preferred pH and buffer type for microvesicular RNA filtration and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 13B.

The following buffer sets are preferred: 100mM Bis Tris Propane, 150mM NaCl, pH6 (2X Loading Buffer) and 50mM Bis Tris Propane, 150mM NaCl, pH6.5 (Equilibration and Wash Buffer); and 100mM Bis Tris Propane, 150mM NaCl, pH6.8 (2X Loading Buffer) and 50mM Bis Tris Propane, 150mM NaCl, pH7 (Equilibration and Wash Buffer).

### Example 14: Microvesicle capture and/or microvesicle stability on a charged filter are affected by buffer pH and buffer concentration.

Normal control plasma was obtained from Bioreclamation LLC, as described in EXAMPLE 1.

Isolation of 3.8mL plasma microvesicle RNA was conducted using ultracentrifugation or 3um positively charged Q regenerated cellulose centrifugal filtration.

The ultracentrifugation sample was prepared, pelleted, and lysed as described in EXAMPLE 13.

In the 3um positively charged Q regenerated cellulose centrifugal filtration method, equilibration, loading, and wash buffers were tested with different buffer types and pHs. The following buffer sets were used:
- 117mM Bis Tris, 150mM NaCl, pH1.9 (2X Loading Buffer) and 58.5mM Bis Tris, 150mM NaCl, pH6 (Equilibration and Wash Buffer)
- 117mM Bis Tris, 150mM NaCl, pH6.1 (2X Loading Buffer) and 58.5mM Bis Tris, 150mM NaCl, pH6.5 (Equilibration and Wash Buffer)
- 100mM Bis Tris Propane, 150mM NaCl, pH6 (2X Loading Buffer) and 50mM Bis Tris Propane, 150mM NaCl, pH6.5 (Equilibration and Wash Buffer)
- 100mM Bis Tris Propane, 150mM NaCl, pH6.8 (2X Loading Buffer) and 50mM Bis Tris Propane, 150mM NaCl, pH7 (Equilibration and Wash Buffer)
- 100mM Bis Tris Propane, 150mM NaCl, pH7.4 (2X Loading Buffer) and 50mM Bis Tris Propane, 150mM NaCl, pH7.5 (Equilibration and Wash Buffer)
- 200mM Tris, 150mM NaCl, pH7.5 (2X Loading Buffer) and 100mM Tris, 150mM NaCl, pH7.5 (Equilibration and Wash Buffer)

First, six 1mL aliquots of plasma from the same four subjects used in the ultracentrifugation method were pooled. 3.8mL of the pooled plasma was aliquoted into separate 50mL conical tubes for each buffer set and mixed with 3.8mL of the appropriate 2X loading buffer. The pH of each diluted plasma sample was assessed for accuracy. The filters were equilibrated, loaded with the plasma sample, washed, and lysed as described in EXAMPLE 13.

For the ultracentrifugation and filtration samples, microvesicular RNA was isolated, and the quality and concentration were assessed, as described in EXAMPLE 1. See FIG. 14A. cDNA was synthesized as described in EXAMPLE 1.

To determine the preferred pH and buffer type for microvesicular RNA filtration and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 14B.

The following buffer sets are preferred: 100mM Bis Tris Propane, 150mM NaCl, pH6 (2X Loading Buffer) and 50mM Bis Tris Propane, 150mM NaCl, pH6.5 (Equilibration and Wash Buffer); and 100mM Bis Tris Propane, 150mM NaCl, pH6.8 (2X Loading Buffer) and 50mM Bis Tris Propane, 150mM NaCl, pH7 (Equilibration and Wash Buffer).

### Example 15: Microvesicle capture and microvesicle stability on a charged filter is affected by the concentration of buffer.

Normal control plasma was obtained from Bioreclamation LLC, as described in EXAMPLE 1.

Isolation of 3.8mL plasma microvesicle RNA was conducted using ultracentrifugation or 3um positively charged Q regenerated cellulose centrifugal filtration.

The ultracentrifugation sample was prepared, pelleted, and lysed as described in EXAMPLE 13.

In the 3um positively charged Q regenerated cellulose centrifugal filtration method, equilibration, loading, and wash buffers were tested with varying buffer concentration. The following buffer sets were used:
- 100mM Bis Tris Propane, 0.15mM NaCl, pH6 (2X Loading Buffer) and 50mM Bis Tris Propane, 0.15mM NaCl, pH6.5 (Equilibration and Wash Buffer)
- 500mM Bis Tris Propane, 900mM NaCl, pH6.4 (2X Loading Buffer) and 250mM Bis Tris Propane, 450mM NaCl, pH6.5 (Equilibration and Wash Buffer)

First, two 1mL aliquots of plasma from the same four subjects used in the ultracentrifugation method were pooled. 3.8mL of the pooled plasma was aliquoted into separate 50mL conical tubes for each buffer set and mixed with 3.8mL of the appropriate 2X loading buffer. The pH of each diluted plasma sample was assessed for accuracy. The filters were equilibrated, loaded with the plasma sample, washed, and lysed as described in EXAMPLE 13.

For the ultracentrifugation and filtration samples, microvesicular RNA was isolated, and the quality and concentration were assessed, as described in EXAMPLE 1. See FIG. 15A. cDNA was synthesized as described in EXAMPLE 1.

To determine the preferred buffer concentration for microvesicular RNA filtration and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 15B.

### Example 16: Microvesicles are stable and overall RNA yield is not affected by low to high concentrations of salt.

Normal control plasma was obtained from Bioreclamation LLC, as described in EXAMPLE 1.

To determine the stability of plasma microvesicles in NaCl, microvesicular pellets were resuspended, in duplicate, in 50mM Bis Tris Propane, pH6.5 buffer with the following NaCl concentrations:
- 0.15M NaCl
- 0.3M NaCl
- 0.6M NaCl
- 1.2M NaCl
- 2.4M NaCl

Specifically, 20 1mL aliquots of plasma from one subject were pooled and 1.9mL of the pooled plasma was transferred to ten 2.0mL Eppendorf tubes. Plasma microvesicles were pelleted, resuspended, and lysed as described in EXAMPLE 10.

The microvesicular RNA was isolated, and the quality and concentration were assessed, as described in EXAMPLE 1. See FIG. 16A. cDNA was synthesized as described in EXAMPLE 1.

To determine the stability of plasma microvesicles in varying salt concentrations, RT-PCRs were performed on mRNA and non-coding RNA (including microRNA) using primers and probes specific to GAPDH, HPRT1, RN7SL, RNaseP, miR-16, and let-7a. For GAPDH, RNaseP, and HPRT1, the amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:20 fraction of the cDNA reverse transcribed with the SuperScript VILO cDNA Synthesis Kit. For RN7SL, the amplification reactions were performed in a 20ul volume containing 2X Taqman Gene Expression Master Mix, 900nM RN7SL Forward CAAAACTCCCGTGCTGATCA (SEQ ID NO 1), 900nM RN7SL Reverse GGCTGGAGTGCAGTGGCTAT (SEQ ID NO 2), 250nM RN7SL Probe TGGGATCGCGCCTGT (SEQ ID NO 3), and a 1:20 fraction of the cDNA reverse transcribed with the SuperScript VILO cDNA Synthesis Kit. For miR-16 and let-7a, the amplification reactions were performed in a 20ul volume containing 2X Taqman Universal PCR Master Mix, 20X Taqman MicroRNA Assay (Applied Biosystems, Foster City, Ca., USA), and a 1:20 fraction of the cDNA reverse transcribed with the Taqman MicroRNA Reverse Transcription Kit. Amplification conditions were performed as described in EXAMPLE 1. See FIG. 16B.

### Example 17: Microvesicle capture and microvesicle stability on a charged filter is not affected by salt concentration of the loading buffer.

Normal control plasma was obtained from Bioreclamation LLC, as described in EXAMPLE 1.

Isolation of 3.8mL plasma microvesicle RNA was conducted using ultracentrifugation or 3um positively charged Q regenerated cellulose centrifugal filtration.

The ultracentrifugation sample was prepared, pelleted, and lysed as described in EXAMPLE 13.

In the 3um positively charged Q regenerated cellulose centrifugal filtration method, equilibration and loading buffers were tested with varying salt concentrations. The following buffer sets were used:
- 100mM Bis Tris Propane, 0.15M NaCl, pH6.0 (2X Loading buffer) and 50mM Bis Tris Propane, 0.15M NaCl, pH6.5 (Equilibration buffer)
- 100mM Bis Tris Propane, 1.05M NaCl, pH6.0 (2X Loading buffer) and 50mM Bis Tris Propane, 0.6M NaCl, pH6.5 (Equilibration buffer)
- 100mM Bis Tris Propane, 2.25M NaCl, pH6.0 (2X Loading buffer) and 50mM Bis Tris Propane, 1.2M NaCl, pH6.5 (Equilibration buffer)
- 100mM Bis Tris Propane, 4.65M NaCl, pH6.0 (2X Loading buffer) and 50mM Bis Tris Propane, 2.4M NaCl, pH6.5 (Equilibration buffer)

First, four 1mL aliquots of plasma from the same four subjects used in the ultracentrifugation method were pooled. 3.8mL of the pooled plasma was aliquoted into separate 50mL conical tubes for each buffer set and mixed with 3.8mL of the appropriate 2X loading buffer. Before the plasma sample was applied, the filter was conditioned by passing through 5mL of the appropriate equilibration buffer at 100xg for 5 min. Then, the plasma sample was passed through the filter at 100xg for 5min. The filtrate was saved for further analysis. Without a wash, the microvesicles were lysed with Qiazol. 1mL Qiazol was applied to the membrane and distributed by centrifugation at 100xg for 1 sec. The filter was then incubated for 10 min at room temp and eluted at 200xg for 2min followed by 5000xg for 2min.

3.8mL of the plasma filtrate samples were each transferred to two 5mL polyallomer tubes (Beckman-Coulter, Miami, Fl., USA) containing 8 µL RNasin Plus (40 U/µl, Promega, Madison, Wi., USA) RNase inhibitor and were incubated for 5 min at room temp. Following incubation, each filtrate aliquot was diluted in 1.2mL PBS. Microvesicles were pelleted and lysed as described in EXAMPLE 1.

For the ultracentrifugation and filtration samples, microvesicular RNA was isolated, and the quality and concentration were assessed, as described in EXAMPLE 1. See FIG. 17A. cDNA was synthesized as described in EXAMPLE 1.

To determine the preferred salt concentration for loading the plasma sample in the filtration method, and to compare microvesicular RNA isolated by filtration (filter and filtrate) and ultracentrifugation, RT-PCRs were performed on mRNA and non-coding RNA (including microRNA) using primers and probes specific to GAPDH, HPRT1, RN7SL, BRAF, miR-16, and let-7a. For GAPDH and HPRT1, the amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:20 fraction of the cDNA reverse transcribed with the SuperScript VILO cDNA Synthesis Kit. For RN7SL, the amplification reactions were performed in a 20ul volume containing 2X Taqman Gene Expression Master Mix, 900nM RN7SL Forward CAAAACTCCCGTGCTGATCA (SEQ ID NO 1), 900nM RN7SL Reverse GGCTGGAGTGCAGTGGCTAT (SEQ ID NO 2), 250nM RN7SL Probe TGGGATCGCGCCTGT (SEQ ID NO 3), and a 1:20 fraction of the cDNA reverse transcribed with the SuperScript VILO cDNA Synthesis Kit. For BRAF, the amplification reactions were performed in a 20ul volume containing 2X Taqman Gene Expression Master Mix, 900nM BRAF WT Forward AAAAATAGGTGATTTTGGTCTAGCTACAGT (SEQ ID NO 4), 900nM BRAF JS E15 Reverse TGGATCCAGACAACTGTTCAA (SEQ ID NO 6), 250nM BRAF AZ E15 Probe GATGGAGTGGGTCCCATCAG (SEQ ID NO 7), and a 1:20 fraction of cDNA reverse transcribed with the SuperScript VILO cDNA Synthesis Kit. For miR-16 and let-7a, the amplification reactions were performed in a 20ul volume containing 2X Taqman Universal PCR Master Mix, 20X Taqman MicroRNA Assay (Applied Biosystems, Foster City, Ca., USA), and a 1:20 fraction of the cDNA reverse transcribed with the Taqman MicroRNA Reverse Transcription Kit. Amplification conditions were performed as described in EXAMPLE 1. See FIG. 17B.

### Example 18: Microvesicle capture and microvesicle stability on a charged filter is not affected by salt concentration of the loading or wash buffer.

Normal control plasma was obtained from Bioreclamation LLC, as described in EXAMPLE 1.

Isolation of 3.8mL plasma microvesicle RNA was conducted using ultracentrifugation or 3um positively charged Q regenerated cellulose centrifugal filtration.

The ultracentrifugation sample was prepared, pelleted, and lysed as described in EXAMPLE 13.

In the 3um positively charged Q regenerated cellulose centrifugal filtration method, equilibration and loading buffers were tested with varying salt concentrations. The following buffer sets were used:
- 100mM Bis Tris Propane, 0.15mM NaCl, pH6 (2X Loading Buffer) and 50mM Bis Tris Propane, 0.15mM NaCl, pH6.5 (Equilibration and Wash Buffer)
- 100mM Bis Tris Propane, 1.05M NaCl, pH6 (2X Loading Buffer) and 50mM Bis Tris Propane, 0.6M NaCl, pH6.5 (Equilibration and Wash Buffer)
- 100mM Bis Tris Propane, 2.25M NaCl, pH6 (2X Loading Buffer) and 50mM Bis Tris Propane, 1.2M NaCl, pH6.5 (Equilibration and Wash Buffer)
- 100mM Bis Tris Propane, 4.65M NaCl, pH6 (2X Loading Buffer) and 50mM Bis Tris Propane, 2.4M NaCl, pH6.5 (Equilibration and Wash Buffer)

First, four 1mL aliquots of plasma from the same four subjects used in the ultracentrifugation method were pooled. 3.8mL of the pooled plasma was aliquoted into separate 50mL conical tubes for each buffer set and mixed with 3.8mL of the appropriate 2X loading buffer. Before the plasma sample was applied, the filter was conditioned by passing through 5mL of the appropriate equilibration buffer at 100xg for 5 min. Then, the plasma sample was passed through the filter at 100xg for 5min. The filtrate was saved for further analysis. The filter was washed with 18mL of the appropriate wash buffer at 100xg for 5min. Then the microvesicles were lysed with Qiazol. 1mL Qiazol was applied to the membrane and distributed by centrifugation at 100xg for 1 sec. The filter was then incubated for 10 min at room temp and eluted at 200xg for 2min followed by 5000xg for 2min.

For the ultracentrifugation and filtration samples, microvesicular RNA was isolated, and the quality and concentration were assessed, as described in EXAMPLE 1. See FIG. 18A. cDNA was synthesized as described in EXAMPLE 1.

To determine the preferred salt concentration for washing the plasma sample in the filtration method and to compare microvesicular RNA isolated by filtration and ultracentrifugation, RT-PCRs were performed on mRNA and non-coding RNA (including microRNA) using primers and probes specific to GAPDH, RN7SL, HPRT1, BRAF, miR-16, and let-7a, as described in EXAMPLE 17. See FIG. 18B.

### Example 19: The RNA lysis buffer affects the RNA yield from microvesicles isolated with a charged filter

Normal control plasma was obtained from Bioreclamation LLC, as described in EXAMPLE 1.

Isolation of 4mL plasma microvesicular RNA was conducted using ultracentrifugation or Fast Trap Adenovirus purification and concentration kit (Millipore, Billerica, Ma., USA) 0.65um positively charged Q polyethersulfone vacuum filtration. In the 0.65um positively charged Q polyethersulfone vacuum filtration method, the microvesicular RNA was lysed with either Qiazol (Qiagen) or Promega Lysis Reagent (Promega).

The ultracentrifugation sample was prepared, pelleted, and lysed as described in EXAMPLE 1.

The 4mL plasma for 0.65um positively charged Q polyethersulfone vacuum filtration lysed with Qiazol (Qiagen) was prepared, and the filter was equilibrated, loaded with the plasma sample, washed, and lysed as described in EXAMPLE 1.

For the ultracentrifugation and 0.65um positively charged Q polyethersulfone vacuum filtration lysed with Qiazol samples, microvesicular RNA was isolated, as described in EXAMPLE 1.

In the 0.65um positively charged Q polyethersulfone vacuum filtration lysed with Promega Lysis Reagent (Promega) method, one 1mL aliquots of plasma from the same four subjects used in the ultracentrifugation method were pooled and mixed with 4mL 2X Loading Buffer (20mM Tris, pH7). Before the plasma sample was applied, the filter was conditioned by passing through 25mL Equilibration Buffer (Millipore) by vacuum. Then, the plasma sample was passed through the filter by vacuum. The filter was washed with 20mL Wash Buffer (10mM Tris, pH7) by vacuum. Then the microvesicles were lysed with Promega Lysis Reagent. 2.25mL Promega Lysis Reagent was applied to the membrane and distributed by drawing approximately 5 drops of Promega Lysis Reagent through the filter by vacuum. The filter was then incubated for 5 min at room temp and eluted by vacuum. To isolate the microvesicular RNA, 0.27 volume 100% isopropanol was added to the lysate, mixed by pipet, transferred to a spin column, and centrifuged at 13,000xg for 30 sec until all of the lysate was applied. The column was then washed twice with 500ul RNA Wash Solution (Promega) at 13,000xg for 30 sec. A final 300ul RNA Wash Solution was applied to the column at 13,000xg for 2 min. To dry the membrane, the column was centrifuged at 13,000xg for 2 min. RNA was eluted with 20ul H₂O into a 1.5mL Eppendorf tube, incubated at room temp for 1 min, and centrifuged at 10,000xg for 1 min.

For all microvesicular RNA samples, the quality and concentration were assessed, as described in EXAMPLE 1. cDNA was synthesized as described in EXAMPLE 1.

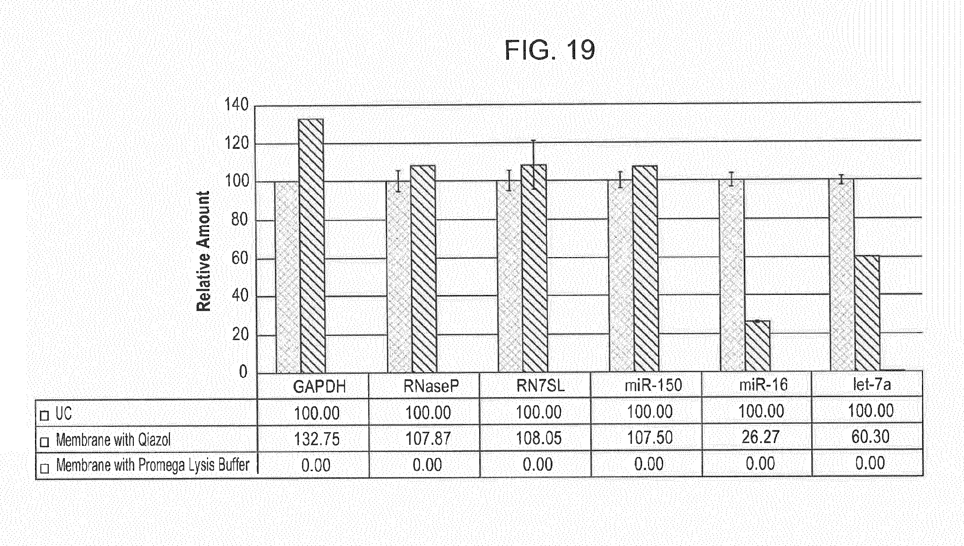

To determine the preferred lysis buffer for microvesicular RNA filtration and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 19.

### Example 20: A second volume of Qiazol does not significantly improve the RNA yields when isolating microvesicles on a charged filter.

Normal control plasma was obtained from Bioreclamation LLC, as described in EXAMPLE 1.

Isolation of 4mL plasma microvesicular RNA was conducted using ultracentrifugation or Fast Trap Adenovirus purification and concentration kit (Millipore, Billerica, Ma., USA) 0.65um positively charged Q polyethersulfone vacuum filtration.

The ultracentrifugation sample was prepared, pelleted, and lysed as described in EXAMPLE 1.

The 4mL plasma for 0.65um positively charged Q polyethersulfone vacuum filtration was prepared, and the filter was equilibrated, loaded with the plasma sample, and washed as described in EXAMPLE 1. The filter microvesicles were lysed with Qiazol. 2.25mL Qiazol was applied to the membrane and distributed by drawing approximately 5 drops of Qiazol through the filter by vacuum. The filter was then incubated for 5 min at room temp and eluted by vacuum. The lysis was repeated with an additional 2.25mL Qiazol. The two lysates were isolated for microvesicular RNA separately.

For the ultracentrifugation and filtration samples, microvesicular RNA was isolated, and the quality and concentration were assessed, as described in EXAMPLE 1. See FIG. 20A. cDNA was synthesized as described in EXAMPLE 1.

To determine the preferred and needed number of volumes of lysis buffer for microvesicular RNA filtration, and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 20B.

### Example 21: A second volume of Qiazol does not significantly improve the RNA yields when isolating microvesicles on a charged filter.

Normal control plasma was obtained from Bioreclamation LLC, as described in EXAMPLE 1.

Isolation of 4mL plasma microvesicular RNA was conducted using ultracentrifugation or 3um positively charged Q regenerated cellulose centrifugal filtration.

The ultracentrifugation sample was prepared, pelleted, and lysed as described in EXAMPLE 1.

The 4mL plasma for 3um positively charged Q regenerated cellulose centrifugal filtration was prepared, and the filter was equilibrated, loaded with the plasma sample, and washed as described in EXAMPLE 1. The filter microvesicles were lysed with Qiazol. 1mL Qiazol was applied to the membrane and distributed at 100xg for 1 sec. The filter was then incubated for 10 min at room temp and eluted at 500xg for 5 min. The lysis was repeated with an additional 1mL Qiazol. The two lysates were isolated for microvesicular RNA separately.

For the ultracentrifugation and filtration samples, microvesicular RNA was isolated, and the quality and concentration were assessed, as described in EXAMPLE 1. See FIG. 21A. cDNA was synthesized as described in EXAMPLE 1.

To determine the preferred and needed number of volumes of lysis buffer for microvesicular RNA filtration, and to compare microvesicular RNA isolated by filtration and ultracentrifugation, RT-PCRs were performed on mRNA and non-coding RNA (including microRNA) using primers and probes specific to GAPDH, RN7SL, RNaseP, miR-16, miR-150, and let-7a, as described in EXAMPLE 1. See FIG. 21B.

### Example 22: Microvesicular RNA can be isolated using a 20nm PES neutral syringe filter

Normal control plasma was obtained in house, as described in EXAMPLE 1.

Isolation of 4mL plasma microvesicular RNA was conducted using ultracentrifugation or ExoMir kit (Bioo Scientific, Austin, Tx., USA) 20nm neutral polyethersulfone syringe filtration.

In the ultracentrifugation method, 4mL plasma from the same subject was transferred to a 5mL polyallomer tube (Beckman-Coulter, Miami, Fl., USA) containing 8 µL RNasin Plus (40 U/µl, Promega, Madison, Wi., USA) RNase inhibitor, and incubated for 5 minutes at room temp. Following incubation, the plasma was diluted with 1mL PBS. Microvesicles were pelleted and lysed as described in EXAMPLE 1.

In the 20nm neutral polyethersulfone syringe filtration method, 4mL from the same subject used in the ultracentrifugation method was treated with 200ul Proteinase K (>600 mAU/ml, Qiagen) for 15 min at room temp. Following incubation, the plasma was passed through a filter stack (0.8um neutral mixed cellulose ester filter (Millipore) + 20nm neutral polyethersulfone filter (Bioo Scientific) by syringe. The filter stack was washed with 20mL PBS by syringe. Then, the 0.8um neutral mixed cellulose ester filter was discarded, and the residual fluid was removed from the 20nm neutral polyethersulfone filter only by syringe. For lysis, 700ul Qiazol was applied to the filter and distributed by syringe. The filter was then incubated for 20 sec at room temp and eluted by syringe.

For the ultracentrifugation and filtration samples, microvesicular RNA was isolated, and the quality and concentration were assessed, as described in EXAMPLE 1. See FIG. 22A. Six-sevenths of the total volume of microvesicular RNA was converted to cDNA using the SuperScript VILO cDNA Synthesis Kit (Invitrogen, Carlsbad, Ca., USA), according to the manufacturer's protocol.

To compare microvesicular RNA isolated by filtration and ultracentrifugation, RT-PCRs were performed on mRNA and non-coding RNA using primers and probes specific to GAPDH, RN7SL, 18S, BRAF, HERV K, and PO. For GAPDH, 18S, and PO, the amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:20 fraction of cDNA. For RN7SL, the amplification reactions were performed in a 20ul volume containing 2X Taqman Gene Expression Master Mix, 900nM RN7SL Forward CAAAACTCCCGTGCTGATCA (SEQ ID NO 1), 900nM RN7SL Reverse GGCTGGAGTGCAGTGGCTAT (SEQ ID NO 2), 250nM RN7SL Probe TGGGATCGCGCCTGT (SEQ ID NO 3), and a 1:20 fraction of cDNA. For BRAF, the amplification reactions were performed in a 20ul volume containing 2X Taqman Gene Expression Master Mix, 900nM BRAF WT Forward AAAAATAGGTGATTTTGGTCTAGCTACAGT (SEQ ID NO 4), 900nM BRAF JS E15 Reverse TGGATCCAGACAACTGTTCAA (SEQ ID NO 6), 250nM BRAF AZ E15 Probe GATGGAGTGGGTCCCATCAG (SEQ ID NO 7), and a 1:20 fraction of cDNA. For HERV K, the amplification reactions were performed in a 20ul volume containing 2X Taqman Gene Expression Master Mix, 900nM HERV K Forward ACCCAACAGCTCCGAAGAGA (SEQ ID NO 8), 900nM HERV K Reverse CCCCACATTTCCCCCTTT (SEQ ID NO 9), HERV K Probe CGACCATCGAGAACAG (SEQ ID NO 10), and a 1:20 fraction of cDNA. Amplification conditions were as described in EXAMPLE 1. See FIG. 22B.

### Example 23: Microvesicular RNA can be isolated using a 20nm PES neutral syringe filter

Normal control plasma was obtained from Bioreclamation LLC, as described in EXAMPLE 1.

Isolation of 4mL plasma microvesicular RNA was conducted using ultracentrifugation or 20nm neutral polyethersulfone syringe filtration (Tisch, North Bend, Oh., USA).

The ultracentrifugation sample was prepared, pelleted, and lysed as described in EXAMPLE 1.

In the 20nm neutral polyethersulfone syringe filtration method, one 1mL aliquots of plasma from the same four subjects used in the ultracentrifugation method were pooled and passed through the filter by syringe. The filter was washed with 20mL PBS by syringe. Then, the residual fluid was removed by syringe. For lysis, 700ul Qiazol was applied to the filter and distributed by syringe. The filter was then incubated for 20 sec at room temp and eluted by syringe.

For the ultracentrifugation and filtration samples, microvesicular RNA was isolated, and the quality and concentration were assessed, as described in EXAMPLE 1. See FIG. 23A. cDNA was synthesized as described in EXAMPLE 22.

To compare microvesicular RNA isolated by filtration and ultracentrifugation, RT-PCRs were performed on mRNA and non-coding RNA using primers and probes specific to GAPDH, RN7SL, and RNaseP. For GAPDH and RNaseP, the amplification reactions were performed in a 20ul volume containing 2X Taqman Gene Expression Master Mix (Applied Biosystems, Foster City, Ca., USA), 20X Taqman Gene Expression Assay (Applied Biosystems, Foster City, Ca., USA), and a 1:10 fraction of cDNA. For RN7SL, the amplification reactions were performed in a 20ul volume containing 2X Taqman Gene Expression Master Mix, 900nM RN7SL Forward (SEQ ID NO 1), 900nM RN7SL Reverse (SEQ ID NO 2), 250nM RN7SL Probe (SEQ ID NO 3), and a 1:10 fraction of cDNA. Amplification conditions were as described in EXAMPLE 1. See FIG. 23B.

### Example 24: Isolation of Microvesicular miRNA using a 20nm PES neutral syringe filter (Tisch)

Microvesicular miRNA can be isolated using a 20nm PES neutral syringe filter (Tisch). See FIG. 24.

### Example 25: Effect of RNA on Microvesicle Capture

The microvesicle capture is RNA dependent. Some RNAs are more efficiently captured on the filter compared to others (GAPDH vs. miR-451). This may depend on whether the RNA is protected by proteins and/or microvesicles and on microvesicle size. See FIG. 25.

### Example 26: Isolation of Microvesicular RNA Using Neutral Filters

Microvesicular RNA can be isolated using a 30nm and a 50nm PES neutral syringe filter. See FIG. 26.

Microvesicular RNA can be isolated using a 0.2um PES neutral filter in a spin column format (Pall). See FIG. 27.

Microvesicular RNA can be isolated using a 0.8um PES neutral syringe filter. See FIG. 28.

Microvesicular RNA can be isolated using a 0.8um PES neutral filter in a spin column format (Pall). See FIG. 29.

Microvesicular RNA yield is affected by a lysis buffer type when isolating microvesicles on a neutral PES filter. See FIG. 30.

An additional elution with Qiazol does not significantly improve the RNA yields in the isolation of microvesicular RNA on a 20nm PES neutral syringe filter. See FIG. 31.

Microvesicle stability and/or microvesicular RNA yield is affected by a wash step when isolating microvesicles on a neutral filter. See FIG. 32.

RNA gets stuck on the 20nm PES filter in a syringe format and cannot be easily eluted off. Larger RNA (ex. GAPDH) is harder to elute off than smaller RNA (let-7a). See FIG. 33.

### Example 27: Isolation of Microvesicular RNA Using Beads

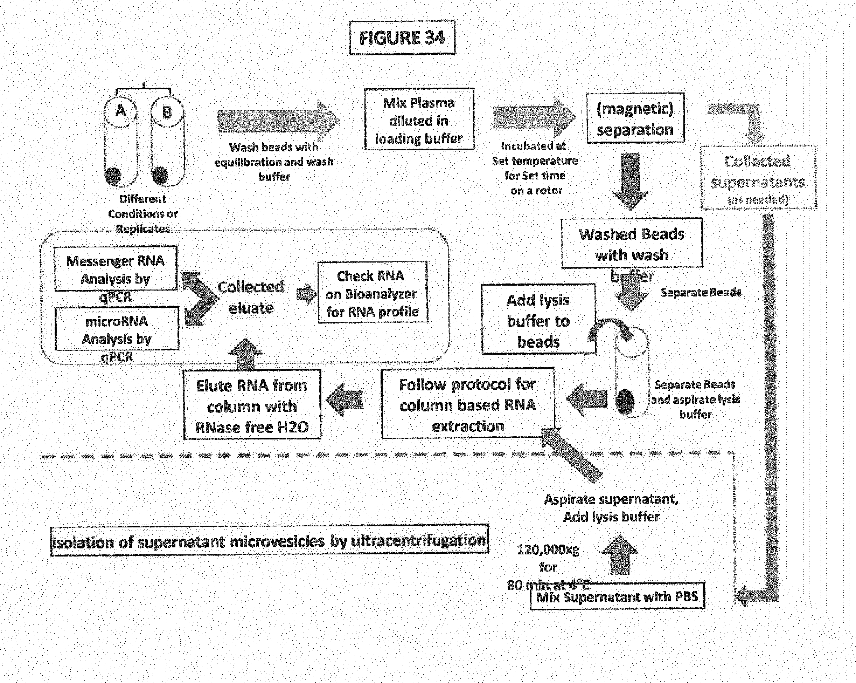

The studies provided herein demonstrate the feasibility of using charged or neutral (magnetic) beads for isolation of microvesicle RNA. These studies demonstrate affinity capture with beads under various different conditions of charges or functional groups, binding and wash buffers, bead loads/concentrations and/or incubation times. The general flow chart for microvesicle isolation with beads and RNA extraction is shown in Figure 34.

*Screening of different types of magnetic beads:* The following four types of magnetic beads were evaluated in the studies described herein:

| **Name of Spherotech Beads** | **Dimension** | **Cat#** | **w/v** | **Amount used in the study** |
|---|---|---|---|---|
| **Dimethylamino magnetic particles** | **3.6 um** | **DM-30-10** | **2.5%** | **5mg (1X 10^8 beads)** |
| **Jeffamines Magnetic Particles** | **3.39 um** | **JAM-30-10** | **2.5%** | **5 mg (1 X 10^8 beads)** |
| **Amino Cross-linked Magnetic Particles** | **1-2 um** | **AMX-10-10** | **2.5%** | **1.5 mg (1 X 10^9 beads)** |
| **Carboxyl Magnetic Particles, Cross-linked** | **1.22 um** | **CMX-10-10** | **2.5%** | **1.5 mg (1 X 10^9 beads** |

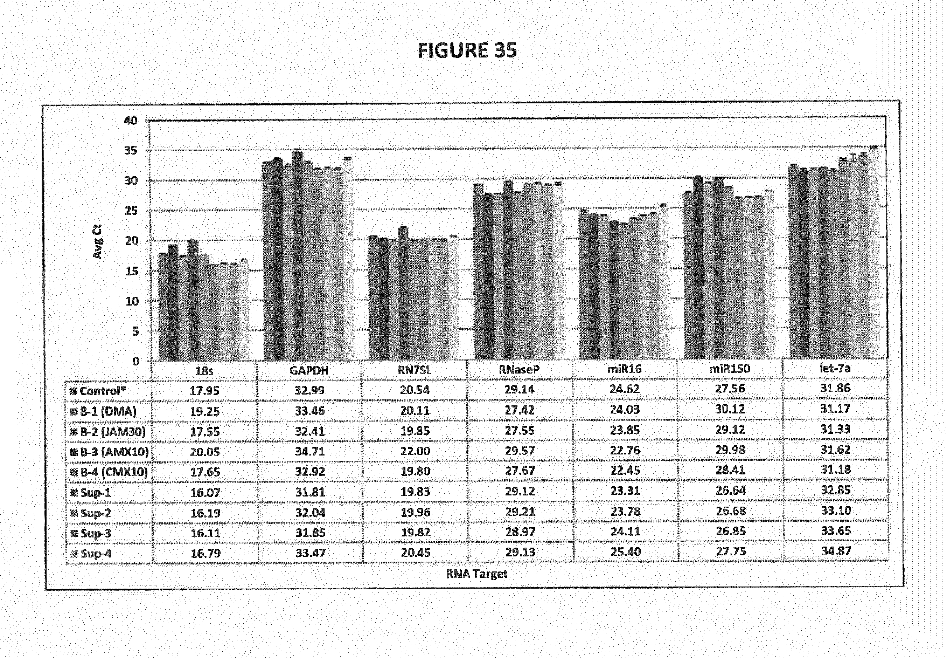

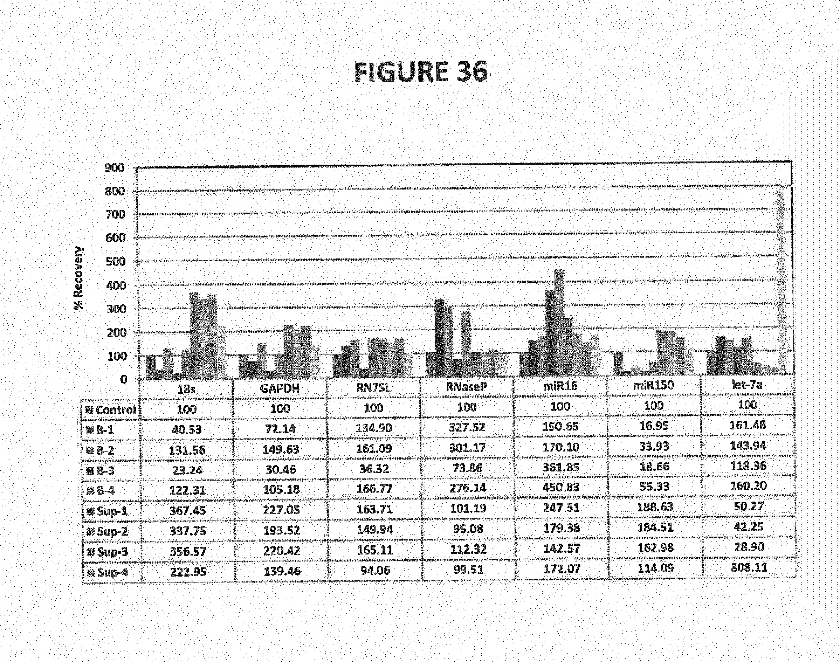

The microvesicle isolation and recovery obtained using each of these types of beads are shown in Figures 35 and 36. Results showed both amine (JAM) functionalized and carboxyl (CMX) functionalized beads captured microvesicles similarly in most targets as evaluated with RT-qPCR.

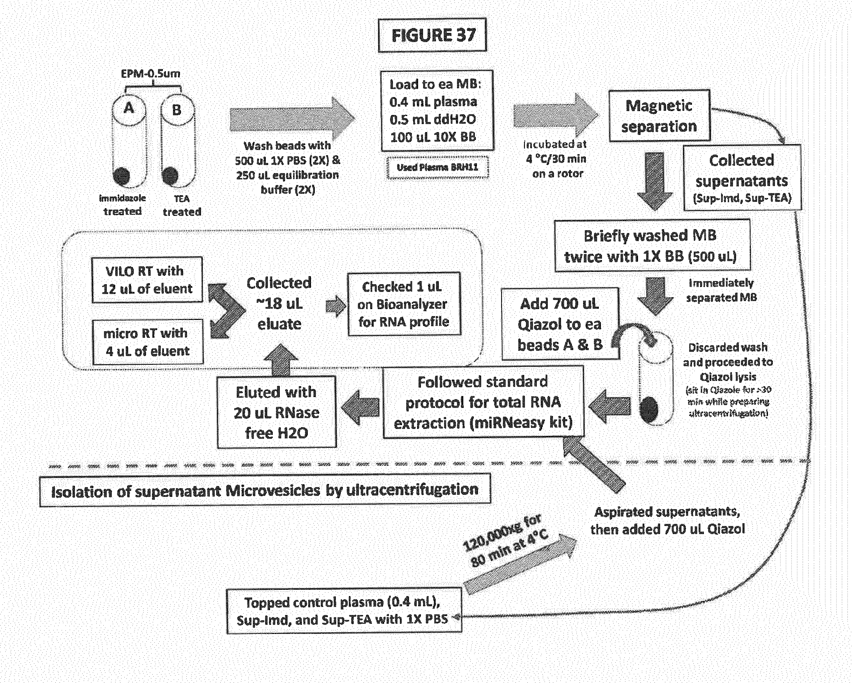

*Microvesicle RNA Isolation with Quaternary Amine Beads:* Epoxy beads were treated with imidazole to generate immidazolium cations or with TEA to produce positively charged quaternary amine with the anticipation that these positively charged amines may capture microvesicles more effectively than the 1° or 2° amines. An overview of the methods used in this study is presented in Figure 37. The following types of amine magnetic beads were evaluated:

| **Name of Beads (Spherotech)** | **Cat #** | **Dimension** | **Estimated beads/mL** | **Total beads #** |
|---|---|---|---|---|
| **Epoxy beads (treated with immidazole in house)** | **EPM-05** | **0.5 um** | **8.5X10¹⁰** | **3.8 X 10⁹** |
| **Epoxy beads (treated with TEA in house)** | **EPM-05** | **0.5 um** | **8.5X10¹⁰** | **3.8 X 10⁹** |

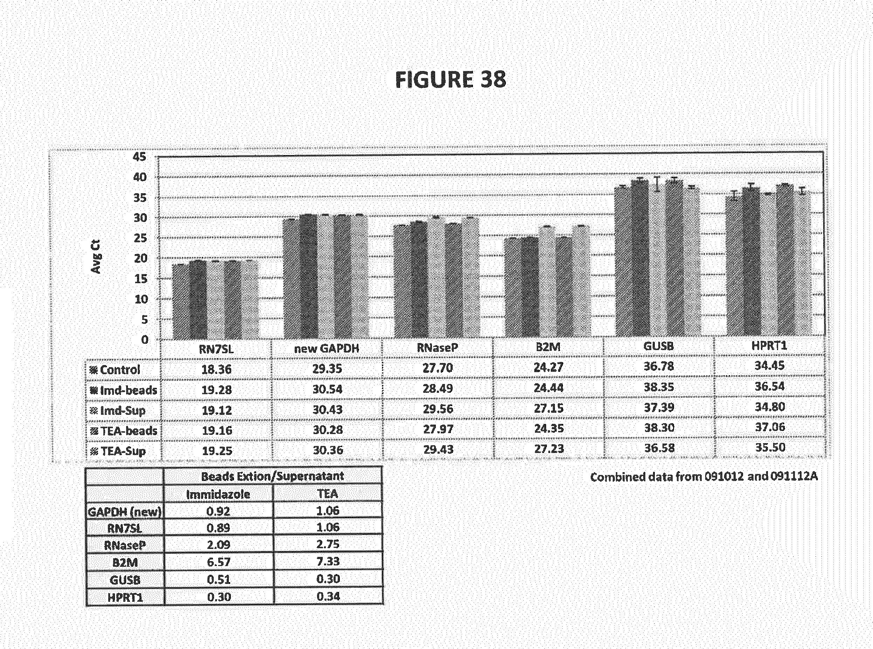

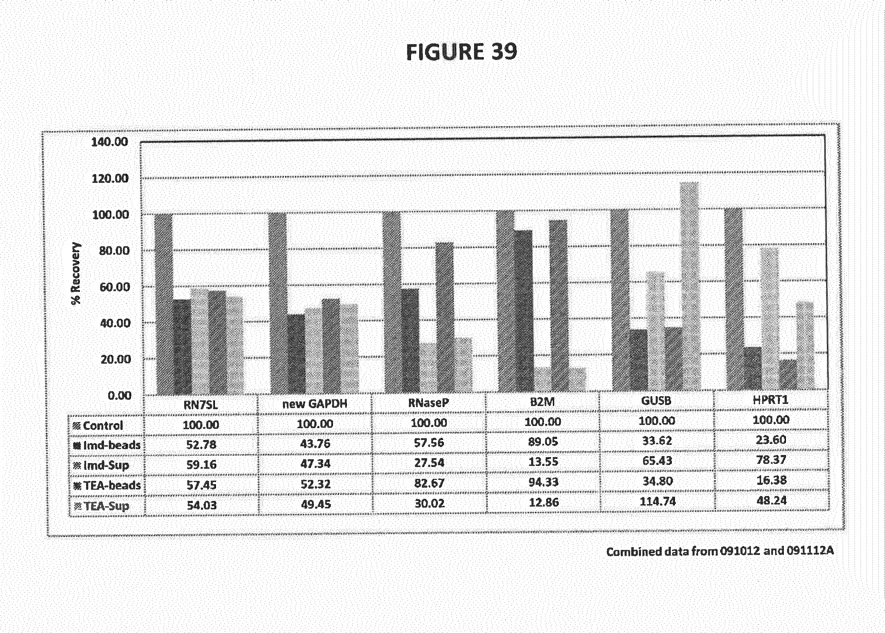

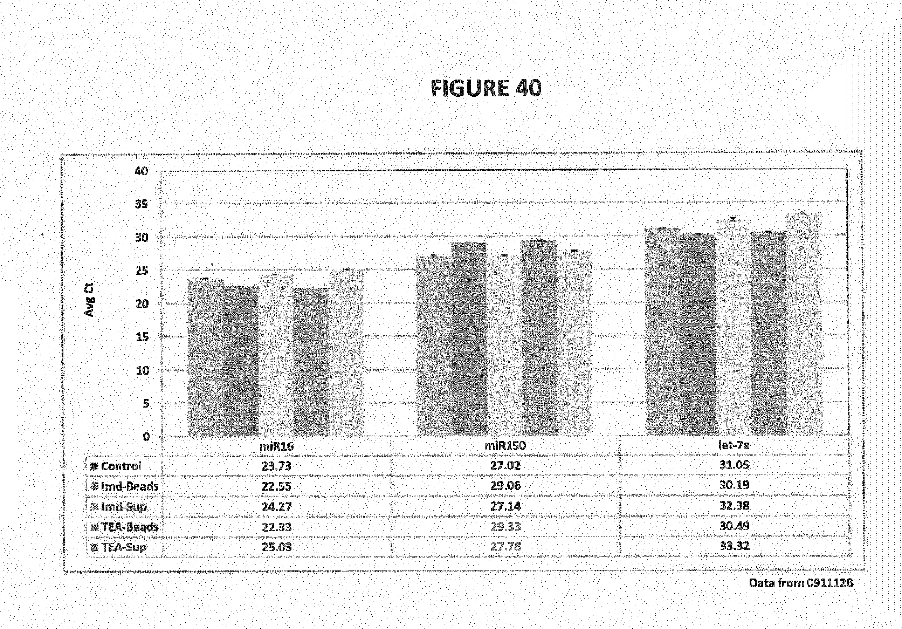

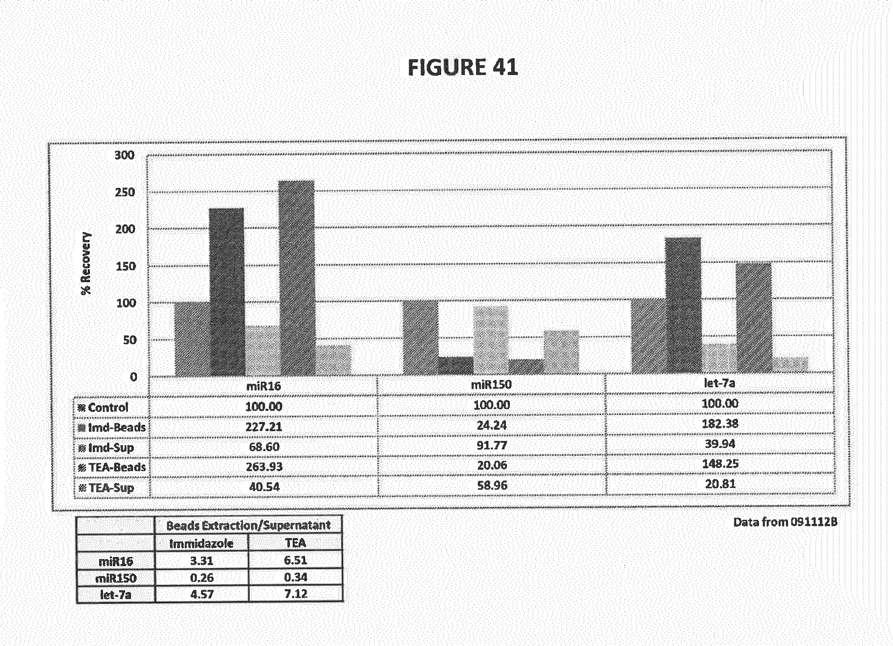

Microvesicle capture was evaluated using RT-qPCR. Briefly, the following steps were performed: lysis of captured microvesicles and extraction of total RNA by Qiazole method; purification of total RNA with Qiagen miRNeasy kit/MinElute column (20 uL); checked RNA profile on Bioanalyzer (1 uL); RT reaction with VILO RT kit (12uL) and Micro RT kit (4 uL); Real time PCR with 2 uL of cDNA on the following targets: VILO products (GAPDH (new assay kits), RN7SL, RNaseP, B2M, GUSB, HPRT1) and Micro RT products (miR16, miR150, Let-7a). The microvesicle isolation results tested using selected mRNA targets are shown in Figure 38, and the recovery of the selected mRNA targets from the isolated microvesicles is shown in Figure 39. The microvesicle isolation results tested using selected micro RNA targets are shown in Figure 40, and the recovery of the selected micro RNA targets from the isolated microvesicles is shown in Figure 41.

*Reproducibility Studies:* The reproducibility of microvesicle isolation using TEA treated beads was evaluated in the studies described herein. Briefly, triplicates of microvesicle isolation were performed with TEA Beads, 0.4 mL plasma pool BH11, qiazole lysis and total RNA extraction (miRNeasy kit). A summary of the results is shown in the table below:
Data Summary: Triplicates of Microvesicle Isolation with TEA Beads

| | **Avg Ct** | | | **%CV** | | |
|---|---|---|---|---|---|---|
| | **UC Control** | **TEA Beads** | **Supernatant** | **UC Control** | **(TEA Beads** | **Supernatant** |
| **RN7SL** | **18.39** | **19.08** | **18.90** | **0.21** | **0.41** | **1.62** |
| **GAPDH** | **29.20** | **30.23** | **29.76** | **0.73** | **0.21** | **1.78** |
| **RNaseP** | **27.51** | **27.56** | **28.52** | **0.98** | **1.30** | **2.80** |
| **B2M** | **23.80** | **24.65** | **27.70** | **2.79** | **9.05** | **17.57** |
| **GUSB** | **36 .11** | **38.37** | **36.00** | **2.62** | **0.26** | **1.41** |
| **HPRT** | **34.26** | **36.95** | **34.28** | **0.81** | **0.35** | **3.09** |
| **miR16** | **23.27** | **21.63** | **24.00** | **2.80** | **2.82** | **3.72** |
| **miR150** | **26.75** | **29.62** | **27.22** | **1.40** | **0.86** | **1.78** |
| **let-7a** | **30.23** | **29.89** | **32.10** | **3.83** | **1.84** | **3.40** |

Preliminary results showed that TEA beads appeared to be 1 to 2 fold better than imidazole beads in microvesicle isolation. RT-qPCR results showed a gene dependent pattern in the distribution of microvesicle RNA between supernatants and beads extraction:
- GAPDH and RN7SL: close to 1:1 distribution
- RNaseP: 2.1 to 2.8 X in beads > in supernatant
- B2M: 6.6 to 7.3X in beads > in supernatant
- miR16: 3.3 to 6.5X in beads > in supernatant
- Let-7a: 4.6 to 7.1X in beads > in supernatant
- GUSB: 2 to 3 X in supernatant > in beads
- HPRT1: 3X in supernatant > in beads
- miR150: 3 to 4X in supernatant > in beads

Without intending to be bound by theory, the data suggest that there might be multiple classes of microvesicles bearing different surface (membrane) characteristics such as charge, polarity, etc.. In addition to its membrane nature, each type of microvesicle may carry different RNA expression patterns as well.

*Confirmation of Microvesicle Isolation with Other Beads:* The studies described herein were designed to confirm microvesicle isolation with other quaternary amine or sulfonate functionalized non-magnetic ionic exchange beads. The following beads were evaluated: Commercially manufactured (Hamilton); Quaternary Amine (Cationic) Resin: PRP-X400 and Sulfonate (Anionic exchange) Resin: RCX30. The characteristics of each bead type are summarized in the tables below:
Cation Exchange Resin Tested

| **Product name (Hamilton)** | **Dimension** | **Hamilton Cat#** | **Stock (w/v)** | **Amount used in the study** |
|---|---|---|---|---|
| **PRP-X400 (sulfonate)** | **12-20 um** | **79591** | **0.2g/2mL** | **20 mg** |
| **PRP-X400 (Sulfonate)** | **12-20 um** | **79591** | **0.2g/2mL** | **50 mg** |

Anion Exchange Resin Tested

| **Product name (Hamilton)** | **Dimension** | **Hamilton Cat#** | **w/v** | **Amount used in the study** |
|---|---|---|---|---|
| **RCX-30 (Quarternary amine)** | **7 um** | **79706** | **0.2g/2mL** | **8 mg** |
| **RCX-30 (Quarternary amine)** | **7 um** | **79706** | **0.2g/2mL** | **20 mg** |

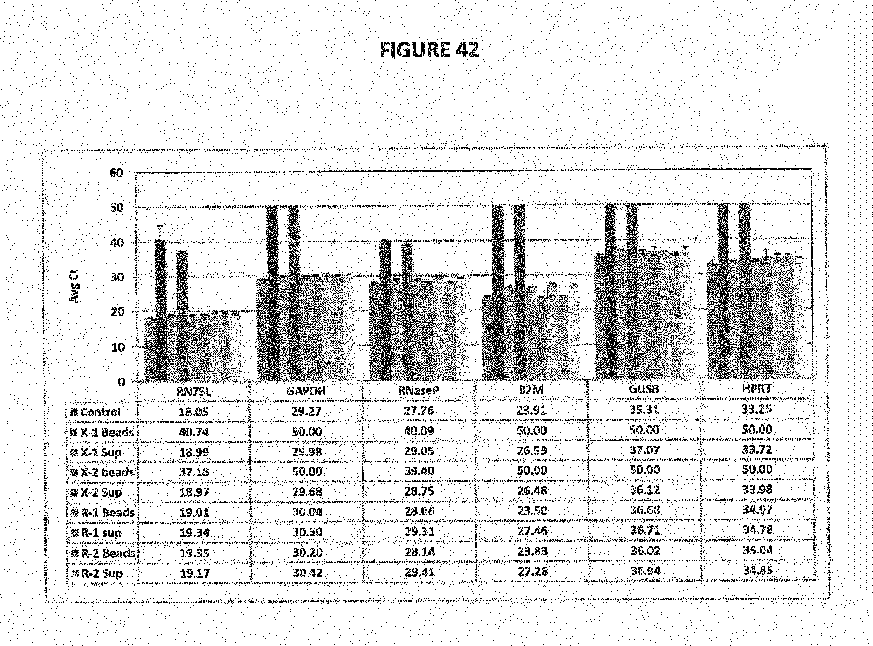

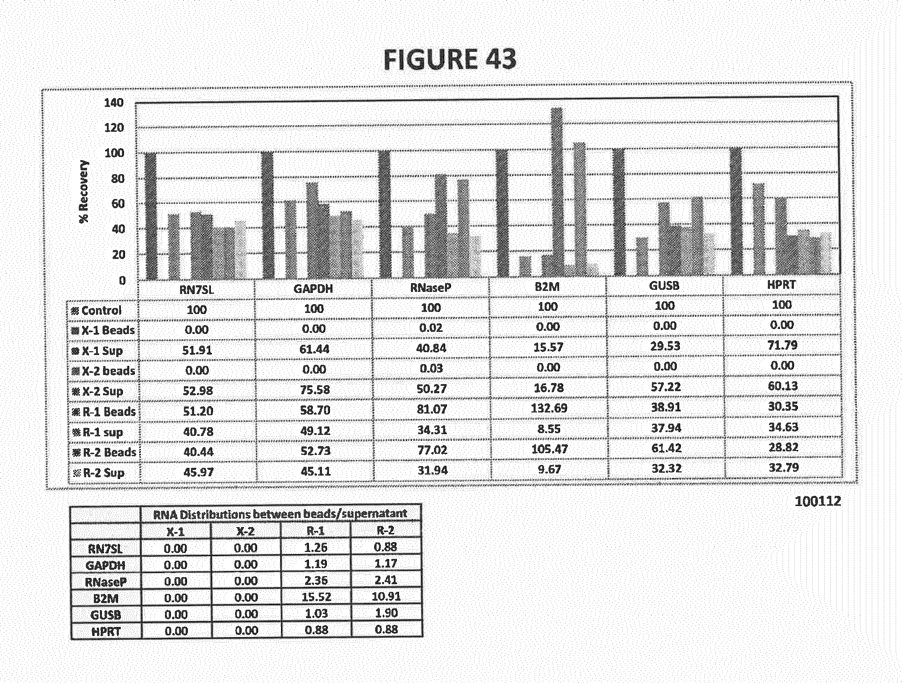

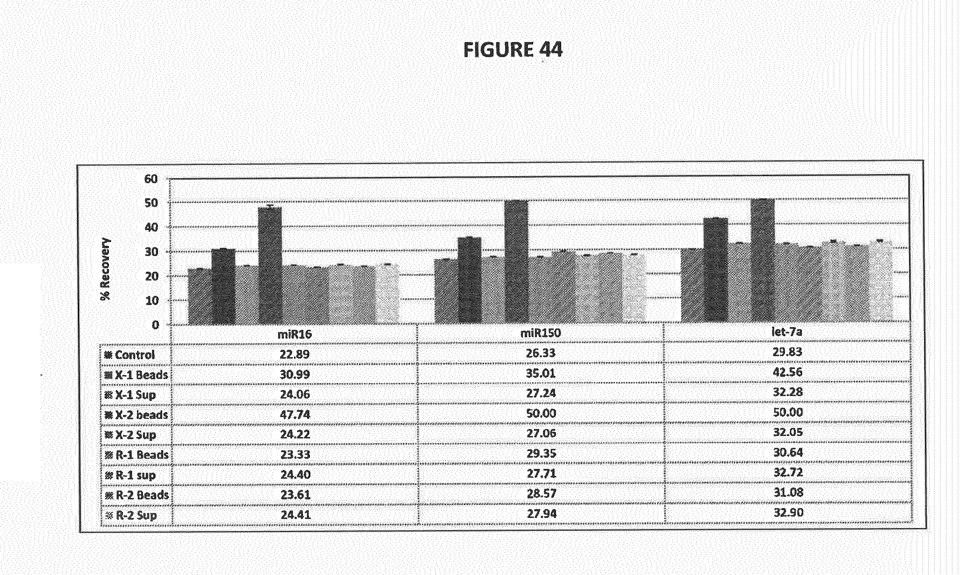

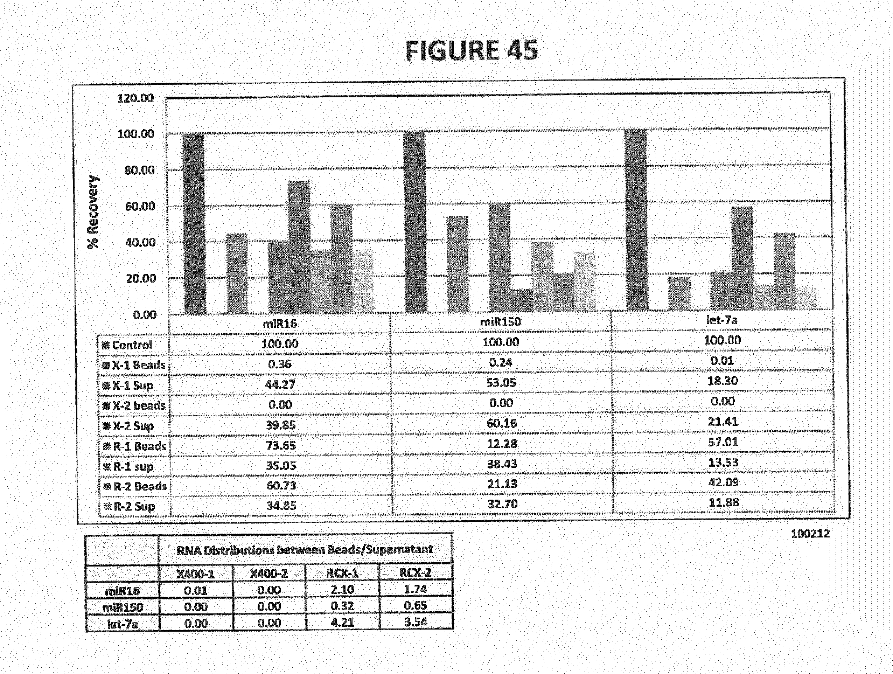

The microvesicle isolation results with non-magnetic beads using selected mRNA targets are shown in Figure 42, where X is cationic and R is anionic. The recovery of these selected mRNA targets is shown in Figure 43. The microvesicle isolation results with non-magnetic beads using selected micro RNA targets are shown in Figure 44, where X is cationic and R is anionic. The recovery of these selected micro RNA targets is shown in Figure 45.

In summary, results showed that microvesicles were not detected from cationic exchange resin as analyzed from RT-qPCR on selected mRNA and micro RNA targets. Anionic exchange resin captures microvesicles similarly to TEA treated magnetic beads on the tested RNA targets. Gene dependent pattern in the distribution of microvesicle RNA between supernatants and beads extraction was observed:
- GAPDH, GUSB, RN7SL, HPRT: close to 1:1 distribution
- RNaseP: 2.5X in beads > in supernatant
- B2M: 10-15X in beads > in supernatant
- miR16: ∼2X in beads > in supernatant
- Let-7a: ∼3.5X in beads > in supernatant
- miR 150: 3 to 4X in supernatant > in beads

*Evaluation of microvesicle biding to selected control beads:* In the studies described herein, the background level of binding to various control beads was evaluated. In these studies, untreated epoxy beads and unfunctionalized polysterene beads were used. In particular, the following beads were tested:
Control Beads Tested:

| **Name of Beads (Spherotech)** | **Cat #** | **Dimension** | **Estimated beads/mL** | **Beads # included** |
|---|---|---|---|---|
| **Epoxy beads (B-Epoxy)** | **EPM-05** | **0.5 um** | **8.5X10¹⁰** | **3.8 X 10⁹** |
| **Polystyrene beads (B-PM)** | **PM-20** | **2.49 um** | **8.5X10¹⁰** | **3.8 X 10⁹** |

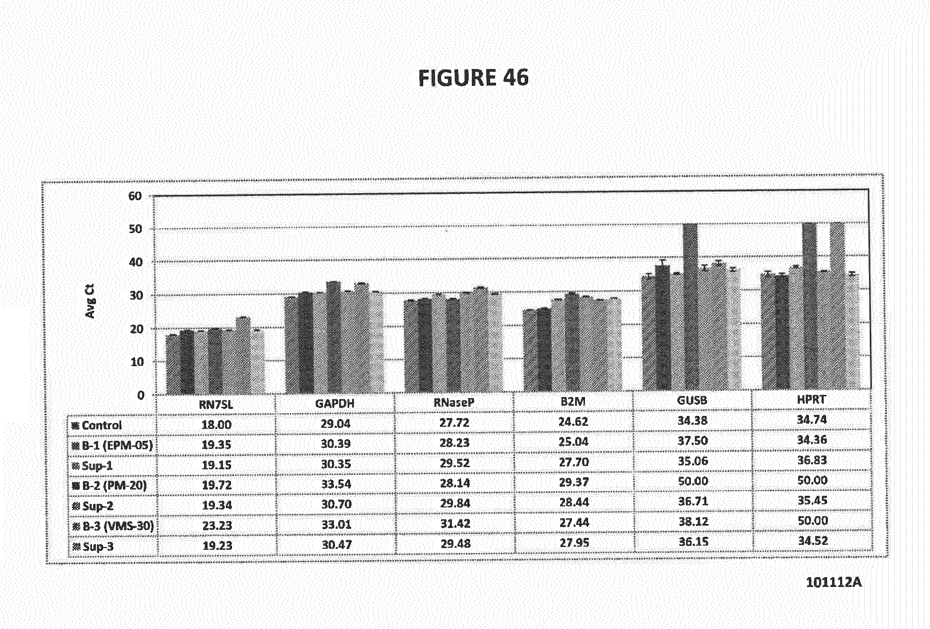

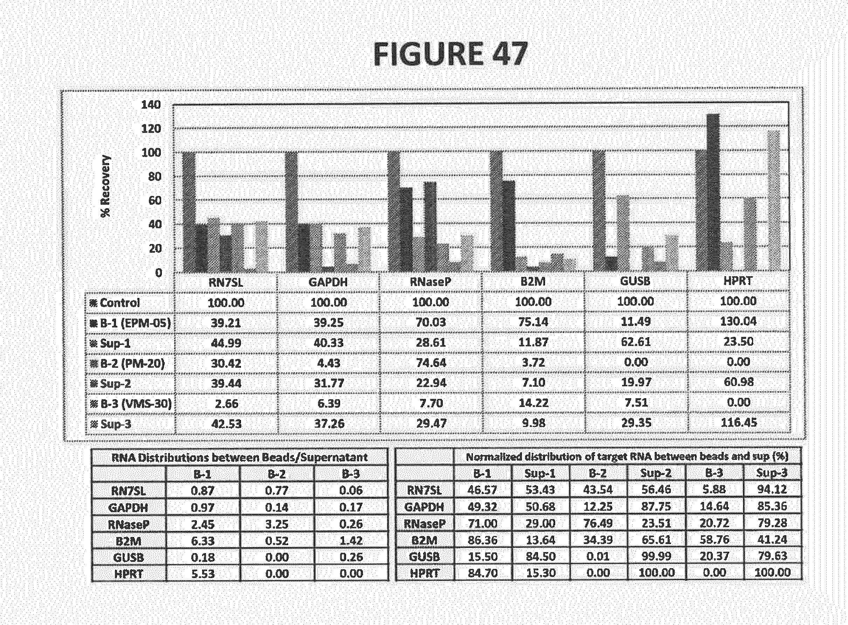

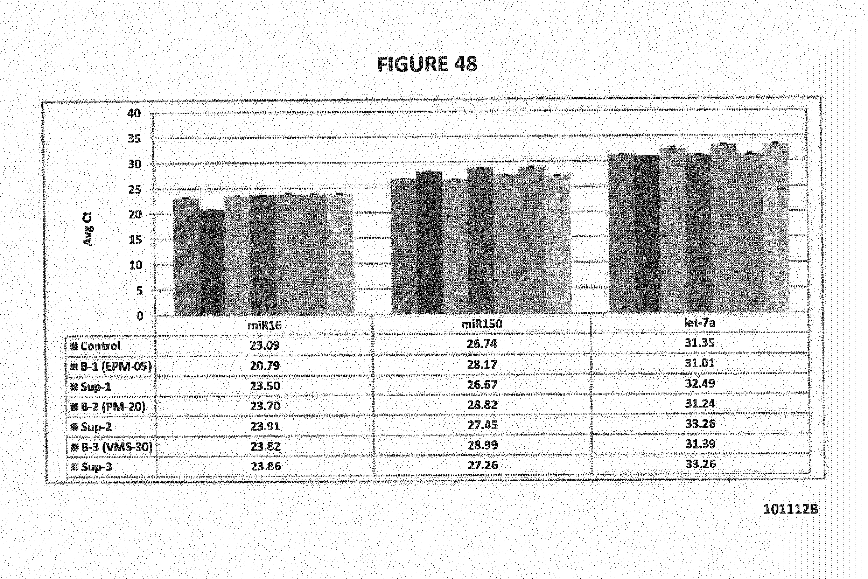

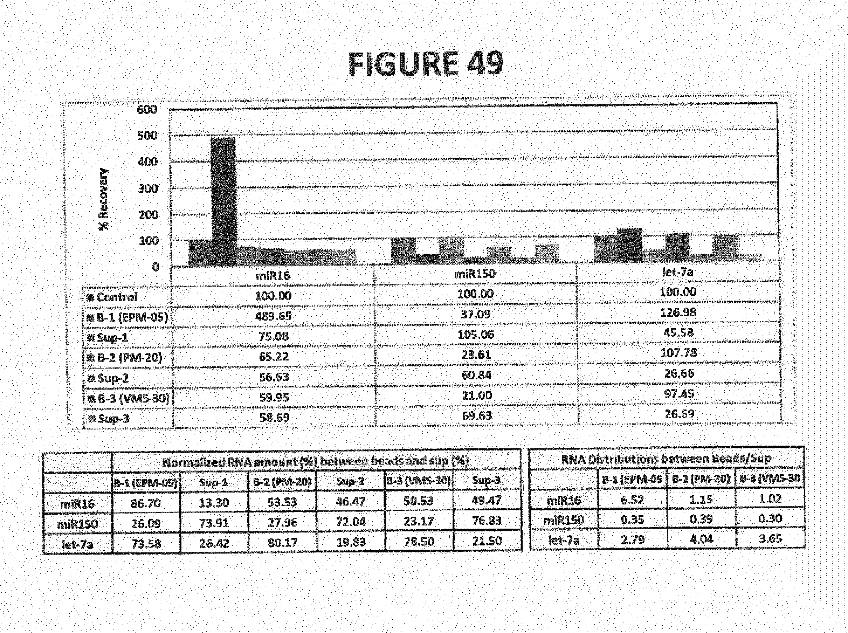

The microvesicle isolation results with control beads using selected mRNA targets are shown in Figure 46, and the recovery of these selected mRNA targets is shown in Figure 47. The microvesicle isolation results with control using selected micro RNA targets are shown in Figure 48, and the recovery of these selected micro RNA targets is shown in Figure 49.

In summary, similar microvesicle isolation pattern was observed with both anion exchangers (Hamilton TEA resin and in-house TEA modified beads). Sulfonate exchanger (cation exchange resin) showed very poor microvesicle recovery as evaluated via RT-qPCR. Without intending to be bound by theory, this could be due to poor microvesicle capturing and/or RT-qPCR inhibitors leached out from cation resin. However, a preliminary study with carboxyl beads (cationic) did not show such effects as sulfonate resin; thus, it may not be a general issue of cation exchangers Results showed a gene dependent pattern in the distribution of microvesicle RNA between supernatants and beads extraction. Again, without intending to be bound by theory, the data suggest that there might be multiple classes of microvesicles bearing different surface (membrane) characteristics such as charge, polarity, proteins (antigens), carbohydrates, etc.. The observed differential RNA expression may indirectly associate to their microvesicle origins.

### Example 28: Effect of Bead Titration of Capturing Efficiency and RNA Yield

In the studies described herein, four types of beads were evaluated using a fixed plasma volume (0.4 mL) and an increasing amount of beads to expand the beads to microvesicle ratio (also referred to herein as B:E ratio) from 0.05:1 to 20:1. The following table presents the characteristics of the magnetic beads used for microvesicle RNA isolation in these studies:

| **Beads Label** | **Name of Beads (Spherotech)** | **Manufacturer** | **Surface Character** | **Cat #** | **Dim ension** | **Estimated Beads/mL** | **uL of beads added** | **Beads # included** | **SA Ratio** |
|---|---|---|---|---|---|---|---|---|---|
| **A** | **TEA treated beads** | **Spherotech** | **Hydrophobic/ 4° amine** | **EPM-05** | **0.5 um** | **8.5 X 10¹⁰** | **45 uL (1 mg)** | **3.8 X 10⁹** | **2.5:1** |
| **B** | **Epoxy beads** | **Spherotech** | **Hydrophilic/ Reactive** | **EPM-05** | **0.5 um** | **2.0 X 10¹¹** | **20 uL (1 mg)** | **4.0 X 10⁹** | **2.5:1** |
| **C** | **Polystyrene beads** | **Spherotech** | **Hydrophobic** | **PM-20** | **2.49 um** | **1.6 X 10⁹** | **200 uL (5 mg)** | **3.2 X 10⁸** | **5:1** |
| **D** | **MyOne Tosylactivated** | **Life Tech (Dynabeads)** | **Hydrophobic/ Reactive** | **655.01D** | **1 um** | **1.0 X 10¹¹** | **40 uL (4 mg)** | **8.0 X 10⁹** | **10:1** |

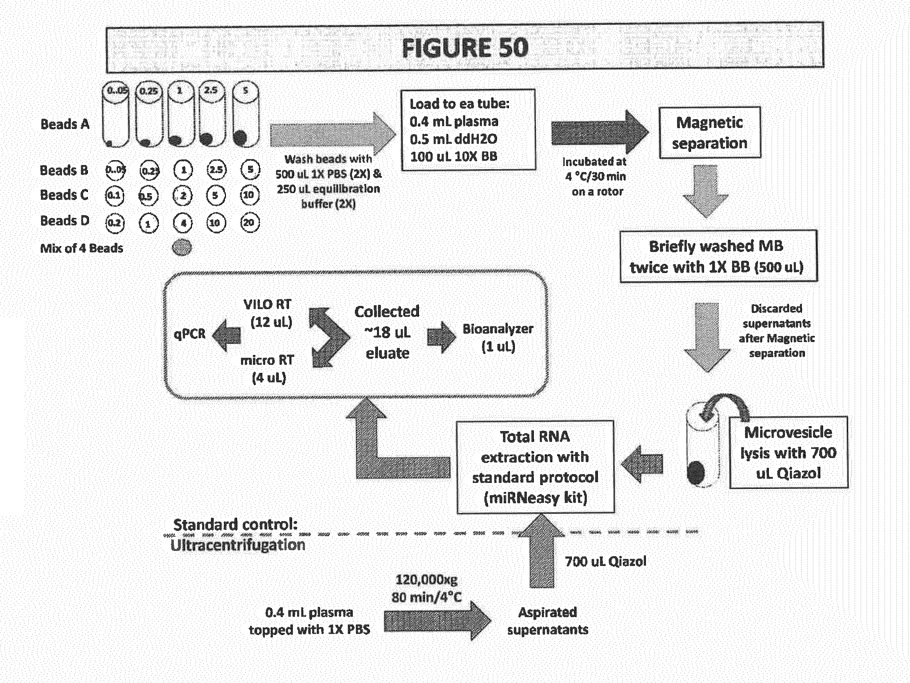

Titrations of Beads in this study give a B/E (= ratio of bead SA to cut SA of microvesicle) from 0.05:1, 0.25:1, 1:1, 2.5:1 to 5:1 for beads A & B; from 0.1:1,0.5:1, 2:1, 5:1, to 10:1 for beads C; and from 0.2:1, 1:1, 4:1, 10:1, to 20:1 for beads D. The standard control was ultracentrifugation with 0.4 mL plasma. An overview of the methods used in this study is shown in Figure 50.

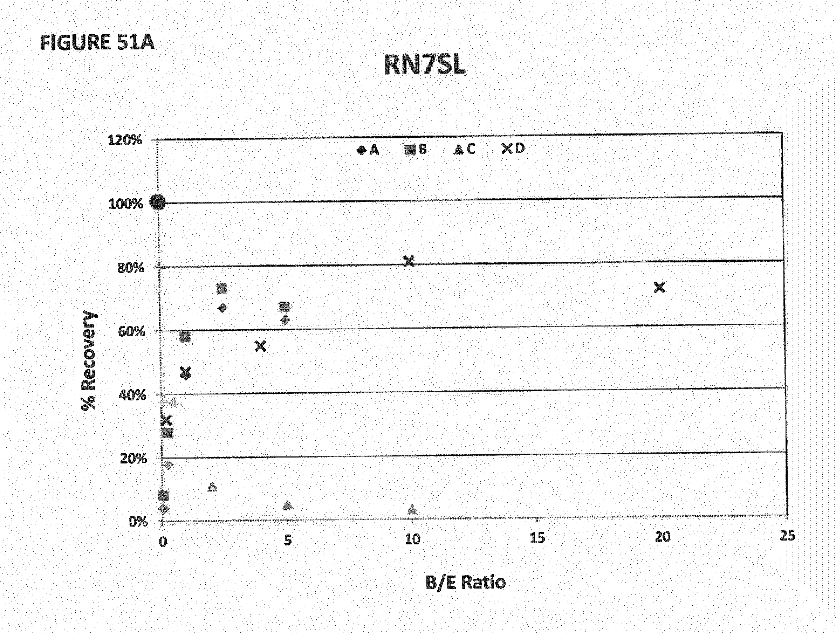

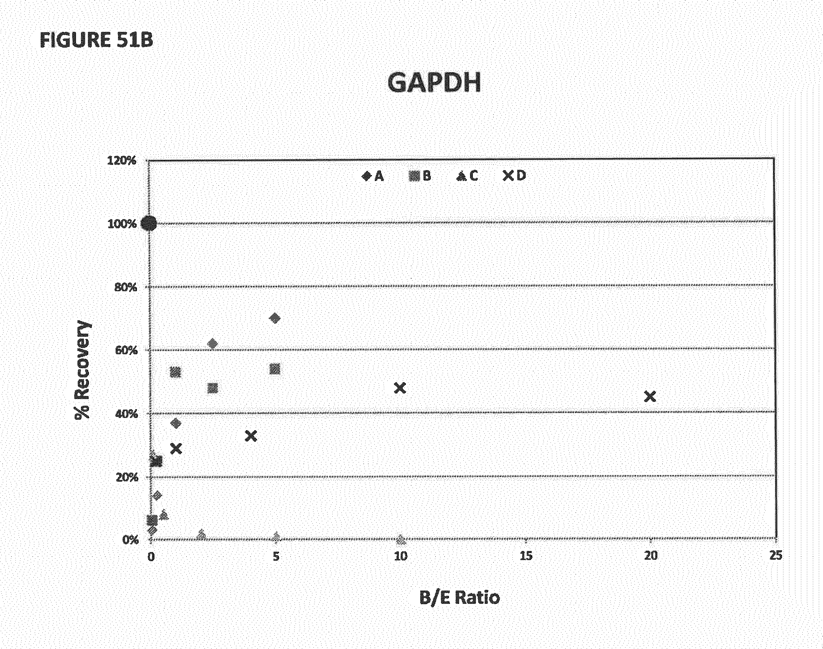

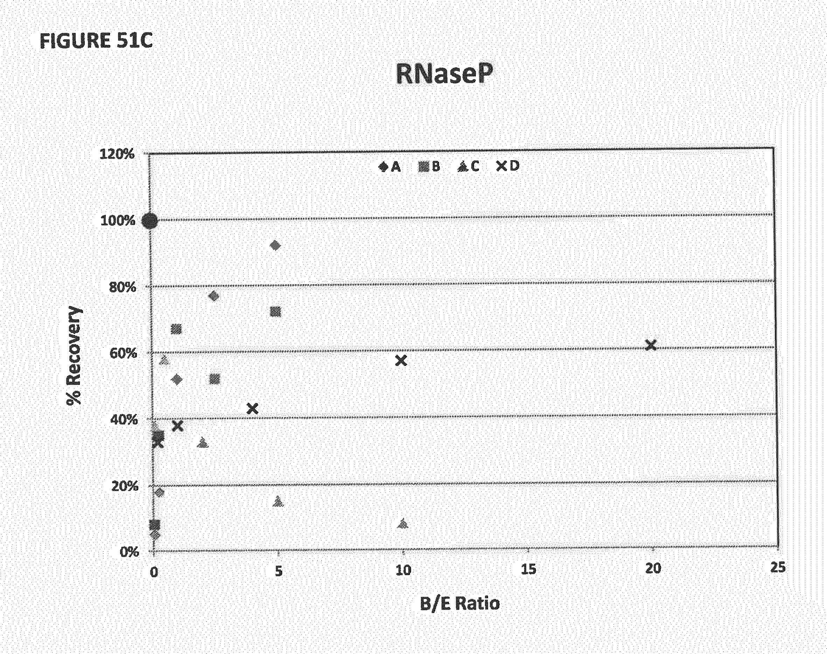

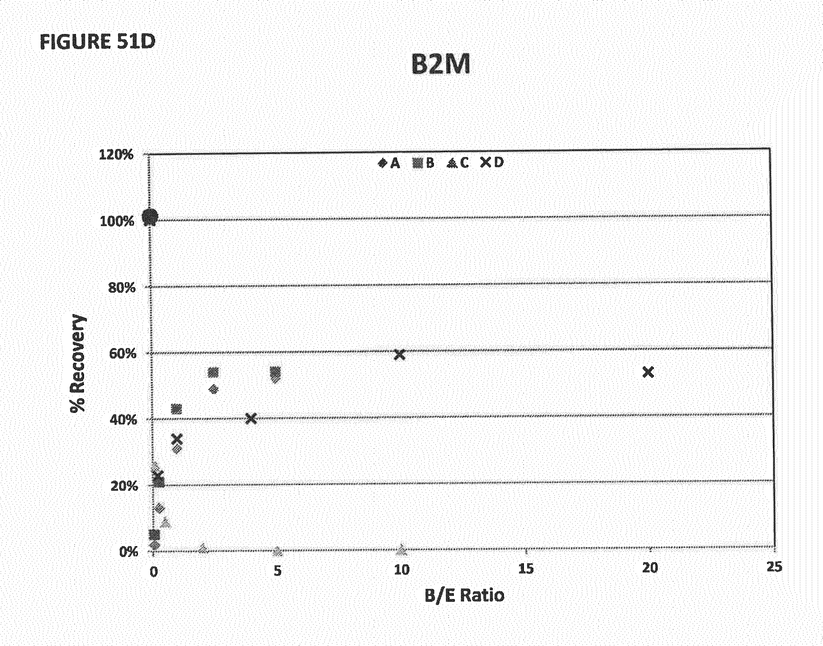

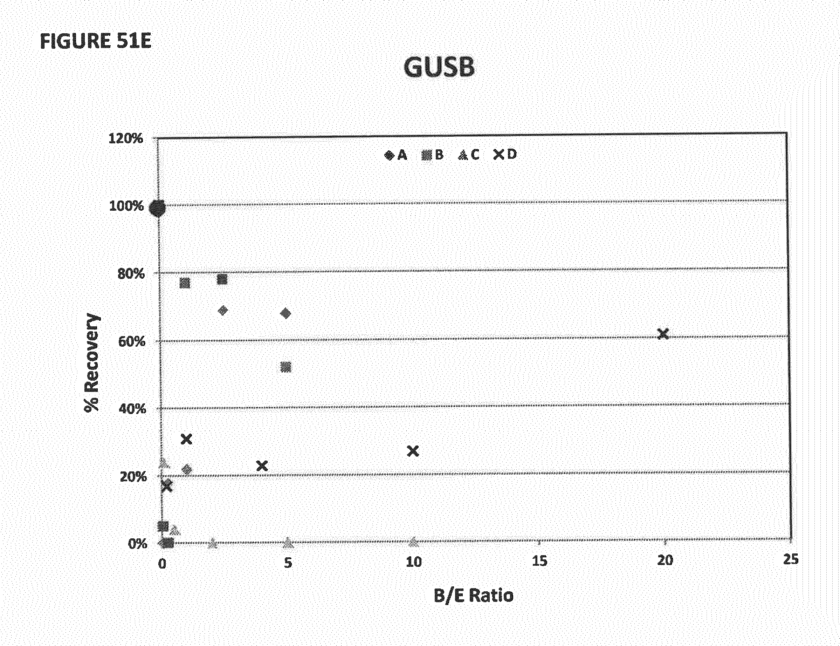

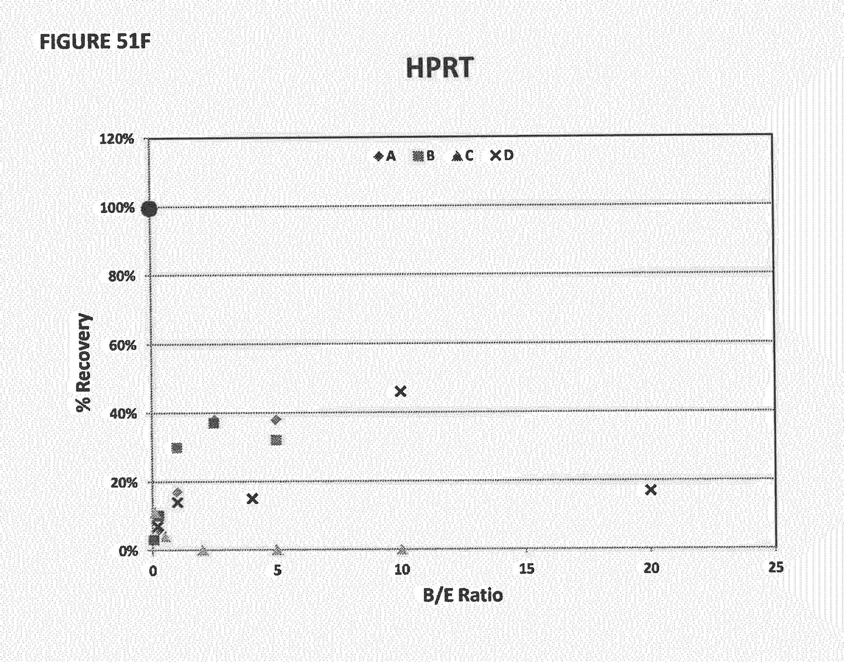

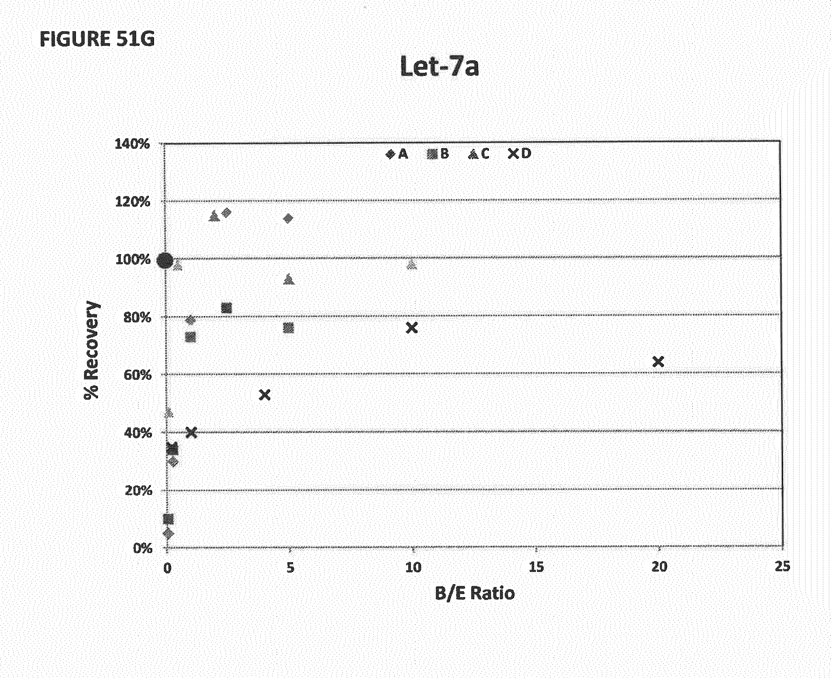

Evaluation of microvesicle capturing was performed via RT-qPCR. Real time PCR was performed on the following 7 targets and recovery calculated using ultracentrifugation results as 100%: VILO products (GAPDH, RN7SL, RNaseP, B2M, GUSB and HPRT1) and Micro RT products (Let-7a). The percent recovery as a function of B:E ratio for each target is shown in Figures 51A-51G.

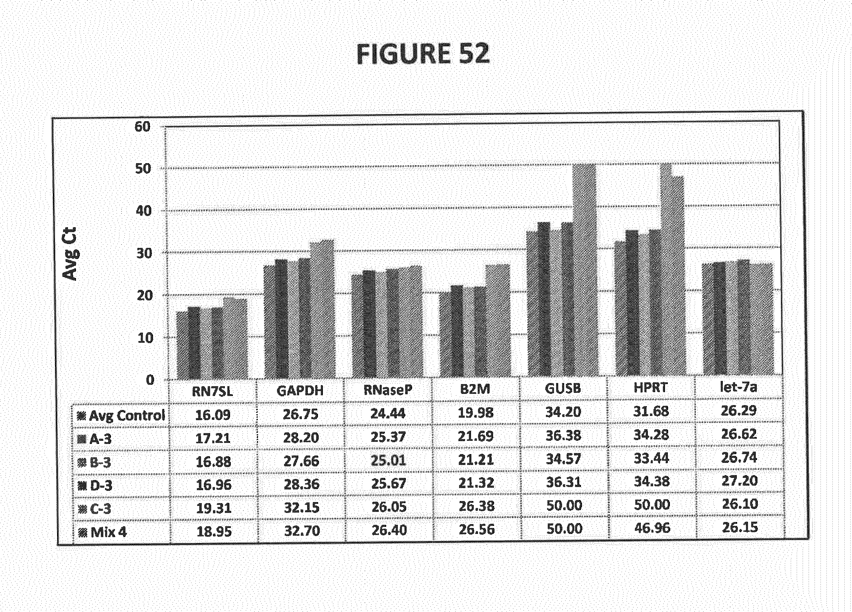

A Ct comparison between individual and mixed beads is shown in Figure 52, where the beads/microvesicle ratio was 2:1 for all targets. Ct values from mixed beads was similar to bead C (PM), i.e., significantly higher than individual beads, except for let-7a target. Without intending to be bound by theory, this is most likely an inhibitory effect of bead C.

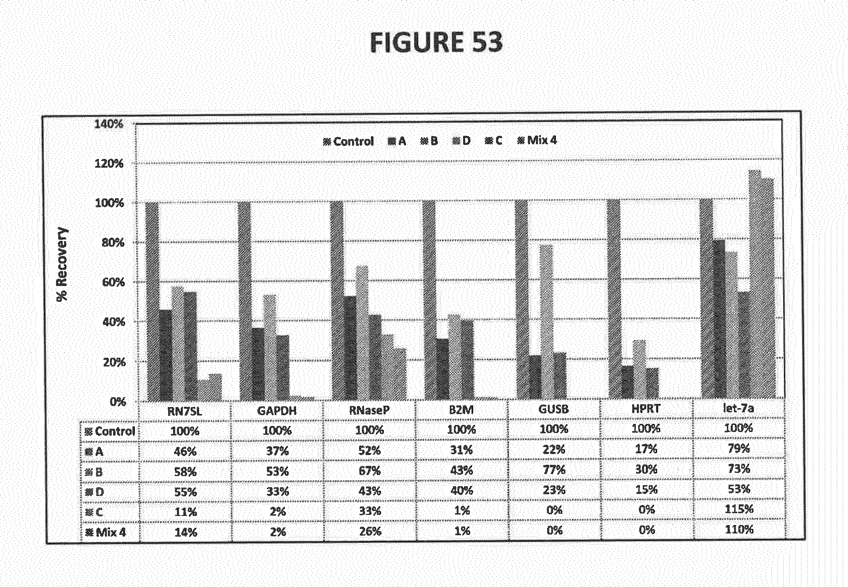

A recovery comparison between individual and mixed beads is shown in Figure 53, where the beads/microvesicle ratio was 2:1 for all targets. The % recovery from mixed beads was similar to bead C, i.e., significantly lower than individual beads for all targets, except let-7a. Without intending to be bound by theory, the poor recovery with mixed beads is most likely an inhibitory effect from bead C. A summary of the % recovery for each target is shown in the table below:

| Target | Beads | B/E ratio | | | | | | | | | | | | |
|---|---|---|---|---|---|---|---|---|---|---|---|---|---|---|
| | | 0 | 0.05 | 0.1 | 0.2 | 0.25 | 0.5 | 1 | 2 | 2.5 | 4 | 5 | 10 | 20 |
| RN7SL | A | 100% | 4% | | | 18% | | 46% | | 67% | | 63% | | |
| | B | 100% | 8% | | | 28% | | 58% | | 73% | | 67% | | |
| | C | 100% | | 39% | | | 38% | | 11% | | | 5% | 3% | |
| | D | 100% | | | 32% | | | 47% | | | 55% | | 81% | 72% |
| GAPDH | | 0 | 0.05 | 0.1 | 0.2 | 0.25 | 0.5 | 1 | 2 | 2.5 | 4 | 5 | 10 | 20 |
| | A | 100% | 3% | | | 14% | | 37% | | 62% | | 70% | | |
| | B | 100% | 6% | | | 25% | | 53% | | 48% | | 54% | | |
| | C | 100% | | 27% | | | 8% | | 2% | | | 1% | 0% | |
| | D | 100% | | | 25% | | | 29% | | | 33% | | 48% | 45% |
| RNaseP | | 0 | 0.05 | 0.1 | 0.2 | 0.25 | 0.5 | 1 | 2 | 2.5 | 4 | 5 | 10 | 20 |
| | A | 100% | 5% | | | 18% | | 52% | | 77% | | 92% | | |
| | B | 100% | 8% | | | 35% | | 67% | | 52% | | 72% | | |
| | C | 100% | | 38% | | | 58% | | 33% | | | 15% | 8% | |
| | D | 100% | | | 33% | | | 38% | | | 43% | | 57% | 61% |
| B2M | | 0 | 0.05 | 0.1 | 0.2 | 0.25 | 0.5 | 1 | 2 | 2.5 | 4 | 5 | 10 | 20 |
| | A | 100% | 2% | | | 13% | | 31% | | 49% | | 52% | | |
| | B | 100% | 5% | | | 21% | | 43% | | 54% | | 54% | | |
| | C | 100% | | 26% | | | 9% | | 1% | | | 0% | 0% | |
| | D | 100% | | | 23% | | | 34% | | | 40% | | 59% | 53% |
| GUSB | | 0 | 0.05 | 0.1 | 0.2 | 0.25 | 0.5 | 1 | 2 | 2.5 | 4 | 5 | 10 | 20 |
| | A | 100% | 0% | | | 18% | | 22% | | 69% | | 68% | | |
| | B | 100% | 5% | | | 0% | | 77% | | 78% | | 52% | | |
| | C | 100% | | 24% | | | 4% | | 0% | | | 0% | 0% | |
| | D | 100% | | | 17% | | | 31% | | | 23% | | 27% | 61% |
| HPRT | | 0 | 0.05 | 0.1 | 0.2 | 0.25 | 0.5 | 1 | 2 | 2.5 | 4 | 5 | 10 | 20 |
| | A | 100% | 3% | | | 6% | | 17% | | 38% | | 38% | | |
| | B | 100% | 3% | | | 10% | | 30% | | 37% | | 32% | | |
| | C | 100% | | 11% | | | 4% | | 0% | | | 0% | 0% | |
| | D | 100% | | | 7% | | | 14% | | | 15% | | 46% | 17% |
| let-7a | | 0 | 0.05 | 0.1 | 0.2 | 0.25 | 0.5 | 1 | 2 | 2.5 | 4 | 5 | 10 | 20 |
| | A | 100% | 5% | | | 30% | | 79% | | 116% | | 114% | | |
| | B | 100% | 10% | | | 34% | | 73% | | 83% | | 76% | | |
| | C | 100% | | 47% | | | 98% | | 115% | | | 93% | 98% | |
| | D | 100% | | | 35% | | | 40% | | | 53% | | 76% | 64% |

In summary, bead titration studies on selected RNA targets showed that a 2.5:1 ratio of bead/microvesicles as chosen in previous studies (tested with beads A & B), achieves a saturation level for most genes (except RNAseP, GAPDH). Results showed microvesicles are captured by beads with different surface characteristics (charged, neutral, hydrophobic, etc.). Without intending to be bound by theory, this agrees with the previous hypothesis: there might be multiple classes of microvesicles bearing different surface (membrane) characteristics such as charge, polarity, etc. In addition to its membrane nature, each type of microvesicle may carry different RNA expression patterns as well.

Bead C (PM) appeared inhibitory to all targets tested except let-7a. The inhibition could be at RT level (microRT kit for let-7a) or at microvesicle capturing. Mix of A, B, C, and D beads performed similarly to bead C, suggesting that inhibition was inherited from bead C. This also reduces the possibility of inhibition acting at microvesicle capture. The source of inhibition can be confirmed by simply eliminating C from the mix of 4 beads.

### Example 29: Titrations of Plasma Volume using a Fixed Beads to Microvesicle Ratio

In the studies described herein, plasma titration was evaluated using four types of beads to evaluate microvesicle recovery at a fixed bead:microvesicle ratio. The characteristics of the beads used are shown in the table below:

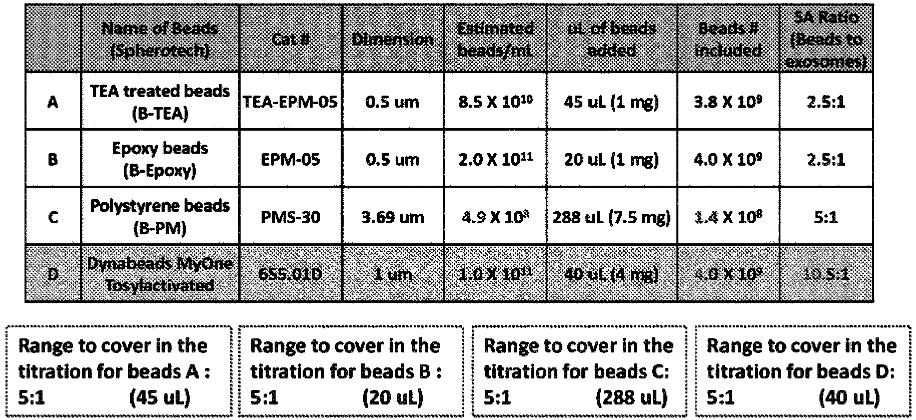

Ultracentrifugation with 0.4, 1, 4 mL plasma in 1X PBS were used as the controls. The bead aliquots were prepared as shown in the table below:

| | mL of plasma pool | 0.4 | 1 | 4 |
|---|---|---|---|---|
| At 5:1 ratio of SA Beads/Exosome | uL of Beads A | 45 (2.5:1) | 112.5* | 450 |
| | uL of Beads B | 20 (2.5:1) | 50* | 200 |
| | uL of Beads C | 288 (5:1) | 720 | |
| | uL of Beads D | 40 (10:1) | 100 | 400 |
| | Mix of A, B, C, D | 393 | | |

| | | | | |
|---|---|---|---|---|
| *Note: Tubes for beads A-1 mL and beads B-1 mL fell from the magnet separator, which resulted in some beads loss in both samples | | | | |

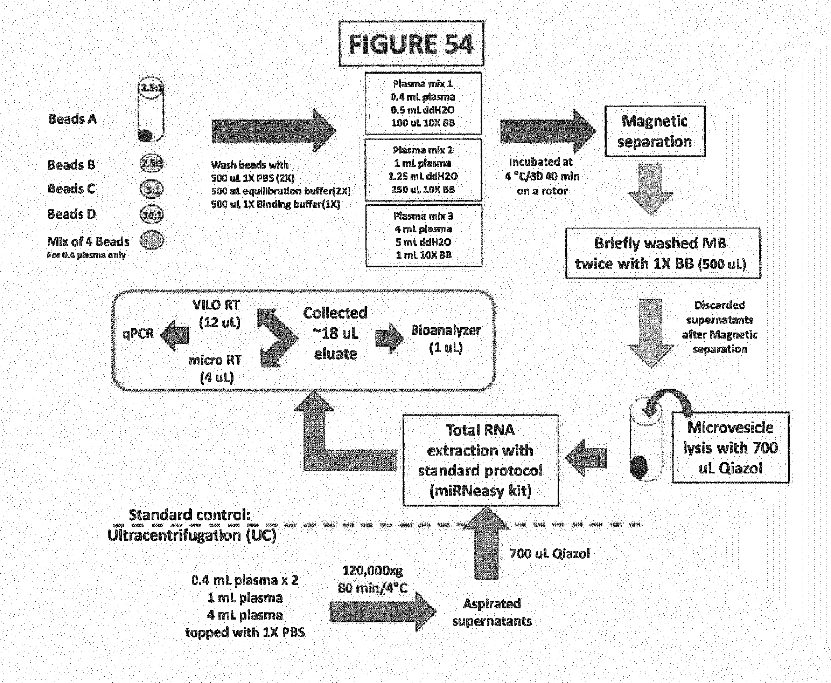

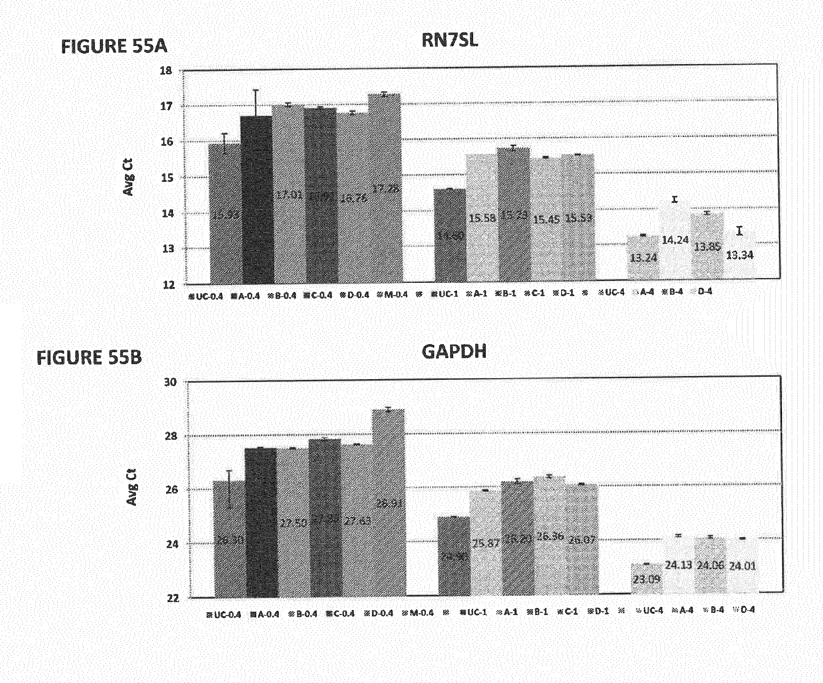

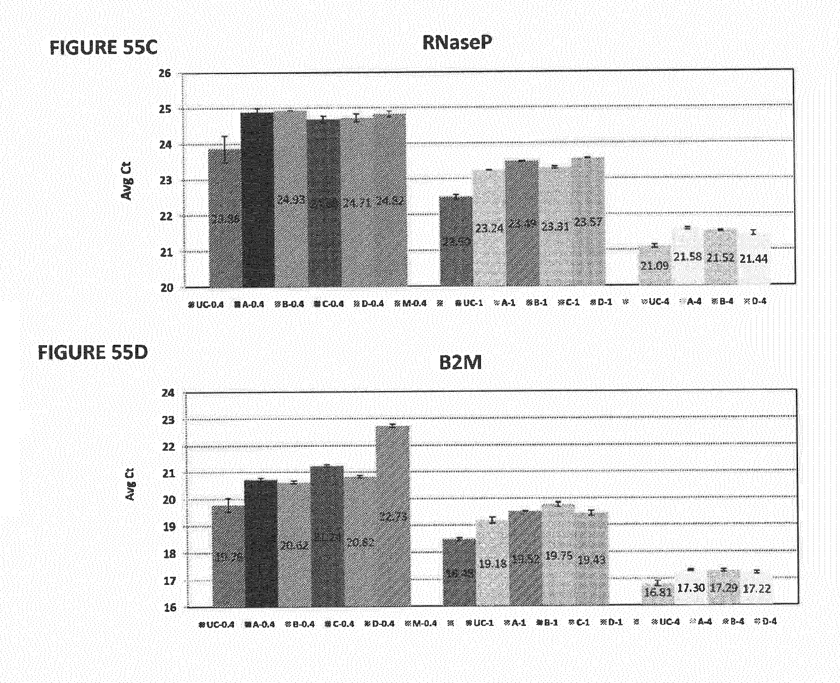

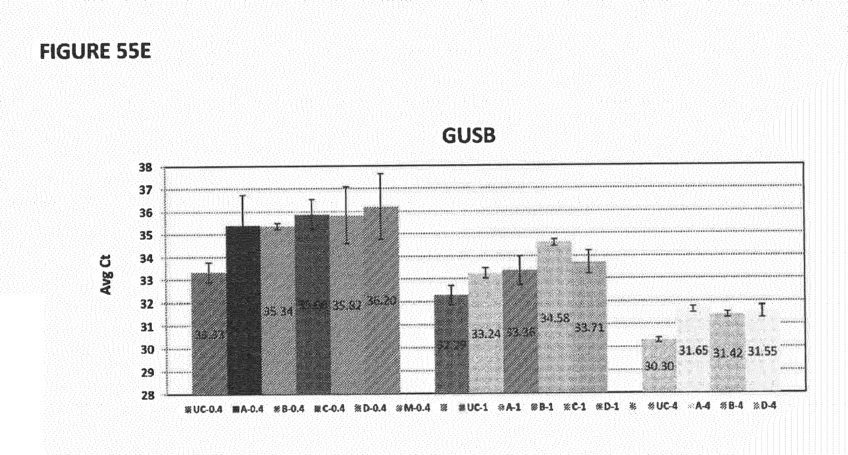

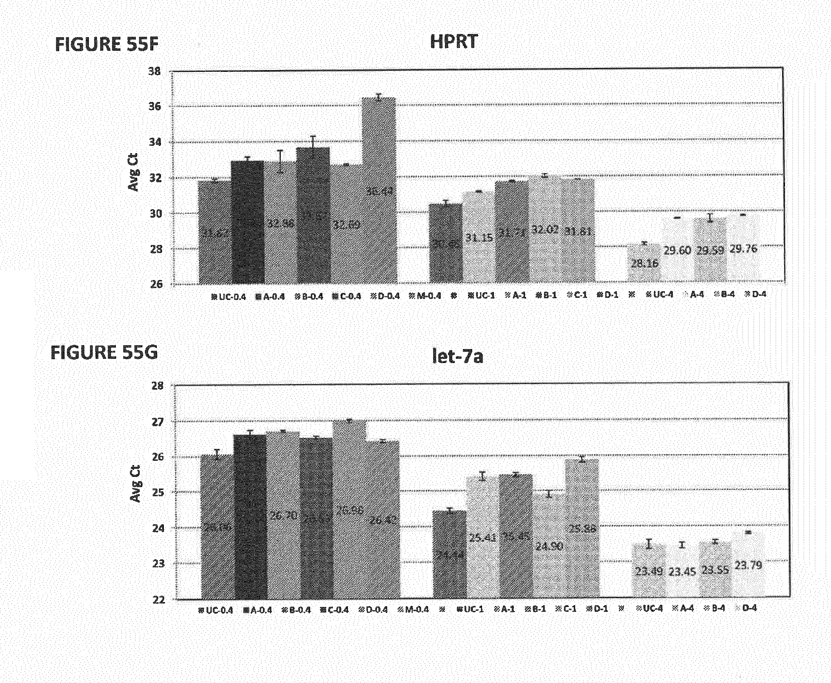

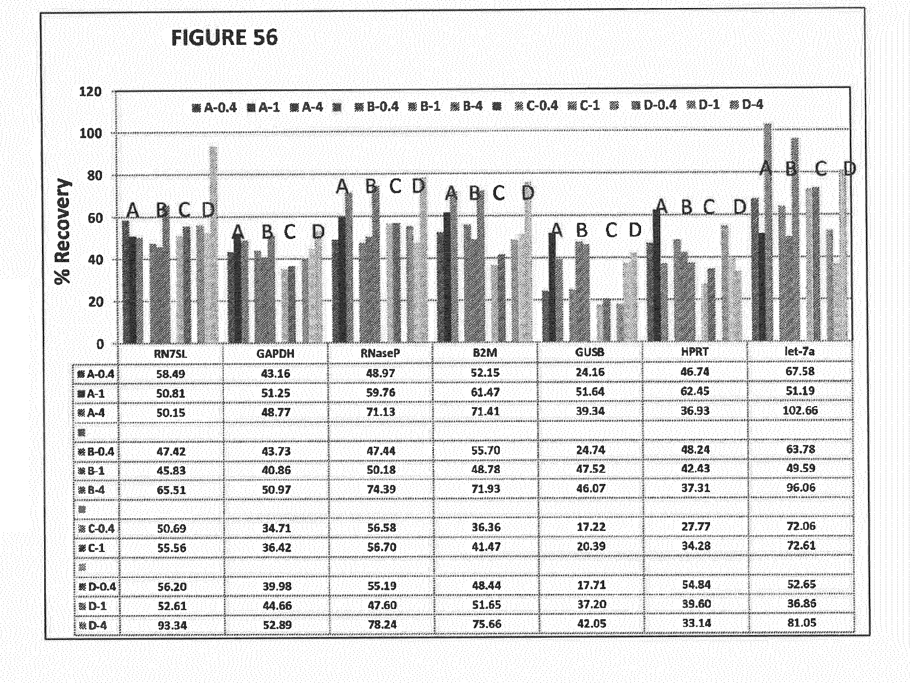

An overview of the methods used in these studies is shown in Figure 54. The average Ct value for each target is shown in Figures 55A-55G. Recovery comparisons for various targets at a bead:microvesicle ratio of 5:1 and plasma titrations from 0.4 mL, 1 mL to 4 mL are shown in Figure 56. A summary of the % recovery results is shown in the table below:

| | | % Recovery | | | | | | |
|---|---|---|---|---|---|---|---|---|
| | Total RNA yield (pg/uL) | RN7SL | GAPDH | RNaseP | B2M | GUSB | HPRT | let-7a |
| A-0.4 | 631 | 58.49 (67%) | 43.16 (62%) | 48.97 (77%) | 52.15 (49%) | 24.16 (69%) | 46.74 (38%) | 67.58 (116%) |
| A-1 | 1052 | 50.81 | 51.25 | 59.76 | 61.47 | 51.64 | 62.45 | 51.19 |
| A-4 | 3249 | 50.15 | 48.77 | 71.13 | 71.41 | 39.34 | 36.93 | 102.66 |
| | | | | | | | | |
| B-0.4 | 630 | 47.42 (73%) | 43.73 (48%) | 47.44 (52%) | 55.70 (54%) | 24.74 (78%) | 48.24 (37%) | 63.78 (83%) |
| B-1 | 898 | 45.83 | 40.86 | 50.18 | 48.78 | 47.52 | 42.43 | 49.59 |
| B-4 | 3062 | 65.51 | 50.97 | 74.39 | 71.93 | 46.07 | 37.31 | 96.06 |
| | | | | | | | | |
| C-0.4 | 593 | 50.69 (5%) | 34.71 (1%) | 56.58 (15%) | 36.36 (0%) | 17.22 (0%) | 27.77 (0%) | 72.06 (93%) |
| C-1 | 973 | 55.56 | 36.42 | 56.70 | 41.47 | 20.39 | 34.28 | 72.61 |
| | | | | | | | | |
| D-0.4 | 202 | 56.20 (81%) | 39.98 (48%) | 55.19 (57%) | 48.44 (59%) | 17.71 (27%) | 54.84 (46%) | 52.65 (76%) |
| D-1 | 818 | 52.61 | 44.66 | 47.60 | 51.65 | 37.20 | 39.60 | 36.86 |
| D-4 | 3484 | 93.34 | 52.89 | 78.24 | 75.66 | 42.05 | 33.14 | 81.05 |
| | | | | | | | | |
| UC-0.4 (1) | 616 | | | | | | | |
| UC-0.4 (2) | 269 | | | | | | | |
| C-1 | 961 | | | | | | | |
| C-4 | 520 | | | | | | | |

In summary, plasma titrations at fixed beads/microvesicle ratio (5:1) showed moderate reproducibility in the range of 0.4, 1, and 4 mL plasma volume.

### Example 30: Time Course Study of Beads-Microvesicle Binding in Plasma

In the studies described herein, epoxy magnetic beads (EPM-30) were evaluated for binding to microvesicles (at a bead:microvesicle ratio of 5:1) from 0.4 mL plasma. The table below presents the magnetic beads used for microvesicle isolation in these studies:

| **Beads Label** | **Name of Beads (Spherotech)** | **Manufacturer** | **Surface Character** | **Cat #** | **Dimension** | **Beads added** | **Beads included** |
|---|---|---|---|---|---|---|---|
| **A** | **Epoxy beads** | **Spherotech** | **Hydrophilic/ Reactive** | **EPM-20** | **2.85 um** | **218 uL (5.5 mg)** | **2.4 X 10⁸** |

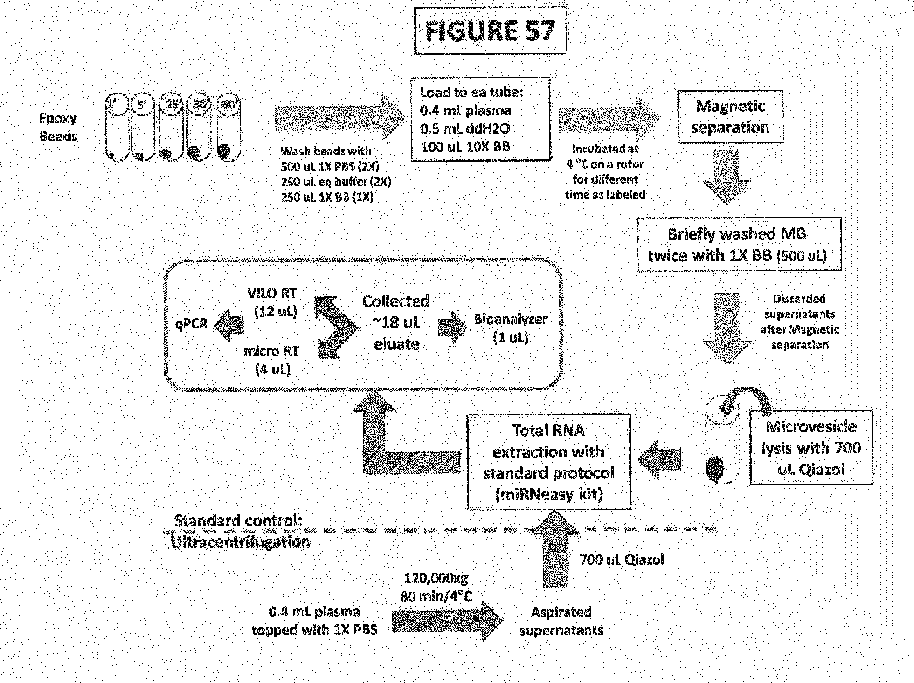

An overview of the methods used in these studies is shown in Figure 57. Five aliquots of EPM-30 beads mixed with 0.4 mL Plasma (B/E = 5:1) were incubated at 4°C (on a rotor) and stopped at 1 min, 5 min, 15 min, 30min, and 60 min. Beads were separated and washed with 1X BB followed by Qiazol lysis and total RNA isolation with miRNeasy kit. RT-qPCR was performed on selected mRNA targets and let-7a microRNA. The standard control was ultracentrifugation with 0.4 mL plasma.

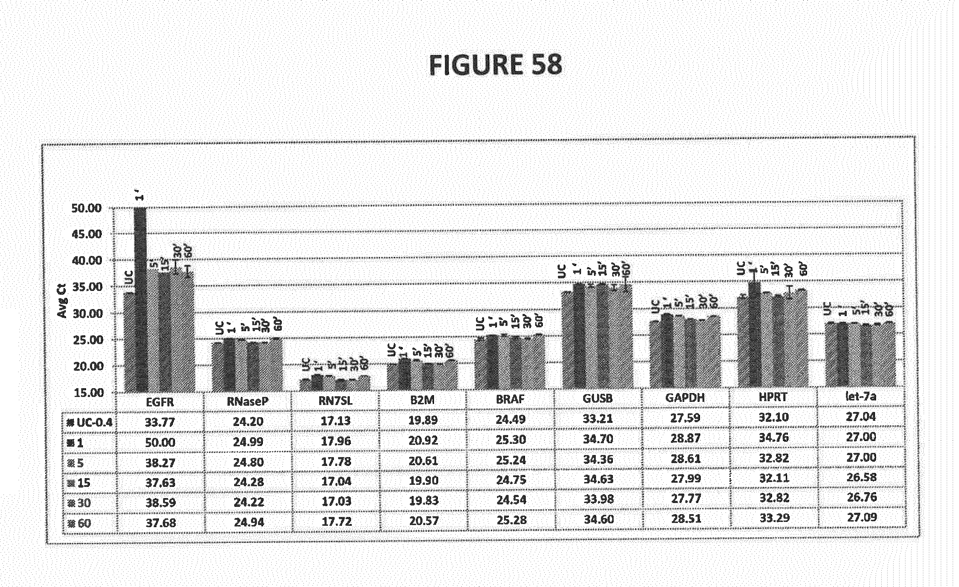

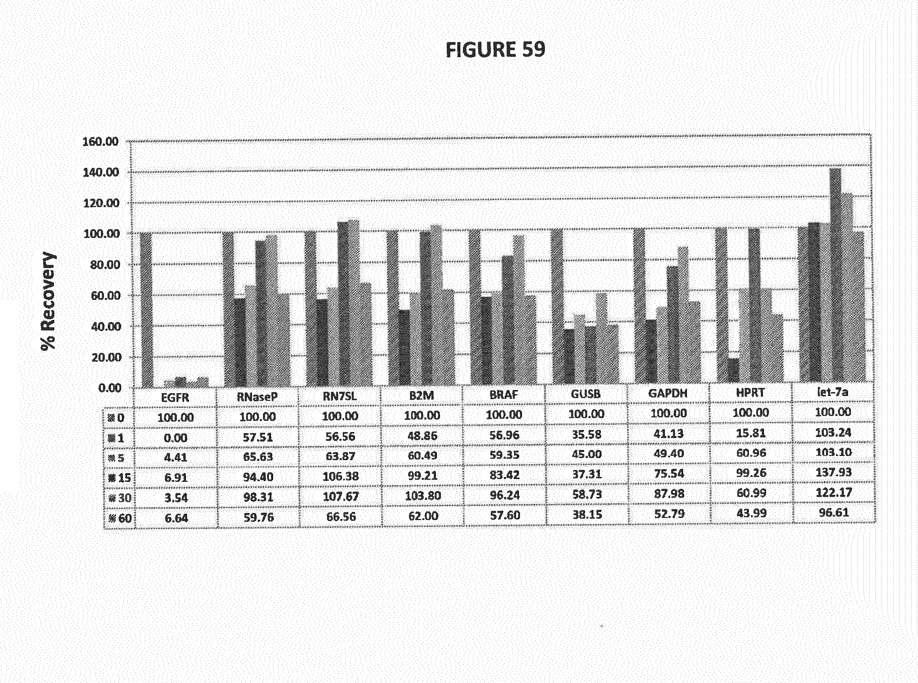

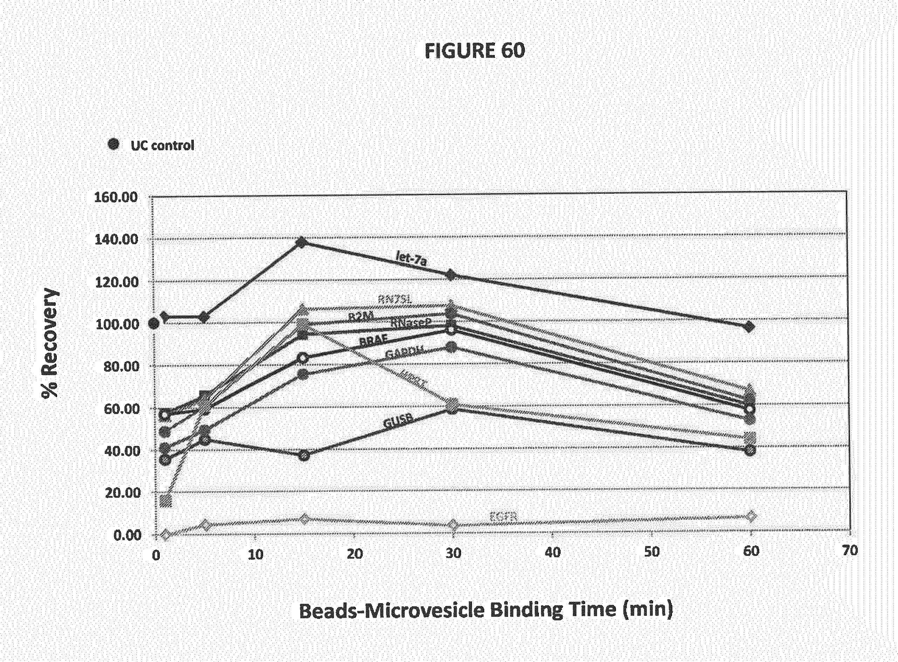

The average Ct values for various targets in the time course study on epoxy bead-microvesicle binding at 1 min, 5 min, 15 min, 30 min and 60 min are shown in Figure 58, and the % recovery values for each target at the same time points are shown in Figure 59. The % recovery as a function of bead-microvesicle binding time is shown in Figure 60.

Results showed that 30 min (15 min for HPRT and let-7a) of incubation gives the highest microvesicle RNA detection for all targets except EGFR. Signals started declining at 60 min. EGFR was detected with very poor signals (∼4.4% recovery at 5 min). Each single type of beads (Epoxy) seems performing similarly (in % recovery) (as observed previously).

Future studies will be designed to evaluate whether microvesicles captured to one beads type are the same as those bound to other types of beads, but through different affinities/interactions.

### Example 31: Additional Studies of Microvesicle Binding to Epoxy Beads

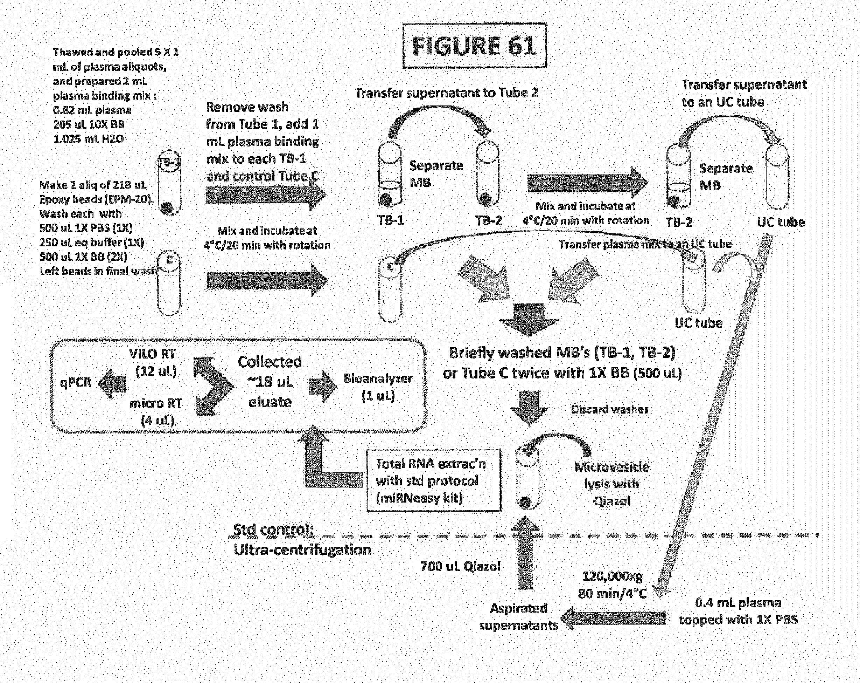

The studies described herein were designed to evaluate, if not captured completely, whether the remaining microvesicles in the supernatant can be further captured by additional fresh beads, and to evaluate the background level of microvesicle adsorption to Eppendorf tube. An overview of these studies is shown in Figure 61. Briefly, these studies were performed using the following steps:
- Performed one plasma (0.4 mL, pre-mixed in 1 mL of 1X BB) binding sequentially to two epoxy beads aliquots (B/E ratio = 5). Monitored beads bound microvesicles (to both aliquots) as well as free un-bound microvesicles present in the supernatant.
- Performed mock beads binding by adding pre-mixed plasma (0.4 mL) to an empty Eppendorf tube. Monitored tube bound microvesicles as well as free un-bound microvesicles present in the supernatant.
- The amounts of microvesicles bound to beads/tube or free in the supernatant were measured and expressed as the amounts of selected RNA targets via RT-qPCR (Note: qPCR was performed as single target assay).

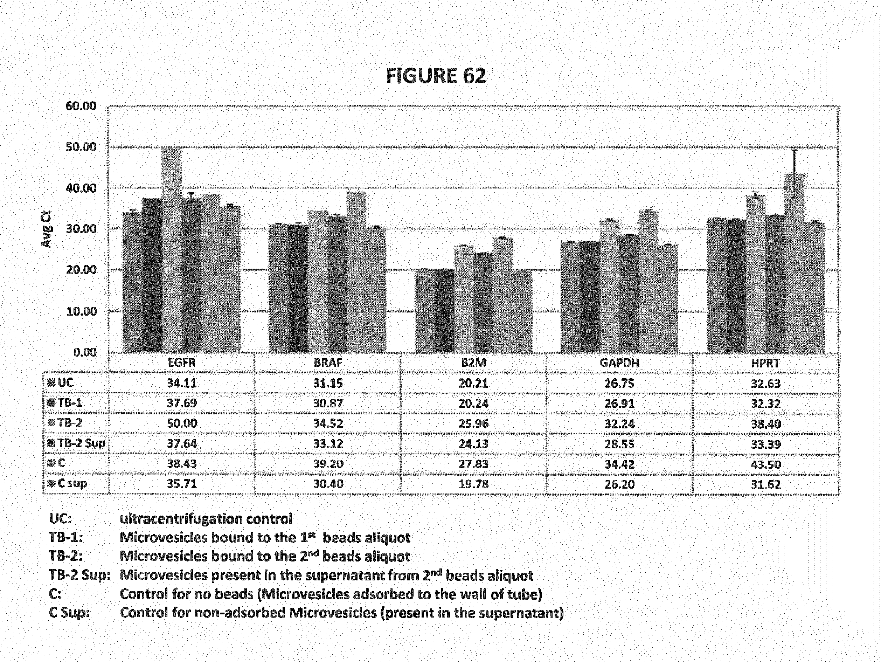

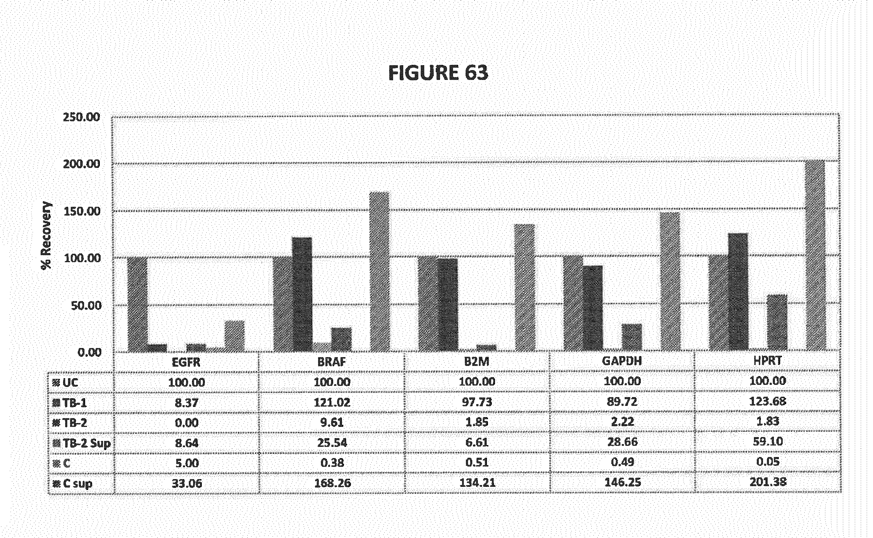

The average Ct values for a variety of RNA targets in collected fractions (epoxy beads only) are shown in Figure 62, and the percent recovery for these RNA targets is shown in Figure 63.

A high linearity (R2>0.99) curve was determined for either EGFR or BRAF standards, indicating a reliable RT-qPCR assay for either target. Results from sequential beads binding study showed that microvesicles are primarily (90∼124%) captured to the first beads aliquot and <10% to the 2nd beads aliquot. However, there are still 7-59% microvesicles present in the supernatant, suggesting that those microvesicles are either inactive or not in the optimal conditions for beads binding. Mock beads binding results showed that microvesicles are considered exclusively in the supernatants, only 0.05∼0.51% of them are adsorbed to the tube. While the capturing of EGFR microvesicles remains problematic, future studies for screening and testing a variety of beads will be performed.

The studies provided herein have demonstrated the feasibility of microvesicle isolation with beads (magnetic or non-magnetic) that would be a more suitable format in clinical utility. Results showed that positively charged, negatively charged, and neutral beads all showed good microvesicle capturing efficiencies. This observation suggests that they may act on different ligands on the surface of microvesicle, thus one microvesicle can be captured with different (functionalized) particles via different (multiple) forces.

### Example 32: EXO50 Microvesicle Isolation and RNA Extraction

This example describes EXO50 spin column and the protocol for microvesicle isolation, subsequent RNA extraction, and target expression analysis of the RNA preparation. The method described in this example was used for the following experiments, optionally with some variation as described in the specific working example.

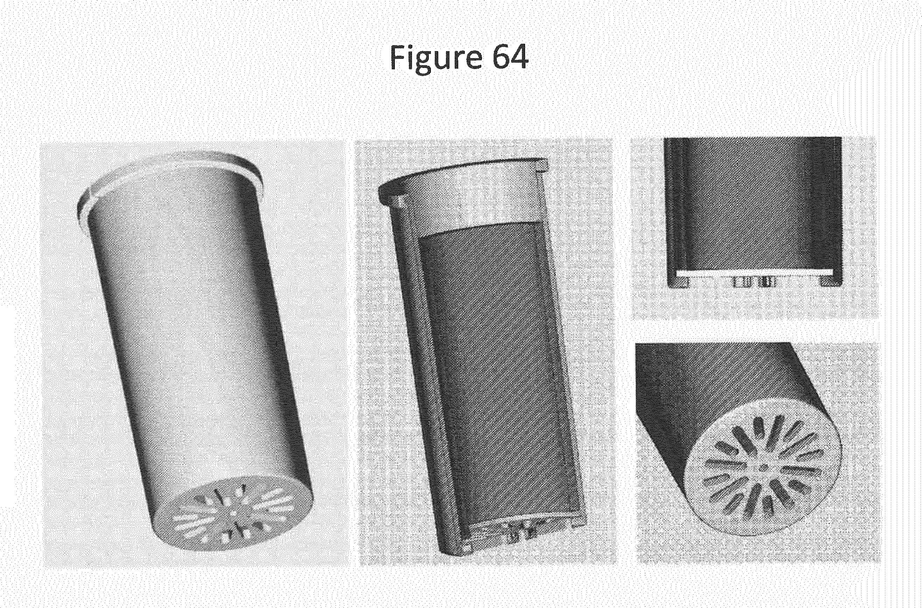

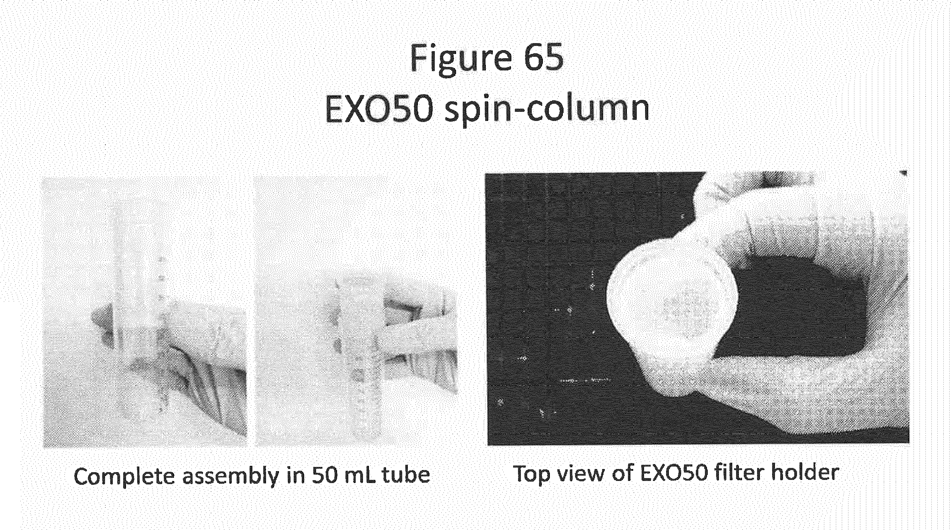

The EXO50 spin column, herein referred to as EXO50, comprises a membrane filter (preferably, Q functionalized). The column can also comprise a column holder that holds the membrane filter between the outer frit and an inner tube (FIG. 64). The column is placed in a collection tube (FIG. 65). After adding the biological sample to the membrane filter, the column is spun, and the flow-through is discarded. Wash buffer is added to the membrane and the column is spun, and the flow-through is discarded. The wash step may be repeated if desired. A lysis buffer, *i.e*., Qiazol, is then added to the membrane for lysis of the microvesicles and subsequent release of microvesicle RNA. The column is spun again to collect the microvesicle RNA. RNA extraction can be performed by chloroform extraction and ethanol conditioning, and/or using silica columns and eluting the isolated RNAs from the silica columns.

Subsequent detection of RNA targets by quantitative PCR is utilized to measure the quality of microvesicle isolation and/or RNA extraction. Once the RNA is extracted from the microvesicle fraction, the RNA is reverse transcribed into cDNA, for example by using SuperscriptII ® VILO cDNA Synthesis kit (Life Technologies) for mRNA or miScriptII RT kit (Qiagen) for short RNAs. Quantitative PCR or Taqman analysis is performed using specific RNA target primers, and optionally probes, to determine Ct values. Expression analysis is performed in duplicate or triplicate.

A variety of RNA targets were selected for expression analysis. Both large (*i.e.,* ribosomal RNA and messenger RNA) and small RNAs (*i.e.*, miRNAs) were detected. Examples of ribosomal RNAs detected include 18S and 28S. Target messenger RNAs included housekeeping genes, such as HPRT1, GAPDH. Other messenger RNAs included cancer specific genes, such as wildtype (wt) BRAF, mutated BRAF V600E. Examples of target miRNAs include let7a, miR16. In some experiments, target genes from control particles, such as Qbeta were assessed (*i.e.*, Qbeta coat protein).

### Example 33: Analysis of Different Membrane Filters

Membrane filters suitable for isolating microvesicles are preferably charged. The table below presents the various types of membrane filters that are suitable of isolating microvesicles:

| **Sample No**. | **Membrane** | **Separation Method** | **Functional group** |
|---|---|---|---|
| (1) + (7) | Regenerated Cellulose, >3 µm pore size ("RC/WBAE") | Weak basic anion exchanger | Diethylamine R-CH₂-NH⁺(C₂H₅)₂ |
| (2) + (8) | Regenerated Cellulose, >3 µm pore size ("RC/SACE") | Strong acidic cation exchanger | Sulfonic acid R-CH₂-SO₃" |
| (3) + (9) | Regenerated Cellulose, >3 µm pore size ("RC/SBAE") | Strong basic anion exchanger | Quaternary ammonium R-CH₂-N⁺(CH₃)₃ |
| (4) + (10) | Metal Chelate Membrane, 3-5 µm, pore size ("MCM") | *Metal chelate membrane* | Iminodiacetic acid - N(CH₂COOH)₂ |
| (5) + (11) | Regenerated Cellulose Coupling Membrane, 0.45 µm pore size ("RCCM") | *Microporous Membrane* | Aldehyde -CHO |
| (6) + (12) | DEAE Cellulose Paper ("DEAE") | Weak basic anion exchanger | DEAE cellulose paper |

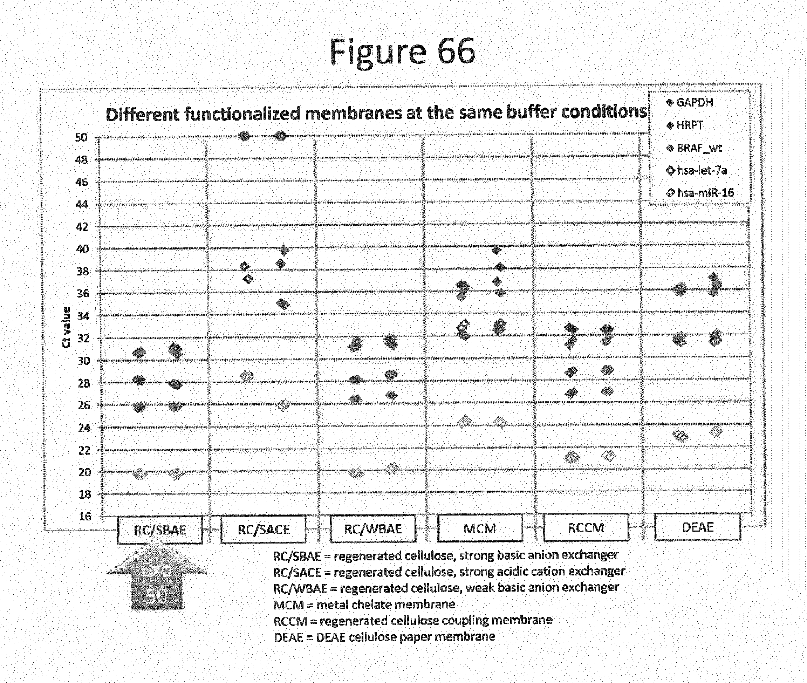

The ability of each of the membranes listed in the table above for isolating microvesicles was assessed. Specifically, assessment of the RNA target signal (Ct values) was determined as a means to quantify the quality of microvesicle isolation. Microvesicles were isolated using the following membranes: regenerated cellulose membranes, e.g., reinforced stabilize cellulose membranes that are reinforced with regenerated cellulose, a metal chelate membrane, a regenerated cellulose coupling membrane, and DEAE cellulose paper. The separation methods for each membrane tested are shown in the table above. Biological samples were added to columns containing the different membranes, similar to the column described in EXAMPLE 32. The flow-through was discarded and the microvesicles were lysed, for example, on the membrane using lysis buffer. Microvesicle RNA was subsequently extracted using a silica column. Detection of mRNA target genes GAPDH, HPRT, and wildtype (wt) BRAF, and miRNA target genes hsa-let-7a and hsa-miR16 was determined by qPCR analysis (FIG. 66). Comparison of the Ct values obtained for each membrane filter for each specific target genes demonstrated the capability of each of the membranes to successfully isolate microvesicles from a biological sample. All buffer conditions for each membrane were the same.

As shown in FIG. 66, all of the membranes were able to isolate microvesicles sufficient for RNA extraction and detection of at least one of the RNA targets. However, membranes RC/SBAE; RC/WBAE; and RCCM performed better than the other membranes tested. The RC/SBAE functionalized membrane performed the best in comparison to all other membranes. Specifically, for each of the mRNA and miRNA targets assayed, lower Ct values were obtained from the RNA preparation from microvesicles isolated by RC/SBAE membrane compared to any other membrane from a microvesicle fraction. The capture surface of the EXO50 columns described in the following examples is comprised of the RC/SBAE membrane.

These results demonstrate that positively charged (anionic) ion exchange membranes purified vesicles at physiological pH. However, negatively charged ion exchange inefficiently purified vesicles at physiological pH.

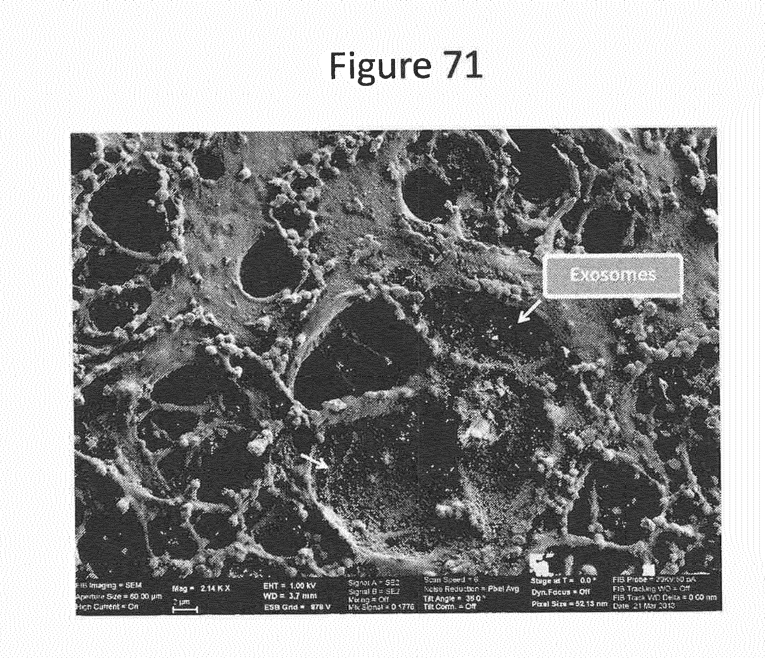

FIG. 71 shows the microvesicles bound to the EXO50 membrane, as detected by scanning electron microscopy. The arrows indicate the captured microvesicles.

### Example 34: EXO50 Membrane Input Capacity

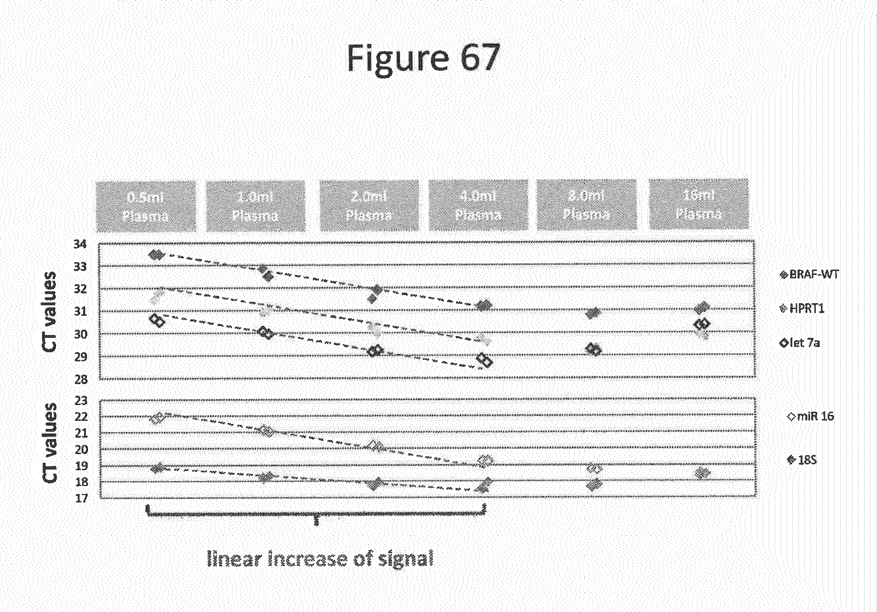

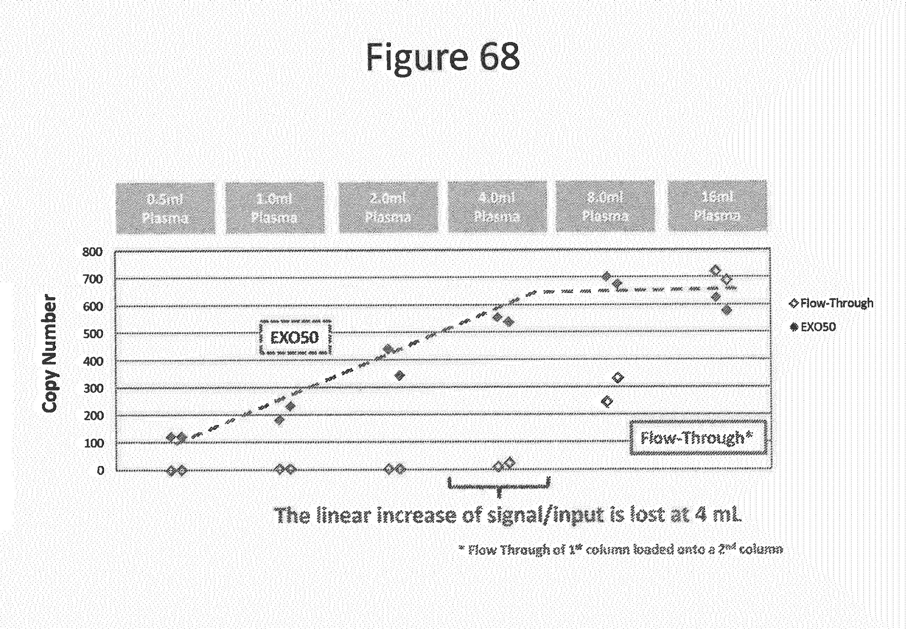

Next, the input capacity of the RC/SBAE membrane was assessed by titrating the amount of input plasma volume added to the membrane. Increasing volumes of sample input was added to the RC/SBAE membrane; for example, 0.5ml, 1.0ml, 2.0ml, 4.0ml, 8.0ml, and 16ml of plasma. Microvesicle fractions were isolated and microvesicle RNA was extracted as described in Example 32. QPCR analysis was performed to detect mRNA genes wt BRAF and HPRT1 and miRNAs let7a, miR16, and 18S. As shown in FIG. 67, the qPCR signal for both mRNA and miRNA genes increased linearly (*i.e.*, a linear decrease in Ct value) from samples between 0.5ml and 4.0ml. However, samples greater than 4.0ml (*i.e.*, 8.0ml and 16ml) failed to demonstrate additional increase in detected signal. These results indicate that sample volumes greater than 4ml would not exhibit any increased In FIG 68, the copy number from the RNA extracted from the microvesicle fraction isolated by EXO50 was compared to the copy number from the flow-through. This experiment identifies the maximal volume of sample input that would saturate the membrane, such that microvesicles would be present in the flow-through and RNA signal from the flow-through would be detected. For example, input volumes greater than 4.0ml did not demonstrate linearly increased signal when compared to the signal detected from the sample of 4.0ml. Consistent with these results, the signal detected from the flow through increased with volumes greater than 4.0ml.

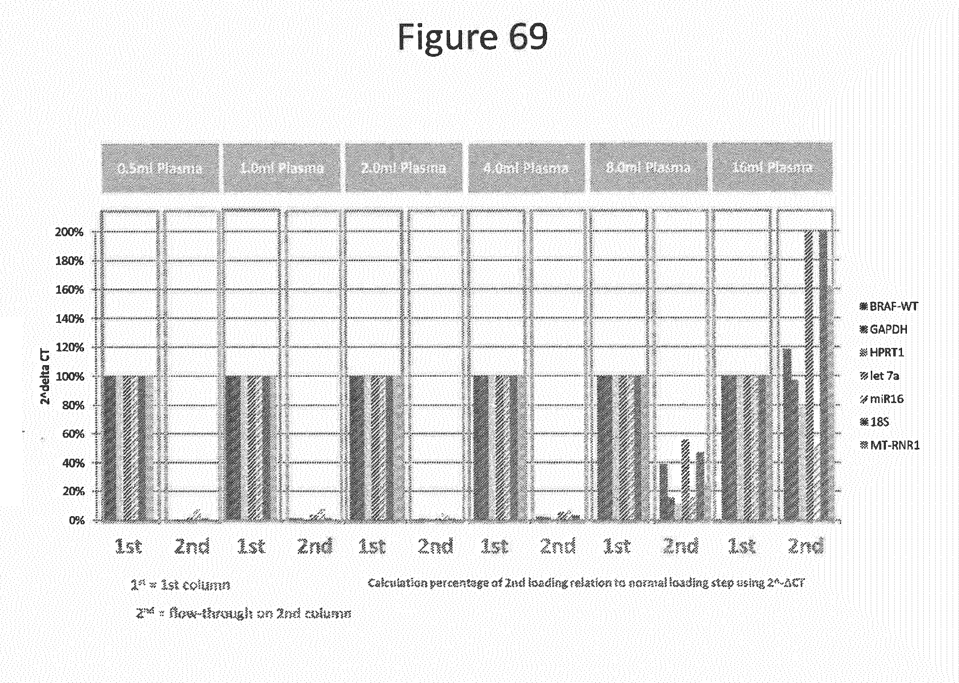

Relative expression levels of various target genes were compared between initial loading and isolation of the biological sample in a first EXO50 column to the flow-through in a second EXO50 column. Biological sample was added to EXO50, with microvesicle isolation, RNA extraction and target gene analysis performed as described in EXAMPLE 32. The flow-through from the first EXO50 column was then loaded onto a second EXO50 column for microvesicle isolation, RNA extraction, and target gene analysis with the same methods as used for the initial biological sample with the first EXO50 column. Varying volumes of initial biological sample (0.5ml - 16ml of plasma) was tested. The target genes include mRNA targets: wt BRAF, GAPDH, and HPRT1; and miRNAs: let7a, miR16; and ribosomal RNA 18S. The expression levels of each target gene were normalized to the detected expression level of the corresponding target gene from the first loading step. As shown in FIG. 69, the flow-through from samples between 0.5ml and 4.0ml had very little expression of any target genes, indicating that the EXO50 had efficiently captured all of the microvesicles from the biological sample In contrast, the flow-through from samples of 8.0ml and 16ml showed at least 10% increase in expression detected for some target genes, and up to 200% detected for other target genes. These results show that the flow-through from the larger sample input volumes still contained microvesicles that were isolated from the second EXO50 column, thereby demonstrating that sample volumes of 8ml and 16ml are not completely depleted of microvesicles. Furthermore, these results demonstrate that two isolation and lysis steps are not needed to isolate all of the microvesicles from a biological sample between 0.5ml and 4ml.

### Example 35: Determining the Number of Membrane Layers to Deplete Sample

The number of membrane layers was assessed for complete depletion of microvesicle RNA from a 4.0ml plasma sample. One to six layers of RC/SBAE were placed adjacent to one another in an EXO50 column. The microvesicle RNA was extracted and analyzed as described in EXAMPLE 32 from the sample and from the flow-through. The target genes assessed were wt BRAF and GAPDH.

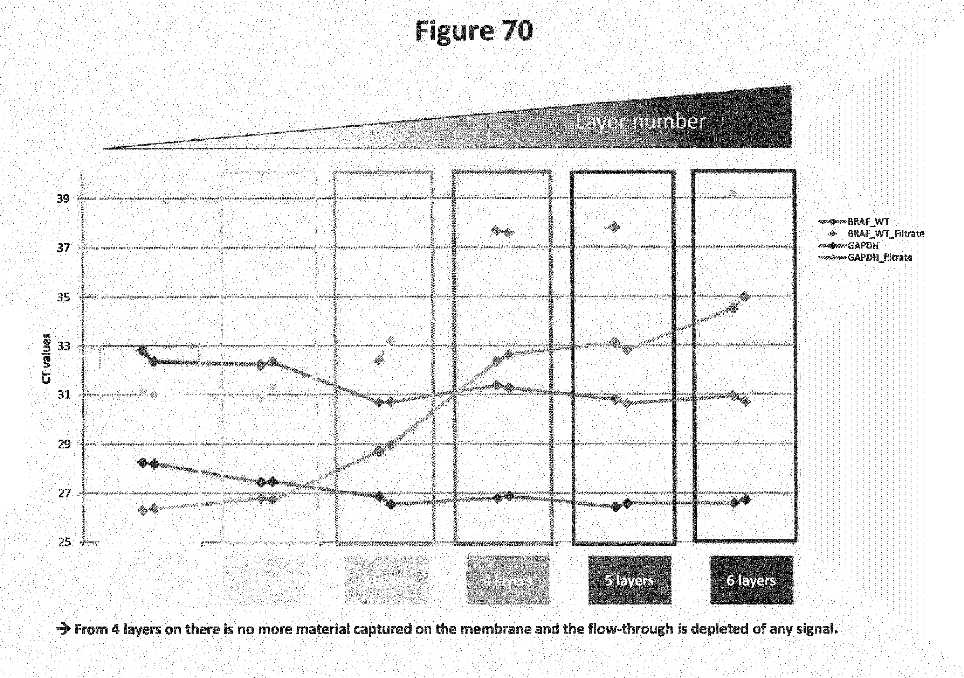

The results shown in FIG. 70 show that one and two layers were insufficient to capture all of the microvesicles from the sample, as demonstrated by the signal detected from the flow-through (filtrate) data points. However, the columns with 4 layers or more did not show any increase in signal from the input data points, while the flow-through (filtrate) was completely depleted of any signal. Thus, these results show that 3 layers is optimal to ensure sufficient microvesicle capture, while increasing the number of layers to 4 or greater does not yield any added benefit for microvesicle capture.

### Example 36: EXO50 Buffer Conditions for Microvesicle Isolation

Varying buffer conditions were assessed for optimal microvesicle isolation using the EXO50 columns and methods as described in EXAMPLE 32. Different loading buffers that were added to each sample for loading onto the column were assessed. The buffers tested included:
- 160mM Tris, 142mM Cl⁻, pH 7.0
- 192mM imidazole; 159mM Cl⁻, pH 6.5-6.8
- 300mM Bis Tris, 211mM Cl⁻, pH 6.3-6.4
- 40mM Citrate, 124mM Na⁺, pH 6.7-6.9
- 50mM Phosphate, 265mM Na⁺, 185mM Cl⁻, pH 7.0-7.2
- 80mM Bis Tris Propane, 14mM Cl⁻, pH 6.7.

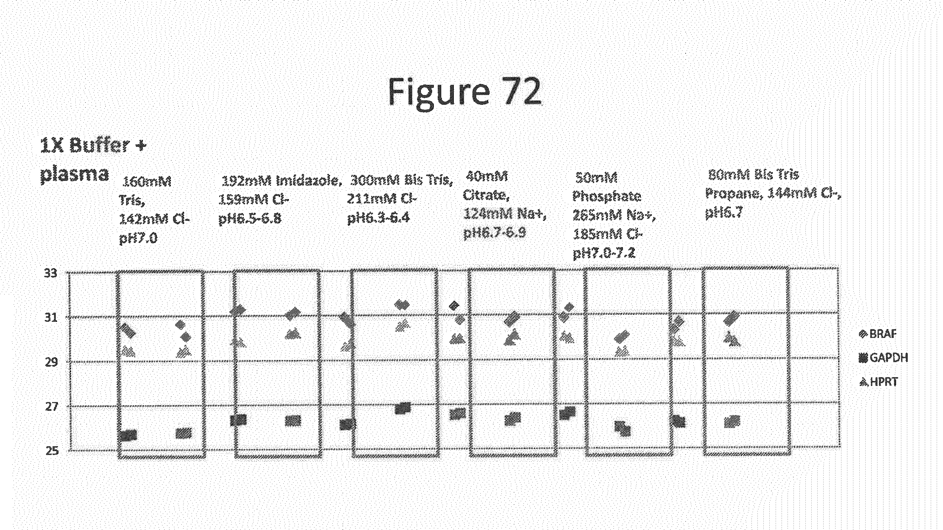

As shown in FIG. 72, all buffers resulted in successful microvesicle isolation, such that RNA targets BRAF, GAPDH, and HPRT were detected.

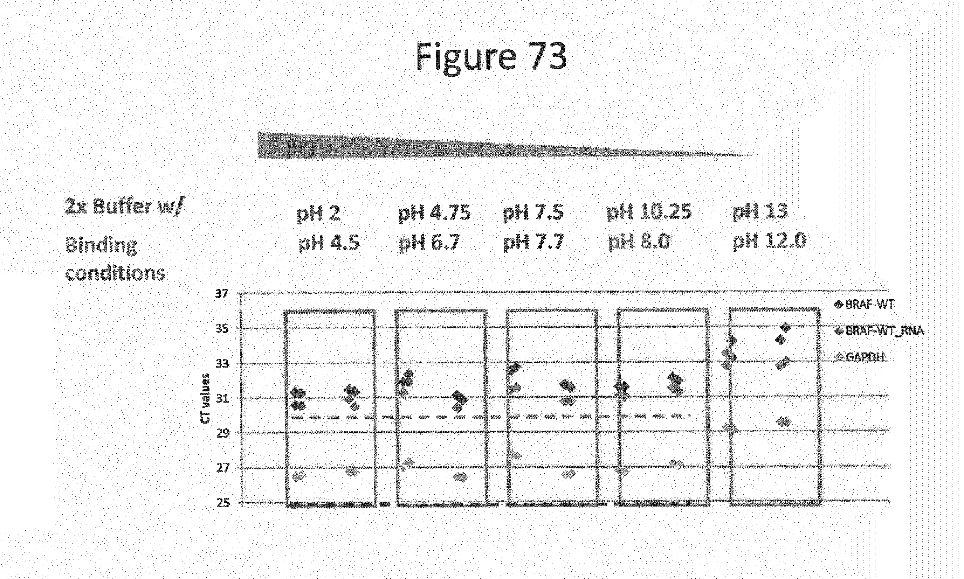

Varying pH conditions were also assessed. Buffers were prepared at 2X PBS pH 2.0, pH 4.75, pH 10.25, and pH 13.0 for loading the sample. Wash buffers were at 1X. 1000 copies of Qbeta was spiked with the sample upon loading into EXO50 column. Buffers with increasing pH demonstrated that optimal buffer conditions are between pH 2-10.25, while very basic buffers resulted in less efficient microvesicle isolation (FIG. 73). The optimal range for isolation is between pH 4-8.

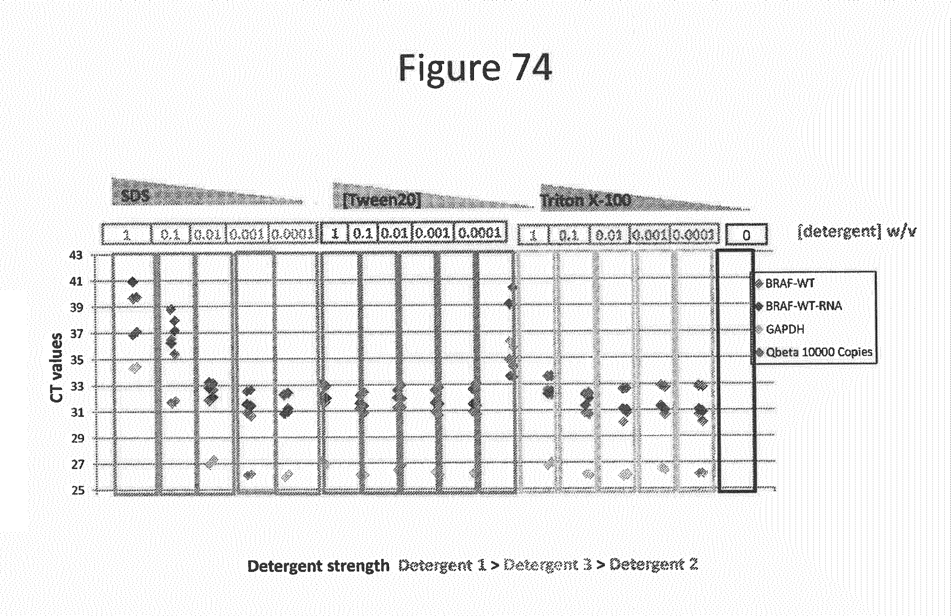

Buffers were tested with varying amounts of detergent to show that the process is tolerant to buffers with detergents of varying strength. FIG. 74 demonstrates that SDS up to 0.001%, Triton X-100 up to 0.01%, and Tween up to 0.1% detergent in both the binding and wash buffer resulted in successful microvesicle isolation and subsequent microvesicle RNA extraction analysis.

### Example 37: EXO50 RNA preparation

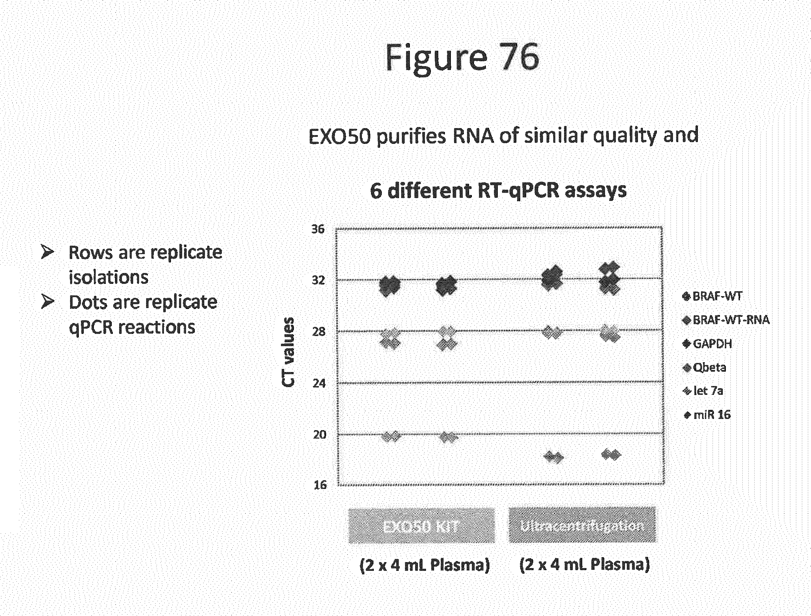

The results shown in FIG. 76 demonstrate that the RNA purified by EXO50 is PCR-amplifiable RNA, i.e., suitable for amplification and PCR processing, and that EXO50 isolates the same amount and quality of RNA as ultracentrifugation from a large volume of plasma. 4ml of plasma sample were processed either by EXO50 or by ultracentrifugation. Comparison of isolation and detection of RNA targets (wt BRAF, GAPDH, Qbeta, let7a, miR16) showed that EXO50 purifies RNA of similar quality as obtained by ultracentrifugation. With respect to some target genes, EXO50 was shown to have improved RNA quality, as indicated by increased Ct signal.

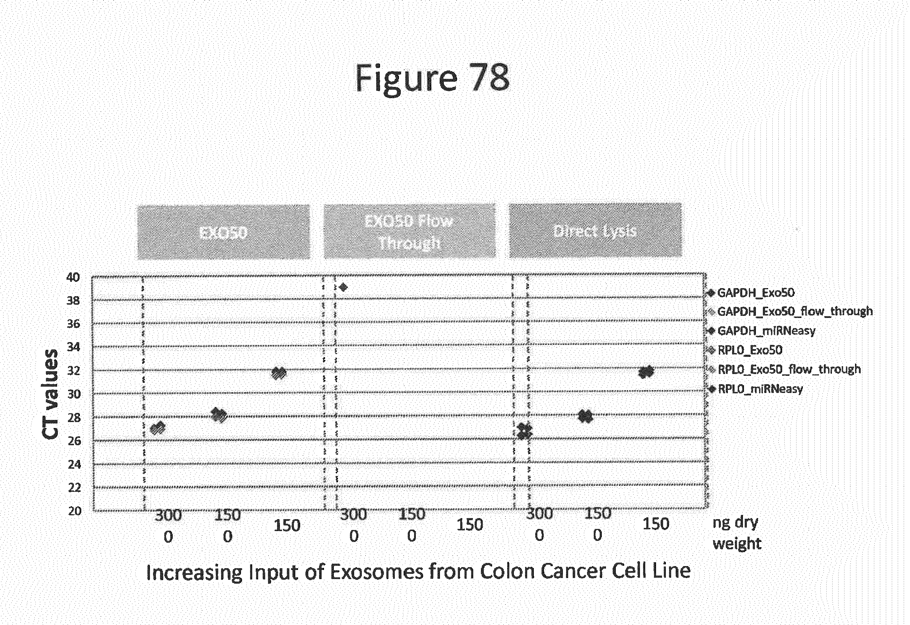

Furthermore, EXO50 purifies a high percentage of mRNA from microvesicles. For example, EXO50 purifies 100% of mRNA from commercially available cancer exosomes or cancer cell culture supernatant. Increasing input of commercial available cancer exosomes from a colon cancer cell line (150, 1500, and 3000 ng) was processed by EXO50 or by direct lysis (using Qiagen miRNeasy RNA isolation kit). The results shown in FIG. 78 demonstrate that the flow-through fro EXO50 processing showed no detection of mRNA (no Ct signal for RPL0 and very high Ct signal for GAPDH), therefore indicating that 100% of the mRNA was successfully extracted from the microvesicle fraction of the sample.

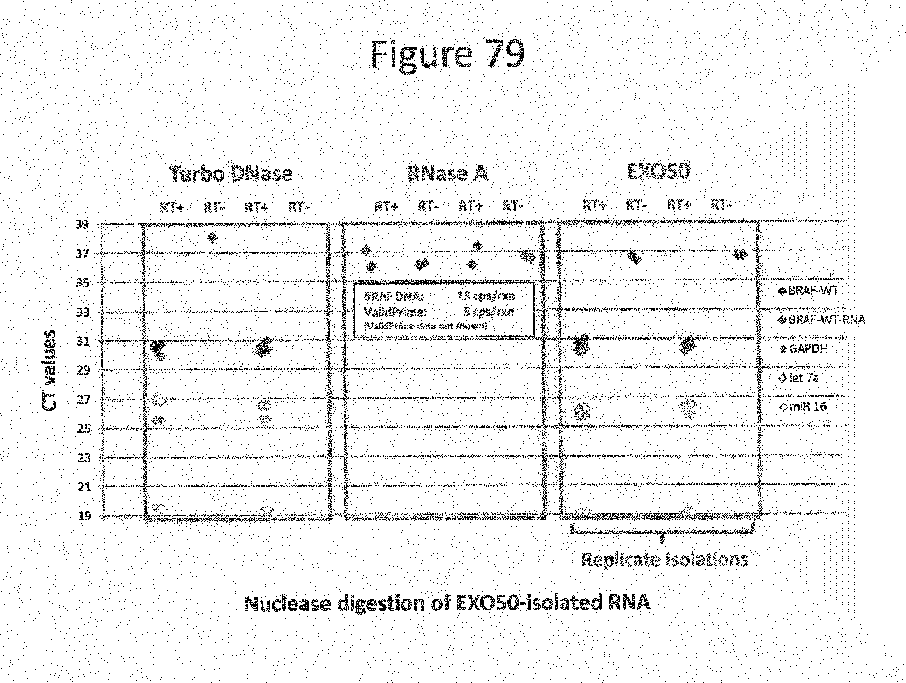

The EXO50 RNA preparation also contains very little contaminating DNA. The background level of detection of certain targets from the RNA preparation that is not reverse transcribed (-RT) shows that there is some background level of contaminating DNA (FIG. 79). EXO50 RNA preparations were digested with RNase A, which showed loss of Ct signal for the target genes, to show that the preparation mostly contains RNA and very little contaminating DNA. QPCR analysis of the RNase-digested RNA prep showed very low background level of BRAF similar to that detected when the sample is not reverse-transcribed. Addition of DNase was shown to remove the background DNA signal, as shown by the loss of signal in the DNase-digested samples that were not reverse-transcribed.

### Example 38: EXO 50 Operability Experiments

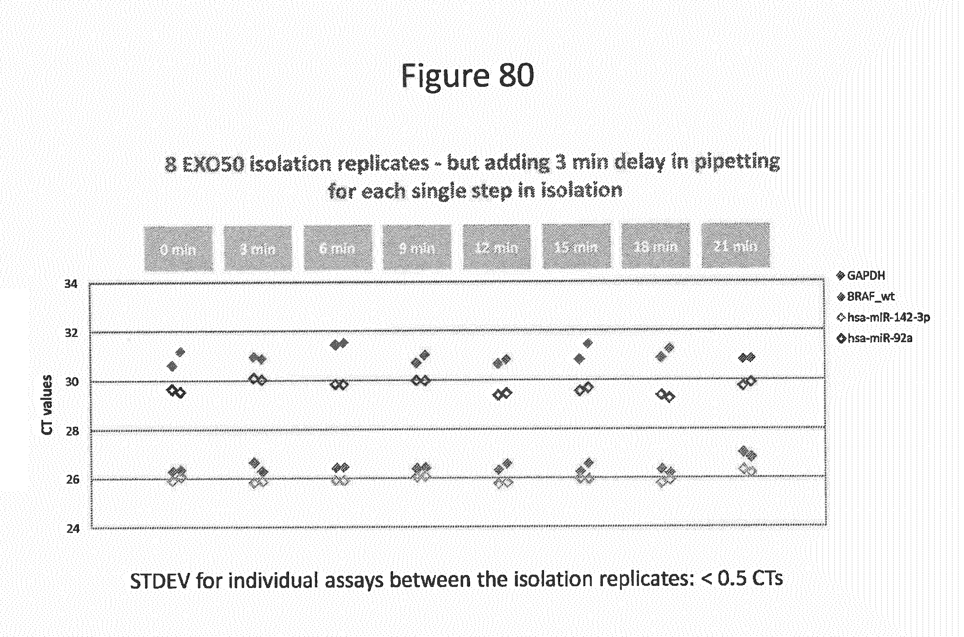

EXO50 is robust for parallel processing of many samples. Eight 4ml of plasma samples were processed in parallel using EXO50, with each sample having added 3 minutes of delay in pipetting for each single step in isolation. Thus, one sample was processed as normal (0 minutes), a second sample was processed with 3 minutes between each step (3 minutes), a third sample was processed with 6 minutes between each step (6 minutes), etc. with the eight sample processed with 21 minutes between each step. There was no significant difference in the detected Ct values for targets GAPDH, wt BRAF, hsa-miR-142-3p, and hsa-miR-92a, even with 21 minutes between each step (FIG. 80). The standard deviation for the individual assays between the isolation replicates were less than 0.5 Cts. This experiment demonstrates that the EXO50 process allows for processing of a long row of replicates without change in isolation efficiency - as long as 21 minutes pipetting time allows per step.

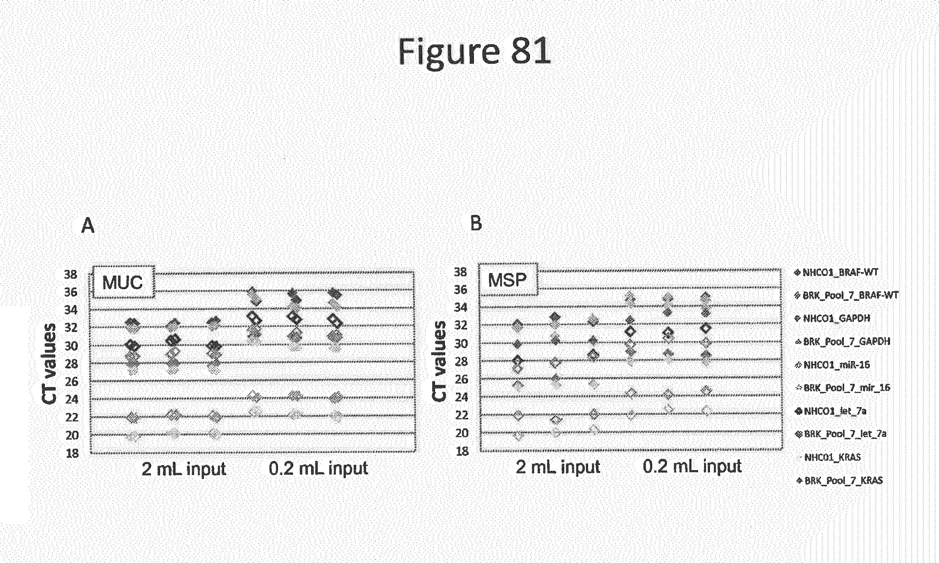

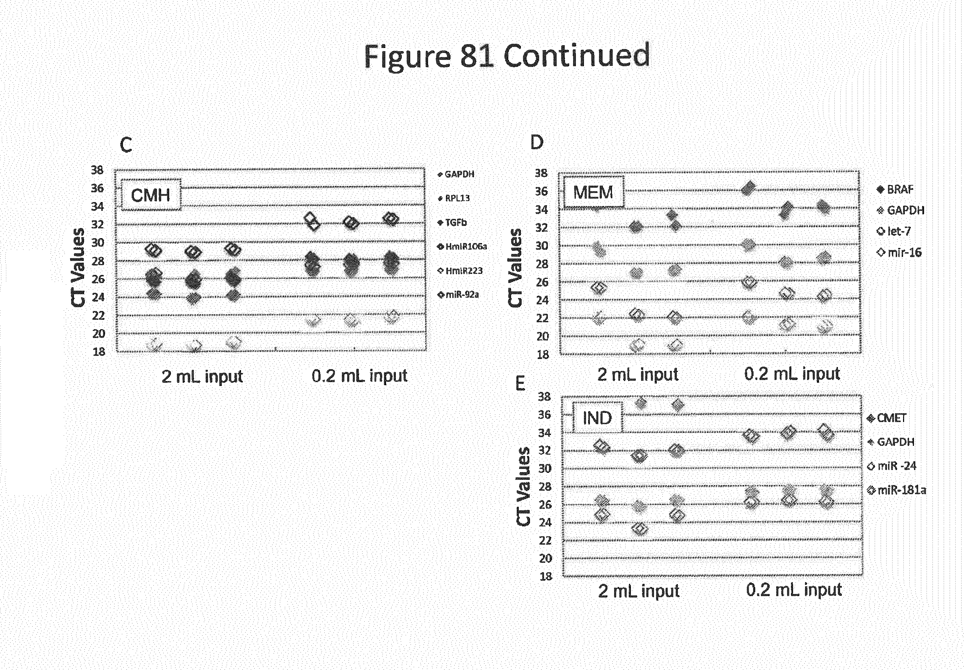

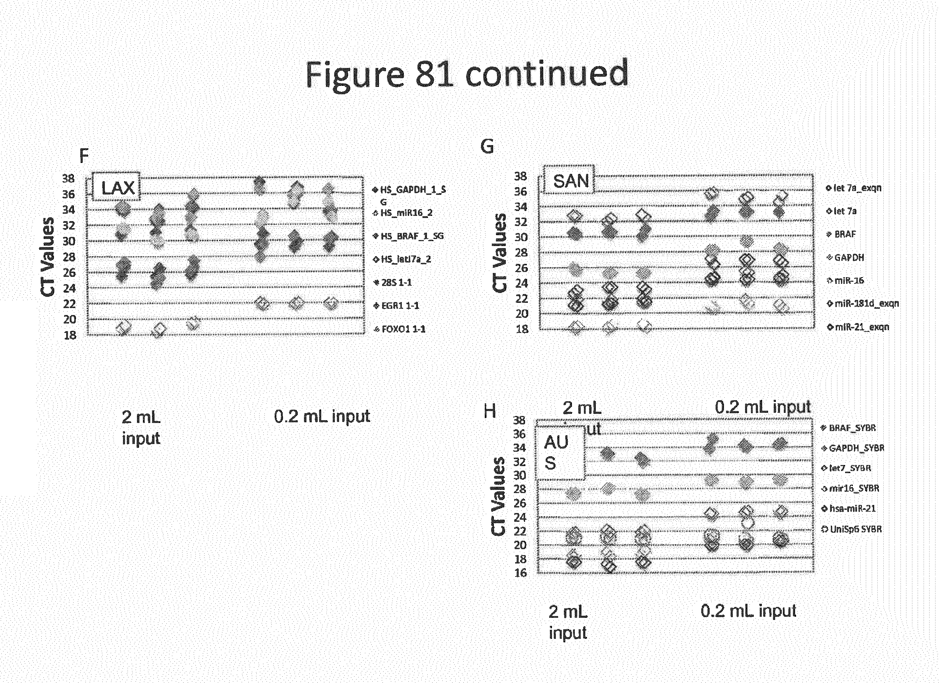

The operability of the EXO50 process by different users at different research sites, using different qPCR analysis reagents was compared in an on-site experiment in order to assess the ease of usability of the EXO50 process and the reproducibility of the results. The sample used in this experiment comprised a pool of plasma from 900 single blood draws. The sample was shipped on dry-ice to 8 different labs, some on different continents. Each lab performed the EXO50 process with 2ml or 0.2ml plasma in triplicate. RT-qPCR analysis on 2 mRNAs (*i.e.*, wt BRAF, GAPDH) and 2 miRNAs (*i.e.*, miR-16 and let-7a) was performed, and the results were compared in FIG. 81. In all labs the EXO50 procedure is reproducible and the PCR output scales linear with plasma input from 0.2 mL to 2 mL. First-time users using the same PCR primers showed very similar output PCR signal (FIG. 81). These results demonstrate the robustness of the EXO50 for reproducibility independent of user and research site.

### Example 39: EXO50 Mini Spin-Column Format

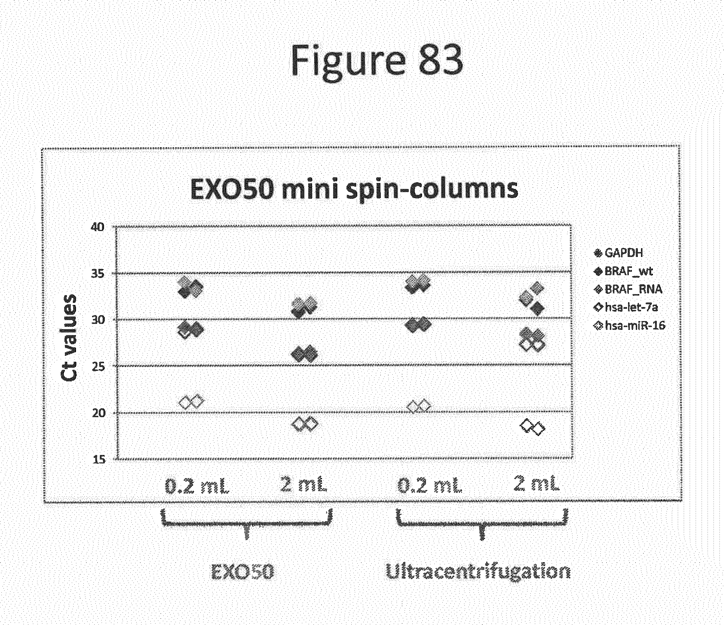

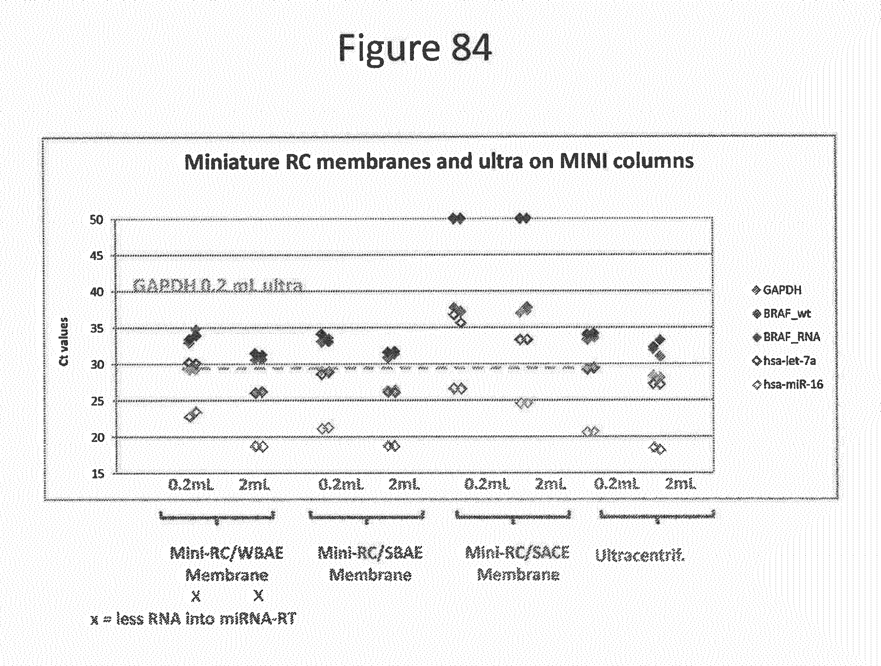

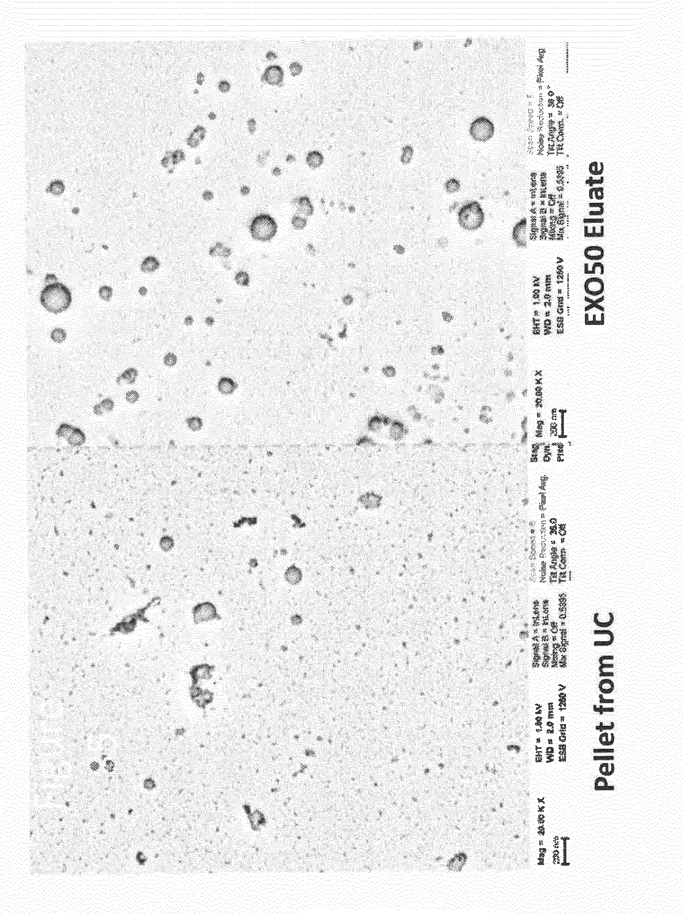

The EXO50 format was also adapted to a mini-spin column format to assess its reproducibility and scalability. The EXO50 mini format comprises a miniature RC/SBAE membrane. Miniature RC/WBAE and RC/SACE membranes were also tested for comparison. 2ml and 0.2ml of plasma sample was tested. The standard buffers were used: binding buffer (2X) 100mM Phosphate Buffer, 370mM NaCl; wash buffer (IX) 50mM phosphate buffer, 185mM NaCl. The results from the mini-format was also compared to ultracentrifugation. Detection of target mRNAs and miRNAs showed that the miniature RC/SBAE membrane had the best Ct signal compared to the miniature RC/SACE and miniature RC/WBAE membranes (FIG. 84). The comparison between EXO50 mini format (RC/SBAE membrane) and ultracentrifugation is shown in FIG. 83.

### Example 40: Comparison of EXO50 to Other Methods

The standard ultracentrifugation process comprises pre-filtering the biological sample, *i.e.,* plasma through a 0.8µm filter. The filtrate is then loaded into an ultracentrifuge and spun at >100,000xg for 1-3 hours. The supernatant is removed and the pellet containing the microvesicles is lysed. The microvesicle RNA is then purified on a silica column, *i.e.,* a commercially available RNA isolation column (Qiagen), using manufacturer's protocol.

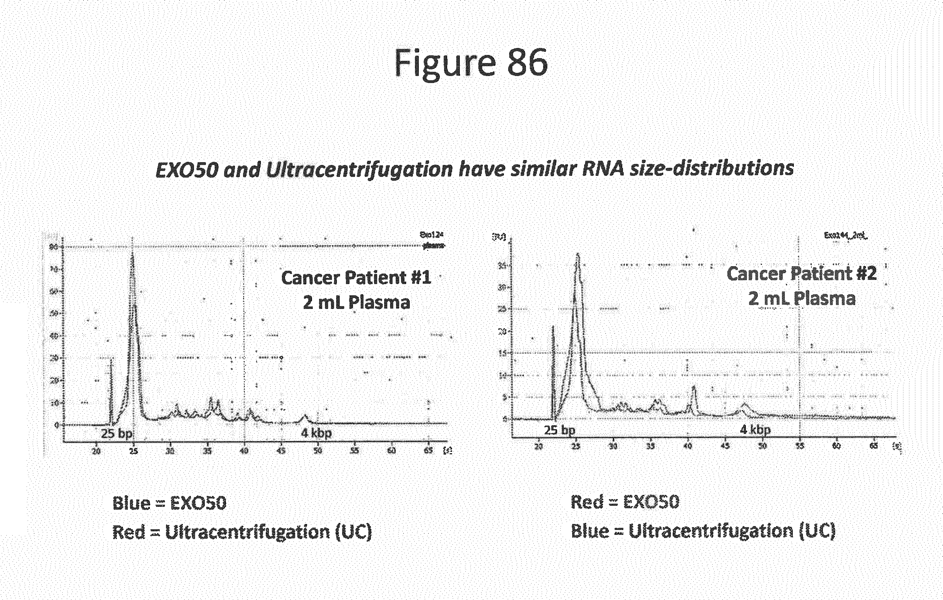

Comparison of bioanalyzer profiles from the RNA extracted by ultracentrifugation or by EXO50 were similar with respect to RNA-size distributions (FIG. 86). EXO50 was shown to isolate both small and large RNAs. Separation of the microvesicle RNA was achieved by ethanol fractionation. rRNA (*i.e*., large RNA) peaks were clearly visible at 2Kb and 4Kb. The majority of the isolated small RNAs were between 50-200 base pairs.

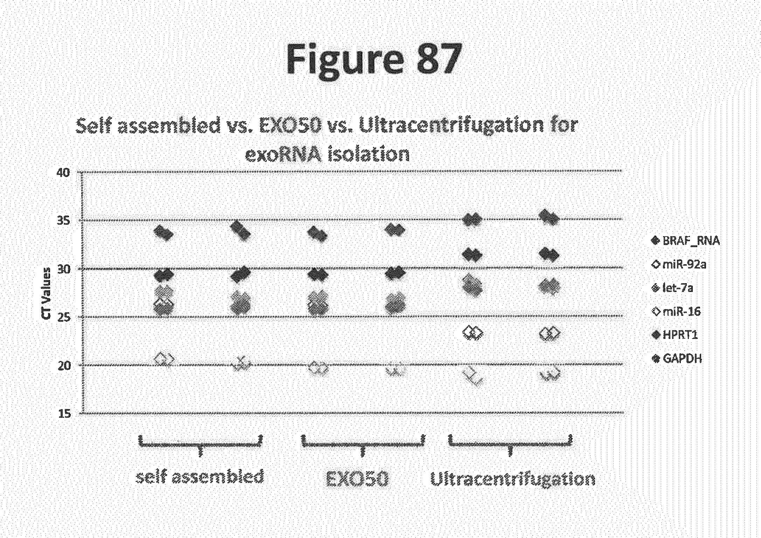

Comparison between ultracentrifugation, EXO50, and self-assembled membrane capture system is shown in FIG. 87. The self-assembled membrane capture system also comprises Q-functionalized membranes. Two separate technical replicate extractions are shown for each column. The non-filled circles (miR-16 and miR-92a) are known to exist as free circulating protein bound miRNAs. The results shown herein demonstrate that EXO50 and the self-assembled Q membranes result in increased Ct signal for microvesicle RNA targets.

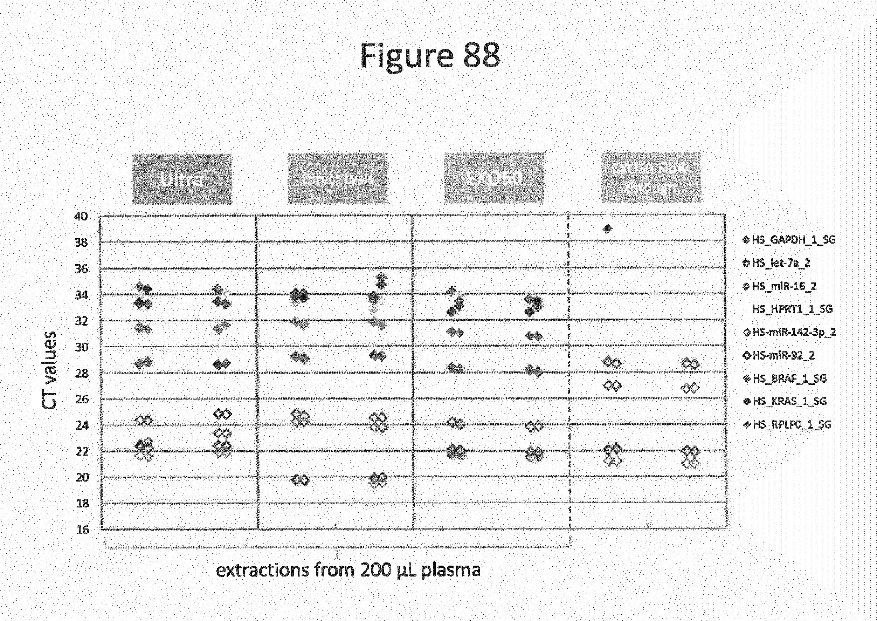

A similar comparison experiment was also performed using 0.2ml of plasma, comparing ultracentrifugation, direct lysis, and EX050. As shown in FIG. 88, EXO50 also performed better than direct lysis for most all genes. EXO50 flow-through demonstrates that some miRNA signal was still detected in the flow-through.

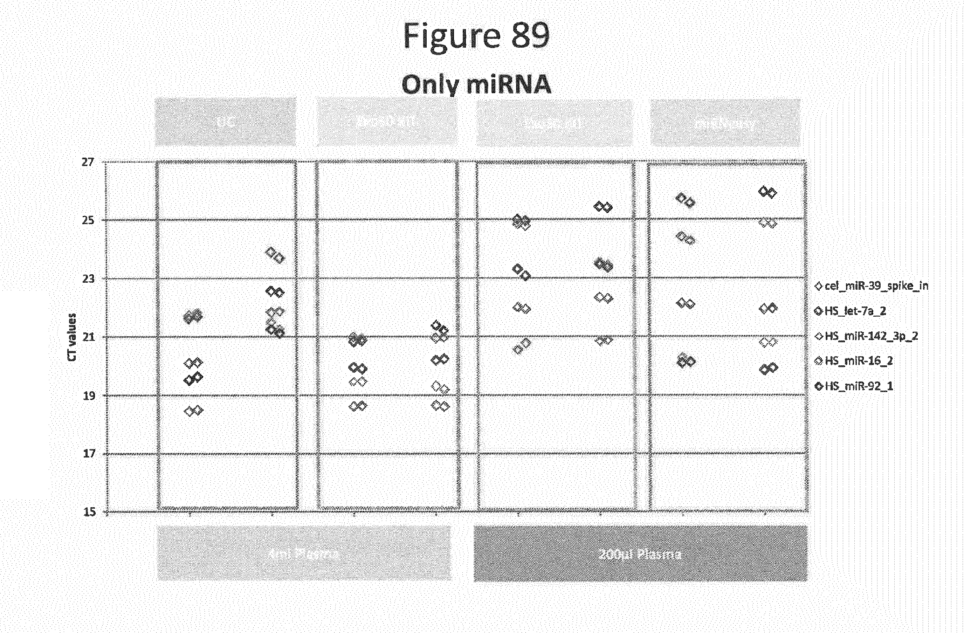

However, increasing sample volumes to 4ml plasma showed that EXO50 had increased detected Ct signal in comparison to ultracentrifugation (FIG. 89).

### Example 41: EXO50 Detection of Cancer Mutations

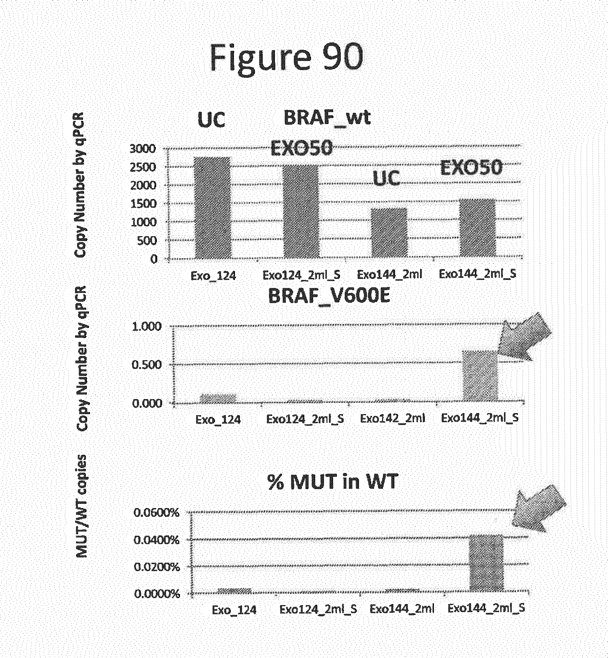

Importantly, EXO50 was shown to successfully detect cancer mutations from 2ml plasma samples from a melanoma patient. Samples were processed by ultracentrifugation (UC) or EXO50. The copy number was determined by qPCR analysis of wild-type BRAF (wt BRAF) and compared to the copy number of mutated BRAF (BRAF V600E). As shown in FIG. 90, ultracentrifugation and EXO50 were both able to detect wt BRAF copies. However, only EXO50 was able to detect the presence of mutated BRAF V600E. These results demonstrate that EXO50 was able to detect cancer mutations from microvesicle RNA, while ultracentrifugation was unable to resolve the presence of the cancer mutation.

### Example 42: EXO51 Elution Step

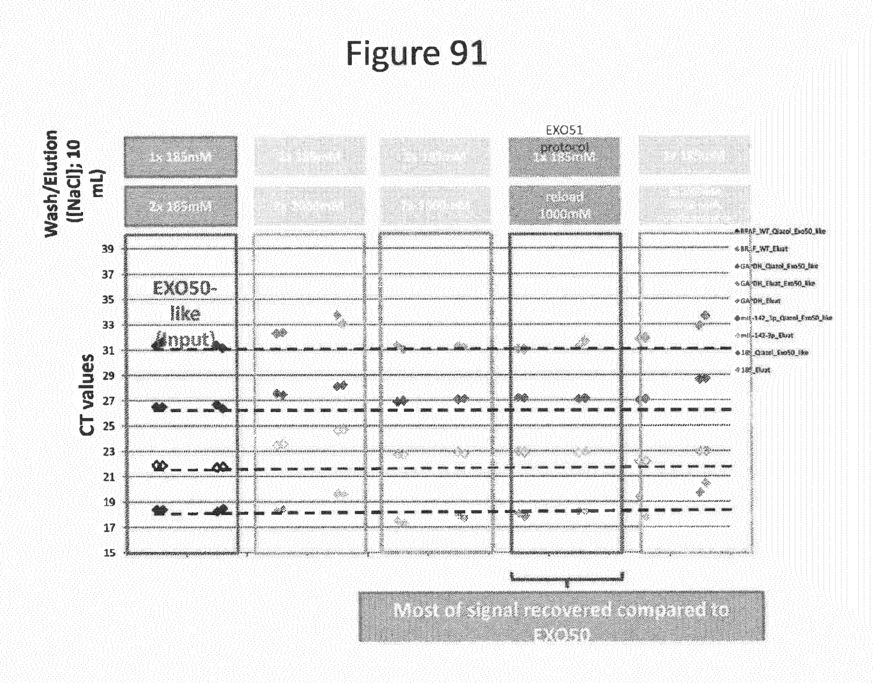

This example shows the optional elution step for isolating the microvesicles from the filter membrane prior to RNA lysis and extraction, also referred to herein as EXO51. Specifically, the microvesicles were isolated from the EXO50 column by a wash/elution step to isolate intact isolated microvesicles from the membrane. 10ml of wash/elution buffer was added to the column twice. In some cases, a different NaCl concentration was used the first time compared to the second time, while in some cases, the same NaCl concentrations were used. For example, 185mM, 1000mM, and 2000mM NaCl buffer was used for the first wash. For the second was, 185mM, 1000mM, and 2000mM NaCl buffer was used. The columns were spun at 5,000xg for 1 minute. Comparison of Ct values for various genes (*i.e.*, GAPDH, BRAF, Kras, etc.) using the various elution protocols compared to EXO50 demonstrated that RNA isolation of EXO51 was comparable to that of EXO50 (FIG. 91).

Scanning electron microscopy of the eluted microvesicles from EXO51 showed that microvesicles from 50-200 nm in size (FIG. 85). In comparison, microvesicles isolated by ultracentrifugation revealed more fibrous protein structure-like materials in the isolated microvesicles, which can potentially affect RNA lysis and extraction, RNA purity, and ability to detect/amply RNA sequences from the RNA preparation. The EXO51 isolation contained particles that are similar to purified material obtained using UC procedures, but not similar to source material. The flow-through from the EXO51 method still contained a large amount of particles that were different from purified vesicle material.

An additional concentrating step can be utilized with the EXO51 protocol. Various spin concentrators (for example, VivaSpin 20 columns) with different size cutoff values were used, for example, 10,000 MW, 50,000 MW, and 100,000 MW cutoff concentrators were used. The eluates were spun for 5 minutes. If the retentate volume was too high (> 200 µL), the retentate was spin in 5 min increments to the desired volume, *i.e.,* less than 200 µL. The concentrated eluate was transferred from the upper chamber of the concentrator (filter side) with gentle resuspending. The concentrated eluate was filled up to 200 µL total volume by using 1x wash buffer. Then the samples were ready for lysis and RNA extraction. Concentration spin speeds and times ranged from 800xg and 4500xg for concentrating the microvesicle eluate without significant changes to RNA yield or subsequent Ct values obtained from RNA expression analysis.

### Example 43: EXO52 Isolation of DNA

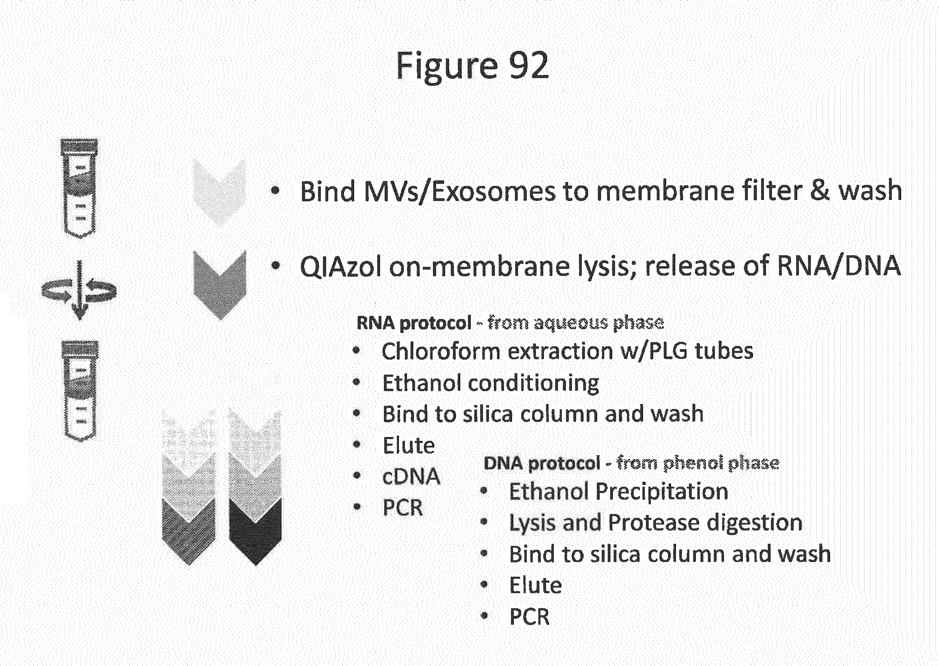

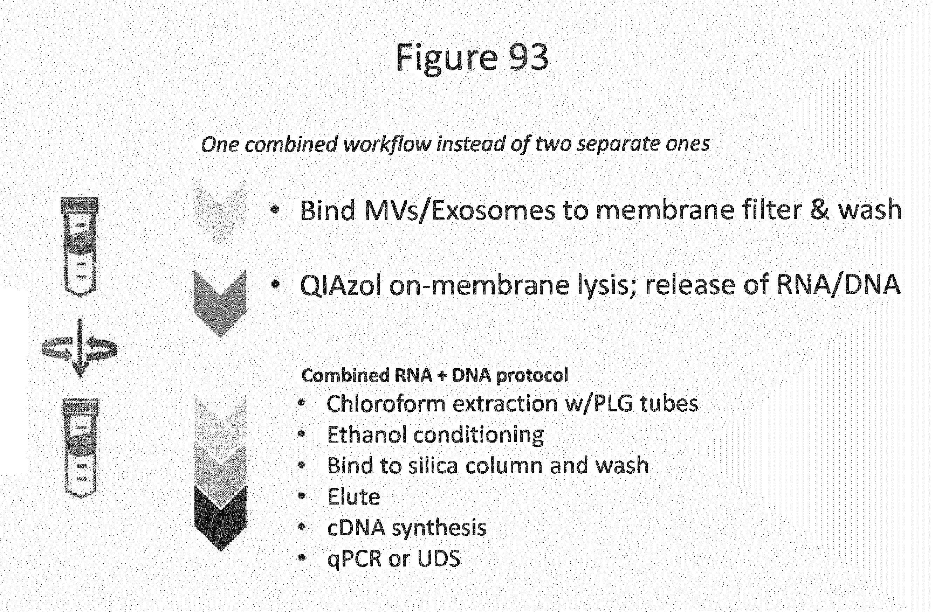

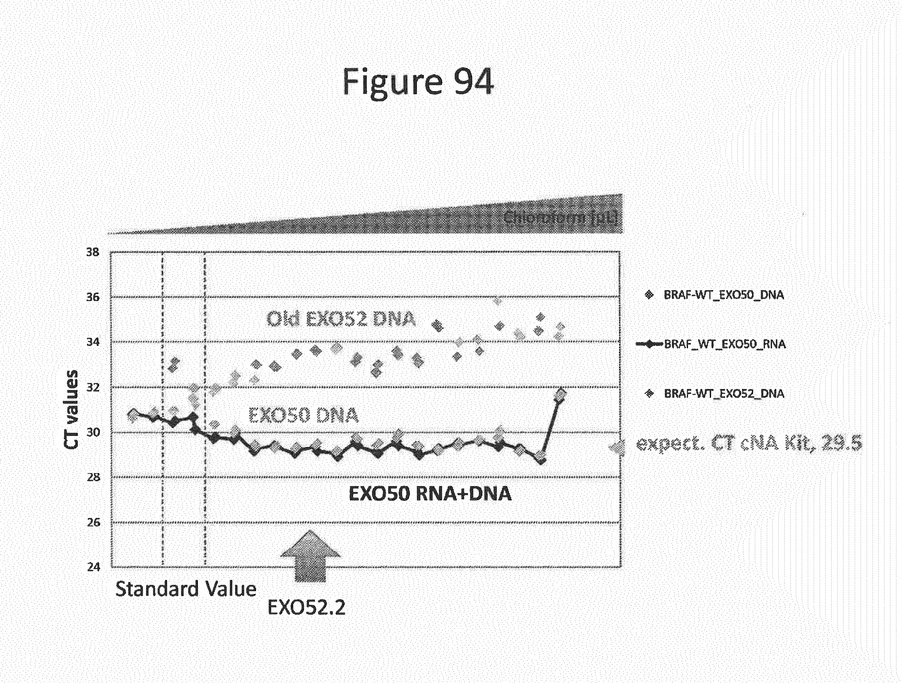

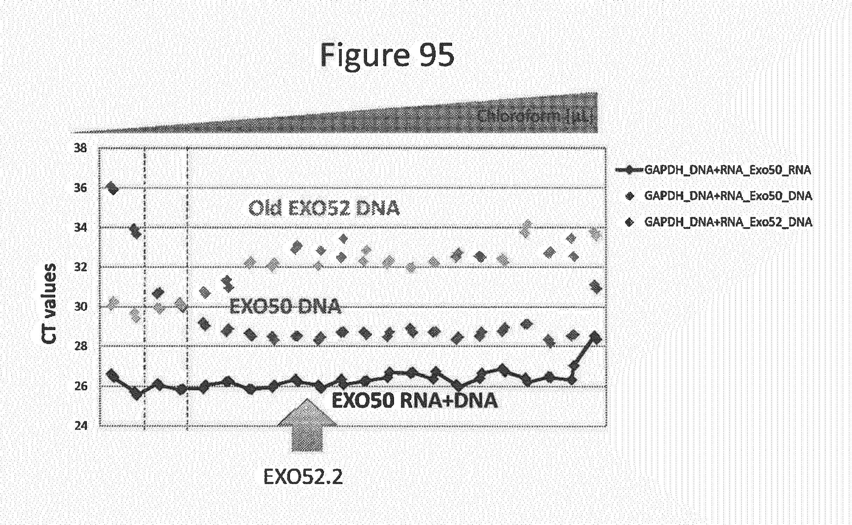

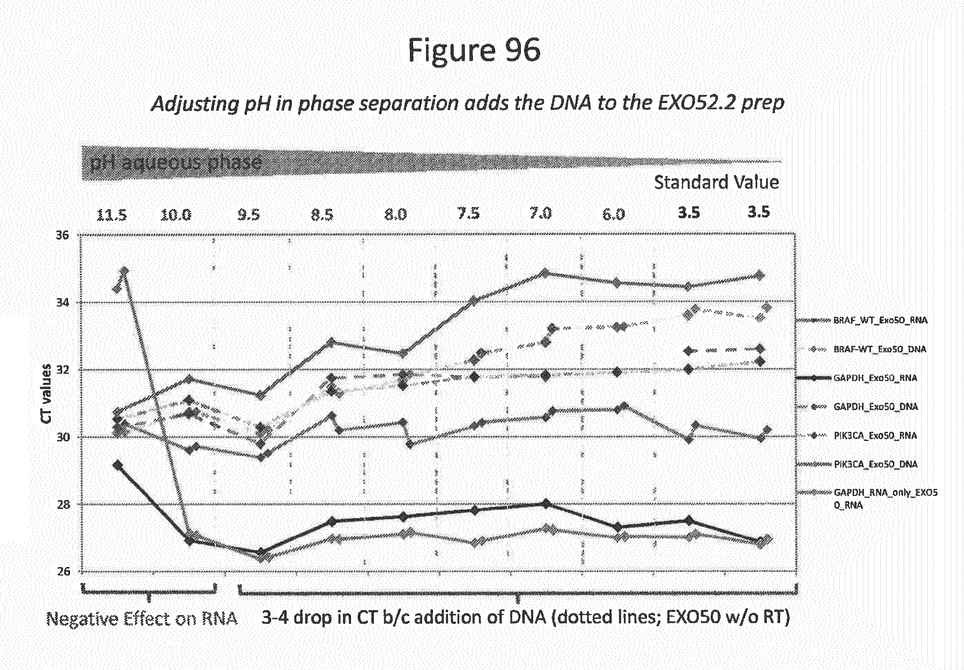

The EXO50 column can also be used to isolate all of the DNA from the plasma. Two methods for utilizing the EXO50 column for DNA isolation in addition to RNA is depicted in FIG. 92 (EXO52) and FIG. 93 (EXO52.2). Specifically, the difference between the two processes EXO52 and EXO52.2, is that the RNA and DNA extraction is combined in one tube in EXO52.2, for ease in usability, streamlining of the protocol, and increased reproducibility. FIG. 94 shows a gain of 1.5 Cts in EXO50 RNA + DNA (EXO52.2). FIG. 95 shows that increasing the amount of chloroform during phase separation adds the DNA back to the aqueous phase, such that the DNA is co-isolated with the normal EXO50 procedure. Further optimization of pH levels during phase separation also adds the DNA to the prep, as shown in FIG. 96.

Thus, the EXO50 methods can be used, as shown in EXO52 and EXO52.2, to isolate all DNA from plasma samples. The DNA is recovered from the lower, hydrophobic phase of the QIAzol lysis after phase separation. The EXO50 methods, in combination with the EXO52 methods (e.g., EXO52 or EXO52.2), separate RNA and DNA at similar levels for the same sample volume, and the RNA and DNA can be separated from each other. These methods of the disclosure capture the same or more mRNA and much more miRNA than a commercially available isolation kit, e.g., Qiagen.

### Example 44: EXO60 Microvesicle Isolation and RNA Extraction from CSF

This example demonstrates that microvesicle RNA can be isolated and extracted from cerebrospinal fluid (CSF) using the EXO50 RC/SBAE membrane - containing column, in the process referred to herein as EXO60.

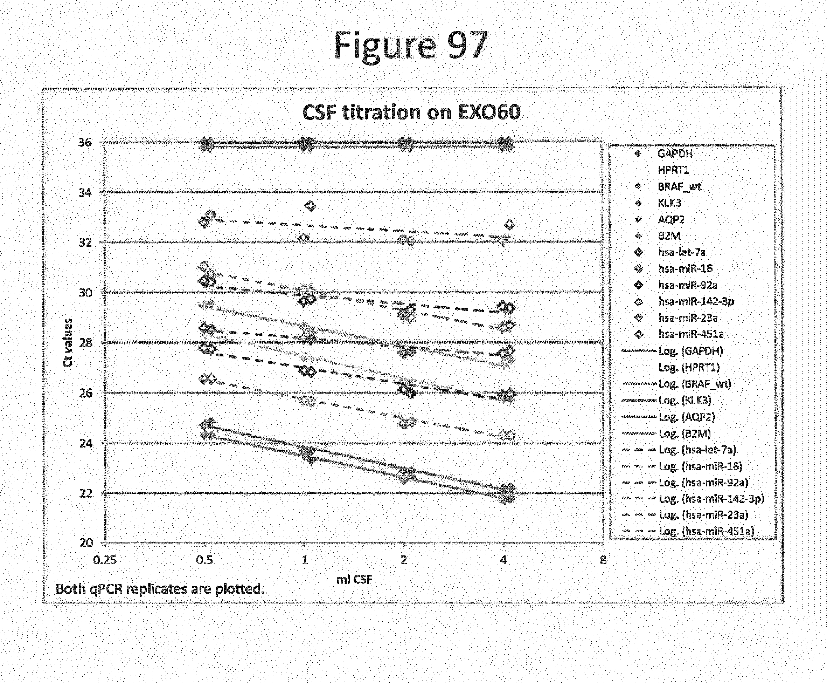

A titration experiment was performed, with increasing volumes of CSF sample (0.5ml, 1ml, 2ml, and 4ml) from a patient. The CSF sample was pre-filtered by 0.8um filter before freezing. The CSF was processed through the column as described in EXAMPLE 32. The results from qPCR analysis are shown in FIG. 97 and show that Ct signal for target genes (both mRNA and miRNAs) increased linearly with sample volume. This experiment demonstrates that EXO60 successfully isolated microvesicles from CSF samples.

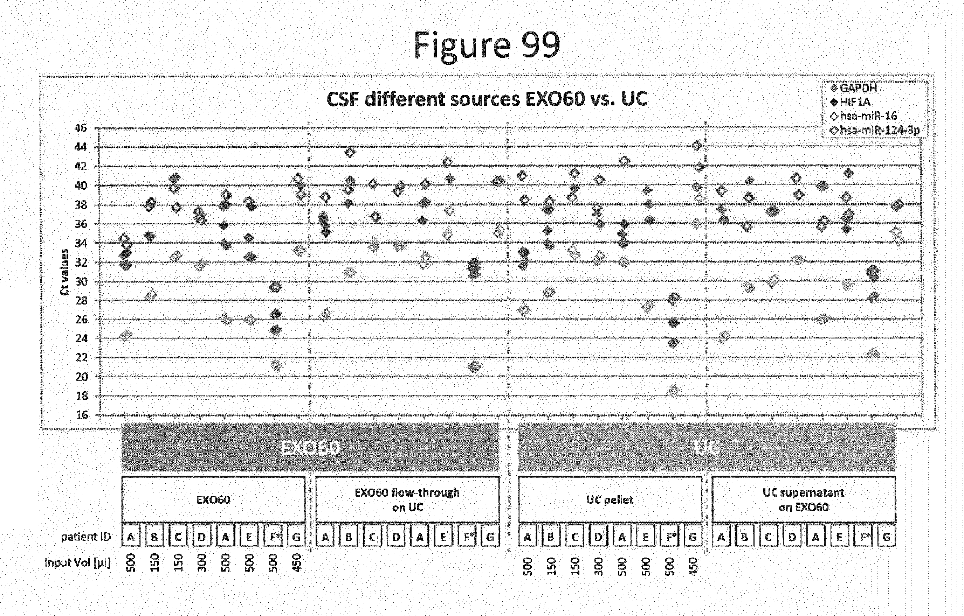

An additional experiment was performed, comparing EXO60 and ultracentrifugation from different patient samples and sources. Flow-through from the EXO60 isolation and supernatant from the ultracentrifugation were also assessed to determine the quality of the microvesicle extraction. Genes assessed were mRNA targets GAPDH and HIF1A, and miRNA targets hsa-miR-16 and hsa-miR-124-3p. As shown in Figure 99, the microvesicle RNA was successfully isolated, similar to results obtained from ultracentrifugation.

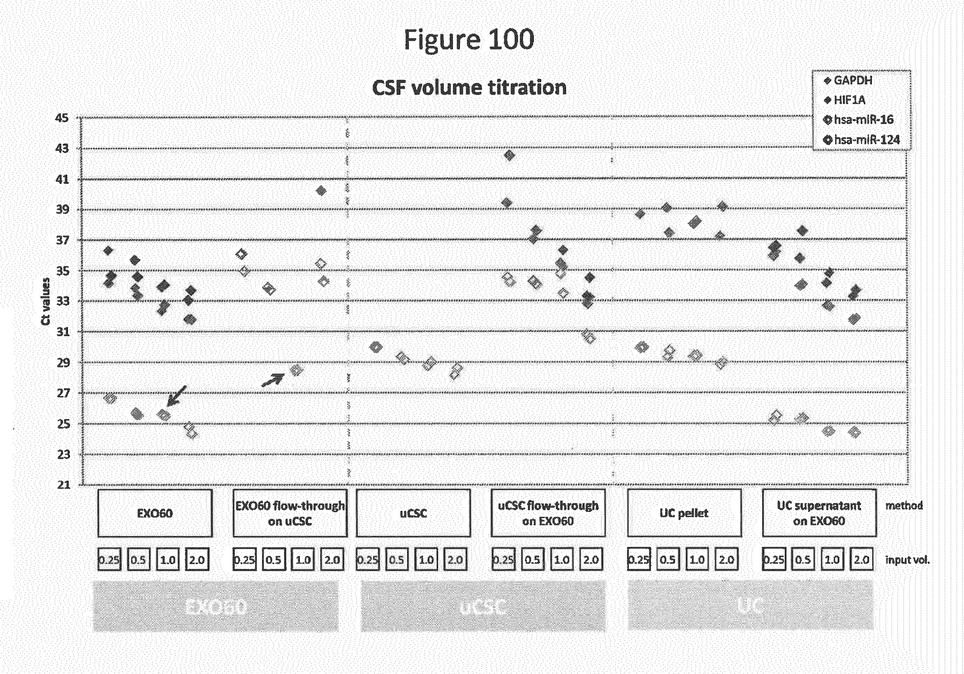

As shown in Figure 100, EXO60 isolates RNA from CSF better than methods based on size filtration or ultracentrifugation. For EXO60, the RNA was extracted using 70% EtOH, while the uCSC extraction used 30% isopropanol, and the UC extraction used 60% EtOH. EXO60 demonstrated a linear PCR output in a range from at least 0.2 to 2 mL of CSF input. In the flow-through of EXO60, only some miR-16 miRNA was found, and no GAPDH and HIF1A mRNA was found. In contrast for the uCSC extractions, miR-16 was found only in the columns fraction, and GAPDH and HIF1A and some miR-16 were found in the flow-through fraction. The UC extraction showed that most of the mRNA and miRNA remained in the supernatant. No HIF1a and only some GAPDH were found in the pellet fraction.

### Example 45: EXO70 Microvesicle Isolation and RNA Extraction from Urine

The studies presented herein were designed to compare EXO50-based isolation of microvesicle RNA, a method referred to herein as EXO70, with filtration based isolation (uCSC). Each isolation method was run using a 20 ml urine sample. For the EXO70 isolation, the RNA was extracted isolated according to EXAMPLE 32, but with 30% EtOH for mRNA-only capture. For the uCSC isolation, the RNA was extracted and isolated according to EXAMPLE 32.

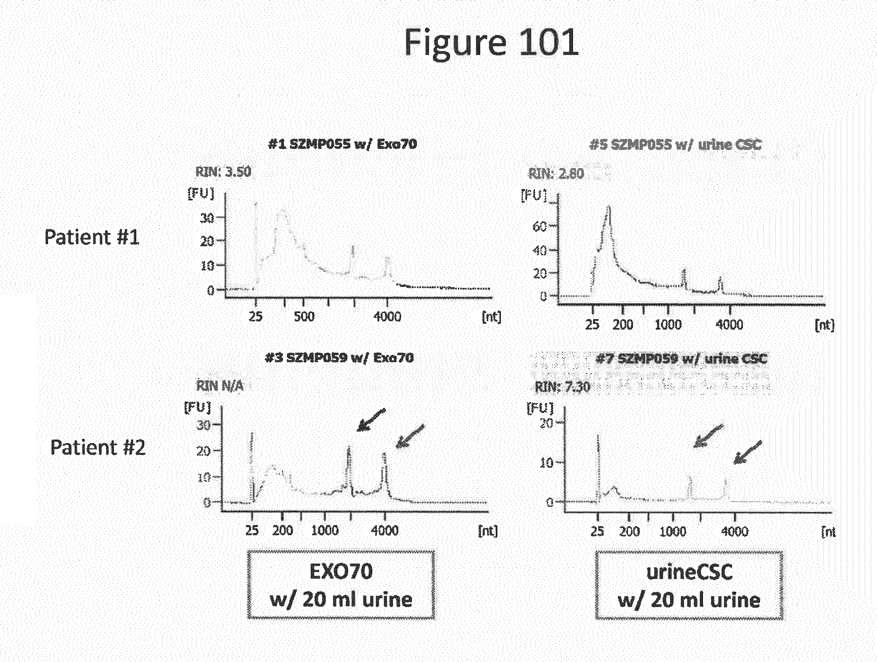

As shown in Figure 101, RNA isolated by EXO70 and filtration demonstrated similar RNA size-distributions when the ethanol conditioning was adjusted to only isolate large RNAs. The studies also demonstrated that both methods yielded PCR signal of the same strength.

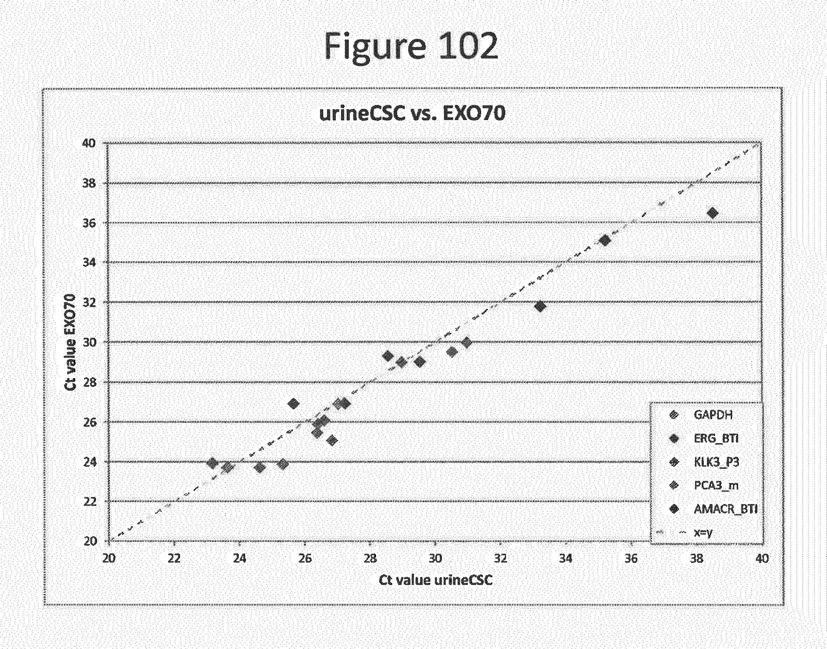

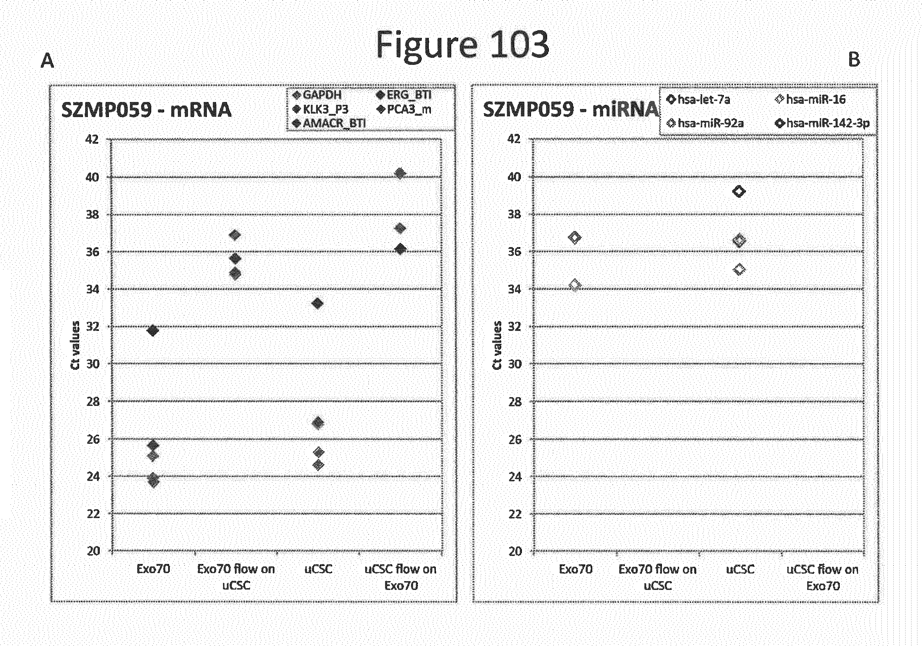

As shown in Figures 102, 103A and 103B, the load of the flow-throughs from EXO70 to a fresh uCSC column yielded much higher Ct values (about 8 Ct values) for the mRNA signal, signifying that in the flow-through are only less than 1% signal is left. Thus, EXO70 captured mRNA and miRNA with > 99% efficiency since there was less than 1% of mRNA and miRNAs left in the flow through.

The flow-through from the uCSC loaded on an EXO70 column yielded little to no signal for the mRNAs in 2 of 4 patients, and if there was a signal detectable then it was about 15 Ct values higher than the on-column data point. There is less than 1% mRNA signal left in the urine flow-through both EXO70 and uCSC.

### Example 46: EXO50 Microvesicle Isolation in Cell Supernatant Samples

The studies presented herein were designed to test the use the EXO50 methods and kits with cell supernatant samples. The samples were processed as set forth above in EXAMPLE 32.

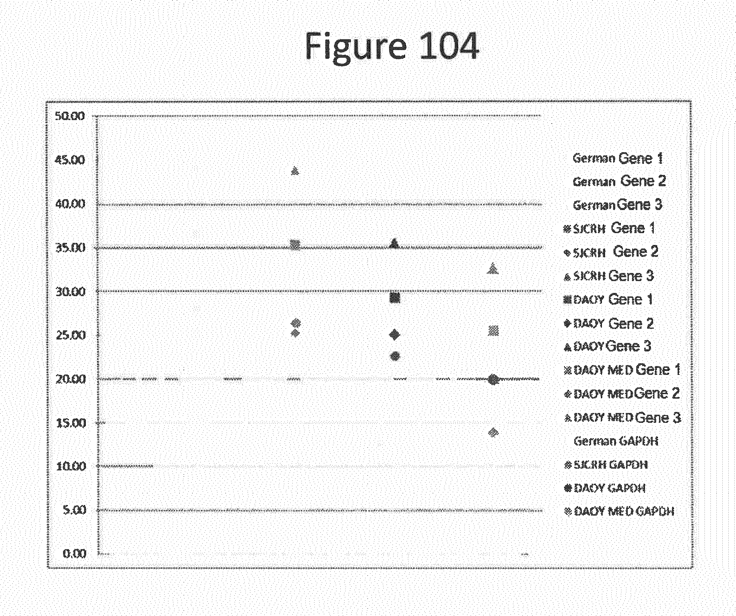

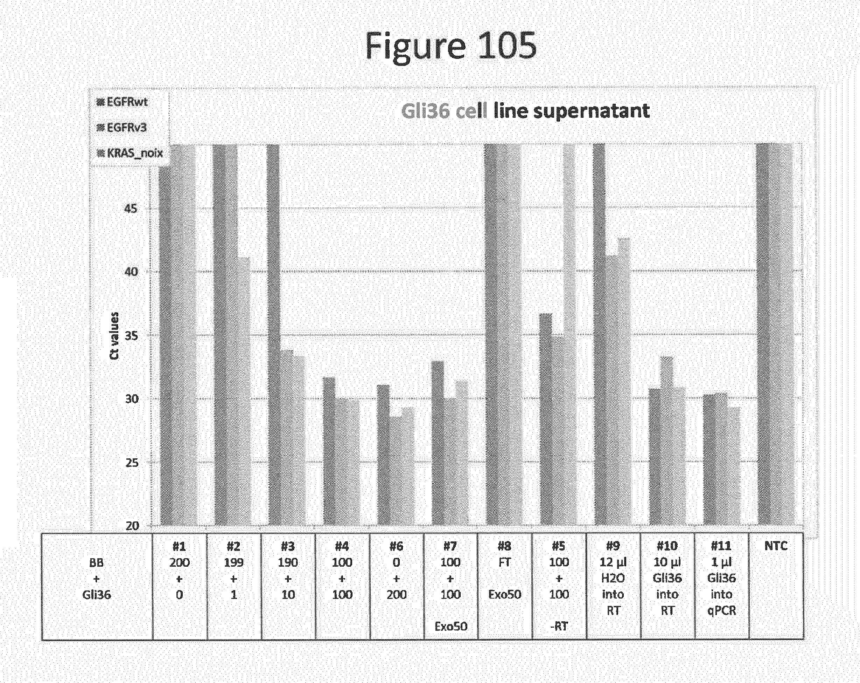

As seen in Figures 104 and 105, EXO50 isolated vesicle RNA from human cell culture supernatant. Different concentrations of 2x Binding Buffer (BB) with the Gli36 cell line supernatant were used, as shown in Figure 105.

## Claims

1. A device for isolating microvesicles comprising;
a column comprising an inner tube and an outer frit;
a capture surface configured within the column, the capture surface having at least one membrane that is positively charged and functionalized with quaternary ammonium R-CH₂-N⁺(CH₃)₃;
a holder that secures the capture surface between the outer frit and the inner tube; and
a collection tube.

2. A device as claimed in claim 1, wherein the capture surface has two, three, four, five or six membranes, preferably three.

3. A device as claimed in claim 2, wherein the membranes are all adjacent to one another.

4. A device as claimed in any one of claims 1 to 3, wherein the outer frit comprises a large net structure.

5. A device as claimed in any one of claims 1 to 4, wherein the outer frit is configured at a first end of the column.

6. A device as claimed in any one of claims 1 to 5, wherein the inner tube is slightly conus-shaped.

7. A device as claimed in any one of claims 1 to 6, wherein the holder is configured between a first end of the inner tube and the outer frit.

8. A device as claimed in claim 7 wherein:
the outer frit is configured at a first end of the column;
the first end of the inner tube is configured towards the first end of the column; and
the holder secures the capture surface such that the capture surface is parallel to the outer frit.

9. A device as claimed in any one of claims 1 to 8, wherein the outer frit is configured at an end of the column and is substantially perpendicular to a central axis of the inner tube and the column.

10. A device as claimed in any one of claims 1 to 9, wherein the column is configured to fit within the collection tube.

11. A device as claimed in any one of claims 1 to 10, wherein the collection tube is configured to receive the column.

12. A device as claimed in any one of claims 1 to 11, wherein the membrane/s has/have a pore size in a range between about 3 and 5 micrometres.

13. A device as claimed in any one of claims 2 to 12, wherein the membranes are identical to each other.

14. A device as claimed in any one of claims 1 to 13, wherein the membrane/s comprise/s regenerated cellulose.

15. A device as claimed in any of claims 1 to 14, wherein the inner tube is configured to receive a biological sample, the biological sample having a volume of between about 0.1 mL to about 4 mL.

## Patentansprüche

1. Vorrichtung zur Isolierung von Mikrovesikeln, umfassend:
eine Säule, die ein inneres Rohr und eine äußere Fritte umfasst;
eine Erfassungsoberfläche, die innerhalb der Säule konfiguriert ist, wobei die Erfassungsoberfläche mindestens eine Membran aufweist, die positiv geladen und mit quartärem Ammonium R-CH₂-N⁺(CH₃)₃ funktionalisiert ist;
eine Halterung, die die Erfassungsoberfläche zwischen der äußeren Fritte und dem inneren Rohr sichert; und
ein Sammelrohr.

2. Vorrichtung nach Anspruch 1, wobei die Erfassungsoberfläche zwei, drei, vier, fünf oder sechs Membranen, vorzugsweise drei aufweist.

3. Vorrichtung nach Anspruch 2, wobei die Membranen alle aneinander angrenzen.

4. Vorrichtung nach einem der Ansprüche 1 bis 3, wobei die äußere Fritte eine große Netzstruktur umfasst.

5. Vorrichtung nach einem der Ansprüche 1 bis 4, wobei die äußere Fritte an einem ersten Ende der Säule konfiguriert ist.

6. Vorrichtung nach einem der Ansprüche 1 bis 5, wobei das innere Rohr leicht kegelförmig ist.

7. Vorrichtung nach einem der Ansprüche 1 bis 6, wobei die Halterung zwischen einem ersten Ende des inneren Rohrs und der äußeren Fritte konfiguriert ist.

8. Vorrichtung nach Anspruch 7, wobei:
die äußere Fritte an einem ersten Ende der Säule konfiguriert ist; das erste Ende des inneren Rohrs zu dem ersten Ende der Säule hin konfiguriert ist und
die Halterung die Erfassungsoberfläche sichert, so dass die Erfassungsoberfläche parallel zu der äußeren Fritte ist.

9. Vorrichtung nach einem der Ansprüche 1 bis 8, wobei die äußere Fritte an einem Ende der Säule konfiguriert ist und im Wesentlichen senkrecht zu einer Mittelachse des inneren Rohrs und der Säule ist.

10. Vorrichtung nach einem der Ansprüche 1 bis 9, wobei die Säule dazu konfiguriert ist, in das Sammelrohr zu passen.

11. Vorrichtung nach einem der Ansprüche 1 bis 10, wobei das Sammelrohr dazu konfiguriert ist, die Säule aufzunehmen.

12. Vorrichtung nach einem der Ansprüche 1 bis 11, wobei die Membran/en eine Porengröße in einem Bereich zwischen etwa 3 und 5 Mikrometer aufweist/aufweisen.

13. Vorrichtung nach einem der Ansprüche 2 bis 12, wobei die Membranen zueinander identisch sind.

14. Vorrichtung nach einem der Ansprüche 1 bis 13, wobei die Membran/en regenerierte Cellulose umfasst/umfassen.

15. Vorrichtung nach einem der Ansprüche 1 bis 14, wobei das innere Rohr dazu konfiguriert ist, eine biologische Probe aufzunehmen, wobei die biologische Probe ein Volumen zwischen etwa 0,1 mL und etwa 4 mL aufweist.

## Revendications

1. Dispositif pour l'isolement de microvésicules comprenant :
une colonne comprenant un tube intérieur et un fritté extérieur ;
une surface de capture configurée à l'intérieur de la colonne, la surface de capture présentant au moins une membrane qui est chargée positivement et fonctionnalisée par un ammonium quaternaire R-CH₂-N⁺(CH₃)₃ ;
un support qui maintient la surface de capture en place entre le fritté extérieur et le tube intérieur ; et
un tube de collecte.

2. Dispositif tel que revendiqué à la revendication 1, où la surface de capture présente deux, trois, quatre, cinq ou six membranes, préférablement trois.

3. Dispositif tel que revendiqué à la revendication 2, où les membranes sont toutes adjacentes les unes aux autres.

4. Dispositif tel que revendiqué à l'une quelconque des revendications 1 à 3, où le fritté extérieur comprend une structure à mailles de grande taille.

5. Dispositif tel que revendiqué à l'une quelconque des revendications 1 à 4, où le fritté extérieur est configuré à une première extrémité de la colonne.

6. Dispositif tel que revendiqué à l'une quelconque des revendications 1 à 5, où le tube intérieur est légèrement en forme de cône.

7. Dispositif tel que revendiqué à l'une quelconque des revendications 1 à 6, où le support est configuré entre une première extrémité du tube intérieur et le fritté extérieur.

8. Dispositif tel que revendiqué à la revendication 7, où :
le fritté extérieur est configuré à une première extrémité de la colonne ;
la première extrémité du tube intérieur est configurée dans la direction de la première extrémité de la colonne ; et
le support maintient la surface de capture en place de sorte que la surface de capture est parallèle au fritté extérieur.

9. Dispositif tel que revendiqué à l'une quelconque des revendications 1 à 8, où le fritté extérieur est configuré à une extrémité de la colonne et est essentiellement perpendiculaire à un axe central du tube intérieur et de la colonne.

10. Dispositif tel que revendiqué à l'une quelconque des revendications 1 à 9, où la colonne est configurée pour s'emboîter dans le tube de collecte.

11. Dispositif tel que revendiqué à l'une quelconque des revendications 1 à 10, où le tube de collecte est configuré pour recevoir la colonne.

12. Dispositif tel que revendiqué à l'une quelconque des revendications 1 à 11, où la(les) membrane(s) a(ont) des pores d'une taille dans une plage qui va d'entre environ 3 et 5 micromètres.

13. Dispositif tel que revendiqué à l'une quelconque des revendications 2 à 12, où les membranes sont identiques les unes aux autres.

14. Dispositif tel que revendiqué à l'une quelconque des revendications 1 à 13, où la(les) membrane(s) comprend(nent) une cellulose régénérée.

15. Dispositif tel que revendiqué à l'une quelconque des revendications 1 à 14, où le tube intérieur est configuré pour recevoir un échantillon biologique, l'échantillon biologique ayant un volume d'entre environ 0,1 ml et environ 4 ml.
